# EUROPEAN PATENT APPLICATION

(11) **EP 2 253 721 A1**
(43) Date of publication of application: **24.11.2010**
(21) Application number: 10175619.5
(22) Date of filing: 26.02.2008
(51) Int. Cl.: C12Q 1/68

(54) **Method for predicting the occurence of lung metastasis in breast cancer patients**

(30) Priority: 26.02.2007 EP 07300823
(62) Divisional of application: 08709219.3
(71) Applicant: INSERM (Institut National de la Santé et de la Recherche Medicale), 75013 Paris (FR); Centre Rene Huguenin, 92210 Saint-Cloud (FR)
(72) Inventor: Lidereau, Rosette, 92230, Gennevilliers (FR); Driouch, Keltouma, 93260, Les Lilas (FR); Landemaine, Thomas, 92100, Boulogne-Billancourt (FR)
(74) Representative: Catherine, Alain

(57) **Abstract**

The present invention relates to the prognosis of the progression of breast cancer in a patient, and more particularly to the prediction of the occurrence of metastasis in one or more tissue or organ of patients affected with a breast cancer.

SEQUENCE LISTING

<110> INSERM (Institut National de la Santé et de la Recherche Médicale)

<120> Method for predicting the occurrence of metastasis in breast cancer
patients

<130> U605EP - INSERM

<160> 114

<170> PatentIn version 3.1

<210> 1
<211> 25
<212> DNA
<213> artificial sequence

<220>
<223> primer
<400> 1
aaataccctg aatccagaat cggaa 25

<210> 2
<211> 26
<212> DNA
<213> artificial sequence

<220>
<223> primer
<400> 2
tgctgtttga gactgtccaa aacaat 26

<210>  3

<211>  20

<212>  DNA

<213>  artificial sequence



<220>

<223>  primer

<400>  3
agaccgcggc tcctaacgat                                                   20


<210>  4

<211>  21

<212>  DNA

<213>  artificial sequence



<220>

<223>  primer

<400>  4
agtcgggcgg agtttcacag t                                                 21


<210>  5

<211>  23

<212>  DNA

<213>  artificial sequence



<220>

<223>  primer

<400>  5
ctgtggtgga aatgctccca aga                                               23


<210>  6

<211>  22

<212>  DNA

<213>  artificial sequence

<220>

<223>  primer

<400>  6
gctgctctac cccacgcgta ct                                              22

<210>  7

<211>  19

<212>  DNA

<213>  artificial sequence

<220>

<223>  primer

<400>  7
acagccagcc cgacaccat                                                  19

<210>  8

<211>  21

<212>  DNA

<213>  artificial sequence

<220>

<223>  primer

<400>  8
tggccttctc cgacagctgt a                                               21

<210>  9

<211>  26

<212>  DNA

<213>  artificial sequence

<220>

<223>  primer

<400>  9
gtacagcttc ccagaagaag agcata                                            26

<210>  10

<211>  25

<212>  DNA

<213>  artificial sequence

<220>

<223>  primer

<400>  10
cattcagctt ggatggacct ttagt                                             25

<210>  11

<211>  24

<212>  DNA

<213>  artificial sequence

<220>

<223>  primer

<400>  11
gaaacaacaa ctgcccagag aaga                                              24

<210>  12

<211>  19

<212>  DNA

<213>  artificial sequence

<220>

<223>  primer

<400>  12
ccgaggtgtc cagcacgaa                                                    19

<210>  13

<211>  25

<212>  DNA

<213>  artificial sequence

<220>

<223>  primer

<400>  13
cctcagttac ctcagggaaa gttca                                          25

<210>  14

<211>  24

<212>  DNA

<213>  artificial sequence

<220>

<223>  primer

<400>  14
ggtcggtgtg ggaggttgta ttat                                           24

<210>  15

<211>  23

<212>  DNA

<213>  artificial sequence

<220>

<223>  primer

<400>  15
caagagtttg accggctgac cat                                            23

<210>  16

<211>  23

<212>  DNA

<213>  artificial sequence

<220>

<223>  primer

<400>  16
ccgcacttcc tcgtagagct cat                                              23

<210>  17

<211>  19

<212>  DNA

<213>  artificial sequence

<220>

<223>  primer

<400>  17
tgcacgcggc acagagtca                                                   19

<210>  18

<211>  24

<212>  DNA

<213>  artificial sequence

<220>

<223>  primer

<400>  18
cccgctttca taaaatccag gaca                                             24

<210>  19

<211>  25

<212>  DNA

<213>  artificial sequence

<220>

<223>  primer

<400>  19

accatggaaa attcaacatc ttcaa 25

<210> 20

<211> 27

<212> DNA

<213> artificial sequence

<220>

<223> primer

<400> 20
gtttcgcaaa attttcaaac tcttact 27

<210> 21

<211> 25

<212> DNA

<213> artificial sequence

<220>

<223> primer

<400> 21
agccacatgg agttctacaa ccagt 25

<210> 22

<211> 22

<212> DNA

<213> artificial sequence

<220>

<223> primer

<400> 22
agtcgctgat ggcttggatc tt 22

<210> 23

<211> 22

<212>  DNA

<213>  artificial sequence

<220>

<223>  primer

<400>  23
cgacgtcaat tccttcgtgg ta                                                22

<210>  24

<211>  25

<212>  DNA

<213>  artificial sequence

<220>

<223>  primer

<400>  24
gaaaaacaca gcaatgtgct tgtct                                             25

<210>  25

<211>  22

<212>  DNA

<213>  artificial sequence

<220>

<223>  primer

<400>  25
ccaggaccag ggaacagaca tt                                                22

<210>  26

<211>  20

<212>  DNA

<213>  artificial sequence

<220>

<223>  primer

<400>  26
cggagcagtt ggtgaccaca                                                   20


<210>  27

<211>  27

<212>  DNA

<213>  artificial sequence



<220>

<223>  primer

<400>  27
caccatacaa tgaaagttcc acattct                                           27


<210>  28

<211>  26

<212>  DNA

<213>  artificial sequence



<220>

<223>  primer

<400>  28
gggtgattta cgaaaattta gcacaa                                            26


<210>  29

<211>  26

<212>  DNA

<213>  artificial sequence



<220>

<223>  primer

<400>  29
aaggaacttg aacaaggaga accact                                            26

<210> 30

<211> 21

<212> DNA

<213> artificial sequence

<220>

<223> primer

<400> 30
ggcacaactt cgcagcctct a 21

<210> 31

<211> 25

<212> DNA

<213> artificial sequence

<220>

<223> primer

<400> 31
aaagccaact taaagcaggg agaat 25

<210> 32

<211> 20

<212> DNA

<213> artificial sequence

<220>

<223> primer

<400> 32
agcccaggaa ggccaaagaa 20

<210> 33

<211> 25

<212> DNA

<213>  artificial sequence

<220>

<223>  primer

<400>  33
ttttgaacta ctgcagcaaa tcaca                                              25

<210>  34

<211>  22

<212>  DNA

<213>  artificial sequence

<220>

<223>  primer

<400>  34
tcatcttcat cgccatcctc at                                                 22

<210>  35

<211>  25

<212>  DNA

<213>  artificial sequence

<220>

<223>  primer

<400>  35
tcgaagatgg atggcattag attat                                              25

<210>  36

<211>  26

<212>  DNA

<213>  artificial sequence

<220>

<223>  primer

<400>  36
caagtacacc tgacgccagt agttat                                          26

<210>  37

<211>  21

<212>  DNA

<213>  artificial sequence

<220>

<223>  primer

<400>  37
gactgggcca aggtgaagac a                                               21

<210>  38

<211>  24

<212>  DNA

<213>  artificial sequence

<220>

<223>  primer

<400>  38
cctcttgaac acaccatcca catt                                            24

<210>  39

<211>  24

<212>  DNA

<213>  artificial sequence

<220>

<223>  primer

<400>  39
gaggctgaga ctcactggtg tcat                                            24

<210>  40

<211>  21

<212>  DNA

<213>  artificial sequence

<220>

<223>  primer

<400>  40
cgaaggccca acaagtgttt t                                                21

<210>  41

<211>  25

<212>  DNA

<213>  artificial sequence

<220>

<223>  primer

<400>  41
cttgcaacag gaaaacagag agcta                                            25

<210>  42

<211>  26

<212>  DNA

<213>  artificial sequence

<220>

<223>  primer

<400>  42
ctgtttccga tatttgctca tgataa                                           26

<210>  43

<211>  24

<212>  DNA

<213>  artificial sequence

<220>

<223>  primer

<400>  43
aaaggcatgg ggatattgct agaa                                              24

<210>  44

<211>  21

<212>  DNA

<213>  artificial sequence

<220>

<223>  primer

<400>  44
ccctctgacg gtagctggga t                                                 21

<210>  45

<211>  23

<212>  DNA

<213>  artificial sequence

<220>

<223>  primer

<400>  45
gaaaggctct ggcatgatgt caa                                               23

<210>  46

<211>  23

<212>  DNA

<213>  artificial sequence

<220>

<223>  primer

<400>  46

ctgccatctc gattttccca tct                                              23

<210>  47

<211>  22

<212>  DNA

<213>  artificial sequence

<220>

<223>  primer

<400>  47
ctggataggg ctttggagga at                                               22

<210>  48

<211>  26

<212>  DNA

<213>  artificial sequence

<220>

<223>  primer

<400>  48
ttcatctgtg taccagtttc ggtact                                           26

<210>  49

<211>  25

<212>  DNA

<213>  artificial sequence

<220>

<223>  primer

<400>  49
aagaaacact ctccttctgc cacat                                            25

<210>  50

<211>  24

<212>  DNA

<213>  artificial sequence

<220>

<223>  primer

<400>  50
   atcagacttt ggtaccccct caat                                              24

<210>  51

<211>  19

<212>  DNA

<213>  artificial sequence

<220>

<223>  primer

<400>  51
cagtggcttc accgcaaca                                                     19

<210>  52

<211>  22

<212>  DNA

<213>  artificial sequence

<220>

<223>  primer

<400>  52
ttcagcaagt cagcacctga ga                                                 22

<210>  53

<211>  25

<212>  DNA

<213>  artificial sequence

<220>

<223>  primer

<400>  53
caaacaaagg cttgaccatt ttaca                                             25

<210>  54

<211>  25

<212>  DNA

<213>  artificial sequence

<220>

<223>  primer

<400>  54
gtttcagaca acttggcttc ctgat                                             25

<210>  55

<211>  26

<212>  DNA

<213>  artificial sequence

<220>

<223>  primer

<400>  55
caacatgaat ctgggagaat agtcct                                            26

<210>  56

<211>  23

<212>  DNA

<213>  artificial sequence

<220>

<223>  primer

<400>  56
tcctttttgg cactgactct tga                                               23

<210>  57

<211>  20

<212>  DNA

<213>  artificial sequence

<220>

<223>  primer

<400>  57
ggtctccgct gtcccctgta                                                    20

<210>  58

<211>  25

<212>  DNA

<213>  artificial sequence

<220>

<223>  primer

<400>  58
gatctctgag tcctcagcag tcaaa                                              25

<210>  59

<211>  26

<212>  DNA

<213>  artificial sequence

<220>

<223>  primer

<400>  59
aatcaaagtt ttcagaagcc tggata                                             26

<210>  60

<211>  23

<212>  DNA

<213>  artificial sequence

<220>

<223>  primer

<400>  60
cgtacatgtt ctccctcctt ggt                                              23

<210>  61

<211>  23

<212>  DNA

<213>  artificial sequence

<220>

<223>  primer

<400>  61
cacgactaca gcagcggact ctt                                              23

<210>  62

<211>  20

<212>  DNA

<213>  artificial sequence

<220>

<223>  primer

<400>  62
cactcccggc cttctgaaca                                                  20

<210>  63

<211>  25

<212>  DNA

<213>  artificial sequence

<220>

<223>  primer

<400>  63
ctgctcaagt tccagatttg tgttt                                             25

<210>  64

<211>  23

<212>  DNA

<213>  artificial sequence

<220>

<223>  primer

<400>  64
attctctcct tcaatgcgga tgt                                               23

<210>  65

<211>  25

<212>  DNA

<213>  artificial sequence

<220>

<223>  primer

<400>  65
tactaaaaga ggaagcccca gttca                                             25

<210>  66

<211>  21

<212>  DNA

<213>  artificial sequence

<220>

<223>  primer

<400>  66
cccaccttgg accacacaag a                                                 21

<210>  67

<211>  25

<212>  DNA

<213>  artificial sequence

<220>

<223>  primer

<400>  67
ccacgacaat cctgcagaag attat                                          25

<210>  68

<211>  23

<212>  DNA

<213>  artificial sequence

<220>

<223>  primer

<400>  68
tccacttgct gctcactttt gct                                            23

<210>  69

<211>  28

<212>  DNA

<213>  artificial sequence

<220>

<223>  primer

<400>  69
gcaaggaagt gaacatcaaa ttaaagaa                                       28

<210>  70

<211>  28

<212>  DNA

<213>  artificial sequence

<220>

<223>  primer

<400>  70
aatctgcaca ctcattttgg taagagtc                                          28

<210>  71

<211>  23

<212>  DNA

<213>  artificial sequence

<220>

<223>  primer

<400>  71
accttcctgt ttccatgcaa caa                                               23

<210>  72

<211>  23

<212>  DNA

<213>  artificial sequence

<220>

<223>  primer

<400>  72
gtcacacact gggaccacgt tct                                               23

<210>  73

<211>  25

<212>  DNA

<213>  artificial sequence

<220>

<223>  primer

<400>  73

tcaggagaaa tccaaggtgt gatgt                                          25

<210>  74

<211>  29

<212>  DNA

<213>  artificial sequence

<220>

<223>  primer

<400>  74
caatactatt tcgaatctgg cttaaggtt                                      29

<210>  75

<211>  24

<212>  DNA

<213>  artificial sequence

<220>

<223>  primer

<400>  75
gcagacattt ggaaccaaca acct                                           24

<210>  76

<211>  24

<212>  DNA

<213>  artificial sequence

<220>

<223>  primer

<400>  76
ccccttcctt ggcaattatt tcat                                           24

<210>  77

<211>  22

<212>  DNA

<213>  artificial sequence

<220>

<223>  primer

<400>  77
cagcaactct gccggagaaa ct                                                22

<210>  78

<211>  21

<212>  DNA

<213>  artificial sequence

<220>

<223>  primer

<400>  78
cgttggcttc ggatcctgaa t                                                 21

<210>  79

<211>  19

<212>  DNA

<213>  artificial sequence

<220>

<223>  primer

<400>  79
gatgcggagg accgtgtca                                                    19

<210>  80

<211>  24

<212>  DNA

<213>  artificial sequence

<220>

<223>  priimer

<400>  80
tctcttcacc tcctgtgcac ttct                                              24

<210>  81

<211>  25

<212>  DNA

<213>  artificial sequence

<220>

<223>  primer

<400>  81
cctctgatga gtcctccagt gaaca                                             25

<210>  82

<211>  24

<212>  DNA

<213>  artificial sequence

<220>

<223>  primer

<400>  82
cctcgctggg acataaatct ttga                                              24

<210>  83

<211>  25

<212>  DNA

<213>  artificial sequence

<220>

<223>  primer

<400>  83
ctgtgggtga tgataaagca atgaa                                             25

<210>  84

<211>  25

<212>  DNA

<213>  artificial sequence



<220>

<223>  primer

<400>  84
cactgtccag gaaaatagcc aagtt                                             25


<210>  85

<211>  25

<212>  DNA

<213>  artificial sequence



<220>

<223>  primer

<400>  85
catttacatc tatgtgccag accca                                             25


<210>  86

<211>  23

<212>  DNA

<213>  artificial sequence



<220>

<223>  primer

<400>  86
atggcagaat catcatcctc cac                                               23


<210>  87

<211>  24

<212>  DNA

<213>  artificial sequence

<220>

<223>  primer

<400>  87
cgaatgaatg tccatcccca gata                                              24

<210>  88

<211>  21

<212>  DNA

<213>  artificial sequence

<220>

<223>  primer

<400>  88
tccaggtgaa ccaggcttgc t                                                 21

<210>  89

<211>  26

<212>  DNA

<213>  artificial sequence

<220>

<223>  primer

<400>  89
ggaatcgtgc tggaaaaagt aggagt                                            26

<210>  90

<211>  25

<212>  DNA

<213>  artificial sequence

<220>

<223>  primer

<400>  90
ccattcttga tcagctggac cctta                                          25

<210>  91

<211>  21

<212>  DNA

<213>  artificial sequence

<220>

<223>  primer

<400>  91
attccctgag ccgcgaaatc t                                              21

<210>  92

<211>  23

<212>  DNA

<213>  artificial sequence

<220>

<223>  primer

<400>  92
caaggcatcg agttttccct tga                                            23

<210>  93

<211>  24

<212>  DNA

<213>  artificial sequence

<220>

<223>  primer

<400>  93
tgggcacgaa agagatgatg ataa                                           24

<210>  94

<211>  25

<212>  DNA

<213>  artificial sequence

<220>

<223>  primer

<400>  94
gaatgaggta gcgagtggtg tctgt                                              25

<210>  95

<211>  22

<212>  DNA

<213>  artificial sequence

<220>

<223>  primer

<400>  95
cgaggtgtgc agagtgctga ga                                                 22

<210>  96

<211>  26

<212>  DNA

<213>  artificial sequence

<220>

<223>  primer

<400>  96
atccaggact tgttgccaga agtaat                                             26

<210>  97

<211>  21

<212>  DNA

<213>  artificial sequence

<220>

<223>  primer

<400>  97
gcaatggaga gagcagcaac a                                                21

<210>  98

<211>  26

<212>  DNA

<213>  artificial sequence

<220>

<223>  primer

<400>  98
ttctttggtc tccctaaaaa tcttga                                           26

<210>  99

<211>  25

<212>  DNA

<213>  artificial sequence

<220>

<223>  primer

<400>  99
ccactttcag aagatgcaga aatca                                            25

<210>  100

<211>  19

<212>  DNA

<213>  artificial sequence

<220>

<223>  primer

<400>  100

ccagaggttg gagcgcaca                                                    19

<210>  101

<211>  21

<212>  DNA

<213>  artificial sequence

<220>

<223>  primer

<400>  101
atgcaggaag gtgccaaagt t                                                21

<210>  102

<211>  25

<212>  DNA

<213>  artificial sequence

<220>

<223>  primer

<400>  102
cactagtgtc atctctgggc attct                                            25

<210>  103

<211>  24

<212>  DNA

<213>  artificial sequence

<220>

<223>  primer

<400>  103
gatgtgttgc cagggagaac agaa                                             24

<210>  104

<211>  20

<212>  DNA

<213>  artificial sequence

<220>

<223>  primer

<400>  104
gcccgctgga gtctgcaaat                                                    20

<210>  105

<211>  24

<212>  DNA

<213>  artificial sequence

<220>

<223>  primer

<400>  105
tgagttcagt gctgaatcgc aaca                                               24

<210>  106

<211>  19

<212>  DNA

<213>  artificial sequence

<220>

<223>  primer

<400>  106
ctgggagggg ccctggtct                                                     19

<210>  107

<211>  22

<212>  DNA

<213>  artificial sequence

<220>

<223>  primer

<400>  107
ccagtgagag gtcctgccaa ga                                                22

<210>  108

<211>  26

<212>  DNA

<213>  artificial sequence

<220>

<223>  primer

<400>  108
aaaggaaggg ctagctcgat actgtt                                            26

<210>  109

<211>  22

<212>  DNA

<213>  artificial sequence

<220>

<223>  primer

<400>  109
cacccgaaag atgctgcatg tt                                                22

<210>  110

<211>  25

<212>  DNA

<213>  artificial sequence

<220>

<223>  primer

<400>  110
agggagagtt tctttggcaa tttca                                             25

<210>  111

<211>  21

<212>  DNA

<213>  artificial sequence

<220>

<223>  primer

<400>  111
gcccgaggaa gaggaggatg t                                                21

<210>  112

<211>  21

<212>  DNA

<213>  artificial sequence

<220>

<223>  primer

<400>  112
gccctctggc agctgacttg t                                                21

<210>  113

<211>  21

<212>  DNA

<213>  artificial sequence

<220>

<223>  primer

<400>  113
tgcacaggag ccaagagtga a                                                21

<210>  114

<211>  20

<212>  DNA

<213>  artificial sequenceartificial sequence

<400>  114
cacatcacag ctccccacca                                                    20

## Description

### FIELD OF THE INVENTION

The present invention relates to the prognosis of the progression of breast cancer in a patient, and more particularly to the prediction of the occurrence of metastasis in one or more tissue or organ of patients affected with a breast cancer.

### BACKGROUND OF THE INVENTION

Breast cancer is the most common malignant disease in Western women. It is not the primary tumour, but the occurrence of metastases in distant organs that is the major cause of death for cancer patients. Once solid secondary tumours are established, the chances of long-term survival fall from 90% to around 5%. Despite the progress in the development of targeted therapies, approximately 40% of the treated patients relapse and ultimately die of metastatic breast cancer. A better understanding of the molecular and cellular mechanisms underlying metastasis might improve the development of effective therapies that would ameliorate breast cancer care.

Malignant breast tumours can invade and damage nearby tissues and organs. Malignant tumour cells may metastasise, entering the bloodstream or lymphatic system. When breast cancer cells metastasise outside the breast, they are often found in the lymph nodes under the arm (axillary lymph nodes). If the cancer has reached these nodes, it means that cancer cells may have spread to other lymph nodes or other organs, such as bones, liver, brain or lungs. Breast cancer metastasis of various organs also occurs without previous spreading to lymph nodes. Major and intensive research has been focussed on early detection, treatment and prevention of metastatic breast cancer.

The rational development of preventive, diagnostic and therapeutic strategies for women at risk for breast cancer would be aided by a molecular map of the tumorigenesis process. Relatively little is known of the molecular events that mediate the transition of normal breast cells to the various stages of breast cancer progression. Similarly, little is known of the molecular events that mediate the transition of cells from one stage of breast cancer to another, and finally to metastasis.

Molecular means of identifying the differences between normal, non-cancerous cells and cancerous cells have been the focus of intense study. The use of cDNA libraries to analyse differences in gene expression patterns in normal versus tumourigenic cells has been described. Gene expression patterns in human breast cancers have been described by Perou et al. (1999, Proc. Natl. Acad. Sci. USA, Vol. 96 : 9212-9217), who studied gene expression between cultured human "normal" mammary epithelia cells (HMEC) and breast tissue samples by using microarrays comprising about 5000 genes. They used a clustering algorithm to identify differential patterns of expression in HMEC and tissue samples. Perou et al. (2000, Nature, Vol. 406 : 747-752) described the use of clustered gene expression profiles to classify subtypes of human breast tumours. Hedenfalk et al. (2001, New Engl. J. Medicine, Vol. 344(8) : 539-548) described gene expression profiles in BRCA1 mutation positive, BRCA2 mutation positive, and sporadic tumours. Using gene expression patterns to distinguish breast tumour subclasses and predict clinical implications is described by Sorlie et al. (2001, Proc. Natl. Acad. Sci USA, Vol. 98(19) : 10869-10874) and West et al. (2001, Proc. Natl. Acad. Sci USA, Vol. 98(20) : 11462-11467).

Based on the assumption that primary tumours may already contain genes that are predictive of metastastatic process, several groups performed genome wide microarray studies to identify expression profiles associated with the occurrence of distant relapses and poor survival. Several highly prognostic gene signatures were reported containing genes potentially involved in metastatic processes and/or markers of distant relapses. These studies mainly tackled overall relapses problems.

Searches aimed at identifying biological markers that would be involved in breast cancer metastasis in several tissues or organs have also been performed in the art. For this purpose, *in vivo* experiments using human breast cancer xenographs in nude mice were performed, as described below.

Kang et al. (2003, Cancer Cell, Vol. 3 : 537-549) identified a set of genes potentially involved in breast cancer metastasis to bone, by comparing (i) the gene expression profile obtained from *in vitro* cultured cells of the MDBA-MB-231 human breast cancer cell line with (ii) the gene expression profile obtained from a subclone of the same cell line previously experimentally selected *in vivo* for their ability to form bone metastasis in mice. Using microarray gene expression analysis techniques, these authors showed that several genes were underexpressed and several other genes were overexpressed in the cell sublines selected for their ability to form bone metastasis in mice, as compared to the parental MDBA-MB-231 human breast cancer cell line. These authors concluded that, in the case of the human MDA-MB-231 cell line, cell functions that are relevant for metastasis to bone might be carried out by CXCR4, CTGF, IL-11 and OPN genes, with possible contributions of other genes.

Minn et al. (2005, Nature, Vol. 436(28) : 518-524) used the same human parental MDA-MB-231 breast cancer cell line for selecting *in vivo* cell sublines having the ability to form lung metastasis in mice. Then, these authors have performed a transcriptomic microarray analysis of the highly and weakly lung-metastatic cell populations, with the view of identifying patterns of gene expression that would be associated with aggressive lung metastatic behaviour. A final list of 54 candidate lung metastagenicity and virulence genes was selected, twelve of them having been further identified for their significant association with lung-metastasis-free survival, including MMP1, CXCL1 and PTGS2.

In the studies reported above, the authors identified and functionally validated a set of genes that specifically mediate bone or lung metastasis in the animal model. The organ-specific gene signatures that were identified allowed to distinguish between (i) primary breast carcinomas that preferentially metastasized to bone or lung from (ii) those that metastasized elsewhere.

One study recently reported a molecular signature allowing for an bone-specific metastasis prognosis of human breast cancer. This work was performed by comparing primary breast tumors relapsing to bone to those relapsing elsewhere. The authors thereby identified a panel of genes associated to breast cancer metastasis to bone (Smid et al., 2006, J Clin Oncol, Vol. 24(15) : 2261-2267).

However, testing the organ-specific signature on mixed cohort of primary tumors did not allow robust classification of those tumors that gave rise to specific metastases versus those that did not. This is probably due to little predictive value of these gene signatures.

An early detection of metastasis in breast cancer patients is crucial for adapting the anti-cancer therapeutic treatment correspondingly, with the view of increasing the chances of long term disease-free survival.

However, because detection of metastasis in breast cancer patients, even if the said detection is performed at an early stage of metastasis, consists of a poor prognosis of the cancer outcome, there is an increasing need in the art for the availability of reliable methods for detecting the metastasis potentiality of a breast cancer tumour, for both medical treatment and medical survey purposes. There is also a need in the art for methods that would provide a prediction of the one or more tissues or organs in which the formation and development of a breast cancer metastasis would be likely to occur.

### SUMMARY OF THE INVENTION

The present invention relates to methods and kits for predicting the occurrence of metastasis in patients affected with a breast cancer.

The breast cancer metastasis prediction methods of the invention comprise a step of determining, in a tumour tissue sample previously collected from a breast cancer patient to be tested the level of expression of one or more biological markers that are indicative of cancer progression towards metastasis, and more precisely, one or more biological markers that are indicative of cancer progression towards metastasis to specific tissues, and especially to bones, brain, lungs and liver.

This invention also relates to breast cancer metastasis prediction kits that are specifically designed for performing the metastasis prediction methods above.

This invention also pertains to methods for selecting one or more biological markers that are indicative of cancer progression towards metastasis in specific tissues, including bones, brain, lungs and liver.

### BRIEF DESCRIPTION OF THE FIGURES

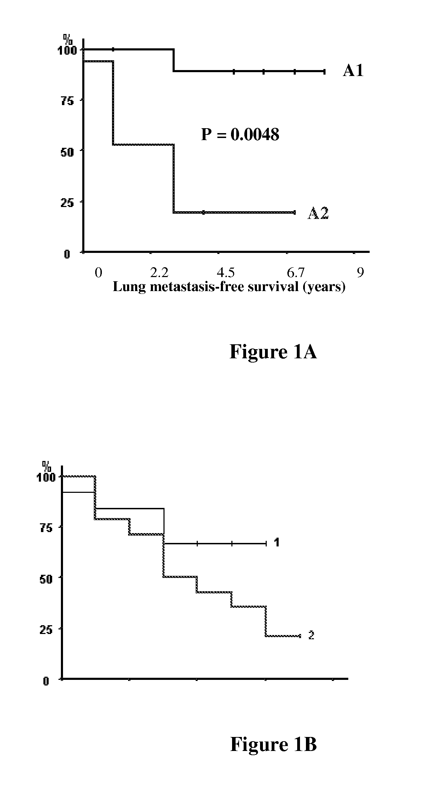

**Figure 1**: Test of the bone and lung metastatic associated genes on primary tumors. Hierarchical clustering of relapsing primary breast carcinomas from a cohort of 27 patients was performed with the lung (A) and bone (B) metastatic signatures. Lung-metastasis-free survival and bone-metastasis-free survival analysis of corresponding patients was performed. Tumors from patients A2 (bottom line) express lung metastatic associated genes in a manner resembling lung relapses. Tumors from patients B2 (bottom line line) express bone metastatic associated genes in a manner resembling bone relapses.

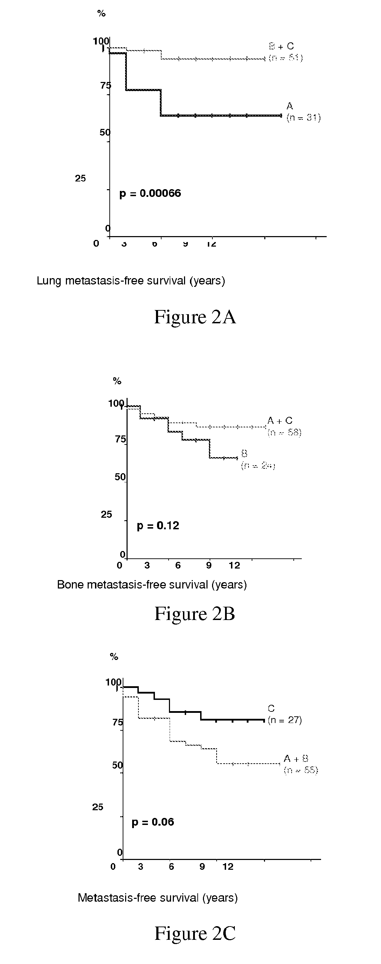

**Figure 2****:** Segregation of primary breast carcinomas using the organ-specific signature. Figure 2A depicts lung metastasis-free survival. Figure 2B depicts bone metastasis-free survival. Figure 2C depicts overall metastasis-free survival. Lung-metastasis-free survival (Kaplan Meier analysis) for 82 breast cancer patients who either expressed (Fig. 2A, corresponding to cluster A) or did not express (green line) the lung metastasis signature (cluster B + C).

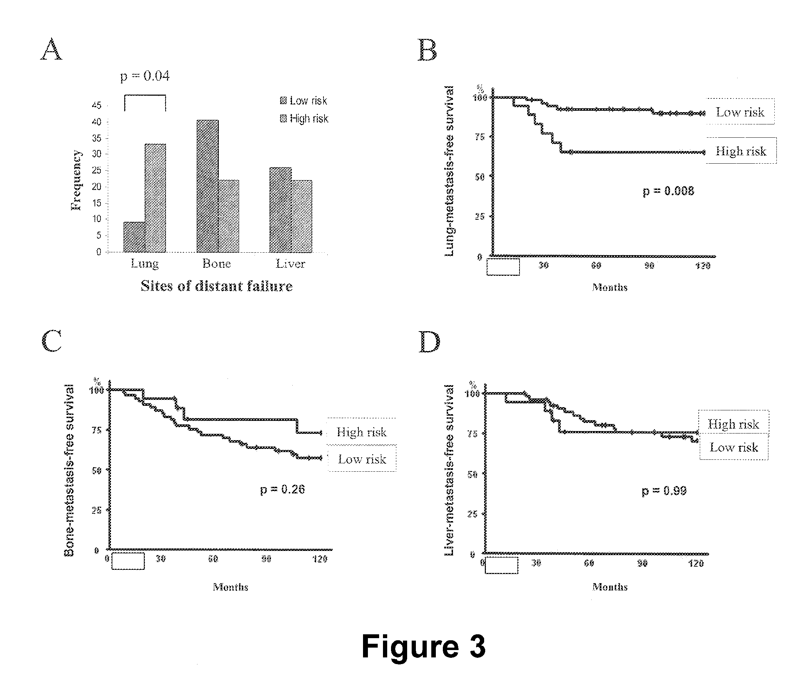

**Figure 3****:** Performance of the six-gene lung metastasis signature in a series of 72 node-negative breast tumors (the CRH cohort) (A) Distribution of distant metastases (first and/or only metastatic sites) in the high-risk and low-risk groups identified by the six-gene signature (Chi² test). (B) Patients with tumors expressing the six-gene signature (high-risk group) had shorter lung-metastasis-free survival (log-rank test). (C and D) Kaplan-Meier curves of bone-metastasis-free survival and liver metastasis-free survival showed no difference between the high- and low-risk groups identified by the six-gene signature.

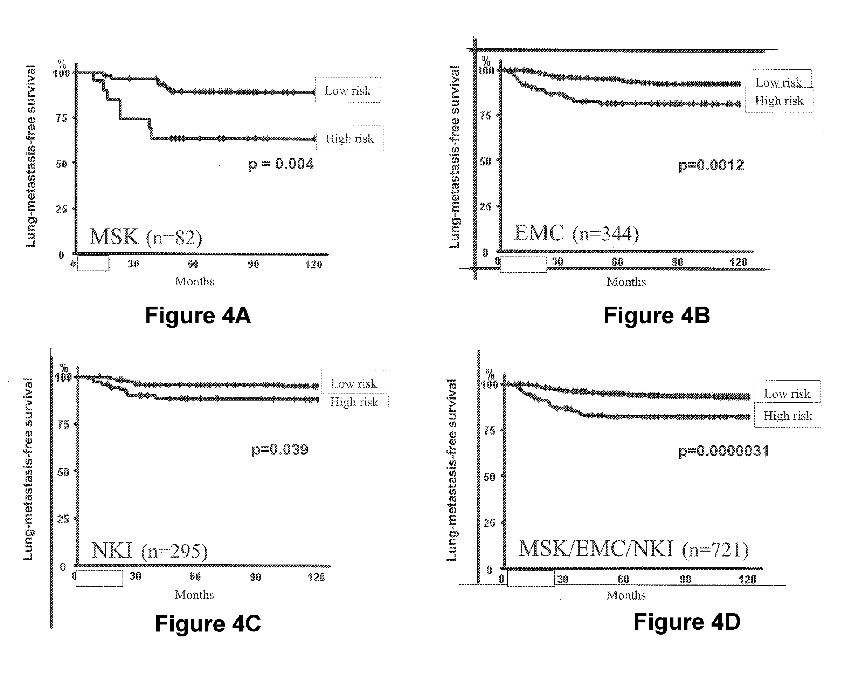

**Figure 4****:** Validation of the six-gene lung metastasis signature in 3 independent series of breast cancer patients. Lung-metastasis-free survival was analyzed for MSK (Fig. 4A), EMC (Fig. 4B) and NKI (Fig. 4C) cohorts (82, 344 and 295 patients respectively) and the combined cohort of 721 breast cancer patients (Fig. 4D). Kaplan-Meier analysis distinguished patients who expressed (high-risk group) and did not express (low-risk group) the six-gene signature. Patients with a high predicted risk of lung metastasis had shorter lung-metastasis-free survival.

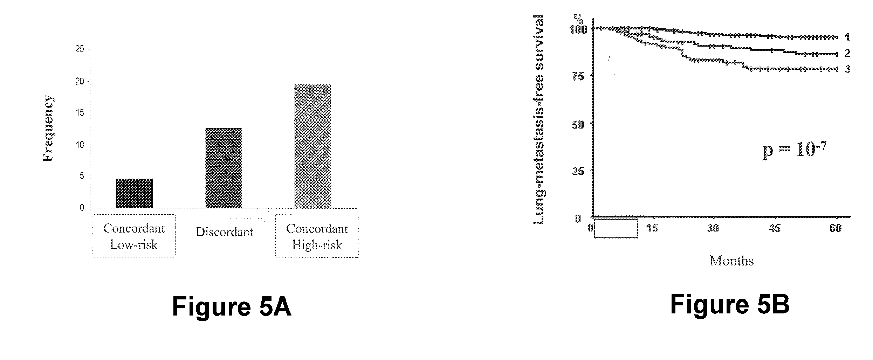

**Figure 5****:** Integration of diverse clinicopathological markers and gene expression signatures for lung metastasis risk prediction. (A) Prediction of breast cancer lung metastasis is improved by using a combination of predictors derived from 2 distinct models. (B) Distribution of lung metastases in the combined cohort of 721 breast cancer patients (MSK/EMC/NKI) according to the clinically and experimentally derived signatures. Patients that are negative for both signatures are shown in black; those with divergent assignement are indicated in blue; and patients found positive for both signatures are shown in green. The six-gene and LMS signatures showed 85% agreement in outcome classification of breast cancer patients with respect to lung metastasis (Kappa coefficient = 0.57). (B) Kaplan-Meier analysis of breast cancer patients (n=721) according to the six-gene and MDA-derived lung metastasis signatures.

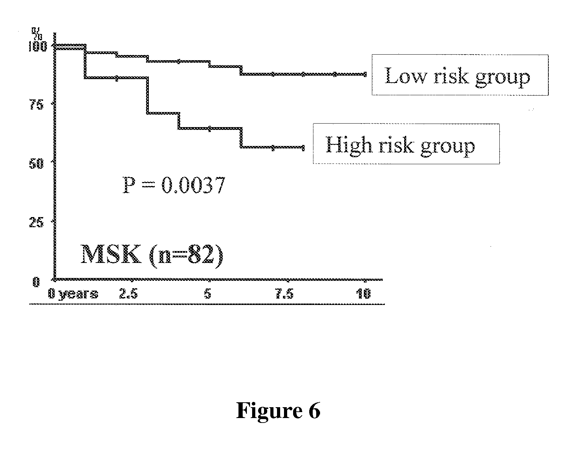

**Figure 6** **: Test of bone metastatic associated genes on primary tumors : Hierarchical clustering of relapsing primary breast carcinomas from a cohort of patients was performed with the eleven-gene bone metastatic signature. Bone metsatsis-free survival analysis of corresponding patients was performed.**

### DETAILED DESCRIPTION OF THE INVENTION

Methods allowing an early prediction of the likelihood of development of metastasis in breast cancer patients are provided by the present invention.

Particularly, it is provided herein methods and kits allowing prediction of the occurrence of metastasis to one or more specific tissue or organ in breast cancer patients, notably in bone, lung, liver and brain tissues or organs.

According to the present invention, highly reliable tissue-specific sets of biological markers that are indicative of a high probability of metastasis occurrence in breast cancer patients have been identified.

The identification of these highly reliable tissue-specific sets of biological markers was permitted, due to the use of a highly accurate method for the screening of such metastasis biological markers that is described below in the present specification.

Thus, an object of the present invention consists of an *in vitro* method for predicting the occurrence of metastasis in a patient affected with a breast cancer, comprising the steps of :
a) providing a breast tumour tissue sample previously collected from the patient to be tested;
b) determining, in the said breast tumour tissue sample, the expression of one or more biological markers that are indicative of an occurrence of metastasis in one or more tissue or organ, wherein the said one or more biological markers are selected from the group consisting of :
   (i) one or more biological markers indicative of an occurrence of metastasis in the bone tissue that are selected from the group consisting of : CLEC4A, MFNG, NXF1, FAM78A, KCTD1, BAIAP2L1, PTPN22, MEGF10, PERP, PSTPIP1, FL11, COL6A2, CD4, CFD, ZFHX1B, CD33, LST1, MMRN2, SH2D3C, RAMP3, FAM26B, ILK, TM6SF1, C10orf54, CLEC3B, IL2RG, HOM-TES-103, ZNF23, STK35, , TNFAIP8L2, RAMP2, ENG, ACRBP, TBC1D10C, C11orf48, EBNA1BP2, HSPE1, GAS6, HCK, SLC2A5, RASA3, ZNF57, WASPIP, KCNK1, GPSM3, ADCY4, AIF1, , NCKAP1, AMICA1, POP7, GMFG, PPM1M, CDGAP, , GIMAP1, ARHGAP9, APOB48R, OCIAD2, FLRT2, P2RY8, RIPK4, PECAM1, , URP2, BTK, , , APBB1IP, CD37, STARD8, GIMAP6, E2F6, WAS, , HLA-DQB1, HVCN1, LOC56902, ORC5L, MEF2C, PLCL2, PLAC9, RAC2, SYNE1, DPEP2, MYEF2, HSPD1, PSCD4, , NXT1, , LOC340061, ITGB3, AP1S2, SNRPG, , , CSF1, BIN2, , ANKRD47, , , LIMS2, , DARC, PTPN7, MSH6, GGTA1, LRRC33, GDPD5, CALCOCO1, FAM110C, BCL6B, LOC641700, ARHGDIB, DAAM2, TNFRSF14, TPSAB1, CSF2RA, RCSD1, FLJ21438, LOC133874, GSN, SLIT3, FYN, NCF4, PTPRC, EVI2B, SCRIB, C11orf31, LOC440731, TFAM, ARPC5L, PARVG, GRN, LMO2, CRSP8, EHBP1L1, HEATR2, , NAALADL1, , LTB, STRBP, FAM65A, ADARB1, TMEM140, DENND1 C, PRPF19, , CASP10, , SLC37A2, RHOJ, MPHOSPH10, PPIH, RASSF1, , HCST, C16orf54, EPB41 L4B, LRMP, LAPTM5, PRDM2, CYGB, LYCAT, ACP5, CMKLR1, UBE1L, MAN2C1, TNFSF12, C7orf24, Cxorf15, CUL1, SMAD7, ITGB7, APOL3, PGRMC1, PPA1, YES1, FBLN1, MRC2, PTK9L, LRP1, IGFBP5, , WDR3, GTPBP4, , SPI1, SELPLG, OSCAR, LYL1, POLR2H, YWHAQ, ISG20L2, LGI14, KIF5B, , NGRN, TYROBP, C5orf4, COX7A2, S100A4, MATK, TMEM33, DOK3, LOC150166, CIRBP, NIN, C10orf72, FMNL1, FAT3, CHKB/CPT1B, SNRPA1, GIMAP4, C20orf18, LTBP2, GAB3, NQO1, MARCH2, MYO1F, CDS1, SRD5A1, C20orf160, SLAMF7, ACTL6A, ABP1, RAE1, MAF, SEMA3G, P2RY13, ZDHHC7, ERG, , FHL1, CLEC10A, INTS5, MYO15B, CTSW, PILRA, HN1, SCARA5, PRAM1, EBP, , SIGLEC9, LGP1, DGUOK, GGCX, , RABL5, ZBTB16, , NOP5/NOP58, CCND2, CD200, EPPK1, , DKFZp586CO721, CCT6A, RIPK3, ARHGAP25, GNAI2, USP4, FAHD2A, LOC399959, LOC133308, HKDC1, CD93, GTF3C4, ITGB2, ELOVL6, TGFB1I1 ASCC3L1, FES, , KCNMB1, AACS, ATP6VOD2, TMEM97, NUDT15, ATP6V1B2, CCDC86, FLJ10154, SCARF2, PRELP, ACHE, , GIMAP8, PDE4DIP, NKG7, , C20orf59, RHOG, TRPV2, TCP1, TNRC8, TNS1,IBSP, MMP9, NRIP2, OLFML2B, OMD, WIF1, ZEB2, ARL8, COL12A1, EBF and EBF3 ;
   (ii) one or more biological markers indicative of an occurrence of metastasis in the lung tissue that are selected from the group consisting of : DSC2, HORMAD1, PLEKHG4, ODF2L, C21orf91, TFCP2L1, TTMA, CHODL, CALB2, UGT8, LOC146795, C1orf210, SIKE, ITGB8, PAQR3, ANP32E, C20orf42/ FERMT1, ELAC1, GYLTL1B, SPSB1, CHRM3, PTEN, PIGL, CHRM3, CDH3 ;
   (iii) one or more biological markers indicative of an occurrence of metastasis in the liver tissue that are selected from the group consisting of : TBX3, , SYT17, LOC90355, AGXT2L1, LETM2, LOC145820, ZNF44, IL20RA, ZMAT1, MYRIP, WHSC1L1, SELT, , GATA2, ARPC2, CAB39L, SLCI6A3, DHFRL1, PRRT3, CYP3A5, RPS6KA5, KIAA1505, ATP5S, ZFYVE16, KIAA0701, PEBP1, DDHD2, WWP1, CCNL1, ROBO2, FAM111B, THRAP2, CRSP9, KARCA1, SLC16A3, , ARID4A, TCEAL1, SCAMP1, KIAAO701, EIF5A, DDX46, PEX7, , BCL2L11, YBX1, UBE21, REXO2, AXUD1, C10orf2, ZNF548, FBXL16, LOC439911, LOC283874, ZNF587, FLJ20366, KIAAO888, BAG4, CALU, KIAA1961, USP30, NR4A2, FOXA1, FBXO15, WNK4, CDIPT, NUDT16L1, SMAD5, STXBP4, TTC6, LOC113386, , TSPYL1, CIP29, C8orf1, , SYDE2, SLC12A8, SLC25A18, C7, STAU2, TSC22D2, GADD45G, PHF3, TNRC6C, TCEAL3, , RRN3, C5orf24, AHCTF1, LOC92497 ; and
   (iv) one or more biological markers indicative of an occurrence of metastasis in the brain tissue that are selected from the group consisting of : LOC644215, BAT1, GPR75, PPWD1, INHA, PDGFRA, MLL5, RPS23, ANTXR1, ARRDC3, PTK2, SQSTM1, METTL7A, NPHP3, PKP2, DDX31, FAM119A, LLGL2, DDX27, TRA16, HOXB13, GNAS, CSPP1, COL8A1, RSHL1, DCBLD2, UBXD8, SURF2, ZNF655, RAC3, AP4M1, HEG1, PCBP2, , SLC30A7, ATAD3A/ATAD3B, CHI3L1, MUC6, HMG20B, BCL7A, GGN, ARHGEF3, PALLD, TOP1, PCTK1, C20orf4, ZBTB1, MSH6, SETD5, POSTN, MOCS3, GABPA, ZSWIM1, ZNHIT2, LOC653352, ELL, ARPC4, ZNF277, VAV2, HNRPH3, LHX1, FAM83A, DIP2B, RBM10, PMPCA, TYSND1, RAB4B, DLC1, KIAA2018, TES, TFDP2, C3orf10, ZBTB38, PSMD7, RECK, JMJD1C, FLJ20273, CENPB, PLAC2, C6orf111, ATP10D, RNF146, XRRA1, NPAS2, APBA2BP, WDR34, SLK, SBF2, , SON, MORC3, C3orf63, WDR54, STX7, ZNF512, KLHL9, LOC284889, ETV4, RMND5B, ARMCX1, SLC29A4, TRIB3, LRRC23, DDIT3, THUMPD3, MICAL-L2, PA2G4, TSEN54, LAS1L, MEA1, S100PBP, TRAF2, EMILIN3, KIAA1712, PRPF6, CHD9, JMJD1B, ANKS1A, CAPN5, EPC2, WBSCR27, CYB561, LLGL1, EDD1.
c) predicting the occurrence of metastasis in one or more tissue or organ when one or more of the said biological markers has (have) a deregulated expression level, as compared to its corresponding expression level measured in a breast tumour sample of a patient that has not undergone metastasis in the corresponding tissue or organ.

As previously mentioned herein, prior art studies disclosed several gene signatures containing genes potentially involved in metastatic processes and/or markers of distant relapses. However, these prior art studies tackled overall relapses problems. As there are multiple types of metastases and potentially multiple distinct pathological processes leading to metastases, these prior art studies suffered for lack of accuracy.

Notably, according to a plurality of these prior art methods, selection of metastasis-specific biological markers was performed by human marker expression analysis in artificial *in vivo* systems, namely in non-human animals, especially in mice.

Further, the screening of metastasis-specific biological markers were generally performed using artificial human cell systems, namely established human cancer cell lines, wherein expression artefacts of one or more genes or proteins cannot be excluded. The probability of introduction of biological markers expression artefacts was further increased by various features of the screening methods which were used, including the selection of multiple human cell sublines having a metastatic potency derived from the parental cell line, by reiterating *in vivo* cycles of cell administration/selection in non-human animal systems, including mice.

Still further, according to these prior art techniques, once pertinent cell sublines were finally selected for their high metastatic potency in non-human animals, differential gene expression analysis was performed by comparing the expression levels of genes between (i) the parental human breast cancer cell line and (ii) the various cell sublines having a metastatic potency.

In view of improving the accuracy of the prior art techniques of selecting metastasis-specific markers for breast cancer, the inventors have designed an original method for selecting highly reliable tissue-specific biological markers of metastasis in breast cancer, using a whole human system of analysis and including, among other features, a step of comparing the expression level of candidate biological markers between (i) a metastatic tissue or organ of interest and (ii) one or more distinct metastatic tissue(s) or organ(s), thus allowing a high selectivity and statistical relevance of the tissue-specific metastatic biological markers that are positively selected, at the end of the marker selection method.

As it is shown in the examples herein, when using a marker selection method comprising a step of comparing the expression level of candidate biological markers between (i) a metastatic tissue or organ of interest selected from the group consisting of bone, lung, liver and brain and (ii) all the other metastatic tissue(s) or organ(s) selected from the group consisting of bone, lung, liver and brain, the inventors have identified tissue-specific breast cancer metastasis biological markers endowed with a high statistical relevance, with P values always lower than 1.10⁻⁴, the said P values being lower than 10⁻⁶ for the most statistically relevant biological markers. Statistical relevancy of the biological markers primarily selected was fully corroborated by Kaplan-Meier analysis, after having assayed for the expression of the said tissue-specific markers in tumour tissue samples of breast cancer patients, as it is shown in the examples herein.

As intended herein; a "biological marker" encompasses any detectable product that is synthesized upon the expression of a specific gene, and thus includes gene-specific mRNA, cDNA and protein.

As used herein, a "biological marker indicative of an occurrence of metastasis", or a "metastasis-specific marker", encompasses any biological marker which is differentially expressed in breast tumors that generate metastasis, or will generate metastasis, in a specific given tissue or in a specific given organ, as compared to the expression of the same biological marker in breast tumors that do not.generate metastasis, or will not generate metastasis, in the said specific given tissue or in the said specific given organ. Preferably, tissues and organs are selected from the group consisting of bone, lung, liver and brain.

The terms "tissue-specific" marker and "tissue metastasis-specific" marker are used interchangeably herein. Similarly, the terms "organ-specific" marker and "organ metastasis-specific" marker are used interchangeably herein.

As intended herein, a "prediction of the occurrence of metastasis" does not consist of an absolute value, but in contrast consists of a relative value allowing to quantify the probability of occurrence of a metastasis to one or more specific tissue(s) or organ(s), in a breast cancer patient. In certain embodiments, the prediction of the occurrence of metastasis is expressed as a statistical value, including a P value, as calculated from the expression values obtained for each of the one or more biological markers that have been tested.

As intended herein, a "tumour tissue sample" encompasses (i) a global primary tumour (as a whole), (ii) a tissue sample from the center of the tumour, (iii) a tissue sample from a location in the tumour, other than the center of the tumour and (iv) any tumor cell located outside the tumor tissue *per se.* In certain embodiments, the said tumour tissue sample originates from a surgical act of tumour resection performed on the breast cancer patient. In certain other embodiments, the said tissue sample originates from a biopsy surgical act wherein a piece of tumour tissue is collected from the breast cancer patient for further analysis. In further embodiments, the said tumor sample consists of a blood sample, including a whole blood sample, a serum sample and a plasma sample, containing tumour cells originating from the primary tumor tissue, or alternatively from metastasis that have already occurred. In still further embodiments, the said tumor sample consists of a blood sample, including a whole blood sample, a serum sample and a plasma sample, containing tumor proteins produced by tumor cells originating from the primary tumor tissue, or alternatively from metastasis that have already occurred.

The various biological markers names specified herein correspond to their internationally recognised acronyms that are usable to get access to their complete amino acid and nucleic acid sequences, including their complementary DNA (cDNA) and genomic DNA (gDNA) sequences. Illustratively, the corresponding amino acid and nucleic acid sequences of each of the biological markers specified herein may be retrieved, on the basis of their acronym names, that are also termed herein "gene symbols", in the GenBank or EMBL sequence databases. All gene symbols listed in the present specification correspond to the GenBank nomenclature. Their DNA (cDNA and gDNA) sequences, as well as their amino acid sequences are thus fully avaialble to the one skilled in the art from the GenBank database, notably at the following Website address : "http://www.ncbi.nlm.nih.gov/". The same sequences may also be retrieved from the Hugo Gene Nomenclature Committee (HGCN) database that is available at the following Website address :

### http://www.gene.ucl.ac.uk/nomenclature/.

At step b) of the *in vitro* prediction method according to the invention, one or more of the specified biological markers is (are) quantified. Quantification of a biological marker includes detection of the expression level of the said biological marker. Detection of the expression level of a specific biological marker encompasses the assessment of the amount of the corresponding specific mRNA or cDNA that is expressed in the tumour tissue sample tested, as well as the assessment of the amount of the corresponding protein that is produced in the said tumour tissue sample.

At the end of step b) of the method according to the invention, a quantification value is obtained for each of the one or more biological markers that are used.

As it has been previously specified, specific embodiments of step b) include :
(i) quantifying one or more biological markers by immunochemical methods, which encompasses quantification of one or more protein markers of interest by *in situ* immunohistochemical methods on a tumor tissue sample, for example using antibodies directed specifically against each of the said one or more protein markers.
(ii) quantifying one or more biological markers by gene expression analysis, which encompasses quantification of one or more marker mRNAs of interest, for example by performing a Real-Time PCR Taqman PCR analysis, as well as by using specifically dedicated DNA microarrays, i.e. DNA microarrays comprising a substrate onto which are bound nucleic acids that specifically hybridize with the cDNA corresponding to every one of the biological markers of interest, among the biological markers listed herein..

In certain other embodiments of the method, step b) consists of quantifying, in a tumor tissue sample, the expression level of one or more marker genes among those specified above. Generally, the assessment of the expression level for a combination of at least two marker genes is performed. In these embodiments of step b) of the method, what is obtained at the end of step b) consists of the expression level values found for each marker gene (nucleic acid or protein expression level) specifically found in cells contained in the tumour tissue sample.

The expression level of a metastasis-specific biological marker according to the present invention may be expressed as any arbitrary unit that reflects the amount of the corresponding mRNA of interest that has been detected in the tissue sample, such as intensity of a radioactive or of a fluorescence signal emitted by the cDNA material generated by PCR analysis of the mRNA content of the tissue sample, including (i) by Real-time PCR analysis of the mRNA content of the tissue sample and (ii) hybridization of the amplified nucleic acids to DNA microarrays.Preferably, the said expression level value consists of a normalised relative value which is obtained after comparison of the absolute expression level value with a control value, the said control value consisting of the expression level value of a gene having the same expression level value in any breast tissue sample, regardless of whether it consists of normal or tumour breast tissue, and/or regardless whether it consists of a non-metastatic or a metastatic breast tissue. Illustratively, the said control value may consist of the amount of mRNA encoding the TATA-box-binding protein (TBP), as it is shown in the examples herein.

Alternatively, the said expression level may be expressed as any arbitrary unit that reflects the amount of the protein of interest that has been detected in the tissue sample, such as intensity of a radioactive or of a fluorescence signal emitted by a labelled antibody specifically bound to the protein of interest. Alternatively, the value obtained at the end of step b) may consist of a concentration of protein(s) of interest that could be measured by various protein detection methods well known in the art, such as. ELISA, SELDI-TOF, FACS or Western blotting.

Because every one of the biological markers that are specified herein consists of a biological marker (i) that is specifically expressed, including at a given expression level, exclusively in a given breast cancer metastatic tissue or organ and (ii) that is expressed at another expression level, in a distinct breast cancer metastatic tissue or organ, then the sole quantification of its expression in a tumour tissue sample originating from a primary tumour specimen allows to predict whether the breast cancer-bearing patient is likely to undergo generation of metastasis in the said tissue or organ.

Additionally, quantifying the said one or more biological markers that are specified herein brings further prediction data relating to the probability of occurrence of metastasis in one or more specific tissue or organ in a breast cancer-bearing patient, since the probability of occurrence of metastasis increases with an increased deregulation of the expression level of the said one or more biological markers tested, as compared to their control expression level that is previously measured in a breast tumor sample from a patient that has not undergone metastasis, at least in the tissue or organ of interest that is considered.

Thus, as used herein, a biological marker of interest having a "deregulated expression level" consists of a metastasis-specific biological marker for which is found, when performing step b) of the *in vitro* prediction method according to the invention, an expression level value that is distinct from the expression level value (that may also be termed the "control" expression value) for the said biological marker that has been previously determined (i) in tumor tissue samples originating from breast cancer patients that have never undergone metastasis, or alternatively (ii) in tumor tissue samples originating from breast cancer patients that have never undergone metastasis in the tissue or organ from which the said biological marker is metastasis-specific.

For performing step c) of the prediction method according to the invention, the one skilled in the art may refer to the deregulated expression values for each of the metsatasis-specific markers described herein, as they are found notably in Tables 1, 2, 5 and 8.

As it is shown in the examples, every one of the biological markers listed herein is highly relevant for predicting the occurrence of metastasis in a specific tissue, since the markers having the lowest statistical relevance possess a P value lower than 1.10⁻⁴.

Further, accuracy of the metastasis prediction increases, at step c) of the method, when more than one tissue-specific biological marker for a given tissue or organ is detected and/or quantified at step b).

Thus, in preferred embodiments of the prediction method according to the invention, more than one biological marker for a given tissue or organ is detected and/or quantified, at step b) of the method.

The more biological markers specific for a given tissue or organ are quantified at step b) of the method, the more accurate are the prediction results that are obtained at step c) of the said method.

Quantifying a plurality of biological markers specific for a given tissue or organ at step b) of the method allows to generate an experimental expression profile of the said plurality of markers, which expreimental expression profile is then compared with at least one reference expression profile that has been previously determined from tissue-specific metastasis patients. Illustratively, if bone metastasis is suspected in the patient tested, then the experimental expression profile that is generated from the results of quantification of the bone metastasis-specific marker genes used at step b) of the method is compared with the pre-existing reference expression profile corresponding to bone metastasis, for prediction. Preferably, the experimental expression profile of the bone-specific metastasis markers is compared with reference expression profiles of the same genes that have been previously determined in patients having bone metastasis, as well as with reference expression profiles obtained from patients having other tissue- or organ-specific metastasis, so as to ensure that the experimental expression profile is the most close from the reference expression profile predetermined from patients having bone metastasis.

In certain embodiments of the prediction method, step b) comprises quantifying at least one tissue-specific biological marker for each tissue or organ. Illustratively, in those embodiments of the method, step b) comprises quantifying (i) at least one bone-specific biological marker, (ii) at least one lung-specific biological marker, (iii) at least one liver-specific biological marker and (iv) at least one brain-specific biological marker.

In certain embodiments of the method, step b) comprises quantifying at least two tissue-specific biological markers for a given tissue or organ. The higher the number of tissue-specific markers for a given tissue or organ are quantified at step b), the more accurate is the prediction of occurrence of metastasis in the said given tissue in the breast cancer-bearing patient tested.

Thus, in certain embodiments of the prediction method according to the invention, the number of biological markers tested for a given tissue or organ is of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40,41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199; 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299 or 300 distinct tissue-specific biological markers for a given tissue or organ selected from the group consisting of the tissue-biological markers disclosed in the present specification, the maximum number of distinct tissue-specific biological markers for a given tissue or organ being limited by the number of markers that are disclosed herein for the said given tissue or organ.

Any combination of two or more of the biological marker described herein is fully encompassed by the present invention.

When a plurality of biological markers for each ot the tissues or organs are quantified, at step b) of the method, then expermimental expression profiles of the patient tested may be generated, that are then compared to the reference expression profiles of the same biological markers, so as to determine to which reference expression profile the experimental expression profile is the most similar, and thus which tisue- or organ-specific metastasis may eventually be predicted.

In some embodiments of the prediction method according to the invention, step c) may be performed by the one skilled in the art by calculating a risk index of organ-specific metastasis of the patient tested, starting from the expression level values of the one or more biological markers that ave been determined at step b). Numerous methods for calculating a risk index are well known from the one skilled in the art.

Illustratively, the one skilled in the art may calculate the risk index of the patient tested, wherein the said risk index is defined as a linear combination of weighted (or not, depending one the genes tested) expression level values with the standardized Cox's regression coefficient as the weight. $Risk index = A + {\sum }_{i = 1}^{n} {w}_{i} ⁢ {x}_{i}$
wherein :
- A is a constant
- Wᵢ is the standardized Cox's regression coefficient for the markers
- Xᵢ is the expression value of the marker (log scale)
- n is the number of genes to predict the risk

The threshold is determined from the ROC curve of the training set to ensure the highest sensitivity and specificity. The constant value A is chosen to center the threshold of the risk index to zero. Patients with positive risk index are classified into the high risk of organ-specific group and patients with negative risk index are classified into the low risk of organ-specific group.

Illustrative embodiments of the prediction method according to the invention, wherein step c) is performed by calculating a risk index of organ-specific metastasis of the patient tested are illustrated in **Tables 9, 10, 11 and 12.**

**Table 9** illustrates the predictive values of various lung-specific biological markers that may be used in the method according to the invention. Details of Table 9 are given hereunder.
- Cohort: EMC-344 TP
- Summary of Results:
   - Number of genes significant at 0.99 level of the univariate test: 23
   - Genes significantly associated with survival:
   - Table 9 - Sorted by p-value of the univariate test.
   - The first 23 genes are significant at the nominal 0.99 level of the univariate test
   - Hazard ratio is the ratio of hazards for a two-fold change in the gene expression level.
   - It is equal to exp(b) where b is the Cox regression coefficient. SD is the standard deviation of the log 2 of the gene expression level.

**Table 10** illustrates the predictive values of various lung-specific biological markers that may be used in the method according to the invention. Details of Table 10 are given hereunder.
- Cohort: MSK-82 TP
- Summary of Results:
   - Number of genes significant at 0.99 level of the univariate test: 23
   - Genes significantly associated with survival:
   - Table 10 - Sorted by p-value of the univariate test.
   - The first 23 genes are significant at the nominal 0.99 level of the univariate test
   - Hazard ratio is the ratio of hazards for a two-fold change in the gene expression level.2 of the gene expression level.

It is equal to exp(b) where b is the Cox regression coefficient. SD is the standard deviation of the log

**Table 11** illustrates the predictive values of various lung-specific biological markers that may be used in the method according to the invention. Details of Table 11 are given hereunder.
- Cohort : NKI-295
- Summary of Results:
   - Number of genes significant at 0.9 level of the univariate test: 20
   - Genes significantly associated with survival:
   - able 11 - Sorted by p-value of the univariate test.
   - The first 20 genes are significant at the nominal 0.9 level of the univariate test
   - Hazard ratio is the ratio of hazards for a two-fold change in the gene expression level. 2 of the gene expression level.
   - It is equal to exp(b) where b is the Cox regression coefficient. SD is the standard deviation of the log 2 of the gene expression level.

**Table 12** illustrates the predictive values of various bone-specific biological markers that may be used in the method according to the invention. Details of Table 12are given hereunder.
- Cohort : NKI-295
- Summary of Results:
   - Number of genes significant at 0.99 level of the univariate test: 51
   - Genes significantly associated with survival:
   - Table 1 - Sorted by p-value of the univariate test.
   - The first 51 genes are significant at the nominal 0.99 level of the univariate test
   - Hazard ratio is the ratio of hazards for a two-fold change in the gene expression level.
   - It is equal to exp(b) where b is the Cox regression coefficient. SD is the standard deviation of the log 2 of the gene expression level.

Also, the 40 highest ranking bone-specific biological markers that have been identified according to the present invention are shown in **Table 13** hereunder.

In certain embodiments of the prediction method according to the invention, the one or more bone-specific biological markers that are quantified at step b) are selected from the group consisting of CLEC4A, MFNG, NXF1, FAM78A, KCTD1, BAIAP2L1, PTPN22, MEGF10, PERP, PSTPIP1, FL11, COL6A2, CD4, CFD, ZFHX1B, CD33, MMRN2, LST1, SH2D3C and RAMP3. Those bone-specific markers possess a high statistical relevance for predicting the occurrence of metastasis in bone tissue, with P values obtained after DNA microarray analysis that are always lower than 10⁻⁶, as shown in **Table** 2

In certain embodiments of the prediction method according to the invention, the one or more lung-specific biological markers that are quantified at step b) are selected from the group consisting of DSC2, HORMAD1, PLEKHG4, ODF2L, c21orf91, TFCP2L1, CHODL, CALB2, UGT8, c1orf210, SIKE, ITGB8, PAQR3, ANP32E, KIND1, ELAC1, GYLTL1B, SPSB1, PTEN and CHRM3. Those lung-specific markers possess a high statistical relevance for predicting the occurrence of metastasis in lung tissue or organ, with P values obtained after DNA microarray analysis that are always lower than 10⁻⁴, as shown in **Table 2**

In certain embodiments of the prediction method according to the invention, the one or more liver-specific biological markers that are quantified at step b) are selected from the group consisting of TBX3, c5orf30, AGXT2L1, LETM2, ZNF44, IL20RA, ZMAT1, MYRIP, WHSC1L1, SELT, GATA2, DHFRL1, ARPC2, SLC16A3, GNPTG, PRRT3, RPS6KA5, K1AA1505, ZFYVE16 and K1 AA0701. Those liver-specific markers possess a high statistical relevance for predicting the occurrence of metastasis in liver tissue or organ, with P values obtained after DNA microarray analysis that are always lower than 10⁻⁵, as shown in **Table 2**

In certain embodiments of the prediction method according to the invention, the one or more brain-specific biological markers that are quantified at step b) are selected from the group consisting of PPWD1, INHA, PDGFRA, MLL5, RPS23, ANTXR1, ARRDC3, METTL7A, NPHP3, PKP2, DDX27, TRA16, HOXB13, CSPP1, RSHL1, DCBLD2, UBXD8, SURF2, ZNF655 and RAC3. Those brain-specific markers possess a high statistical relevance for predicting the occurrence of metastasis in brain tissue or organ, with P values obtained after DNA microarray analysis that are always equal to, or lower than, 10⁻⁵, as shown in **Table 2.**

In certain other embodiments of the prediction method according to the invention, the one or more tissue-specific biological markers are selected from the following groups of markers :
(i) the group of bone metastasis-specific markers consisting of KTCD1, BAIAP2L1, PERP, CFD, CD4, COL6A2, FLI1, PSTPIP1, MGF10, PTPN22, FAM78A, NXF1, MFNG and CLEC4A;
(ii) the group of lung metastasis-specific markers consisting of KIND1, ELAC1, ANP32E, PAQR3, ITGB8, c1orf210, SIKE, UGT8, CALB2, CHODL, c21 orf91, TFCP2L1, ODF2L, HORMAD1, PLEKHG4 and DSC2;
(iii) the group of liver metastasis-specific makers consisting of GATA2, SELT, WHSC1L1, MYRIP, ZMAT1, IL20RA, ZNF44, LETM2, AGXT2L1, c5orf30 and TBX3.
(iv) the group of brain metastasis-specific markers consisting of PPWD1, PDGFRA, MLL5, RPS23, ANTXR1, ARRDC3, METTL7A, NPHP3, RSHL1, CSPP1, HOXB13, TRA16, DDX27, PKP2 and INHA.

In still other embodiments of the prediction method according to the invention, the one or more tissue-specific biological markers are selected from the following groups of markers :
(i) the group of bone metastasis-specific markers consisting of BAIAP2L1, PERP, CFD, CD4, COL6A2, FLI1, PSTPIP1, MGF10, PTPN22, FAM78A, NXF1, MFNG and CLEC4A;
(ii) the group of lung metastasis-specific markers consisting of KIND1, ANP32E, ITGB8, UGT8, TFCP2L1, HORMAD1 and DSC2;
(iii) the group of liver metastasis-specific makers consisting of GATA2, WHSC1L1, LETM2, AGXT2L1 and TBX3.
(iv) the group of brain metastasis-specific markers consisting of PPWD1, PDGFRA, ANTXR1, ARRDC3 and DDX27.

In yet further embodiments of the prediction method according to the invention, the one or more lung-specific biological markers that are detected and/or quantified at step b) are selected from the group consisting of DSC2, TFCP2L1, UGT8, ITGB8, ANP32E and FERMT1.

In still further embodiments of the prediction method according to the invention, the one or more bone-specific biological markers that are detected and/or quantified at step b) are selected from the group consisting of KCNK1, CLEC4A, MFNG, NXF1, PTPN22, PERP, PSTPIP1, FL11, COL6A2, CD4 and CFD. Predictive results of these bone-specific biological markers are shown in **Table 8** hereunder.

As it is shown in examples 3 to 6 herein, the said lung-specific markers possess a high statistical relevance for predicting the occurrence of metastasis in lung tissue or organ, with P values obtained after DNA microarray analysis that are always lower than 10⁻⁴, as shown notably in **Table 5.**

The lung-specific markers DSC2, TFCP2L1, UGT8, ITGB8, ANP32E and FERMT1 are all up-regulated in individuals affected with breast cancer and bearing lung metastasis, as compared with breast cancer patients who have no lung metastasis or with individuals who are not affected with a breast cancer, as shown in **Table 5.**

As shown in examples 3 to 6 herein, the DSC2, TFCP2L1, UGT8, ITGB8, ANP32E and FERMT1 lung-specific markers consists of a six-gene signature which is predictive of selective breast cancer relapse to the lungs.

As shown in the examples herein, this six-gene signature (DSC2, TFCP2L1, UGT8, ITGB8, ANP32E and FERMT1) was generated from a series of lymph node-negative breast cancer patients who did not receive any neoadjuvant or adjuvant therapy to allow the analysis of the signature prognostic impact along with the natural history of the disease. The predictor would thus not be influenced by factors related to systemic treatment.

This six-gene signature was validated in three independent cohort of breast cancers consisting of a total of 721 patients, as detailed hereafter and as illustrated in the examples herein.The said six-gene signature was thus validated in two independent data sets of patients of early stage (n=295 and 344) generated from two distinct microarray platforms and a third series of locally advanced breast cancer patients (n=82). In all tested individual series and in the combined cohort (n=721), the six gene signature had a strong predictive ability for breast cancer lung metastasis.

The results described in the examples herein show that the said six-gene signature improves risk stratification independently of known standard clinical parameters and previously established lung metastasis signature based on an experimental model.

Although there is no targeted therapy for lung metastasis, such as bisphosphonates for bone metastasis, the knowledge of organ-specific metastasis has been emphasized the last few years and might lead to targeted therapeutics in the near future. By delineating the risk for lung metastasis based on gene signatures, it might be possible that these high-risk breast cancer patients may benefit from these therapies targeting specific secondary failures.

Thus, in a further embodiment of the prediction method according to the invention, step b) consists of determining, in the breast tumour tissue sample, the expression of one or more biological markers that are indicative of an occurrence of metastasis in the lung tissue that are selected from the group consisting of DSC2, TFCP2L1, UGT8, ITGB8, ANP32E and FERMT1.

In the above-embodiment of the prediction method according to the invention, the expression of 2, 3, 4, 5 or all of the six lung-specific markers is determined, i.e. detected end/or quantified.

In certain embodiments of the prediction method according to the invention, every one of the tissue-specific biological markers comprised in each group of markers disclosed herein is submitted to quantification. In those embodiments, step b) comprises quantifying every biological marker contained in each group of markers disclosed herein. More precisely, according to those embodiments of the method, step b) comprises quantifying every biological marker contained in (i) one group of bone-specific markers, (ii) one group of lung-specific markers, (iii) one group of liver-specific markers and (iv) one group of brain-specific markers, selected among the various groups of (i) bone-specific markers, (ii) lung-specific markers, (iii) liver-specific markers and (iv) brain-specific markers disclosed in the present specification.

Indeed, the use of every possible combination of at least two metastasis-specific biological markers, that are selected from the group consisting of all the metastasis-specific biological markers disclosed in the present specification, is encompassed herein, for the purpose of performing the *in vitro* prediction method according to the invention.

Preferably, the use of every possible combination of four or more metastasis-specific biological markers, provided that each possible combination comprises (i) one or more bone metastasis-specific biological marker, (ii) one or more lung metastasis-specific biological marker, (iii) one or more liver metastasis-specific biological marker and (iv) one or more liver metastasis-specific biological marker, is encompassed herein, for the purpose of performing the *in vitro* prediction method according to the invention.

Thus, in certain embodiments of the prediction method according to the invention, at least one biological marker selected from each of the groups (i), (ii), (iii) and (iv) is quantified at step b).

In certain embodiments of the prediction method, all biological markers from specific groups (i), (ii), (iii) and (iv) are quantified at step b).

Thus, in certain embodiments of the prediction method according to the invention, step b) consists of determining (i.e. detecting and/or quantifying), in the breast tumour tissue sample, the expression of every one of the following lung-specific markers : DSC2, TFCP2L1, UGT8, ITGB8, ANP32E and FERMT1.

Also, in certain embodiments of the prediction method according to the invention, step b) consists of detremining (i.e. detecting and/or quantifying), in the breast tumor sample, the expression of every one of the following bone-specific markers : KCNK1, CLEC4A, MFNG, NXF1, PTPN22, PERP, PSTPIP1, FL11, COL6A2, CD4 and CFD.

At step b), the said one or more biological markers may be quantified by submitting the said breast tumour tissue sample to a gene expression analysis method.

At step b), the said one or more biological markers may alternatively be quantified by submitting the said breast tumour tissue sample to an immunohistochemical analysis method.

General methods for quantifying the tissue-specific biological markers disclosed herein are detailed below.

### General methods for quantifying biological markers

Any one of the methods known by the one skilled in the art for quantifying a protein biological marker or a nucleic acid biological marker encompassed herein may be used for performing the metastasis prediction method of the invention. Thus any one of the standard and non-standard (emerging) techniques well known in the art for detecting and quantifying a protein or a nucleic acid in a sample can readily be applied.

Such techniques include detection and quantification of nucleic acid biological markers with nucleic probes or primers.

Such techniques also include detection and quantification of protein biological markers with any type of ligand molecule that specifically binds thereto, including nucleic acids (e.g. nucleic acids selected for binding through the well known Selex method), and antibodies including antibody fragments. In certain embodiments wherein the biological marker of interest consists of an enzyme, these detection and quantification methods may also include detection and quantification of the corresponding enzyme activity.

Noticeably, antibodies are presently already available for most, if not all, the biological markers described in the present specification, including those biological markers that are listed in **Table 4**

- Further, in situations wherein no antibody is yet available for a given biological marker, or in situations wherein the production of further antibodies to a given biological marker is sought, then antibodies directed against the said given biological markers may be easily obtained with the conventional techniques, including generation of antibody-producing hybridomas. In this method, a protein or peptide comprising the entirety or a segment of a biological marker protein is synthesised or isolated (e.g. by purification from a cell in which

it is expressed or by transcription and translation of a nucleic acid encoding the protein or peptide *in vivo* or *in vitro* using known methods). A vertebrate, preferably a mammal such as a mouse, rat, rabbit, or sheep, is immunised using the protein or peptide. The vertebrate may optionally (and preferably) be immunised at least one additional time with the protein or peptide, so that the vertebrate exhibits a robust immune response to the protein or peptide. Splenocytes are isolated from the immunised vertebrate and fused with an immortalised cell line to form hybridomas, using any of a variety of methods well known in the art. Hybridomas formed in this manner are then screened using standard methods to identify one or more hybridomas which produce an antibody which specifically binds with the biological marker protein or a fragment thereof. The invention also encompasses hybridomas made by this method and antibodies made using such hybridomas. Polyclonal antibodies may be used as well.

Expression of a tissue-specific biological marker described herein may be assessed by any one of a wide variety of well known methods for detecting expression of a transcribed nucleic acid or protein. Non-limiting examples of such methods include immunological methods for detection of secreted, cell-surface, cytoplasmic, or nuclear proteins, protein purification methods, protein function or activity assays, nucleic acid hybridisation methods, nucleic acid reverse transcription methods, and nucleic acid amplification methods.

In one preferred embodiment, expression of a marker is assessed using an antibody (e.g. a radio-labelled, chromophore-labelled, fluorophore-labeled, polymer-backbone-antibody, or enzyme-labelled antibody), an antibody derivative (e.g. an antibody conjugated with a substrate or with the protein or ligand of a protein-ligand pair {e.g. biotin-streptavidin}), or an antibody fragment (e.g. a single-chain antibody, an isolated antibody hypervariable domain, etc.)
which binds specifically with a marker protein or fragment thereof, including a marker protein which has undergone all or a portion of its normal post-translational modification.

In another preferred embodiment, expression of a marker is assessed by preparing mRNA/cDNA (i.e. a transcribed polynucleotide) from cells in a patient tumour tissue sample, and by hybridising the mRNA/cDNA with a reference polynucleotide which is a complement of a marker nucleic acid, or a fragment thereof. cDNA can, optionally, be amplified using any of a variety of polymerase chain reaction methods prior to hybridisation with the reference polynucleotide.

In a preferred embodiment of the *in vitro* prediction method according to the invention, step b) of detection and/or quantification of the one or more biological markers is performed using DNA microarrays. Illustratively, according to this preferred embodiment, a mixture of transcribed polynucleotides obtained from the sample is contacted with a substrate having fixed thereto a polynucleotide complementary to or homologous with at least a portion (e.g. at least 7, 10, 15, 20, 25, 30, 40, 50, 100, 500, or more nucleotide residues) of a biological marker nucleic acid. If polynucleotides complementary to or homologous with are differentially detectable on the substrate (e.g. detectable using different chromophores or fluorophores, or fixed to different selected positions), then the levels of expression of a plurality of markers can be assessed simultaneously using a single substrate (e.g. a "gene chip" microarray of polynucleotides fixed at selected positions). When a method of assessing marker expression is used which involves hybridisation of one nucleic acid with another, it is preferred that the hybridisation be performed under stringent hybridisation conditions.

An exemplary method for detecting and/or quantifying a biological marker protein or nucleic acid in a tumour tissue sample involves obtaining a tumour tissue sample. Said method includes further steps of contacting the biological sample with a compound or an agent capable of detecting the polypeptide or nucleic acid (e.g., mRNA or cDNA). The detection methods of the invention can thus be used to detect mRNA, protein, or cDNA, for example, in a tumour tissue sample *in vitro.* For example, *in vitro* techniques for detection of mRNA include Northern hybridisation and in situ hybridisation. *In vitro* techniques for detection of a biological marker protein include enzyme linked immunosorbent assays (ELISAs), Western blots, immunoprecipitations, immunofluorescence, and RT-PCR.. A general principle of such detection and/or quantification assays involves preparing a sample or reaction mixture that may contain a biological marker, and a probe, under appropriate conditions and for a time sufficient to allow the marker and probe to interact and bind, thus forming a complex that can be removed and/or detected in the reaction mixture.

As used herein, the term "probe" refers to any molecule which is capable of selectively binding to a specifically intended target molecule, for example, a nucleotide transcript or protein encoded by or corresponding to a biological marker. Probes can be either synthesised by one skilled in the art, or derived from appropriate biological preparations. For purposes of detection of the target molecule, probes may be specifically designed to be labelled, as described herein. Examples of molecules that can be utilised as probes include, but are not limited to, RNA, DNA, proteins, antibodies, and organic molecules.

These detection and/or quantification assays of a biological marker can be conducted in a variety of ways.

For example, one method to conduct such an assay would involve anchoring the probe onto a solid phase support, also referred to as a substrate, and detecting target marker/probe complexes anchored on the solid phase at the end of the reaction. In one embodiment of such a method, a sample from a subject, which is to be assayed for quantification of the biological marker, can be anchored onto a carrier or solid phase support. In another embodiment, the reverse situation is possible, in which the probe can be anchored to a solid phase and a sample from a subject can be allowed to react as an unanchored component of the assay.

There are many established methods for anchoring assay components to a solid phase. These include, without limitation, marker or probe molecules which are immobilised through conjugation of biotin and streptavidin. Such biotinylated assay components can be prepared from biotin-NHS (N-hydroxysuccinimide) using techniques known in the art (e.g., biotinylation kit, Pierce Chemicals, Rockford, III.), and immobilised in the wells of streptavidin-coated 96 well plates (Pierce Chemical). In certain embodiments, the surfaces with immobilised assay components can be prepared in advance and stored.

Other suitable carriers or solid phase supports for such assays include any material capable of binding the class of molecule to which the marker or probe belongs. Well-known supports or carriers include, but are not limited to, glass, polystyrene, nylon, polypropylene, nylon, polyethylene, dextran, amylases, natural and modified celluloses, polyacrylamides, gabbros, and magnetite.

In order to conduct assays with the above mentioned approaches, the non-immobilised component is added to the solid phase upon which the second component is anchored. After the reaction is complete, uncomplexed components may be removed (e.g., by washing) under conditions such that any complexes formed will remain immobilised upon the solid phase. The detection of marker/probe complexes anchored to the solid phase can be accomplished in a number of methods outlined herein.

In a preferred embodiment, the probe, when it is the unanchored assay component, can be labelled for the purpose of detection and readout of the assay, either directly or indirectly, with detectable labels discussed herein and which are well-known to one skilled in the art.

It is also possible to directly detect marker/probe complex formation without further manipulation or labelling of either component (marker or probe), for example by utilising the technique of fluorescence energy transfer (see, for example, Lakowicz et al., U.S. Pat. No. 5,631,169; Stavrianopoulos, et al., U.S. Pat. No. 4,868,103). A fluorophore label on the first, 'donor' molecule is selected such that, upon excitation with incident light of appropriate wavelength, its emitted fluorescent energy will be absorbed by a fluorescent label on a second 'acceptor' molecule, which in turn is able to fluoresce due to the absorbed energy. Alternately, the 'donor' protein molecule may simply utilize the natural fluorescent energy of tryptophan residues. Labels are chosen that emit different wavelengths of light, such that the 'acceptor' molecule label may be differentiated from that of the 'donor'. Since the efficiency of energy transfer between the labels is related to the distance separating the molecules, spatial relationships between the molecules can be assessed. In a situation in which binding occurs between the molecules, the fluorescent emission of the 'acceptor' molecule label in the assay should be maximal. A FRET binding event can be conveniently measured through standard fluorometric detection means well known in the art (e.g., using a fluorimeter).

In another embodiment, determination of the ability of a probe to recognise a marker can be accomplished without labelling either assay component (probe or marker) by utilising a technology such as real-time Biomolecular Interaction Analysis (BIA) (see, e.g., Sjolander, S. and Urbaniczky, C., 1991, Anal. Chem. 63:2338-2345 and Szabo et al., 1995, Curr. Opin. Struct. Biol. 5:699-705). As used herein, "BIA" or "surface plasmon resonance" is a technology for studying biospecific interactions in real time, without labelling any of the interactants (e.g., BIAcore). Changes in the mass at the binding surface (indicative of a binding event) result in alterations of the refractive index of light near the surface (the optical phenomenon of surface plasmon resonance (SPR)), resulting in a detectable signal which can be used as an indication of real-time reactions between biological molecules.

Alternatively, in another embodiment, analogous diagnostic and prognostic assays can be conducted with marker and probe as solutes in a liquid phase. In such an assay, the complexed marker and probe are separated from uncomplexed components by any of a number of standard techniques, including but not limited to: differential centrifugation, chromatography, electrophoresis and immunoprecipitation. In differential centrifugation, marker/probe complexes may be separated from uncomplexed assay components through a series of centrifugal steps, due to the different sedimentation equilibria of complexes based on their different sizes and densities (see, for example, Rivas, G., and Minton, A. P., 1993, Trends Biochem Sci. 18(8):284-7). Standard chromatographic techniques may also be utilized to separate complexed molecules from uncomplexed ones. For example, gel filtration chromatography separates molecules based on size, and through the utilization of an appropriate gel filtration resin in a column format, for example, the relatively larger complex may be separated from the relatively smaller uncomplexed components. Similarly, the relatively different charge properties of the marker/probe complex as compared to the uncomplexed components may be exploited to differentiate the complex from uncomplexed components, for example through the utilization of ion-exchange chromatography resins. Such resins and chromatographic techniques are well known to one skilled in the art (see, e.g., Heegaard, N. H., 1998, J. Mol. Recognit. Winter 11(1-6):141-8; Hage, D. S., and Tweed, S. A. J Chromatogr B Biomed Sci Appl 1997 Oct. 10;699(1-2):499-525). Gel electrophoresis may also be employed to separate complexed assay components from unbound components (see, e.g., Ausubel et al., ed., Current Protocols in Molecular Biology, John Wiley & Sons, New York, 1987-1999). In this technique, protein or nucleic acid complexes are separated based on size or charge, for example. In order to maintain the binding interaction during the electrophoretic process, non-denaturing gel matrix materials and conditions in the absence of reducing agent are typically preferred. SELDI-TOF technique may also be employed on matrix or beads coupled with active surface, or not, or antibody coated surface, or beads.

Appropriate conditions to the particular assay and components thereof will be well known to one skilled in the art.

In a particular embodiment, the level of marker mRNA can be determined both by in situ and by in vitro formats in a biological sample using methods known in the art. The term "biological sample" is intended to include tissues, cells, biological fluids and isolates thereof, isolated from a subject, as well as tissues, cells and fluids present within a subject, provided that the said biological sample is susceptible to contain (i) cells originating from the breast cancer or (ii) nucleic acids or proteins that are produced by the breast cancer cells from the patient. Many expression detection methods use isolated RNA. For *in vitro* methods, any RNA isolation technique that does not select against the isolation of mRNA can be utilised for the purification of RNA from breast cancer (see, e.g., Ausubel et al., ed., Current Protocols in Molecular Biology, John Wiley & Sons, New York 1987-1999). Additionally, large numbers of tissue samples can readily be processed using techniques well known to those of skill in the art, such as, for example, the single-step RNA isolation process of Chomczynski (1989, U.S. Pat. No. 4,843,155).

The isolated mRNA can be used in hybridisation or amplification assays that include, but are not limited to, Southern or Northern analyses, polymerase chain reaction analyses and probe arrays. One preferred diagnostic method for the detection of mRNA levels involves contacting the isolated mRNA with a nucleic acid molecule (probe) that can hybridise to the mRNA encoded by the gene being detected. The nucleic acid probe can be, for example, a full-length cDNA, or a portion thereof, such as an oligonucleotide of at least 7, 15, 30, 50, 100, 250 or 500 nucleotides in length and sufficient to specifically hybridise under stringent conditions to a mRNA encoding a marker of the present invention. Other suitable probes for use in the pronostic assays of the invention are described herein. Hybridisation of an mRNA with the probe indicates that the marker in question is being expressed.

In most preferred embodiments of step b) of the *in vitro* prediction method according to the invention, detection and/or quantification of the metastasis-specific biological markers is performed by using suitable DNA microarrays. In such a marker detection/quantification format, the mRNA is immobilised on a solid surface and contacted with a probe, for example by running the isolated mRNA on an agarose gel and transferring the mRNA from the gel to a membrane, such as nitrocellulose. In an alternative format, the probe(s) are immobilized on a solid surface and the mRNA is contacted with the probe(s), for example, in an Affymetrix gene chip array. A skilled artisan can readily adapt known mRNA detection methods for use in detecting the level of mRNA encoded by the markers of the present invention. Specific hybridization technology which may be practiced to generate the expression profiles employed in the subject methods includes the technology described in U.S. Pat. Nos. 5,143,854; 5,288,644; 5,324,633; 5,432,049; 5,470,710; 5,492,806; 5,503,980; 5,510,270; 5,525,464; 5,547,839; 5,580,732; 5,661,028; 5,800,992; the disclosures of which are herein incorporated by reference; as well as WO 95/21265; WO 96/31622; WO 97/10365; WO 97/27317; EP 373 203; and EP 785 280. In these methods, an array of "probe" nucleic acids that includes a probe for each of the phenotype determinative genes whose expression is being assayed is contacted with target nucleic acids as described above. Contact is carried out under hybridization conditions, e.g., stringent hybridization conditions as described above, and unbound nucleic acid is then removed. The resultant pattern of hybridized nucleic acid provides information regarding expression for each of the genes that have been probed, where the expression information is in terms of whether or not the gene is expressed and, typically, at what level, where the expression data, i.e., expression profile, may be both qualitative and quantitative.

An alternative method for determining the level of mRNA marker in a sample involves the process of nucleic acid amplification, e.g., by rtPCR (the experimental embodiment set forth in Mullis, 1987, U.S. Pat. No. 4,683,202), ligase chain reaction (Barany, 1991, Proc. Natl. Acad. Sci. USA, 88:189-193), self sustained sequence replication (Guatelli et al., 1990, Proc. Natl. Acad. Sci. USA 87:1874-1878), transcriptional amplification system (Kwoh et al., 1989, Proc. Natl. Acad. Sci. USA 86:1173-1177), Q-Beta Replicase (Lizardi et al., 1988, Bio/Technology 6:1197), rolling circle replication (Lizardi et al., U.S. Pat. No. 5,854,033) or any other nucleic acid amplification method, followed by the detection of the amplified molecules using techniques well known to those of skill in the art. These detection schemes are especially useful for the detection of nucleic acid molecules if such molecules are present in very low numbers. As used herein, amplification primers are defined as being a pair of nucleic acid molecules that can anneal to 5' or 3' regions of a gene (plus and minus strands, respectively, or vice-versa) and contain a short region in between. In general, amplification primers are from about 10 to 30 nucleotides in length and flank a region from about 50 to 200 nucleotides in length. Under appropriate conditions and with appropriate reagents, such primers permit the amplification of a nucleic acid molecule comprising the nucleotide sequence flanked by the primers.

For *in situ* methods, mRNA does not need to be isolated from the breast cancer prior to detection. In such methods, a cell or tissue sample is prepared/processed using known histological methods. The sample is then immobilised on a support, typically a glass slide, and then contacted with a probe that can hybridise to mRNA that encodes the marker.

As an alternative to making determinations based on the absolute expression level of the marker, determinations may be based on the normalised expression level of the marker. Expression levels are normalised by correcting the absolute expression level of a marker by comparing its expression to the expression of a gene that is not a marker, e.g., a housekeeping gene that is constitutively expressed. Suitable genes for normalisation include housekeeping genes such as the actin gene, ribosomal 18S gene, GAPDH gene and TATA-box-binding protein (TBP). This normalisation allows the comparison of the expression level of one or more tissue-specific biological marker of interest in one sample.

Alternatively, the expression level can be provided as a relative expression level. To determine a relative expression level of a marker, the level of expression of the marker is determined for 10 or more samples of normal versus cancer cell isolates, preferably 50 or more samples, prior to the determination of the expression level for the sample in question. The median expression level of each of the genes assayed in the larger number of samples is determined and this is used as a baseline expression level for the marker. The expression level of the marker determined for the test sample (absolute level of expression) is then divided by the mean expression value obtained for that marker. This provides a relative expression level.

As already mentioned previously in the present specification, the detection/quantificattion reagent for detecting and/or quantifying a biological marker protein when performing the metastasis prediction method of the invention may consist of an antibody that specifically bind to such a biological marker protein or a fragment thereof, preferably an antibody with a detectable label. Antibodies can be polyclonal, or more preferably, monoclonal. An intact antibody, or a fragment or derivative thereof (e.g., Fab or F(ab').sub.2) can be used. The term "labelled", with regard to the probe or antibody, is intended to encompass direct labelling of the probe or antibody by coupling (i.e., physically linking) a detectable substance to the probe or antibody, as well as indirect labelling of the probe or antibody by reactivity with another reagent that is directly labelled. Examples of indirect labelling include detection of a primary antibody using a fluorescently labelled secondary antibody and end-labelling of a DNA probe with biotin such that it can be detected with fluorescently labelled streptavidin.

One skilled in the art will know many other suitable carriers for binding antibody or antigen, and will be able to adapt such support for use with the present invention. For example, protein isolated from breast cancer can be run on a polyacrylamide gel electrophoresis and immobilised onto a solid phase support such as nitrocellulose. The support can then be washed with suitable buffers followed by treatment with the detectably labeled antibody. The solid phase support can then be washed with the buffer a second time to remove unbound antibody. The amount of bound label on the solid support can then be detected by conventional means.

The most preferred methods for quantifying a biological marker for the purpose of carrying out the metastasis prediction method of the invention are described hereunder.

### Quantifying biological markers by cDNA microarrays

According to this embodiment, a microarray may be constructed based on the metastasis-specific markers that are disclosed throughout the present specification. Metastasis-specific detection reagents including these markers may be placed on the microarray. These cancer metastasis-specific detection reagents may be different than those used in PCR methods. However, they should be designed and used in conditions such that only nucleic acids having the metastasis-specific marker may hybridize and give a positive result.

Most existing microarrays, such as those provided by Affymetrix (California), may be used with the present invention.

One of skill in the art will appreciate that an enormous number of array designs are suitable. The high density array will typically include a number of probes that specifically hybridize to the sequences of interest. See WO 99/32660 for methods of producing probes for a given gene or genes. In a preferred embodiment, the array will include one or more control probes.

### Nucleic acid probes immobilized on the microarray devices

High density array chips include « test probes » that specifically hybridize with mRNAs or cDNAs consisting of the products of expression of the meatstasis-specific biological markers that are described herein.

Test probes may be oligonucleotides that range from about 5 to about 500 or about 5 to about 200 nucleotides, more preferably from about 10 to about 100 nucleotides and most preferably from about 15 to about 70 nucleotides in length. In other particularly preferred embodiments, the probes are about 20 or 25 nucleotides in length. In another preferred embodiment, test probes are double or single strand DNA sequences. DNA sequences may be isolated or cloned from natural sources or amplified from natural sources using natural nucleic acid as templates. These probes have sequences complementary to particular subsequences of the metastasis-specific markers whose expression they are designed to detect.

In addition to test probes that bind the target nucleic acid(s) of interest, the high density array can contain a number of control probes. The control probes fall into three categories referred to herein as normalization controls; expression level controls; and mismatch controls. Normalization controls are oligonucleotide or other nucleic acid probes that are complementary to labeled reference oligonucleotides or other nucleic acid sequences that are added to the nucleic acid sample. The signals obtained from the normalization controls after hybridization provide a control for variations in hybridization conditions, label intensity, "reading" efficiency and other factors that may cause the signal of a perfect hybridization to vary between arrays. In a preferred embodiment, signals (e.g. fluorescence intensity) read from all other probes in the array are divided by the signal (, fluorescence intensity) from the control probes thereby normalizing the measurements. Virtually any probe may serve as a normalization control. However, it is recognized that hybridization efficiency varies with base composition and probe length. Preferred normalization probes are selected to reflect the average length of the other probes present in the array; however, they can be selected to cover a range of lengths. The normalization control(s) can also be selected to reflect the (average) base composition of the other probes in the array, however in a preferred embodiment, only one or a few probes are used and they are selected such that they hybridize well (i.e., no secondary structure) and do not match any target-specific probes. Expression level controls are probes that hybridize specifically with constitutively expressed genes in the biological sample. Virtually any constitutively expressed gene provides a suitable target for expression level controls. Typical expression level control probes have sequences complementary to subsequences of constitutively expressed "housekeeping genes" including the .beta.-actin gene, the transferrin receptor gene, and the GAPDH gene. Mismatch controls may also be provided for the probes to the target genes, for expression level controls or for normalization controls. Mismatch controls are oligonucleotide probes or other nucleic acid probes identical to their corresponding test or control probes except for the presence of one or more mismatched bases. A mismatched base is a base selected so that it is not complementary to the corresponding base in the target sequence to which the probe would otherwise specifically hybridize. One or more mismatches are selected such that under appropriate hybridization conditions (e.g., stringent conditions) the test or control probe would be expected to hybridize with its target sequence, but the mismatch probe would not hybridize (or would hybridize to a significantly lesser extent). Preferred mismatch probes contain a central mismatch. Thus, for example, where a probe is a twenty-mer, a corresponding mismatch probe may have the identical sequence except for a single base mismatch (e.g., substituting a G, a C or a T for an A) at any of positions 6 through 14 (the central mismatch). Mismatch probes thus provide a control for non-specific binding or cross hybridization to a nucleic acid in the sample other than the target to which the probe is directed. Mismatch probes also indicate whether hybridization is specific or not.

### Solid Supports for DNA microarrays

Solid supports containing oligonucleotide probes for differentially expressed genes can be any solid or semisolid support material known to those skilled in the art. Suitable examples include, but are not limited to, membranes, filters, tissue culture dishes, polyvinyl chloride dishes, beads, test strips, silicon or glass based chips and the like. Suitable glass wafers and hybridization methods are widely available. Any solid surface to which oligonucleotides can be bound, either directly or indirectly, either covalently or non-covalently, can be used. In some embodiments, it may be desirable to attach some oligonucleotides covalently and others non-covalently to the same solid support. A preferred solid support is a high density array or DNA chip. These contain a particular oligonucleotide probe in a predetermined location on the array. Each predetermined location may contain more than one molecule of the probe, but each molecule within the predetermined location has an identical sequence. Such predetermined locations are termed features. There may be, for example, from 2, 10, 100, 1000 to 10,000, 100,000 or 400,000 of such features on a single solid support. The solid support or the area within which the probes are attached may be on the order of a square centimeter. Methods of forming high density arrays of oligonucleotides with a minimal number of synthetic steps are known. The oligonucleotide analogue array can be synthesized on a solid substrate by a variety of methods, including, but not limited to, light-directed chemical coupling, and mechanically directed coupling (see U.S. Pat. No. 5,143,854 to Pirrung et al.; U.S. Pat. No. 5,800,992 to Fodor et al.; U.S. Pat. No. 5,837,832 to Chee et al.

In brief, the light-directed combinatorial synthesis of oligonucleotide arrays on a glass surface proceeds using automated phosphoramidite chemistry and chip masking techniques. In one specific implementation, a glass surface is derivatized with a silane reagent containing a functional group, e.g., a hydroxyl or amine group blocked by a photolabile protecting group. Photolysis through a photolithographic mask is used selectively to expose functional groups which are then ready to react with incoming 5' photoprotected nucleoside phosphoramidites. The phosphoramidites react only with those sites which are illuminated (and thus exposed by removal of the photolabile blocking group). Thus, the phosphoramidites only add to those areas selectively exposed from the preceding step. These steps are repeated until the desired array of sequences has been synthesized on the solid surface. Combinatorial synthesis of different oligonucleotide analogues at different locations on the array is determined by the pattern of illumination during synthesis and the order of addition of coupling reagents.

In addition to the foregoing, methods which can be used to generate an array of oligonucleotides on a single substrate are described in WO 93/09668 to Fodor et al. High density nucleic acid arrays can also be fabricated by depositing premade or natural nucleic acids in predetermined positions. Synthesized or natural nucleic acids are deposited on specific locations of a substrate by light directed targeting and oligonucleotide directed targeting. Another embodiment uses a dispenser that moves from region to region to deposit nucleic acids in specific spots.

Oligonucleotide probe arrays for expression monitoring can be made and used according to any techniques known in the art (see for example, Lockhart et al., Nat. Biotechnol. 14, 1675-1680 (1996); McGall et al., Proc. Nat. Acad. Sci. USA 93, 13555-13460 (1996). Such probe arrays may contain at least two or more oligonucleotides that are complementary to or hybridize to two or more of the genes described herein. Such arrays may also contain oligonucleotides that are complementary to or hybridize to at least 3, 4, 5, 6, 7, 8, 9, 10, 20, 30, 50, 70 or more of the genes described therein.

### Gene Signature Differential analysis.

Gene Signature Differential analysis is a method designed to detect nucleic acids, like mRNAs or cDNAs differentially expressed in different samples.

### Hybridization

Nucleic acid hybridization simply involves contacting a probe and target nucleic acid under conditions where the probe and its complementary target can form stable hybrid duplexes through complementary base pairing (see WO 99/32660 to Lockhart). The nucleic acids that do not form hybrid duplexes are then washed away leaving the hybridized nucleic acids to be detected, typically through detection of an attached detectable label. It is generally recognized that nucleic acids are denatured by increasing the temperature or decreasing the salt concentration of the buffer containing the nucleic acids. Under low stringency conditions (e.g., low temperature and/or high salt) hybrid duplexes (e.g., DNA-DNA, RNA-RNA or RNA-DNA) will form even where the annealed sequences are not perfectly complementary. Thus, specificity of hybridization is reduced at lower stringency. Conversely, at higher stringency (e.g., higher temperature or lower salt) successful hybridization requires fewer mismatches. One of skill in the art will appreciate that hybridization conditions may be selected to provide any degree of stringency. In a preferred embodiment, hybridization is performed at low stringency, in this case in 6.times.SSPE-T at 37.degree. C. (0.005% Triton x-100) to ensure hybridization and then subsequent washes are performed at higher stringency (e.g., 1.times.SSPE-T at 37.degree. C.) to eliminate mismatched hybrid duplexes. Successive washes may be performed at increasingly higher stringency (e.g. down to as low as 0.25.times.SSPE-T at 37.degree. C. to 50.degree. C.) until a desired level of hybridization specificity is obtained. Stringency can also be increased by addition of agents such as formamide. Hybridization specificity may be evaluated by comparison of hybridization to the test probes with hybridization to the various controls that can be present (e.g., expression level controls, normalization controls, mismatch controls, etc.).

In general, there is a tradeoff between hybridization specificity (stringency) and signal intensity. Thus, in a preferred embodiment, the wash is performed at the highest stringency that produces consistent results and that provides a signal intensity greater than approximately 10% of the background intensity. The hybridized array may be washed at successively higher stringency solutions and read between each wash. Analysis of the data sets thus produced will reveal a wash stringency above which the hybridization pattern is not appreciably altered and which provides adequate signal for the particular oligonucleotide probes of interest.

### Signal Detection

The hybridized nucleic acids are typically detected by detecting one or more labels attached to the sample nucleic acids. The labels may be incorporated by any of a number of means well known to those of skill in the art (see WO 99/32660 to Lockhart). Any suitable methods can be used to detect one or more of the markers described herein. For example, gas phase ion spectrometry can be used. This technique includes, e.g., laser desorption/ionization mass spectrometry. In some embodiments, the sample can be prepared prior to gas phase ion spectrometry, e.g., pre-fractionation, two-dimensional gel chromatography, high performance liquid chromatography, etc. to assist detection of markers.

### Quantifying biological markers by immunohistochemistry on conventional tissue slides (paraffin-embedded or frozen specimens)

In certain embodiments, a biological marker, or a set of biological markers, may be quantified with any one of the immunohistochemistry methods known in the art.

Typically, for further analysis, one thin section of the tumour, is firstly incubated with labelled antibodies directed against one biological marker of interest. After washing, the labelled antibodies that are bound to said biological marker of interest are revealed by the appropriate technique, depending of the kind of label, e.g. radioactive, fluorescent or enzyme label. Multiple labelling can be performed simultaneously.

### Quantifying biological markers by nucleic acid amplification

In certain embodiments, a biological marker, or a set of biological markers, may be quantified with any one of the nucleic acid amplification methods known in the art.

The polymerase chain reaction (PCR) is a highly sensitive-and powerful method for such biological markers quantification

For performing any one of the nucleic acid amplification method that is appropriate for quantifying a biological marker when performing the metastasis prediction method of the invention, a pair of primers that specifically hybridise with the target mRNA or with the target cDNA is required.

A pair of primers that specifically hybridise with the target nucleic acid biological marker of interest may be designed by any one of the numerous methods known in the art.

In certain embodiments, for each of the biological markers of the invention, at least one pair of specific primers, as well as the corresponding detection nucleic acid probe, is already referenced and entirely described in the public "Quantitative PCR primer database", notably at the following Internet address : http://Ipgws.nci.nih.gov/cgi-bin/PrimerViewer.

In other embodiments, a specific pair of primers may be designed using the method disclosed in the US Patent n° US 6,892,141 to Nakae et al., the entire disclosure of which is herein incorporated by reference.

Many specific adaptations of the PCR technique are known in the art for both qualitative and quantitative detection purposes. In particular, methods are known to utilise fluorescent dyes for detecting and quantifying amplified PCR products. In situ amplification and detection, also known as homogenous PCR, have also been previously described. See e.g. Higuchi et al., (Kinetics PCR Analysis: Real-time Monitoring of DNA Amplification Reactions, Bio/Technology, Vol 11, pp 1026-1030 (1993)), Ishiguro et al., (Homogeneous quantitative Assay of Hepatitis C Virus RNA by Polymerase Chain Reaction in the Presence of a Fluorescent Intercalater, Anal. Biochemistry 229, pp 20-213 (1995)), and Wittwer et al., (Continuous Fluorescence Monitoring of Rapid cycle DNA Amplification, Biotechniques, vol.22, pp 130-138 (1997.))

A number of other methods have also been developed to quantify nucleic acids (Southern, E. M., J. Mol. Biol., 98:503-517, 1975; Sharp, P. A., et al., Methods Enzymol. 65:750-768, 1980; Thomas, P. S., Proc. Nat. Acad. Sci., 77:5201-5205, 1980). More recently, PCR and RT-PCR methods have been developed which are capable of measuring the amount of a nucleic acid in a sample. One approach, for example, measures PCR product quantity in the log phase of the reaction before the formation of reaction products plateaus (Kellogg, D. E., et al., Anal. Biochem. 189:202-208 (1990); and Pang, S., et al., Nature 343:85-89 (1990)). A gene sequence contained in all samples at relatively constant quantity is typically utilised for sample amplification efficiency normalisation. This approach, however, suffers from several drawbacks. The method requires that each sample have equal input amounts of the nucleic acid and that the amplification efficiency between samples be identical until the time of analysis. Furthermore, it is difficult using the conventional methods of PCR quantitation such as gel electrophoresis or plate capture hybridisation to determine that all samples are in fact analysed during the log phase of the reaction as required by the method.

Another method called quantitative competitive (QC)-PCR, as the name implies, relies on the inclusion of an internal control competitor in each reaction (Becker-Andre, M., Meth. Mol. Cell Biol. 2:189-201 (1991); Piatak, M. J., et al., BioTechniques 14:70-81 (1993); and Piatak, M. J., et al., Science 259:1749-1754 (1993)). The efficiency of each reaction is normalised to the internal competitor. A known amount of internal competitor is typically added to each sample. The unknown target PCR product is compared with the known competitor PCR product to obtain relative quantitation. A difficulty with this general approach lies in developing an internal control that amplifies with the same efficiency of the target molecule.

For instance, the nucleic acid amplification method that is used may consist of Real-Time quantitative PCR analysis.

Real-time or quantitative PCR (QPCR) allows quantification of starting amounts of DNA, cDNA, or RNA templates. QPCR is based on the detection of a fluorescent reporter molecule that increases as PCR product accumulates with each cycle of amplification. Fluorescent reporter molecules include dyes that bind double-stranded DNA (i.e. SYBR Green I) or sequence-specific probes (i.e. Molecular Beacons or TaqMan® Probes).

Preferred nucleic acid amplification methods are quantitative PCR amplification methods, including multiplex quantitative PCR method such as the technique disclosed in the published US patent Application n ° US 2005/0089862, to Therianos et al., the entire disclosure of which is herein incorporated by reference.

Illustratively, for quantifying biological markers of the invention, tumor tissue samples are snap-frozen shortly after biopsy collection. Then, total RNA from a "tumour tissue sample" is isolated and quantified. Then, each sample of the extracted and quantified RNA is reverse-transcribed and the resulting cDNA is amplified by PCR, using a pair of specific primers for each biological marker that is quantified. Control pair of primers are simultaneously used as controls, such as pair of primers that specifically hybridise with TBP cDNA, 18S cDNA and GADPH cDNA, or any other well known "housekeeping" gene.

Illustrative embodiments of detection and quantification of the tissue-specific biological markers using nucleic acid amplification methods are disclosed in the examples herein.

The sequences of specific pairs of nucleic acid primers for detecting and/or quantifying various tissue-specific biological markers specified herein are expressly listed.

Further pairs of primers for detecting and/or quantifying the same tissue-specific biological markers are obtainable by the one skilled in the art, starting from the nucleic acid sequences of the said markers that are publicly available in the sequence databases.

Also, pairs of primers for the other tissue-specific markers disclosed herein are obtainable by the one skilled in the art, starting from the nucleic acid sequences of the said markers that are publicly available in the sequence databases.

Illustratively, specific pairs of primers that may be used for detecting and/or amplifying various tissue-specific biological markers are found below :
(i) bone metastasis-specific markers : KTCD1 (SEQ ID N°1 and 2), BAIAP2L1 (SEQ ID N°3 and 4), PERP (SEQ ID N°5 and 6), CFD (SEQ ID N °7 and 8), CD4 (SEQ ID N°9 and 10), COL6A2 (SEQ ID N°11 and 12), FLI1 (SEQ ID N°13 and 14), PSTPIP1 (SEQ ID N°15 and 16), MGF10 (SEQ ID N°17 and 18), PTPN22 (SEQ ID N°19 and 20), FAM78A (SEQ ID N°21 and 22), NXF1 (SEQ ID N°23 and 24), MFNG (SEQ ID N°25 and 26) and CLEC4A (SEQ ID N°27 and 28);
(ii) lung metastasis-specific markers : KIND1 (SEQ ID N°29 and 30), ELAC1 (SEQ ID N°31 and 32), ANP32E (SEQ ID N°33 and 34), PAQR3 (SEQ ID N °35 and 36), ITGB8 (SEQ ID N°37 and 38), c1orf210 (SEQ ID N°39 and 40), SIKE (SEQ ID N°41 and 42), UGT8 (SEQ ID N°43 and 44), CALB2 (SEQ ID N°45 and 46), CHODL (SEQ ID N°47 and 48), c21orf91 (SEQ ID N°49 and 50), TFCP2L1 (SEQ ID N°51 and 52), ODF2L (SEQ ID N°53 and 54), HORMAD1 (SEQ ID N°55 and 56), PLEKHG4 (SEQ ID N°57 and 58) and DSC2 (SEQ ID N°59 and 60);
(iii) liver metastasis-specific makers : GATA2 (SEQ ID N°61 and 62), SELT (SEQ ID N°63 and 64), WHSC1L1 (SEQ ID N°65 and 66), MYRIP (SEQ ID N°67 and 68), ZMAT1 (SEQ ID N°69 and 70), IL20RA (SEQ ID N°71 and 72), ZNF44 (SEQ ID N°73 and 74), LETM2 (SEQ ID N°75 and 76), AGXT2L1 (SEQ ID N°77 and 78), c5orf30 (SEQ ID N°79 and 80) and TBX3 (SEQ ID N°81 and 82).
(iv) brain metastasis-specific markers : PPWD1 (SEQ ID N°83 and 84), PDGFRA (SEQ ID N°85 and 86), MLL5 (SEQ ID N°87 and 88), RPS23 (SEQ ID N°89 and 90), ANTXR1 (SEQ ID N°91 and 92), ARRDC3 (SEQ ID N°93 and 94), METTL7A (SEQ ID N°95 and 96), NPHP3 (SEQ ID N°97 and 98), RSHL1 (SEQ ID N°99 and 100), CSPP1 (SEQ ID N°101 and 102), HOXB13 (SEQ ID N°103 and 104), TRA16 (SEQ ID N°105 and 106), DDX27 (SEQ ID N°107 and 108), PKP2 (SEQ ID N°109 and 110) and INHA (SEQ ID N°111 and 112).

A pair of primers that may be used for quantifying the TATA-box Binding Protein (TBP) as a control consists of the nucleic acids of SEQ ID N°113-114 disclosed herein.

The primers having the nucleic acid sequences SEQ ID N°1 to SEQ ID N°114 are also described in Table 3 herein.

### Kits for predicting metastasis in breast cancer

The invention also relates to a kit for the *in vitro* prediction of the occurrence of metastasis in one or more tissue or organ in a patient (e.g. in a tumour tissue sample previously collected from a breast cancer patient). The kit comprises a plurality of reagents, each of which is capable of binding specifically with a biological marker nucleic acid or protein.

Suitable reagents for binding with a marker protein include antibodies, antibody derivatives, antibody fragments, and the like.

Suitable reagents for binding with a marker nucleic acid (e.g. a genomic DNA, an mRNA, a spliced mRNA, a cDNA, or the like) include complementary nucleic acids. For example, the nucleic acid reagents may include oligonucleotides (labelled or non-labelled) fixed to a substrate, labelled oligonucleotides not bound with a substrate, pairs of PCR primers, molecular beacon probes, and the like.

Another object of the present invention consists of a kit for the *in vitro* prediction of the occurrence of metastasis in a patient, which kit comprises means for detecting and/or quantifying one or more biological markers that are indicative of an occurrence of metastasis in one or more tissue or organ, wherein the said one or more biological markers are selected from the group consisting of :
(i) one or more biological markers indicative of an occurrence of metastasis in the bone tissue that are selected from the group consisting of : CLEC4A, MFNG, NXF1, FAM78A, KCTD1, BAIAP2L1, PTPN22, MEGF10, PERP, PSTPIP1, FLI1, COL6A2, CD4, CFD, ZFHX1B, CD33, LST1, MMRN2, SH2D3C, RAMP3, FAM26B, ILK, TM6SF1, C10orf54, CLEC3B, IL2RG, HOM-TES-103, ZNF23, STK35,, TNFAIP8L2, RAMP2, ENG, ACRBP, TBC1D10C, C11orf48, EBNA1BP2, HSPE1, GAS6, HCK, SLC2A5, RASA3, ZNF57, WASPIP, KCNK1, GPSM3, ADCY4, , AIF1, , NCKAP1, AMICA1, POP7, GMFG, PPM1M, CDGAP, , GIMAP1, ARHGAP9, APOB48R, OCIAD2, FLRT2, P2RY8, RIPK4, PECAM1, , URP2, BTK, , , APBB1IP, CD37, STARD8, GIMAP6, E2F6, WAS, , HLA-DQB1, HVCN1, LOC56902, ORC5L, MEF2C, PLCL2, PLAC9, RAC2, SYNE1, DPEP2, MYEF2, HSPD1, PSCD4, , , NXT1, , LOC340061, ITGB3, AP1S2, SNRPG, , , CSF1, BIN2, , ANKRD47, , , LIMS2, , DARC, PTPN7, MSH6, GGTA1, LRRC33, GDPD5, CALCOCO1, FAM110C, BCL6B, LOC641700, ARHGDIB, DAAM2, TNFRSF14, TPSAB1, CSF2RA, RCSD1, FLJ21438, LOC133874, GSN, SLIT3, FYN, NCF4, PTPRC, EVI2B, SCRIB, C11orf31, LOC440731, TFAM, ARPC5L, PARVG, GRN, LMO2, CRSP8, EHBP1L1, HEATR2, , NAALADL1, , INPP5D, LTB, STRBP, FAM65A, ADARB1, TMEM140, DENND1C, PRPF19, , CASP10, , SLC37A2, RHOJ, MPHOSPH10, PPIH, RASSF1, , HCST, C16orf54, EPB41 L4B, LRMP, LAPTM5, PRDM2, CYGB, LYCAT, ACP5, CMKLR1, UBE1L, MAN2C1, TNFSF12, C7orf24, Cxorf15, CUL1, SMAD7, ITGB7, APOL3, PGRMC1, PPA1, YES1, FBLN1, MRC2, PTK9L, LRP1, IGFBP5, , WDR3, GTPBP4, , SPI1, SELPLG, OSCAR, LYL1, POLR2H, YWHAQ, ISG20L2, LGI14, KIF5B, , NGRN, TYROBP, C5orf4, COX7A2, S100A4, MATK, TMEM33, DOK3, LOC150166, CIRBP, NIN, C10orf72, FMNL1, FAT3, CHKB/CPT1B, SNRPA1, GIMAP4, C20orf18, LTBP2, GAB3, NQO1, MARCH2, MYO1F, CDS1, SRD5A1, C20orf160, SLAMF7, ACTL6A, ABP1, RAE1, MAF, SEMA3G, P2RY13, ZDHHC7, ERG, , FHL1, CLEC10A, INTS5, MYO15B, CTSW, PILRA, HN1, SCARA5, PRAM1, EBP, , SIGLEC9, LGP1, DGUOK, GGCX, , RABL5, ZBTB16, TPSAB1, NOP5/NOP58, CCND2, CD200, EPPK1, , DKFZp586CO721, CCT6A, RIPK3, ARHGAP25, GNA12, USP4, FAHD2A, LOC399959, LOC133308, HKDC1, CD93, GTF3C4, ITGB2, ELOVL6, TGFB1I1, ASCC3L1, FES, , KCNMB1, AACS, ATP6VOD2, , TMEM97, NUDT15, ATP6V1 B2, CCDC86, FLJ10154, SCARF2, , , , PRELP, ACHE, , GIMAP8, PDE4DIP, NKG7, , C20orf59, RHOG, TRPV2, TCP1, TNRC8, TNS1, IBSP, MMP9, NRIP2, OLFML2B, OMD, WIF1, ZEB2, ARL8, COL12A1, EBF and EBF3 ;
(ii) one or more biological markers indicative of an occurrence of metastasis in the lung tissue that are selected from the group consisting of : DSC2, HORMAD1, PLEKHG4, ODF2L, C21orf91, TFCP2L1, TTMA, CHODL, CALB2, UGT8, LOC146795, C1orf210, SIKE, ITGB8, PAQR3, ANP32E, C20orf42/FERMT1, ELAC1, GYLTL1B, SPSB1, CHRM3, PTEN, PIGL, CHRM3, CDH3 ;
(iii) one or more biological markers indicative of an occurrence of metastasis in the liver tissue that are selected from the group consisting of : TBX3, , SYT17, LOC90355, AGXT2L1, LETM2, LOC145820, ZNF44, IL20RA, ZMAT1, MYRIP, WHSC1L1, SELT, , GATA2, ARPC2, CAB39L, SLCI6A3, DHFRL1, PRRT3, CYP3A5, RPS6KA5, KIAA1505, ATP5S, ZFYVE16, KIAA0701, PEBP1, DDHD2, WWP1, CCNL1, ROBO2, FAM111B, THRAP2, CRSP9, KARCA1, SLC16A3, , ARID4A, TCEAL1, SCAMP1, KIAAO701, EIF5A, DDX46, PEX7, , BCL2L11, YBX1, UBE21, REXO2, AXUD1, C10orf2, ZNF548, FBXL16, LOC439911, LOC283874, ZNF587, FLJ20366, KIAAO888, BAG4, CALU, KIAA1961, USP30, NR4A2, FOXA1, FBXO15, WNK4, CDIPT, NUDT16L1, SMAD5, STXBP4, TTC6, LOC113386, , TSPYL1, CIP29, C8orf1, , SYDE2, SLC12A8, SLC25A18, C7, STAU2, TSC22D2, GADD45G, PHF3, TNRC6C, TCEAL3, , RRN3, C5orf24, AHCTF1, LOC92497 ; and
(iv) one or more biological markers indicative of an occurrence of metastasis in the brain tissue that are selected from the group consisting of : LOC644215, BAT1, GPR75, PPWD1, INHA, PDGFRA, MLL5, RPS23, ANTXR1, ARRDC3, PTK2, SQSTM1, METTL7A, NPHP3, PKP2, DDX31, FAM119A, LLGL2, DDX27, TRA16, HOXB13, GNAS, CSPP1, COL8A1, RSHL1, DCBLD2, UBXD8, SURF2, ZNF655, RAC3, AP4M1, HEG1, PCBP2, , SLC30A7, ATAD3A/ATAD3B, CH13L1, MUC6, HMG20B, BCL7A, GGN, ARHGEF3, PALLD, TOP1, PCTK1, C20orf4, ZBTB1, MSH6, SETD5, POSTN, MOCS3, GABPA, ZSWIM1, ZNHIT2, LOC653352, ELL, ARPC4, ZNF277, VAV2, HNRPH3, LHX1, FAM83A, DIP2B, RBM10, PMPCA, TYSND1, RAB4B, DLC1, KIAA2018, TES, TFDP2, C3orf10, ZBTB38, PSMD7, RECK, JMJD1C, FLJ20273, CENPB, PLAC2, C6orf111, ATP10D, RNF146, XRRA1, NPAS2, APBA2BP, WDR34, SLK, SBF2, , SON, MORC3, C3orf63, WDR54, STX7, ZNF512, KLHL9, LOC284889, ETV4, RMND5B, ARMCX1, SLC29A4, TRIB3, LRRC23, DDIT3, THUMPD3, MICAL-L2, PA2G4, TSEN54, LAS1L, MEA1, S100PBP, TRAF2, EMILIN3, KIAA1712, PRPF6, CHD9, JMJD1B, ANKS1A, CAPN5, EPC2, WBSCR27, CYB561, LLGL1, EDD1.

The present invention also encompasses various alternative embodiments of the said prediction kit, wherein the said prediction kit comprises combination of marker detection and/or marker quantification means, for detecting and/or quantifying various combinations of the markers described in the present specification.

In certain embodiments of the invention, the said prediction kit consists of a kit for the *in vitro* prediction of the occurrence of lung metastasis in a patient who is affected with a breast cancer, wherein the said kit comprises means for detecting and/or quantifying one or more biological markers that are indicative of an occurrence of metastasis in the lung tissue, wherein the said one or more biological markers are selected from the group consisting of DSC2, TFCP2L1, UGT8, ITGB8, ANP32E and FERMT1.

In certain embodiments of the said prediction kit, it comprises means for detecting and/or quantifying 2, 3, 4, 5 or all of the following lung-specific markers : DSC2, TFCP2L1, UGT8, ITGB8, ANP32E and FERMT1.

In further embodiments, the said prediction kit comprises means for detecting and/or quantifying the whole combination of the six following markers : DSC2, TFCP2L1, UGT8, ITGB8, ANP32E and FERMT1.

In yet further embodiments, the said prediction kit comprises means for detecting and/or quantifying exclusively the whole combination of the six following markers : DSC2, TFCP2L1, UGT8, ITGB8, ANP32E and FERMT1, together with means for detecting and/or quantifying one or more reference markers, e.g. markers corresponding to ubiquitously expressed genes or proteins like actin..

Most preferably, a prediction kit according to the invention consists of a DNA microarray comprising probes hybridizing to the nucleic acid expression products (mRNAs or cDNAs) of the metastasis-specific biological markers described herein.

Another object of the present invention consists of a kit for monitoring the anti-metastasis effectiveness of a therapeutic treatment of a patient affected with a breast cancer with a pharmaceutical agent, which kit comprises means for quantifying one or more biological markers that are indicative of an occurrence of metastasis in one or more tissue or organ, wherein the said one or more biological markers are selected from the group consisting of :
(i) one or more biological markers indicative of an occurrence of metastasis in the bone tissue that are selected from the group consisting of : CLEC4A, MFNG, NXF1, FAM78A, KCTD1, BAIAP2L1, PTPN22, MEGF10, PERP, PSTPIP1, FLI1, COL6A2, CD4, CFD, ZFHX1B, CD33, LST1, MMRN2, SH2D3C, RAMP3, FAM26B, ILK, TM6SF1, C10orf54, CLEC3B, IL2RG, HOM-TES-103, ZNF23, STK35, , TNFAIP8L2, RAMP2, ENG, ACRBP, TBC1D10C, C11orf48, EBNA1BP2, HSPE1, GAS6, HCK, SLC2A5, RASA3, ZNF57, WASPIP, KCNK1, GPSM3, ADCY4, , AIF1, , NCKAP1, AMICA1, POP7, GMFG, PPM1M, CDGAP, , GIMAP1, ARHGAP9, APOB48R, OCIAD2, FLRT2, P2RY8, RIPK4, PECAM1, , URP2, BTK, , , APBB1IP, CD37, STARD8, GIMAP6, E2F6, WAS, , HLA-DQB1, HVCN1, LOC56902, ORC5L, MEF2C, PLCL2, PLAC9, RAC2, SYNE1, DPEP2, MYEF2, HSPD1, PSCD4, , , NXT1, , LOC340061, ITGB3, AP1S2, SNRPG, , , CSF1, BIN2, , ANKRD47, , , LIMS2, , DARC, PTPN7, MSH6, GGTA1, LRRC33, GDPD5, CALCOCO1, FAM110C, BCL6B, LOC641700, ARHGDIB, DAAM2, TNFRSF14, TPSAB1, CSF2RA, RCSD1, FLJ21438, LOC133874, GSN, SLIT3, FYN, NCF4, PTPRC, EVI2B, SCRIB, C11orf31, LOC440731, TFAM, ARPC5L, PARVG, GRN, LMO2, CRSP8, EHBP1L1, HEATR2, , NAALADL1, , INPP5D, LTB, STRBP, FAM65A, ADARB1, TMEM140, DENND1C, PRPF19, , CASP10, , SLC37A2, RHOJ, MPHOSPH10, PPIH, RASSF1, , HCST, C16orf54, EPB41 L4B, LRMP, LAPTM5, PRDM2, CYGB, LYCAT, ACP5, CMKLR1, UBE1L, MAN2C1, TNFSF12, C7orf24, Cxorf15, CUL1, SMAD7, ITGB7, APOL3, PGRMC1, PPA1, YES1, FBLN1, MRC2, PTK9L, LRP1, IGFBP5, , WDR3, GTPBP4, , SPI1, SELPLG, OSCAR, LYL1, POLR2H, YWHAQ, ISG20L2, LGI14, KIF5B, , NGRN, TYROBP, C5orf4, COX7A2, S100A4, MATK, TMEM33, DOK3, LOC150166, CIRBP, NIN, C10orf72, FMNL1, FAT3, CHKB/CPT1B, SNRPA1, GIMAP4, C20orf18, LTBP2, GAB3, NQO1, MARCH2, MY01 F, CDS1, SRD5A1, C20orf160, SLAMF7, ACTL6A, ABP1, RAE1, MAF, SEMA3G, P2RY13, ZDHHC7, ERG, , FHL1, CLEC10A, INTS5, MYO15B, CTSW, PILRA, HN1, SCARA5, PRAM1, EBP, , SIGLEC9, LGP1, DGUOK, GGCX, , RABL5, ZBTB16, TPSAB1, NOP5/NOP58, CCND2, CD200, EPPK1, , DKFZp586CO721, CCT6A, RIPK3, ARHGAP25, GNA12, USP4, FAHD2A, LOC399959, LOC133308, HKDC1, CD93, GTF3C4, ITGB2, ELOVL6, TGFB1I1, ASCC3L1, FES, , KCNMB1, AACS, ATP6VOD2, , TMEM97, NUDT15, ATP6V1B2, CCDC86, FLJ10154, SCARF2, , , , PRELP, ACHE, , GIMAP8, PDE4DIP, NKG7, , C20orf59, RHOG, TRPV2, TCP1, TNRC8, TNS1, IBSP, MMP9, NRIP2, OLFML2B, OMD, WIF1, ZEB2, ARL8, COL12A1, EBF and EBF3 ;
(ii) one or more biological markers indicative of an occurrence of metastasis in the lung tissue that are selected from the group consisting of : DSC2, HORMAD1, PLEKHG4, ODF2L, C21orf91, TFCP2L1, TTMA, CHODL, CALB2, UGT8, LOC146795, C1orf210, SIKE, ITGB8, PAQR3, ANP32E, C20orf42, ELAC1, GYLTL1B, SPSB1, CHRM3, PTEN, PIGL, CHRM3, CDH3 ;
(iii) one or more biological markers indicative of an occurrence of metastasis in the liver tissue that are selected from the group consisting of : TBX3, , SYT17, LOC90355, AGXT2L1, LETM2, LOC145820, ZNF44, IL20RA, ZMAT1, MYRIP, WHSC1L1, SELT, , GATA2, ARPC2, CAB39L, SLC16A3, DHFRL1, PRRT3, CYP3A5, RPS6KA5, KIAA1505, ATP5S, ZFYVE16, KIAA0701, PEBP1, DDHD2, WWP1, CCNL1, ROBO2, FAM111B, THRAP2, CRSP9, KARCA1, SLC16A3, , ARID4A, TCEAL1, SCAMP1, KIAAO701, EIF5A, DDX46, PEX7, , BCL2L11, YBX1, UBE21, REXO2, AXUD1, C10orf2, ZNF548, FBXL16, LOC439911, LOC283874, ZNF587, FLJ20366, KIAAO888, BAG4, CALU, KIAA1961, USP30, NR4A2, FOXA1, FBXO15, WNK4, CDIPT, NUDT16L1, SMAD5, STXBP4, TTC6, LOC113386, , TSPYL1, CIP29, C8orf1, , SYDE2, SLC12A8, SLC25A18, C7, STAU2, TSC22D2, GADD45G, PHF3, TNRC6C, TCEAL3, , RRN3, C5orf24, AHCTF1, LOC92497 ; and
(iv) one or more biological markers indicative of an occurrence of metastasis in the brain tissue that are selected from the group consisting of : LOC644215, BAT1, GPR75, PPWD1, INHA, PDGFRA, MLL5, RPS23, ANTXR1, ARRDC3, PTK2, SQSTM1, METTL7A, NPHP3, PKP2, DDX31, FAM119A, LLGL2, DDX27, TRA16, HOXB13, GNAS, CSPP1, COL8A1, RSHL1, DCBLD2, UBXD8, SURF2, ZNF655, RAC3, AP4M1, HEG1, PCBP2, , SLC30A7, ATAD3A/ATAD3B, CH13L1, MUC6, HMG20B, BCL7A, GGN, ARHGEF3, PALLD, TOP1, PCTK1, C20orf4, ZBTB1, MSH6, SETD5, POSTN, MOCS3, GABPA, ZSWIM1, ZNHIT2, LOC653352, ELL, ARPC4, ZNF277, VAV2, HNRPH3, LHX1, FAM83A, DIP2B, RBM10, PMPCA, TYSND1, RAB4B, DLC1, KIAA2018, TES, TFDP2, C3orf10, ZBTB38, PSMD7, RECK, JMJD1C, FLJ20273, CENPB, PLAC2, C6orf111, ATP10D, RNF146, XRRA1, NPAS2, APBA2BP, WDR34, SLK, SBF2, , SON, MORC3, C3orf63, WDR54, STX7, ZNF512, KLHL9, LOC284889, ETV4, RMND5B, ARMCX1, SLC29A4, TRIB3, LRRC23, DDIT3, THUMPD3, MICAL-L2, PA2G4, TSEN54, LAS1L, MEA1, S100PBP, TRAF2, EMILIN3, KIAA1712, PRPF6, CHD9, JMJD1B, ANKS1A, CAPN5, EPC2, WBSCR27, CYB561, LLGL1, EDD1.

The present invention also encompasses various alternative embodiments of the said monitoring kit, wherein the said monitoring kit comprises combination of marker detection and/or marker quantification means, for detecting and/or quantifying various combinations of the markers described in the present specification.

In certain embodiments of the invention, the said monitoring kit consists of a kit for the *in vitro* prediction of the occurrence of lung metastasis in a patient who is affected with a breast cancer, wherein the said kit comprises means for detecting and/or quantifying one or more biological markers that are indicative of an occurrence of metastasis in the lung tissue, wherein the said one or more biological markers are selected from the group consisting of DSC2, TFCP2L1, UGT8, ITGB8, ANP32E and FERMT1.

In certain embodiments of the said monitoring kit, it comprises means for detecting and/or quantifying 2, 3, 4, 5 or all of the following lung-specific markers : DSC2, TFCP2L1, UGT8, ITGB8, ANP32E and FERMT1.

In further embodiments, the said monitoring kit comprises means for detecting and/or quantifying the whole combination of the six following markers : DSC2, TFCP2L1, UGT8, ITGB8, ANP32E and FERMT1.

In yet further embodiments, the said monitoring kit comprises means for detecting and/or quantifying exclusively the whole combination of the six following markers : DSC2, TFCP2L1, UGT8, ITGB8, ANP32E and FERMT1, together with means for detecting and/or quantifying one or more reference markers, e.g. markers corresponding to ubiquitously expressed genes or proteins like actin..

The prediction kit and the monitoring kit of the invention may optionally comprise additional components useful for performing the methods of the invention. By way of example, the kits may comprise fluids (e.g. SSC buffer) suitable for annealing complementary nucleic acids or for binding an antibody with a protein with which it specifically binds, one or more sample compartments, an instructional material which describes performance of the prediction method or of the monitoring method of the invention, and the like.

### Kits comprising antibodies

In certain embodiments, a kit according to the invention comprises one or a combination or a set of antibodies, each kind of antibodies being directed specifically against one biological marker of the invention.

In one embodiment, said kit comprises a combination or a set of antibodies comprising at least two kind of antibodies, each kind of antibodies being selected from the group consisting of antibodies directed against one of the biological markers disclosed herein.

An antibody kit according to the invention may comprise 2 to 20 kinds of antibodies, each kind of antibodies being directed specifically against one biological marker of the invention. For instance, an antibody kit according to the invention may comprise 2, 3, 4, 5, 6, 7, 8, 9, 10, 11, 12, 13, 14, 15, 16, 17, 18, 19 or 20 kinds of antibodies, each kind of antibodies being directed specifically against one biological marker as defined herein.

Various antibodies directed against biological markers according to the invention encompass antibodies directed against biological markers selected from the group consisting of those metastasis-specific markers that are listed in Table 4. Specific embodiments of these antibodies are listed in Table 4 herein.

Specific embodiments of a prediction kit according to the invention which contains detection/quantification means consisting of antibodies include the fluorescent microsphere-bound antibody suspension array technology, e.g. the kit technology that is marketed under the trademark Bio-Plex® by the Bio-Rad Company.

Concurrent with the increasing interest in magnetic microspheres for biological assays is the development of assays conducted on fluorescent microspheres. The use of fluorescent labels or fluorescent material coupled to a surface of the microspheres or incorporated into the microspheres allows preparation of numerous sets of microspheres that are distinguishable based on different dye emission spectra and/or signal intensity. In a biological assay, the fluorescence and light scattering of these microspheres can be measured by a flow cytometer or an imaging system, and the measurement results can be used to determine the size and fluorescence of the microspheres as well as the fluorescence associated with the assay system being studied (e.g., a fluorescently labeled antibody in a "capture sandwich" assay), as described in U.S. Pat. No. 5,948,627 to Lee et al., which is incorporated by reference as if fully set forth herein. By varying the concentrations of multiple dyes incorporated in the microspheres, hundreds, or even thousands, of distinguishable microsphere sets can be produced. In an assay, each microsphere set can be associated with a different target thereby allowing numerous tests to be conducted for a single sample in a single container as described in U.S. Pat. No. 5,981,180 to Chandler et al., which is incorporated by reference as if fully set forth herein.

Fluorescently distinguishable microspheres may be improved by rendering these microspheres magnetically responsive. Examples of methods for forming fluorescent magnetic microspheres are described in U.S. Pat. No. 5,283,079 to Wang et al., which is incorporated by reference as if fully set forth herein. The methods described by Wang et al. include coating a fluorescent core microsphere with magnetite and additional polymer or mixing a core microsphere with magnetite, dye, and polymerizable monomers and initiating polymerization to produce a coated microsphere. These methods are relatively simple approaches to the synthesis of fluorescent magnetic microspheres.

Additionally, for creating a large numbers of precisely dyed microspheres used in relatively large multiplex assays, those as described in U.S. Pat. No. 5,981,180 to Chandler et al. may be used.

Fluorescent magnetic microspheres are also described in U.S. Pat. No. 6,268,222 to Chandler et al., which is incorporated by reference as if fully set forth herein. In this method, nanospheres are coupled to a polymeric core microsphere, and the fluorescent and magnetic materials are associated with either the core microsphere or the nanospheres. This method produces microspheres with desirable characteristics.

A further embodiment of fluorescent magnetic microspheres is a magnetically responsive microspheres that can be dyed using established techniques, such as those described in U.S. Pat. No. 6,514,295 to Chandler et al. In general, this method uses solvents that swell the microsphere thereby allowing migration of the fluorescent material into the microsphere. These dyeing solvents include one or more organic solvents.

Also, magnetic microspheres are described in U.S. Pat. No. 5,091,206 to Wang et al., U.S. Pat. No. 5,648,124 to Sutor, and U.S. Pat. No. 6,013,531 to Wang et al., which are incorporated by reference as if fully set forth herein.

In certain other embodiments, a kit according to the invention comprises one or a combination or a set of pair of ligands or specfic soluble molecules binding with one or more of the biological marker(s), of the invention.

### Kits comprising nucleic acid primers

In certain other embodiments, a kit according to the invention comprises one or a combination or a set of pair of primers, each kind of pair of primers hybridising specifically with one biological marker of the invention.

In one embodiment, said kit comprises a combination or a set of pair of primers comprising at least two kind of pair of primers, each kind of pair of primers being selected from the group consisting of pair of primers hybridising with one of the biological markers disclosed in the present specification, including the pairs of primers of SEQ ID N°1-112 that are detailed earlier in the specification.

A primer kit according to the invention may comprise 2 to 20 kinds of pair or primers, each kind of pair of primers hybridising specifically with one biological marker of the invention. For instance, a primer kit according to the invention may comprise 2, 3, 4, 5, 6, 7, 8, 9, 10, 11, 12, 13, 14, 15, 16, 17, 18, 19 or 20 kinds of pairs of primers, each kind of pair of primers hybridising specifically against one biological marker as defined herein.

Notably, at least one pair of specific primers, as well as the corresponding detection nucleic acid probe, that hybridise specifically with one biological marker of interest, is already referenced and entirely described in the public "Quantitative PCR primer database", notably at the following Internet address : http://Ipgws.nci.nih.gov/cgi-bin/PrimerViewer.

Illustratively, a prediction kit or a monitoring kit according to the invention may comprise one or more specific pairs of primers for detecting and/or amplifying various tissue-specific biological markers, among the primers specified below :
(i) bone metastasis-specific markers : KTCD1 (SEQ ID N°1 and 2), BAIAP2L1 (SEQ ID N°3 and 4), PERP (SEQ ID N°5 and 6), CFD (SEQ ID N°7 and 8), CD4 (SEQ ID N°9 and 10), COL6A2 (SEQ ID N°11 and 12), FLI1 (SEQ ID N°13 and 14), PSTPIP1 (SEQ ID N°15 and 16), MGF10 (SEQ ID N°17 and 18), PTPN22 (SEQ ID N°19 and 20), FAM78A (SEQ ID N°21 and 22), NXF1 (SEQ ID N°23 and 24), MFNG (SEQ ID N°25 and 26) and CLEC4A (SEQ ID N°27 and 28);
(ii) lung metastasis-specific markers : KIND1 (SEQ ID N°29 and 30), ELAC1 (SEQ ID N°31 and 32), ANP32E (SEQ ID N°33 and 34), PAQR3 (SEQ ID N°35 and 36), ITGB8 (SEQ ID N°37 and 38), c1orf210 (SEQ ID N°39 and 40), SIKE (SEQ ID N°41 and 42), UGT8 (SEQ ID N°43 and 44), CALB2 (SEQ ID N°45 and 46), CHODL (SEQ ID N°47 and 48), c21orf91(SEQ ID N°49 and 50), TFCP2L1 (SEQ ID N°51 and 52), ODF2L (SEQ ID N°53 and 54), HORMAD1 (SEQ ID N°55 and 56), PLEKHG4 (SEQ ID N°57 and 58) and DSC2 (SEQ ID N°59 and 60);
(iii) liver metastasis-specific makers : GATA2 (SEQ ID N°61 and 62), SELT (SEQ ID N°63 and 64), WHSC1L1 (SEQ ID N°65 and 66), MYRIP (SEQ ID N°67 and 68), ZMAT1 (SEQ ID N°69 and 70), IL20RA (SEQ ID N°71 and 72), ZNF44 (SEQ ID N°73 and 74), LETM2 (SEQ ID N°75 and 76), AGXT2L1 (SEQ ID N°77 and 78), c5orf3O (SEQ ID N°79 and 80) and TBX3 (SEQ ID N°81 and 82).
(iv) brain metastasis-specific markers : PPWD1 (SEQ ID N°83 and 84), PDGFRA (SEQ ID N°85 and 86), MLL5 (SEQ ID N°87 and 88), RPS23 (SEQ ID N°89 and 90), ANTXR1 (SEQ ID N°91 and 92), ARRDC3 (SEQ ID N°93 and 94), METTL7A (SEQ ID N°95 and 96), NPHP3 (SEQ ID N°97 and 98), RSHL1 (SEQ ID N°99 and 100), CSPP1 (SEQ ID N°101 and 102), HOXB13 (SEQ ID N°103 and 104), TRA16 (SEQ ID N°105 and 106), DDX27 (SEQ ID N°107 and 108), PKP2 (SEQ ID N°109 and 110) and INHA (SEQ ID N°111 and 112).

These kits may also comprise one or more pairs of primers for detecting and/or quantifying a control marker. Illustratively, these kits may comprise a pair of primer for detecting and/or quantifying the TATA-box Binding Protein (TBP), such as the nucleic acids of SEQ ID N°113-114 disclosed herein.

### Monitoring anti-cancer treatments

Monitoring the influence of agents (e.g., drug compounds) on the level of expression of one or more tissue-specific biological markers of the invention can be applied for monitoring the metastatic potency of the treated breast cancer of the patient with time. For example, the effectiveness of an agent to affect biological marker expression can be monitored during treatments of subjects receiving anti-cancer, and especially anti-metastasis, treatments.

In a preferred embodiment, the present invention provides a method for monitoring the effectiveness of treatment of a subject with an agent (e.g., an agonist, antagonist, peptidomimetic, protein, peptide, nucleic acid, small molecule, or other drug candidate) comprising the steps of (i) obtaining a pre-administration sample from a subject prior to administration of the agent; (ii) detecting the level of expression of one or more selected biological markers of the invention in the pre-administration sample; (iii) obtaining one or more post-administration samples from the subject; (iv) detecting the level of expression of the biological marker(s) in the post-administration samples; (v) comparing the level of expression of the biological marker(s) in the pre-administration sample with the level of expression of the marker(s) in the post-administration sample or samples; and (vi) altering the administration of the agent to the subject accordingly. For example, decreased expression of the biological marker gene(s) during the course of treatment may indicate ineffective dosage and the desirability of increasing the dosage. Conversely, increased expression of the biological marker gene(s) may indicate efficacious treatment and no need to change dosage.

Because repeated collection of biological samples from the breast cancer-bearing patient are needed for performing the monitoring method described above, then preferred biological samples consist of blood samples susceptible to contain (i) cells originating from the patient's breast cancer tissue, or (ii) metastasis-specific marker expression products synthesized by cells originating from the patients breast cancer tissue, including nucleic acids and proteins. As used herein, the said "breast cancer patient's tissue includes the primary tumor tissue as well as a organ-specific or tissue-specific metastasis tissue.

As already mentioned previously in the present specification, performing the metastasis prediction method of the invention may indicate, with more precision than the prior art methods, those patients at high-risk of tumor recurrence who may benefit from adjuvant therapy, including immunotherapy.

For example, if, at the end of the metastasis prediction method of the invention, a good prognosis of no metastasis is determined, then the subsequent anti-cancer treatment will not comprise any adjuvant chemotherapy.

However, if, at the end of the metastasis prediction method of the invention, a bad prognosis with is determined, then the patient is administered with the appropriate composition of adjuvant chemotherapy.

Accordingly, the present invention also relates to a method for adapting a cancer treatment in a breast cancer patient, wherein said method comprises the steps of :
a) performing, on at least one tumor tissue sample collected from said patient, the metastasis prediction method that is disclosed herein;
b) adapting the cancer treatment of the said cancer patient by administering to the said patient an adjuvant chemotherapy or an anti-metastasis therapy if a bad cancer prognosis with metastasis in one or more tissue or organ, including bone, lung, liver and brain, is determined at the end of step a).

Another object of the invention consists of a kit for monitoring the effectiveness of treatment (adjuvant or neo-adjuvant) of a subject with an agent, which kit comprises means for quantifying at least one biological marker of the invention that is indicative of the probability of occurrence of metastasis in a breast cancer patient.

### Methods for selecting biological markers indicative of metastasis

This invention also pertains to methods for selecting one or more biological markers that are indicative of the probability of occurrence of metastasis in one or more tissue or organ in a breast cancer-bearing patient.

The said tissue-specific marker selection method according to the invention preferably comprises the steps of :
a) providing means for detecting and/or quantifying one or more biological markers in a tissue sample;
b) providing a plurality of collections of metastasis tissue samples originating from breast cancer patients wherein each of the said collection consists of a plurality of tissue samples originating from metastasis originating from a unique tissue type of metastatic breast cancer patients;
c) detecting and/or quantifying each of the one or more biological markers, separately in every tissue sample contained in each collection of tissue samples;
d) selecting, in the group of biological markers that are detected and/or quantified at step c), those markers that are expressed exclusively in only one collection of tissue samples that is comprised in the plurality of collections of tissue samples provided at step b), whereby a set of markers is selected for each collection of tissue samples, the said set of markers comprising markers, the expression of which is indicative of the probability of occurrence of metastasis in a specific tissue of a breast cancer patient.

For the purpose of performing the marker selection method above exclusively, the term "biological marker" is used in its conventionally acknowledged meaning by the one skilled in the art, i.e. herein a product of expression of any human gene, including nucleic acids and proteins.

Illustrative embodiments of the selection method above are fully described in the examples herein.

For performing step a) of the selection method above, the marker detection and/or quantification means encompass means for detecting or quantifying marker proteins, such as antibodies, or marker gene-specific nucleic acids, such as oligonucleotide primers or probes. Illustratively, DNA or antibodies microarrays may be used at step a) of the selection method above.

Means for specifically detecting and/or quantifying any one of the known biological marker, e.g. any known protein or any gene-specific nucleic acid, may be provided at step a) of the selection method.

Each collection of tissue samples that is provided at step b) of the selection method above comprises a number of metastasis tissue samples originating from a unique metastatic tissue (e.g. bone, lung, liver, brain and skin) that are collected from the same number of breast cancer-bearing individuals. Preferably, each collection of metastasis tissue samples comprises samples originating from at least 10 distinct breast cancer individuals, and most preferably at least 20, 25 or 30 distinct breast cancer individuals. The statistical relevance of the tissue-specific markers that are finally selected at the end of the selection method generally increases with the number of distinct breast cancer individuals tested, and thus with the number of metastasis tissue samples comprised in each collection that is provided at step b).

At step c), detection and/or quantification of the biological markers on the tissue samples provided at step b), using the detection and/or quantification means provided at step a), may be performed according to any one of the detection and/or quantification methods that are described elsewhere in the present specification.

At step d), each marker detected at step c) in a first specific collection of tissue samples (e.g. bone metastasis tissue) is compared to the detection and/or quantification results found for the same marker in all of the other collections of tissue samples (e.g. lung, liver and brain). Then, only those markers that are differentially expressed (i.e. (i) expressed, (ii) not expressed, (iii) over-expressed and (iv) under-expressed) in the said first collection of tissue samples, as compared to the other collections of tissue samples are positively selected as markers indicative of a probability of occurrence of breast cancer metastasis in the said specific tissue (e.g. bone metastasis tissue). At step d), the selection of statistically relevant metastasis tissue-specific markers, by comparing marker expression in one breast cancer metastatic tissue with the expression of the said marker in the group of all other distinct breast metastatic breast cancer tissue(s), is termed a "One Versus All" ("OVA") pairwise comparison, as it is fully described in the examples herein.

The statistical relevance of each marker tested, at step d), may be performed by calculating the p value for the said marker, for example using a univariate t-test, as disclosed in the examples herein. Generally, a marker is selected at step d) of the selection method above, when its p value is lower than 10⁻³_{.}

The statistical relevance of the marker selection, at step d) of the method, may be further increased by performing a multivariate permutation test, to provide 90% confidence that a false marker selection rate is less than 10%, as disclosed in the examples herein.

In view of further improving the relevancy of the marker selection, at step d), further selection filters may be included, such as removing every marker consisting of a tissue-specific gene, i.e. every marker that is selectively differentially expressed in a first specific normal non-cancerous tissue (e.g. bone), as compared to the other normal non-cancerous tissues (e.g. lung, liver, brain, etc.).

For further increasing the statistical relevance of the markers initially selected, at step d) of the selection method above, those markers that were initially selected as described above may be submitted to a further cycle of selection, for example by assaying the initially selected markers on further collections of breast cancer metastasis tissue samples. This further cycle of selection may consist of, for example, performing a further expression analysis of the initially selected markers, for example by technique of quantitative RT-PCR expression analysis, as shown in the examples herein. DNA microarrays may also be used.

According to such a quantitative RT-PCR expression analysis, the quantification measure of expression of each initially selected marker is normalised against a control value, e.g. the quantification measure of expression of a control gene such as TBP. The results may be expressed as N-fold difference of each marker relative to the value in normal breast tissues. Statistical relevance of each initially selected marker is then confirmed, for example at confidence levels of more than 95% (P of less than 0.05) using the Mann-Whitney UTest (SEM), as described in the examples herein.

The present invention is further illustrated by, without in any way being limited to, the examples below.

### EXAMPLES

### EXAMPLES 1 and 2

### A. MATERIALS AND METHODS OF THE EXAMPLES 1 and 2

### A.1. Tissue samples

A total of 33 metastases from human breast cancer were used for microarray and quantitative RT-PCR analyses. These samples were snap-frozen in liquid nitrogen and stored at -196°C. Seven bone metastases (osteolytic type) and 6 normal bone samples were obtained from University of L'Aquila (L'Aquila, Italy), 4 brain metastases and 4 normal brain samples from IDIBELL (Barcelona, Spain). All other metastatic samples (8 lung, 8 liver and 6 skin metastases) and the 35 breast primary tumors samples were obtained from Centre René Huguenin (Saint-Cloud, France). All these primary tumor samples were resected from female patients who did not receive chemotherapy. Normal breast, lung, brain and liver RNA samples were purchased from Clontech, Biochain and Invitrogen. Normal tissue RNA pools were prepared with at least 5 samples each. Publicly available microarray data of a cohort of breast primary tumors with clinical follow up (with known site of relapse: bone, lung and other) were downloaded as on the NCBI website (GSE2603 record).

Additional breast cancer metastatic paraffin-embedded samples were obtained from the Department of Pathology (Pr. J. Boniver) at the University of Liège, Belgium (12 bone, 6 liver, 4 lung), from IDIBELL, Spain (6 brain), from the Anatomopathology Department of the Centre René Huguenin, France (2 lung and 1 liver) and used for the immunohistochemical analysis.

### A.2. RNA Extraction

Total RNA was isolated from human samples using TRIZOL® Reagent from Gibco, as described by the manufacturer. RNA was quantified by absorbance at 260 nm (Nanodrop) . Quality of the RNA samples was assessed by agarose gel electrophoresis and the integrity of 18S and 28S ribosomal RNA bands. Total RNA used for microarray analysis was further purified using RNeasy® spin columns (Qiagen) according to the manufacturers' protocols.

### A.3. Probe preparation and microarray hybridization

Sample labeling, hybridization, and staining were carried out according to the Eukaryotic Target Preparation protocol in the Affymetrix® Technical Manual (701021 Rev. 5) for GeneChip® Expression analysis (Affymetrix). In summary, 500 ng - 10 µg of purified total RNA was used to generate double-stranded cDNA using a GeneChip Expression 3'-Amplification One-Cycle cDNA synthesis Kit and a T7-oligo(dT) primer (Affymetrix). The resulting cDNA was purified using the GeneChip Sample Cleanup Module according to the manufacturers' protocol (Affymetrix). The purified cDNA was amplified using GeneChip Expression 3'-Amplification Reagents For IVT Labelling (Affymetrix) to produce 20-70 µg of biotin labeled cRNA (complement RNA). 13 µg of labeled cRNA (per array) was fragmented at 94°C for 35 min. The fragmented cRNA was hybridized to the Human Genome U133 Plus 2.0 Array for 16 h at 45°C.

The hybridized arrays were washed and stained using Streptavidin-Phycoerythrin (Molecular Probes) and amplified with biotinylated anti-streptavidin (Vector Laboratories) using a GeneChip Fluidics Station 450 (Affymetrix). The arrays were scanned in an Affymetrix GeneChip scanner 3000 at 570 nm. The 5'/3' GAPDH ratios averaged 3.5 and the average background, noise average, % Present calls and average signal of the Present calls were comparable for all the arrays utilized in this experiment. U133 Plus 2.0 expression arrays contain 56000 probe sets corresponding to 47000 transcripts.

### A.4. Microarray statistical analysis

Expression profiles were analyzed with BRB Array tools, version 3.3beta3 (Biometric Research Branch, Division of Cancer Treatment and Diagnosis Molecular Statistics and Bioinformatics Section, National Cancer Institute, Bethesda, Maryland, USA). Microarray data were collated as CEL files, calculation of Affymetrix probe set summaries and quantile normalization were done using RMA function of Bioconductor. We developed 4 raw site-specific metastatic signatures: bone, brain, liver and lung metastases. We used 6 bone, 4 brain, 6 liver, 5 lung and 2 skin metastases. We divided the multiclass problem into a series of 4 "One Versus All" (OVA) pairwise comparisons. For example, the raw bone metastasis signature was defined as follow: we selected probes differentially expressed between "Bone metastases" vs "All other metastases", i.e. "Bone metastases" vs "Lung + Liver + Brain + Skin metastases".

We identified genes that were differentially expressed among the two classes using a univariate t-test. Genes were considered statistically significant if their p value was less than 0.0001. A stringent significance threshold was used to limit the number of false positive findings.

We defined several selection filters. First, we identified potential host-tissue genes that could originate from contamination. Genes are considered as putative host-tissue genes if they are 1.5-fold more expressed in the corresponding normal tissue pool (e.g. bone) than in each of the other 3 normal tissue pools (i.e. liver, brain and lung). Second, upregulated genes with a geometric mean of intensities below 25 in the corresponding class (e.g., bone metastases) are removed. Downregulated genes with a geometric mean of intensities below 25 in the "other metastases" class (e.g. liver, brain, lung and skin metastases) are removed. Finally, probes were aligned against human genome (using Blast software from NCBI website). They were not conserved when they do not recognize transcripts. Metastatic signatures contain remaining most differentially expressed genes (sorted by absolute t-value) and were tested by quantitative RT-PCR.

### A.5. Quantitative RT-PCR expression analysis

cDNA of human samples used for real-time RT-PCR analysis were synthesized as previously described (Bieche et al., 2001). All of the PCR reactions were performed using a ABI Prism 7700 Sequence Detection System (Perkin-Elmer Applied Biosystems) and the SYBR Green PCR Core Reagents kit (Perkin-Elmer Applied Biosystems). 10 µl of diluted sample cDNA (produced from 2 g of total RNA) was added to 15 µl of the PCR master-mix. The thermal cycling conditions were composed of an initial denaturation step at 95°C for 10 min, and 50 cycles at 95°C for 15 s and 65°C for 1 min.

Human RNAs from primary tumors and relapses were analyzed for expression of 56 genes selected among the organ-specific metastasis associated genes by using quantitative real-time reverse transcription-PCR (RT-PCR) assay, as previously described (Bieche et al., 2001). Expression of these genes were measured in 29 metastatic samples from human breast cancer including 19 already tested by microarray and10 new samples (1 bone, 3 lung, 2 liver, and 4 skin relapses). TATA-box-binding protein (TBP) transcripts were used as an endogenous RNA control, and each sample was normalized on the basis of its TBP content. Results, expressed as N-fold differences for each X gene expression relative to the TBP gene (termed "N_{X}"), were obtained with the formula N_{X} = 2^{ΔCtsample}, where the ΔCt (Δ Cycle Threshold) value of the sample was determined by subtracting the average Ct value of the X gene from the average Ct value of the TBP gene. The N_{X} values of the samples were subsequently normalized such that the median of the N_{X} values of the four normal breast samples would have a value of 1. The nucleotide sequences of the primers used for real-time RT-PCR amplification are described in Table 3. Total RNA extraction, cDNA synthesis and the PCR reaction conditions have been previously described in detail (Bieche et al., 2001, Cancer Res, Vol 61 (4) : 1652-1658).

Differences between two populations were judged significant at confidence levels > 95%, (P <0.05), using Mann Whitney *U* Test (SEM)

### ImmunoHistoChemistry

Metastatic biopsies from breast cancer patients were fixed in 4% formaldehyde in 0.1 M phosphate buffer, pH 7.2, and embedded in paraffin. Bone specimens were decalcified either with a solution of ethylenediaminetetraacetic acid (EDTA) and hydrochloric acid (Decalcifier II, Labonord) or with a solution of formalin (20%) and nitric acid (5%). Sections were cut using a Reichert-Jung 1150/Autocut microtome. Slide-mounted tissue sections (4 µm thick) were deparaffinized in xylene and hydrated serially in 100%, 95%, and 80% ethanol. Endogenous peroxidases were quenched in 3% H₂O₂ in PBS for 1 hour, then slides were incubated with the indicated primary antibodies overnight at 4°C. Sections were washed three times in PBS, and antibody binding was revealed using the Ultra-Vision Detection System anti-Polyvalent HRP/DAB kit according to the manufacturer's instructions (Lab Vision). Finally, the slides were counterstained with Mayer's hematoxylin and washed in distilled water.

The anti-OMD and the anti-IBSP antibodies were kindly provided respectively by Dick Heinegard (Department of Experimental Medical Science, Lund University), Sweden and L.W. Fisher (National Institute of Dental Craniofacial Research, NIH, Bethesda, MD, USA). The anti-KIND1 and anti-TOP1 antibodies were purchased from Abcam, the anti-MMP-9 from Chemicon, and anti-DSC2 antibody from Progen respectively.

### Example 1 : Identification of differentially expressed genes

We compared the transcriptional profiles of human breast cancer metastases from the four main target organs of relapse: bone, lung, liver and brain. Therefore, using microarray gene expression data (Affymetrix U133 Plus 2.0 chips), we examined 23 metastatic samples obtained from surgery.

To identify genes that were specifically expressed in each of the 4 metastatic organs, we performed one-versus-all (OVA) class comparisons to distinguish two known subgroups. We used the following combinations: 6 bone metastases versus all 17 others (non-bone metastases), 5 lung metastases versus 18 others, 6 liver metastases versus 17 others and 4 brain metastases versus 19 others. Gene expressions between two defined groups were compared by use of a univariate t-test, in which the critical p-value was set at 10⁻⁴.

After applying filtering criteria as described in Materials and Methods, we identified 4 gene lists corresponding to the genes differentially expressed in bone metastases (325 probes representing 276 genes), lung metastases (28 probes representing 23 genes), liver metastases (114 probes representing 83 genes) and brain metastases (133 probes representing 123 genes) (Table 1). Among the 600 identified probes (representing 505 genes), 77 % were upregulated and 23% were downregulated. The bone and brain metastasis associated genes were downregulated in approximately 30% of the cases whereas the lung and liver metastasis associated genes were downregulated in about 5%. The 20 highest-ranking genes for each organ specific metastasis are illustrated in Table 2.

In order to validate at the protein level the observations made at the RNA level, we proceeded to immunohistochemistry analyses of several organ-specific metastatic gene products: DSC2 and KIND1 associated to lung metastasis and TOP1 corresponding to the brain metastatic process. As expected, proteins were highly expressed most exclusively by metastatic cells. KIND1 and TOP1 are highly and homogeneously expressed by metastatic cells, whereas DSC2 is principally highly expressed by metastatic cells present on the edge of the tumor, suggesting the presence of a cross talk with lung cells.

### 1.1. Main biological processes involving organ-specific metastasis associated genes

All the differentially expressed genes associated to the four sites of relapse were mapped to the Gene Ontology database. Among the 505 genes tested, 326 were annotated for the "Gene Ontology Biological Process Description". The descriptions were classified in major biological processes (i.e., apoptosis, cell adhesion, cell cycle, cell signaling, cytoskeleton organization, RNA processing, transcription regulation) to determine whether certain processes were highly represented. Thereby, we observed that the "cell adhesion" related genes were represented in 4 out of 15 (26,6%) annotations in the lung metastasis list. For the liver and brain metastasis associated genes the "transcription regulation" was the most represented description in 21/58 (32,2%) and 20/63 (31,7%) respectively. In the bone metastasis gene list, the genes related to "cell signaling" were the most frequently found (61/190, i.e. 32,1%). In addition, as previously described, the bone metastasis associated genes contains high proportion of genes involved in immune response (Smid et al., 2006, ). Highly represented annotations in each of the organ-specific metastatic gene list might point to particular biological processes, which are potentially linked to the site of relapse.

Among the "known" proteins identified, several have already been involved in cancer progression and/or metastatic phenotypes, such as the genes *PTEN, PERP, PDGFRA, TBX3* (Attardi et al., 2000 ; Petrocelli et al., 2001; Rowley et al., 2004 ; Oliveira et a., 2005 ; Jechlinger et al., 2006). Among the liver metastasis associated genes, *WHSC1L1* and *LETM2* are of particular interest since they have been described as coamplified in lung cancer. Their upregulation is probably due to a genetic event in cancer cells (Tonon et al, 2005). The brain metastasis associated gene *HOXB13* overexpression was recently associated with the clinical outcome in breast cancer patients (Ma et al., 2006). Moreover, among the bone metastasis associated genes, *MFNG* encodes Manic Fringe protein which modulates the Notch signaling pathway. This pathway has been reported to be involved in bone metastasis of prostate cancer (Zayzafoon et al, 2003), while TNFAIP8L2 and CSF-1 could be implicated in the enhancement of osteoclast formation and activity typically observed in breast cancer bone metastases.

### 1.2. Interaction with the microenvironment in the case of bone metastasis: osteomimicry

Among the genes that were found differentially expressed in bone metastases but that were removed by our filtering criteria to avoid host-tissue genes (due to contamination), we identified several genes known to be expressed by cells of the osteoblastic lineage such as bone sialoprotein (IBSP) and osteocalcin (BGLAP) noncollagenous bone matrix proteins.. We observed that metastatic breast cancer cells localized in bone consistently showed a strong immunoreactivity to IBSP, MMP9 and OMD in the majority of the samples analyzed. This observation is consistent with previous reports indicating that breast and prostate cancer cells metastasizing to bone express bone-related proteins (Koeneman 1999, Waltregny, 2000, Huang 2005). This phenomenon, named « osteomimicry », could explain the propensity of osteotropic cancer cells to metastasize to the skeleton (Koeneman 1999).

### 1.3. Organ-specific metastatic signature

The genes differentially expressed in the 4 target organs (Table 2) were then analyzed to define an organ-specific metastatic signature. Among these genes, 56 were tested by quantitative RT-PCR on a series of 29 breast cancer metastases consisting of 19 distant relapses already used for microarray analysis (4 bone metastases, 5 lung metastases, 6 liver metastases, 4 brain metastases) and 10 additional samples (1 bone metastasis, 3 lung metastases, 2 liver metastases and 4 skin metastases).

Thirty one genes (55%) passed the comparison criteria (OVA comparison, Mann Whitney U test, p value < 0.05) (supplementary table 3). The combination of these validated genes was evaluated as the organ-specific metastatic signature by hierarchical clustering. As shown by the dendrogram, the signature clearly separated the different metastatic classes. Remarkably, the bone metastases cluster seemed separated from the soft tissue metastases clusters. Our restricted "31-gene signature" showed similar patterns than the 80 highest-ranking genes (data not shown).

### 1.4. Predictive value of the organ-specific metastatic signature

The actual assumption is that primary tumors may already contain a gene expression profile that is strongly predictive of metastasis and in addition, tumor cells could also display a tissue-specific expression profile predicting the site of metastasis. To test this hypothesis, a series of publicly available microarray data relative to a cohort of 82 breast cancer patients with follow up (especially site of relapse) was analyzed (Minn et al, 2005a). In this series, 27 tumors relapsed, mainly in bone or lungs. Therefore, our identified lung- and bone- metastasis associated genes (only 6 and 10 genes respectively present on U133A chips) were used to cluster the 27 relapsing tumors (data not shown). We observed, in both cases, that many genes were expressed in the same way in relapsing tumors as the corresponding relapses. For example, genes upregulated or downregulated in bone metastases presented respectively higher or lower expression in breast tumor relapsing to bone than in those relapsing elsewhere.

Furthermore, primary tumors that highly express these bone metastasis genes seemed more susceptible to relapse to bone (p = 0.082, X² test). In the same way, primary tumors that highly express these lung metastasis genes were significantly more susceptible to relapse to lung (p = 0.002, X² test) (**figure 1**).

Finally, we evaluated the predictive value of our organ-specific signature on the large cohort of 82 primary tumors. The organ-specific metastatic signature (represented by only 25 probes present on U133A chips) allowed a classification of those tumors within 3 main clusters corresponding to tumors giving rise mainly to lung metastases, bone metastases and no metastasis

### (figure 2).

Kaplan Meier analyses were performed to assess the prognostic value of the signature with respect to organ-specific-metastasis-free survival. Patients with tumors expressing lung metastasis genes (cluster #1) had worse lung-metastasis-free survival (p = 0.00066) but not bone-metastasis-free survival (data not shown). Patients with tumors expressing bone metastasis genes (cluster #2) showed a tendency to have a worst bone-metastasis free survival (p = 0.12), and the patients included in the cluster #3 (tumors expressing neither bone nor lung metastasis genes) had a better metastasis free survival.

To confirm these results, we performed an analysis of the expression of the organ-specific metastatic signature by quantitative RT-PCR in an independent cohort of 35 patients. These patients were treated at Centre René Huguenin, did not receive chemotherapy and did all present relapses to bone or lung. Tumors that highly express lung metastasis genes (7 genes) significantly relapsed more to lung (p = 0.04, χ² test ). Patients who presented these latter tumors had a significantly worst lung metastasis free survival (p = 0.00043, 10 years after diagnosis of the primary tumor,. However, tumors that express bone metastasis genes did not relapsed more to bone (data not shown).

### EXAMPLES 3 TO 6

### A. MATERIALS AND METHODS OF EXAMPLES 3 to 6

### A.1. Patients and samples

The study was performed according to the local ethical regulations. We first studied the transcriptome of 23 metastases (5 lung, 6 liver, 4 brain, 2 skin and 6 osteolytic bone metastases) from breast cancer patients that undergone surgery (*n=22*). Then, additional 10 samples were used for RT-PCR validation (3 lung, 2 liver, 4 skin and 1 bone metastases) (*n=10*). All metastatic samples were obtained from University of L'Aquila (L'Aquila, Italy), IDIBELL (Barcelona, Spain) and Centre René Huguenin (CRH, Saint-Cloud, France). Normal-tissue RNA pools were prepared from 6 bone (L'Aquila), 4 brain (IDIBELL), and at least 5 breast, lung, and liver normal samples purchased from Clontech (Palo Alto, USA), Biochain (Hayward, USA) and Invitrogen (Frederick, USA).

A series of 72 primary breast tumors ("CRH cohort") was specifically selected from patients with node-negative breast cancer treated at the Centre René Huguenin, and who did not receive systemic neoadjuvant or adjuvant therapy (median follow-up of 132 months, range 22.6 to 294 months). During 10 years of follow-up, 38 patients developed distant metastases. Eleven of these patients developed lung metastases as the first site of distant relapse.

We also analyzed 3 independent breast tumor series; the "MSK" (n=82), "EMC" (n=344) and "NKI" (n=295) cohorts described in detail elsewhere (4, 6, 12, 14) for which microarray data can be freely downloaded from the NCBI website. Briefly, "EMC" and "NKI" cohorts consist of early stage breast cancers 100% and 50% lymph node-negative, respectively whereas "MSK" series is consisting of locally advanced tumors, 66% node-positives).

Finally, paraffin-embedded sections of lung metastases and paired breast tumors were obtained from Liège University (Belgium) and from Centre René Huguenin (France) to perform immunohistological analyses.

### A.2. Gene expression analysis

For microarray analysis, sample labeling, hybridization, and staining were carried out as previously described (Jackson et al., 2005). Human Genome U133 Plus 2.0 arrays were scanned in an Affymetrix GeneChip scanner 3000 at 570 nm. The 5'/3' GAPDH ratios averaged 3.5. The average background, noise average, % Present calls and average signal of Present calls were similar with all the arrays used in this experiment.

For quantitative RT-PCR analysis, we used cDNA synthesis and PCR conditions described in detail elsewhere (34). All PCR reactions were performed with an ABI Prism 7700 Sequence Detection System (Perkin-Elmer Applied Biosystems) and the SYBR Green PCR Core Reagents kit (Perkin-Elmer Applied Biosystems). TATA-box-binding protein (*TBP*) transcripts were used as an endogenous RNA control, and each sample was normalized on the basis of its *TBP* content (Bieche et al., 2001).

### A.3. Immunohistochemistry

Biopsy specimens of primary tumors and matching lung metastases from breast cancer patients were fixed and embedded in paraffin. Sections 4 µm thick were deparaffinized in xylene and hydrated in serial ethanol concentrations. Endogenous peroxidases were quenched in PBS containing 3% H₂O₂ for 1 hour, then the slides were incubated with the indicated primary antibodies overnight at 4°C. Sections were washed, and antibody binding was revealed with the Ultra-Vision Detection System Anti-Polyvalent HRP/DAB kit (Lab Vision). Finally, the slides were counterstained with Mayer's hematoxylin and washed in distilled water. The anti-FERMT1 and anti-DSC2 antibodies were purchased from Abcam (Cambridge, USA) and Progen (Queensland, Australia), respectively.

### A.4. Statistical analysis

Microarray expression profiles were analyzed with BRB Array tools, version 3.3beta3 developed by Richard Simon and Amy Peng Lam (http://linus.nci.nih.gov/BRB-ArrayTools.html). Expression data were collated as CEL files, and calculation of Affymetrix probe set summaries and quantile normalization were done using the RMA function of Bioconductor. Univariate t tests were used to identify genes differentially expressed between 5 lung metastases and 18 non lung metastases (6 bone, 4 brain, 6 liver, and 2 skin). Differences were considered statistically significant if the p value was less than 0.0001. This stringent threshold was used to limit the number of false-positives.

Several selection filters were used to refine the lung metastasis-related gene set. First, we filtered potential host-tissue genes characterized by a 1.5-fold higher expression level in normal lung tissue than in each of the other three normal tissue pools (bone, liver and brain). These genes were considered as potentially expressed by contaminating host tissue. Second, genes with geometric mean intensities below 25 in both lung and non lung metastases were removed. Finally, probes aligned against human genome (using Blast software from the NCBI website) not recognizing any transcripts were excluded.

Genes of interest were mapped between different platforms by using Unigene identifiers. The individual breast cancer series were then analyzed with the same probes when possible. To determine whether gene expression profiles were able to define low- and high-lung metastasis risk populations in the CRH cohort, the six-gene signature was used to create a risk index defined as a linear combination of the gene expression values. The distribution of risk index values was examined to determine the optimal cut point at the 75^{th} percentile to distinguish high and low risk in the CRH series. The risk index function and the high/low risk cutoff point were then applied to the MSK, EMC, NKI and combined cohorts.

Survival times were estimated by the Kaplan-Meier method, and the significance of differences was determined with the log-rank test. Multivariate analyses were performed using the Cox proportional hazards regression model.

### Example 3 : Identification of lung metastasis-associated genes

We first performed a microarray analysis to compare the gene transcript profiles of 5 lung metastases and 18 metastases from other target organs (bone, liver, brain and skin), all obtained from breast cancer patients undergoing surgery. A class comparison was conducted based on a univariate t test, with a stringent p value of 10⁻⁴, to identify genes differentially expressed by the two tissue categories.

After applying filtering criteria (see Patients and Methods), we identified 21 differentially expressed genes (19 known and 2 unknown genes) (**Table 5**), mostly overexpressed in lung metastases. Only one gene, the tumor suppressor gene *PTEN,* was downregulated.

To technically validate the differentially expressed genes, we examined the expression of the highest-ranking genes by quantitative RT-PCR using 19 samples analyzed by microarray (4 had no more RNA available) and 10 additional breast cancer metastases including 3 lung relapses. This validation step led to the selection of 7 genes showing a significant variation of expression, namely *DSC2, HORMAD1, TFCP2L1, UGT8, ITGB8, ANP32E* and *FERMT1* **(Table 5).**

Furthermore, to verify that these observed differentially expressed profiles were due to the metastatic samples but not to differential expression of adjacent host tissues, we evaluated the protein expression for 2 representative genes: *DSC2* and *FERMT1* (corresponding to the upper and lower p values,

Table 5). Strong immunoreactivity was detected for both proteins, almost exclusively in the tumor cells, and not in the surrounding pulmonary tissue.

The characterized genes were mapped into the Gene Ontology and Kyoto Encyclopedia of Genes and Genomes (KEGG) databases to investigate their functions and biological processes (**Table 5**). Interestingly, the lung metastasis-related genes showed an overrepresentation of membrane-bound molecules mainly involved in cell adhesion and/or signal transduction (*DSC2, UGT8, ITGB8, FERMT1*). Very little is known on the other identified genes (*HORMAD1, TFCP2L 1 and ANP32E*). None of these proteins have been previously shown to be specifically involved in metastasis.

### Example 4 : Development of a predictor of selective breast cancer failure to the lungs

To determine whether the 7 lung metastasis-associated genes could already be expressed in primary breast tumor cells and whether they could be predictive of a higher risk of lung metastasis, we analyzed by qRT-PCR their expression patterns in a series of 5 normal breast tissue samples, 6 breast cancer cell lines (MCF7, T47D, SKBR3, MDA-MB-231, MDA-MB-361 and MDA-MB-435) and 44 primary breast tumors. All lung-metastasis-associated genes were expressed in tissues of mammary origin except *HORMAD1* that showed very weak to no expression in all normal breast, all cell lines and a majority of primary tumors (Ct < 35). Therefore, *HORMAD1* was not considered in our attempt to establish a lung metastasis signature.

The 6 remaining genes (*DSC2, TFCP2L1, UGT8, ITGB8, ANP32E* and *FERMT1*) were used to develop a gene signature predictive of a higher risk of lung metastasis. We studied a cohort of 72 lymph-node-negative patients specifically selected on the basis of the treatments and metastatic outcomes: the CRH cohort. All 72 patients had not received neoadjuvant or adjuvant therapy. This meant that the potential prognostic impact of the "lung-metastasis classifier" would not be influenced by factors related to systemic treatment. Thirty-eight patients had developed distant metastases within 10 years (including 11 with lung metastases) and 34 patients remained free of disease after their initial diagnosis for a period of at least 10 years (**Table 6**).

The primary tumors were then assigned to a high-risk group or a low-risk group, with respect to lung metastasis, according to the risk index calculated on the basis of the six-gene signature. The lung metastasis risk index was defined as the linear combination of the expression values of the 6 genes and the appropriate cutoff point was set at the 75^{th} percentile. Tumors expressing high levels of the risk index metastasized significantly more frequently to the lungs than did the other tumors (p = 0.04, χ² test, **Figure 3A****).** In addition, patients with such tumors had significantly shorter lung-metastasis-free survival (p = 0.008 **Figure 3B****).** The six-gene signature did not correlate with the risk of bone metastasis **(****Figure 3C****)** or liver metastasis **(****Figure 3D****).**

### Example 5 : Validation of the Predictive ability of the six-gene signature

To validate the predictive value of the six-gene lung metastasis signature, we analyzed expression profiles of 3 independent cohorts of breast cancer patients which microarray data are publicly available (Wang et al., 2005; Van de Vijver, 2002; Minn et al., 2005, Minn et al., 2007). These 3 datasets correspond to 2 large cohorts of early stage breast cancer patients ("NKI" and "EMC" series of 295 and 344 patients, respectively) and a more locally advanced cohort of breast cancers (the "MSK" cohort of 82 patients) (Wang et al., 2005; Van de Vijver, 2002; Minn et al., 2005, Minn et al., 2007).

Hierarchical clustering analysis was performed on all individual series. Within the 3 different cohorts, the six-gene signature discriminates a subgroup of breast tumors with a higher propensity to metastasize to the lungs (p= 0.04, p = 0.016 and p=0.014, χ² test, for MSK, EMC and NKI respectively). As representative results, the clustering of the MSK and NKI breast tumors have been performed.

The results of hierarchical clustering are only indicative. Thus, using the same procedure as for the CRH cohort, we evaluated the six-gene signature in the 3 independent cohorts. Patients assigned to the high-risk group had significantly shorter lung-metastasis-free survival in all cases (p= 0.004, 0.001 and 0.039 for MSK, EMC and NKI series respectively, **Figure 4****),** whereas there was no difference in bone-metastasis-free survival. It is noteworthy that there is a fewer discrimination in NKI cohort, probably due to the consideration of only 5 genes of the signature since one gene (*ANP32E*) was not present in the corresponding chips. Finally, the Kaplan-Meier analysis was also performed on the combined cohort and resulted in a highly significant correlation of the six-gene signature with the outcome of breast cancer patient with regard to lung metastasis (n=721, p<10⁻⁵).

### Example 6 : Correlation to standard clinico-patholoaical variables and other prognostic signatures

We evaluated whether the six-gene signature provided additional prognostic information that may not be obtained by other models and/or standard markers. First, we analyzed the NKI series (for which the complete clinical data were documented) for the main prognostic molecular signatures reported for breast cancers. Consistent with previous reports, the primary breast tumors expressing the six-gene signature also expressed other poor-prognosis molecular markers (Kang et al., 2003; Minn et al., 2007). The tumors of patients at risk for lung metastasis as defined by the six-gene signature mostly were of poor prognosis on the basis of standard pathological parameters (62% ER-negative and 70% grade 3) and previously reported poor-prognosis signatures as the 70-gene signature (van 't Veer et al., 2002; van de Vijver et al., 2002), the wound-healing signature (Chang et al., 2005; Chang et al., 2005) and the basal-like molecular subtype (23, 24) (80%, 89%, and 58% respectively).

In addition, when analyzing the clinicopathological variables available for each of the cohorts of breast cancer patients, we found no difference between the high- and low-risk groups with respect to age, lymph node status or primary tumor size, whereas most high-risk patients and their tumors appeared to be hormone receptor-negative and grade 3.

To ensure that the six-gene classifier improved risk stratification independently of these standard clinical parameters, we performed a multivariate Cox proportional-hazards analysis on the combined cohort (n=721) (only estrogen-receptor and lymph node status parameters were available for all patients, **Table 7**). The Cox model showed that the six-gene signature and estrogen receptor status were independent predictors of lung metastasis (p=0.01 and 0.04 respectively). The six-gene signature is a significant predictor of lung metastasis in breast cancer. It added new and important prognostic information beyond that provided by ER and lymph node status.

Finally, we compared the predictive value of our lung metastasis signature to the one derived from the MDA-MB-231 mouse model (LMS) (Minn et al., 2007). These two signatures are defined by expression patterns of distinct sets of genes with no overlap. When evaluated on the same series of 721 samples, we observe that despite their different derivations, the signatures gave overlapping and consistent predictions outcome. Each signature assigned the same patients to the high or low risk groups of breast cancer lung metastasis. Indeed, the two models have high level of concordance in their predictions of lung metastasis. Almost all tumors identified as LMS⁺ were also classified as having the six-gene signature. LMS and our six-gene signature showed 85% agreement in outcome classification of breast cancer patients with respect to lung metastasis (Kappa coefficient = 0.57). These results suggest that even though there are no gene overlap and different biological models were used, the outcome predictions still are similar, probably because they track common set of biological characteristics conferring the organ-specific metastatic phenotypes.

Thus, to determine whether the use of the two models together would result in a better model than the use of any one alone, we derived a single model based on the common findings of the 2 models separately. The performance of this model according to the Kaplan-Meier analysis was noticeably better than each of the 2 models **(****Figure 5B****)** demonstrating that gene signatures derived from 2 distinct approaches can be complementary, as recently reported for the 70-gene and WR signatures corresponding to the "top-down" and "bottom-up" strategies respectively (Chang et al., 2005).

Further, as shown in Figure 6, primary that highly express eleven-gene bone metastasis signature are more susceptible to relapse to bone.

Also, **Table 8** shows the highets ranking genes obtained from a class comparison of bone and non-bone metastases of breast cancer.

### REFERENCES

Attardi LD, Reczek EE, Cosmas C, Demicco EG, McCurrach ME, Lowe SW, Jacks T. PERP, an apoptosis-associated target of p53, is a novel member of the PMP-22/gas3 family.Genes Dev. 2000 Mar 15;14(6):704-18
Bieche I, Parfait B, Le Doussal V, Olivi M, Rio MC, Lidereau R, Vidaud M. Identification of CGA as a novel estrogen receptor-responsive gene in breast cancer: an outstanding candidate marker to predict the response to endocrine therapy. Cancer Res. 2001 Feb 15;61 (4):1652-8.
Carlinfante G, Vassiliou D, Svensson O, Wendel M, Heinegard D, Andersson G. Differential expression of osteopontin and bone sialoprotein in bone metastasis of breast and prostate carcinoma. Clin Exp Metastasis 2003; 20:437-44.
Chang, H. Y., Nuyten, D. S., Sneddon, J. B., Hastie, T., Tibshirani, R., Sorlie, T., Dai, H., He, Y. D., van't Veer, L. J., Bartelink, H., van de Rijn, M., Brown, P. O. & van de Vijver, M. J. (2005) Proc Natl Acad Sci U S A 102, 3738-43.
Chang, H. Y., Sneddon, J. B., Alizadeh, A. A., Sood, R., West, R. B., Montgomery, K., Chi, J. T., van de Rijn, M., Botstein, D. & Brown, P. O. (2004) PLoS Biol 2, E7.
Greenberg PA, Hortobagyi GN, Smith TL, Ziegler LD, Frye DK, Buzdar AU. 1996. Long-term follow-up of patients with complete remission following combination chemotherapy for metastatic breast cancer. J Clin Oncol 14:2197-2205.
Gupta GP, Massague J. Cancer metastasis: building a framework. Cell. 2006 Nov 17;127(4):679-95.
Huang WC, Xie Z, Konaka H, Sodek J, Zhau HE, Chung LW. Human osteocalcin and bone sialoprotein mediating osteomimicry of prostate cancer cells: role of cAMP-dependent protein kinase A signaling pathway. Cancer Res. 2005 Mar 15;65(6):2303-13.
Jackson, A., Vayssiere, B., Garcia, T., Newell, W., Baron, R., Roman-Roman, S. & Rawadi, G. (2005) Bone 36, 585-98.
Jechlinger M, Sommer A, Moriggl R, Seither P, Kraut N, Capodiecci P, Donovan M, Cordon-Cardo C, Beug H, Grunert S. Autocrine PDGFR signaling promotes mammary cancer metastasis.J Clin Invest. 2006 Jun;116(6):1561-70
Kakiuchi S, Daigo Y, Tsunoda T, Yano S, Sone S, Nakamura Y. Genome-wide analysis of organ-preferential metastasis of human small cell lung cancer in mice. Mol Cancer Res. 2003 May;1 (7):485-99.
Kang Y, Siegel PM, Shu W, Drobnjak M, Kakonen SM, Cordon-Cardo C, Guise TA, Massague J. A multigenic program mediating breast cancer metastasis to bone. Cancer Cell. 2003 Jun;3(6):537-49.
Knerr K, Ackermann K, Neidhart T, Pyerin W. Bone metastasis: Osteoblasts affect growth and adhesion regulons in prostate tumor cells and provoke osteomimicry. Int J Cancer. 2004 Aug 10;111 (1):152-9.
Koeneman KS, Yeung F, Chung LW (1999) Osteomimetic properties of prostate cancer cells: a hypothesis supporting the predilection of prostate cancer metastasis and growth in the bone environment. Prostate 39:246-261.
Kozlow W, Guise TA. Breast cancer metastasis to bone :mechanism of osteolysis and implications fro therapy. J Mammary Gland Biol Neoplasia 2005;10:169-80.
Ma XJ, Hilsenbeck SG, Wang W, Ding L, Sgroi DC, Bender RA, Osborne CK, Allred DC, Erlander MG. The HOXB13:IL17BR expression index is a prognostic factor in early-stage breast cancer. J Clin Oncol. 2006 Oct 1;24(28):4611-9.
Minn AJ, Gupta GP, Siegel PM, Bos PD, Shu W, Giri DD, Viale A, Olshen AB, Gerald WL, Massague J. Genes that mediate breast cancer metastasis to lung. Nature. 2005 Jul 28;436(7050):518-24.
Minn, A. J., Gupta, G. P., Padua, D., Bos, P., Nguyen, D. X., Nuyten, D., Kreike, B., Zhang, Y., Wang, Y., Ishwaran, H., Foekens, J. A., van de Vijver, M. & Massague, J. (2007) Proc Natl Acad Sci U S A 104, 6740-5.
Minn AJ, Kang Y, Serganova I, Gupta GP, Giri DD, Doubrovin M, Ponomarev V, Gerald WL, Blasberg R, Massague J. Distinct organ-specific metastatic potential of individual breast cancer cells and primary tumors. J Clin Invest. 2005 Jan;115(1):44-55
Oliveira AM, Ross JS, Fletcher JA. Tumor suppressor genes in breast cancer: the gatekeepers and the caretakers. Am J Clin Pathol. 2005 124 Suppl:S16-28. Review.
Petrocelli T, Slingerland JM. PTEN deficiency: a role in mammary carcinogenesis. Breast Cancer Res. 2001;3(6):356-60. Review.
Ramaswamy S, Ross KN, Lander ES, Golub TR. A molecular signature of metastasis in primary solid tumors. Nat Genet. 2003 Jan;33(1):49-54.
Rehn AP, Chalk AM, Wendel M. Differential regulation of osteoadherin (OSAD) by TGF-beta1 and BMP-2. Biochem Biophys Res Commun. 2006 Oct 27;349(3):1057-64.
Roodman GD. Mechanism of bone metastases. N Eng J Med 2004;350:1655-64.
Rowley M, Grothey E, Couch FJ. The role of Tbx2 and Tbx3 in mammary development and tumorigenesis. J Mammary Gland Biol Neoplasia. 2004 Apr;9(2):109-18. Review
Sharp JA, Waltham M, Williams ED, Henderson MA, Thompson EW. Transfection of MDA-MB-231 human breast cancer cells with bone sialoprotein (BSP) stimulates growth of primary and secondary tumors in nude mice. Clin Exp Metastasis 2004;21:19-29.
Smid M, Wang Y, Klijn JG, Sieuwerts AM, Zhang Y, Atkins D, Martens JW, Foekens JA. Genes associated with breast cancer metastatic to bone. J Clin Oncol. 2006 May 20;24(15):2261-7
Steeg PS. Tumor metastasis: mechanistic insights and clinical challenges. Nat Med. 2006 Aug;12(8):895-904.
Tonon G, Wong KK, Maulik G, Brennan C, Feng B, Zhang Y, Khatry DB, Protopopov A, You MJ, Aguirre AJ, Martin ES, Yang Z, Ji H, Chin L, Depinho RA. High-resolution genomic profiles of human lung cancer. Proc Natl Acad Sci U S A. 2005 Jul 5;102(27):9625-30.
van de Vijver, M. J., He, Y. D., van't Veer, L. J., Dai, H., Hart, A. A., Voskuil, D. W., Schreiber, G. J., Peterse, J. L., Roberts, C., Marton, M. J., Parrish, M., Atsma, D., Witteveen, A., Glas, A., Delahaye, L., van der Velde, T., Bartelink, H., Rodenhuis, S., Rutgers, E. T., Friend, S. H. & Bernards, R. (2002) N Engl J Med 347, 1999-2009.
van 't Veer LJ, Dai H, van de Vijver MJ, He YD, Hart AA, Mao M, Peterse HL, van der Kooy K, Marton MJ, Witteveen AT, Schreiber GJ, Kerkhoven RM, Roberts C, Linsley PS, Bernards R, Friend SH. Gene expression profiling predicts clinical outcome of breast cancer. Nature. 2002;415:530-536.
Waltregny D, Bellahcene A, de Leval X, Florkin B, Weidle U, Castronovo V. Increased expression of bone sialoprotein in bone metastases compared with visceral metastases in human breast and prostate cancers. J Bone Miner Res. 2000 May;15(5):834-43.
Wang Y, Klijn JG, Zhang Y, Sieuwerts AM, Look MP, Yang F, Talantov D, Timmermans M, Meijer-van Gelder ME, Yu J, Jatkoe T, Berns EM, Atkins D, Foekens JA. Gene-expression profiles to predict distant metastasis of lymph-node-negative primary breast cancer. Lancet. 2005 Feb 19-25;365(9460):671-9
Weigelt B, Hu Z, He X, Livasy C, Carey LA, Ewend MG, Glas AM, Perou CM, Van't Veer LJ. Molecular portraits and 70-gene prognosis signature are preserved throughout the metastatic process of breast cancer. Cancer Res. 2005 Oct 15;65(20):9155-8.
Weigelt B, Peterse JL, van't Veer LJ. Breast cancer metastasis: markers and models. Nat Rev Cancer. 2005 Aug;5(8):591-602.
Yoneda T, Hiraga T. Cross-talk between cancer cells and bone microenvironment in bone metastasis. Biochem Biophys Res Commun 2005;328:679-87.
Barry, F., Boynton, R.E., Liu, B., Murphy, J.M. Chondrogenic differentiation of mesenchymal stem cells from bone marrow: differentiation-dependent gene expression of matrix components. Exp. Cell. Res. 268, 189-200 (2001).
Beattie, J., Allan, G.J., Lochrie, J.D., Flint, D.J. Insulin-like growth factor-binding protein-5 (IGFBP-5): a critical member of the IGF axis. Biochem. J. 395 1-19 (2006).
Bellahcene, A., et al. Transcriptome analysis reveals an osteoblast-like phenotype for human osteotropic breast cancer cells. Breast Cancer Res. Treat. DOI 10.1007/s10549-006-9279-8
Didier, G., Brezellec, P., Remy, E., Henaut, A. GeneANOVA--gene expression analysis of variance. Bioinformatics 18, 490-491 (2002).
Fisher, L.W. & Fedarko, N.S. Six genes expressed in bones and teeth encode the current members of the SIBLING family of proteins. Connect. Tissue Res. 44 Suppl , 33-40 (2003).
Kanaan, R.A. & Kanaan, L.A. Transforming growth factor beta1, bone connection. Med. Sci. Monit. 12, RA164-169 (2006).
Kikuchi, T. et al. Expression profiles of metastatic brain tumor from lung adenocarcinomas on cDNA microarray. Int. J. Oncol. 28, 799-805 (2006).
Kusu, N. et al. . Sclerostin is a novel secreted osteoclast-derived bone morphogenetic protein antagonist with unique ligand specificity. J. Biol. Chem. 278, 24113-24117 (2003).
Mandelin, J., et al. Human osteoblasts produce cathepsin K. Bone 38, 769-777 (2006).
Stickens, D. et al. Altered endochondral bone development in matrix metalloproteinase 13-deficient mice. Development 131, 5883-5895 (2004).
Weil, R.J., Palmieri, D.C., Bronder, J.L., Stark, A.M., Steeg, P.S. Breast cancer metastasis to the central nervous system. Am. J. Pathol. 167, 913-920 (2005).
Zayzafoon, M., Abdulkadir, S.A., McDonald, J.M. Notch signaling and ERK activation are important for the osteomimetic properties of prostate cancer bone metastatic cell lines. J. Biol. Chem. 279, 3662-3670 (2004).

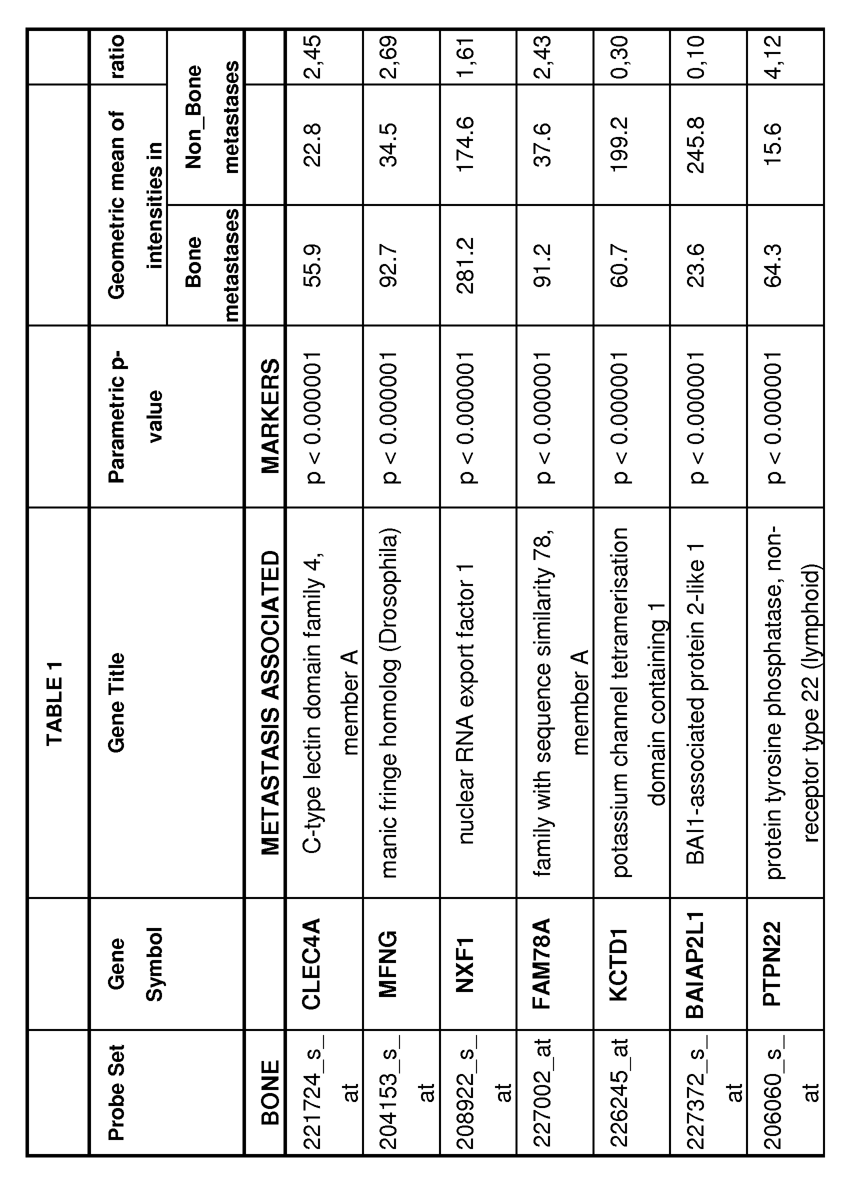

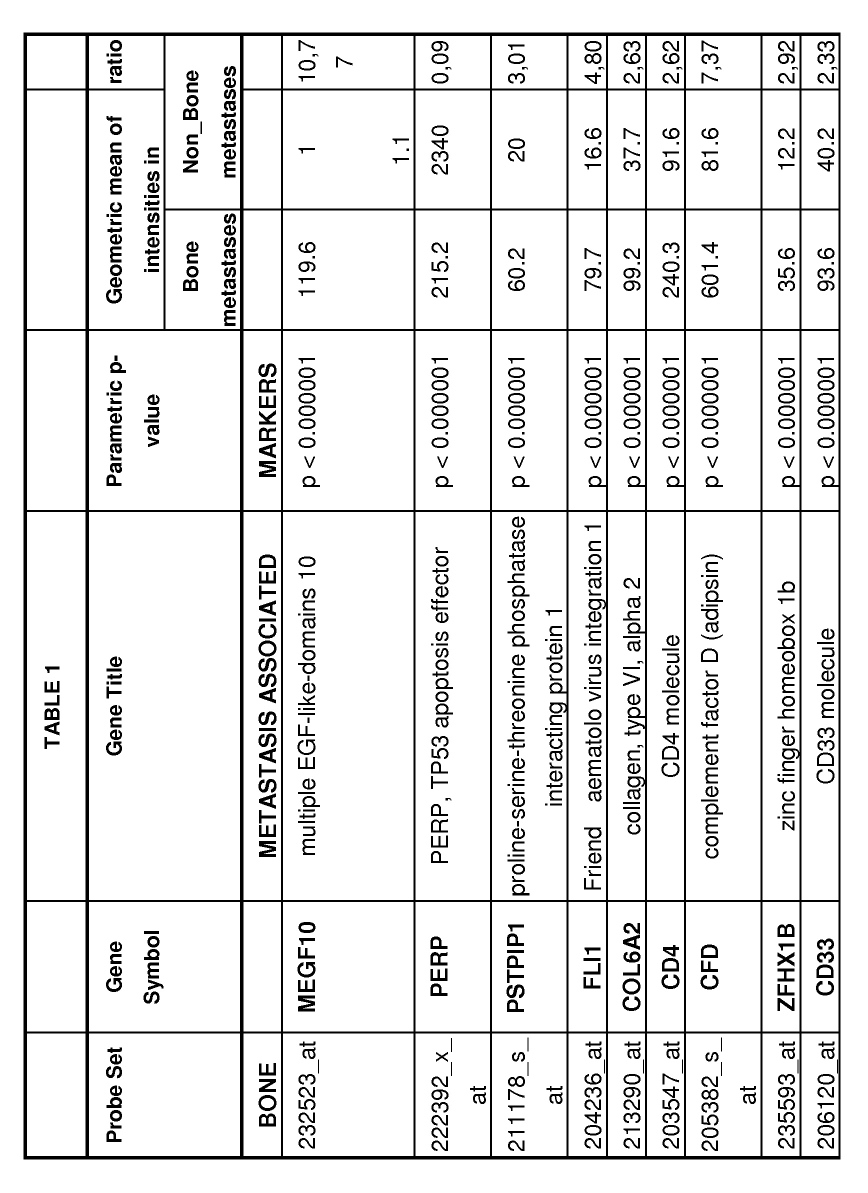

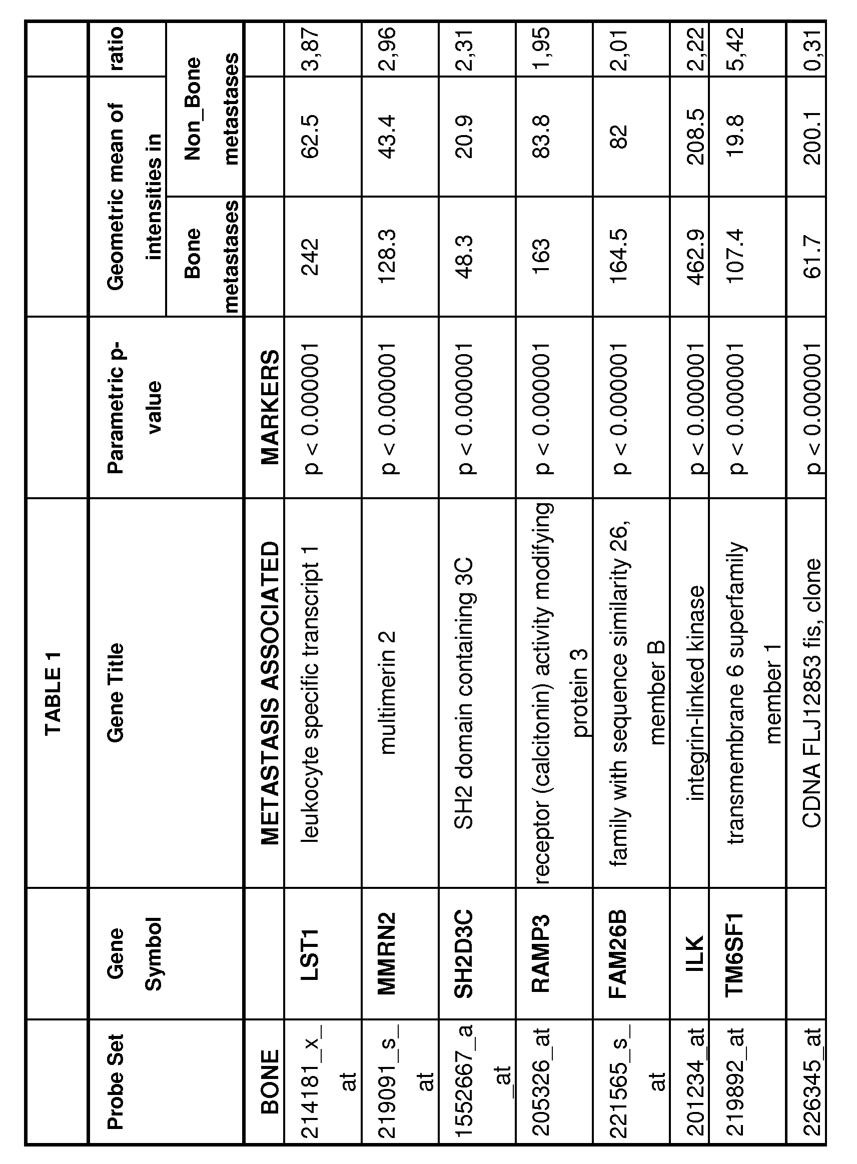

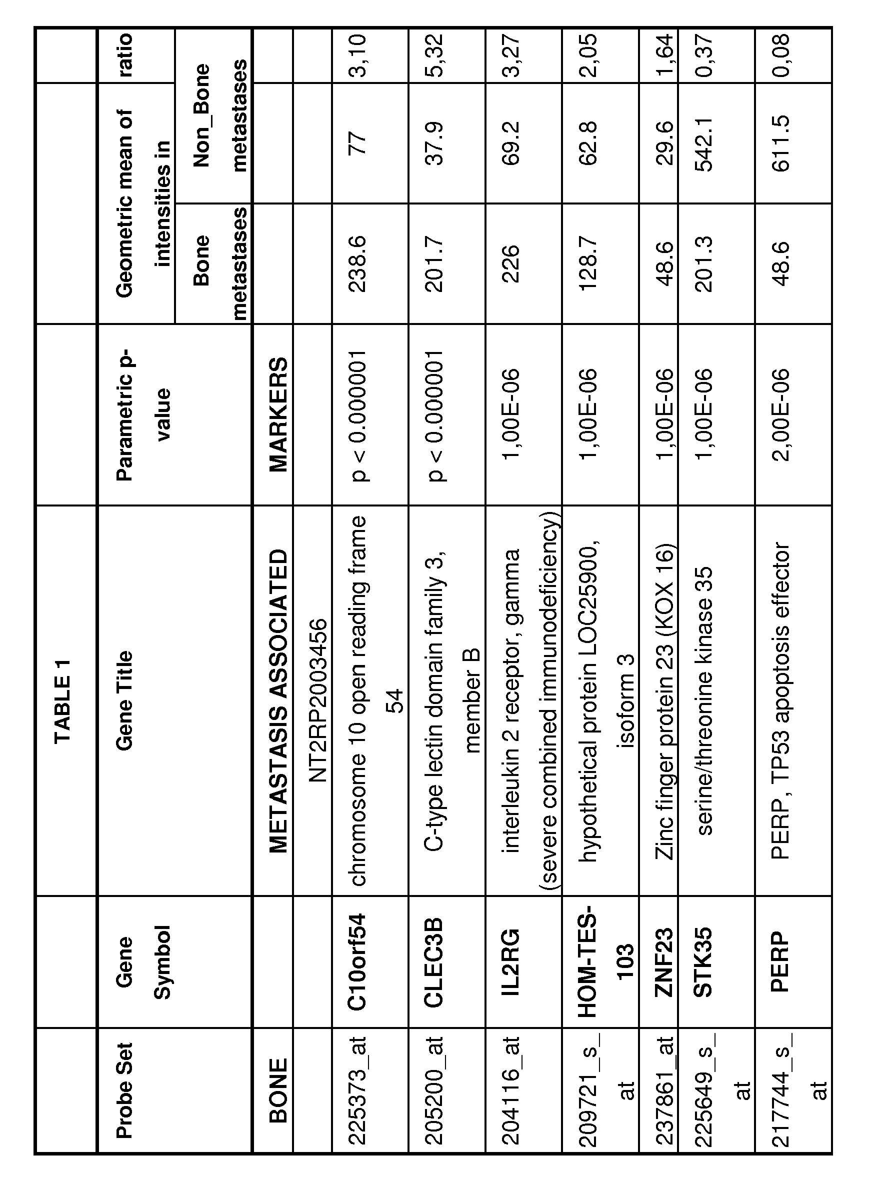

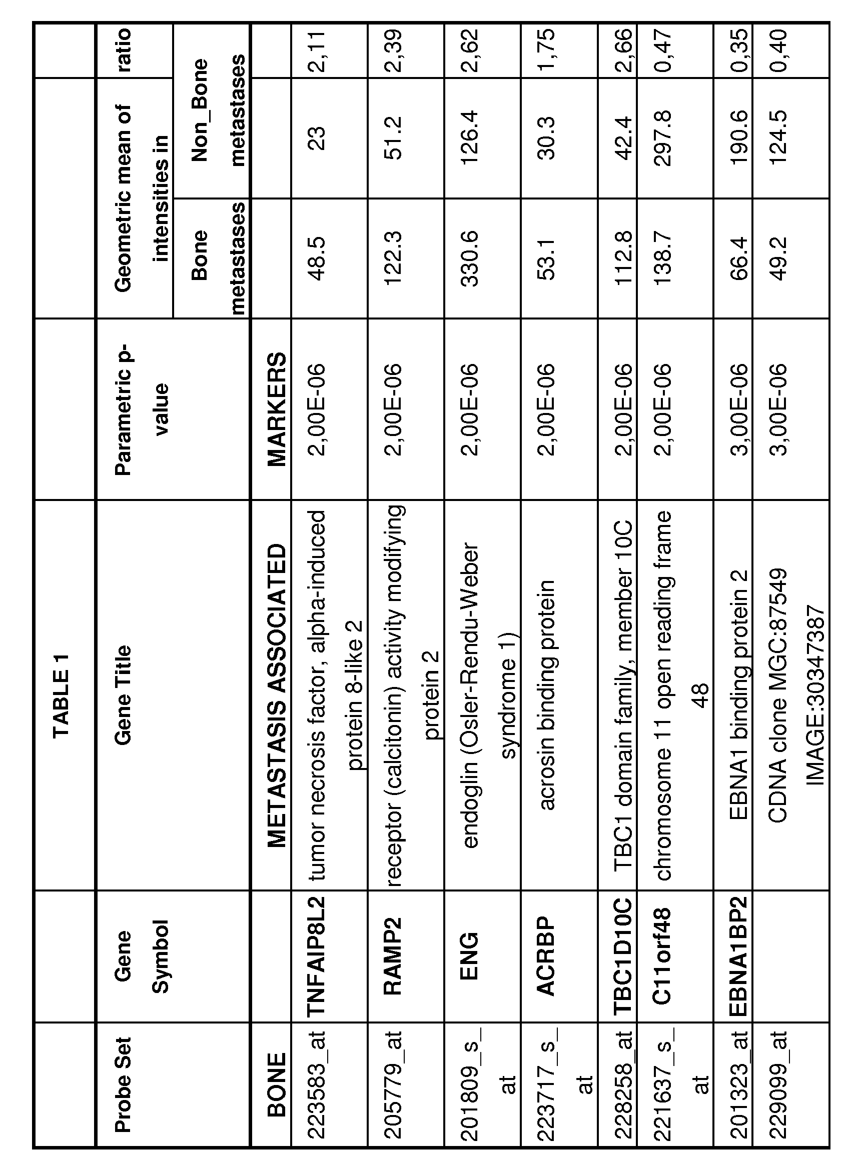

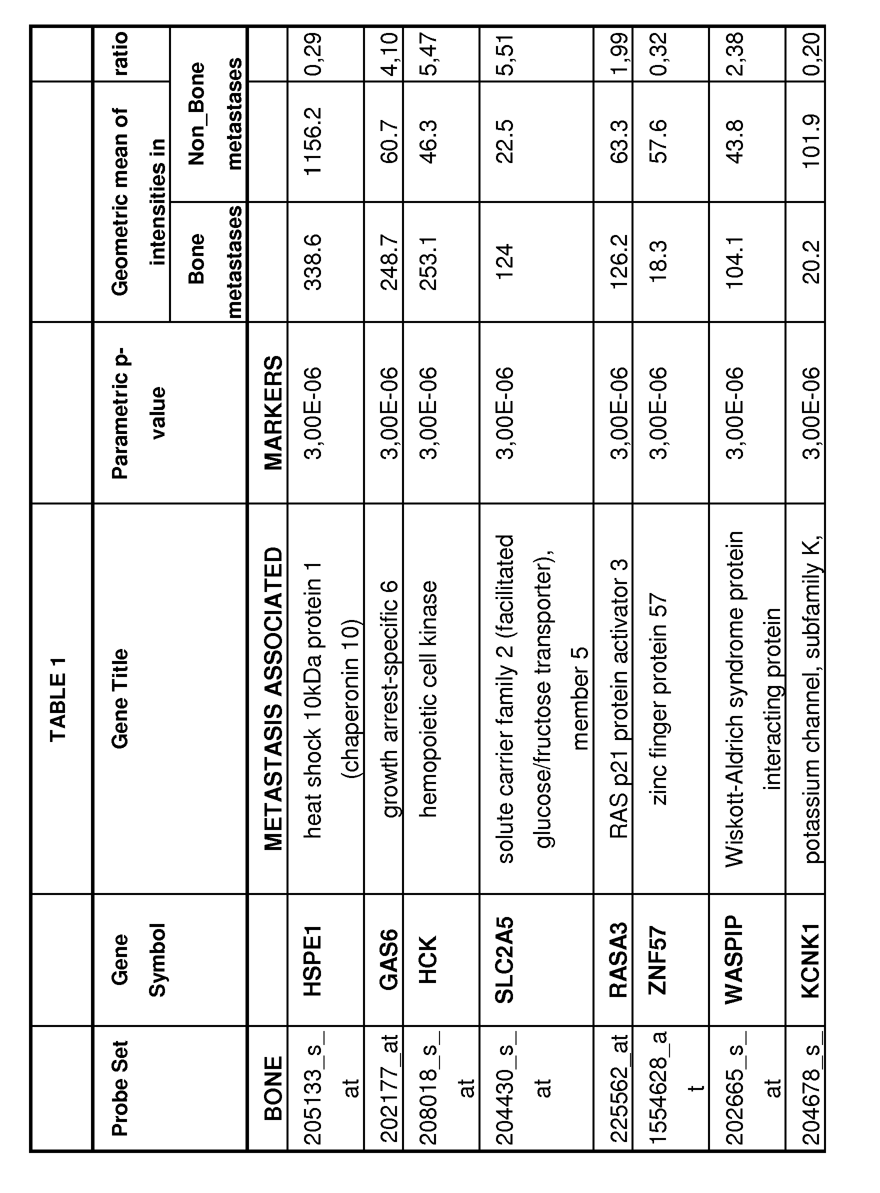

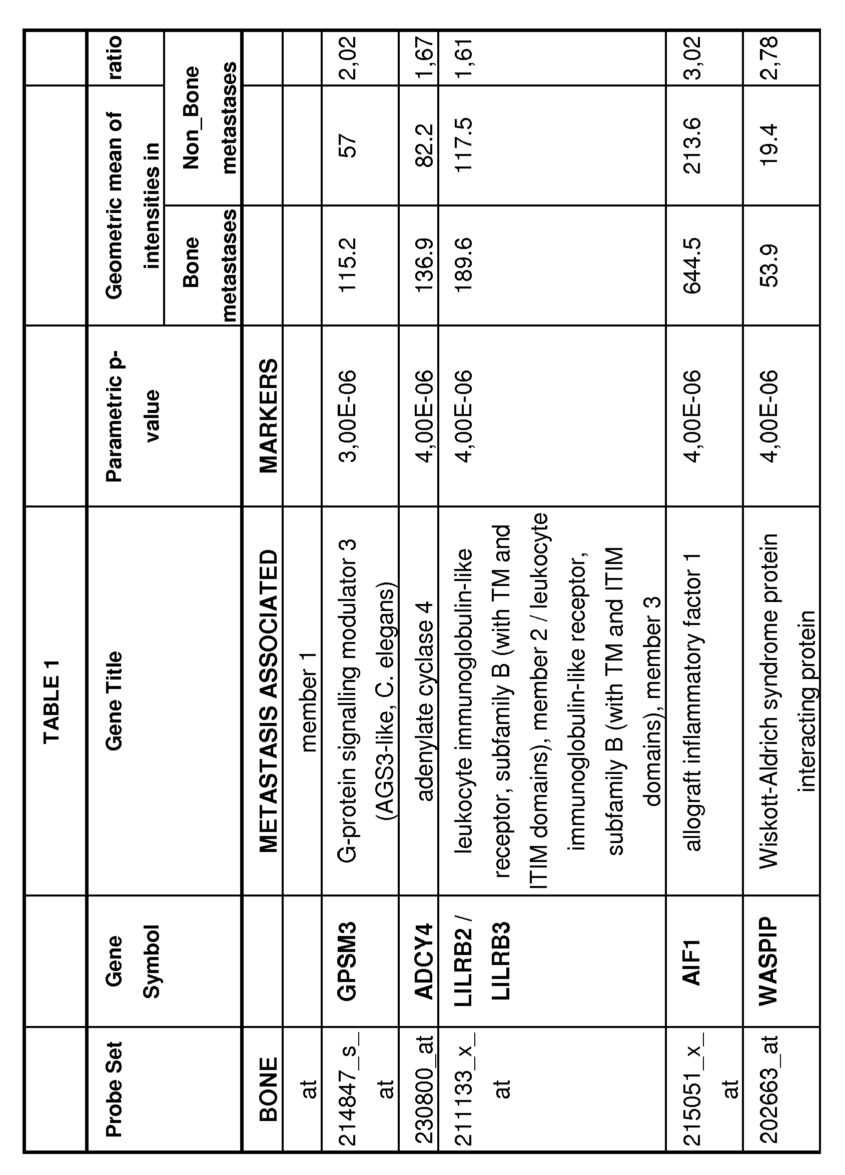

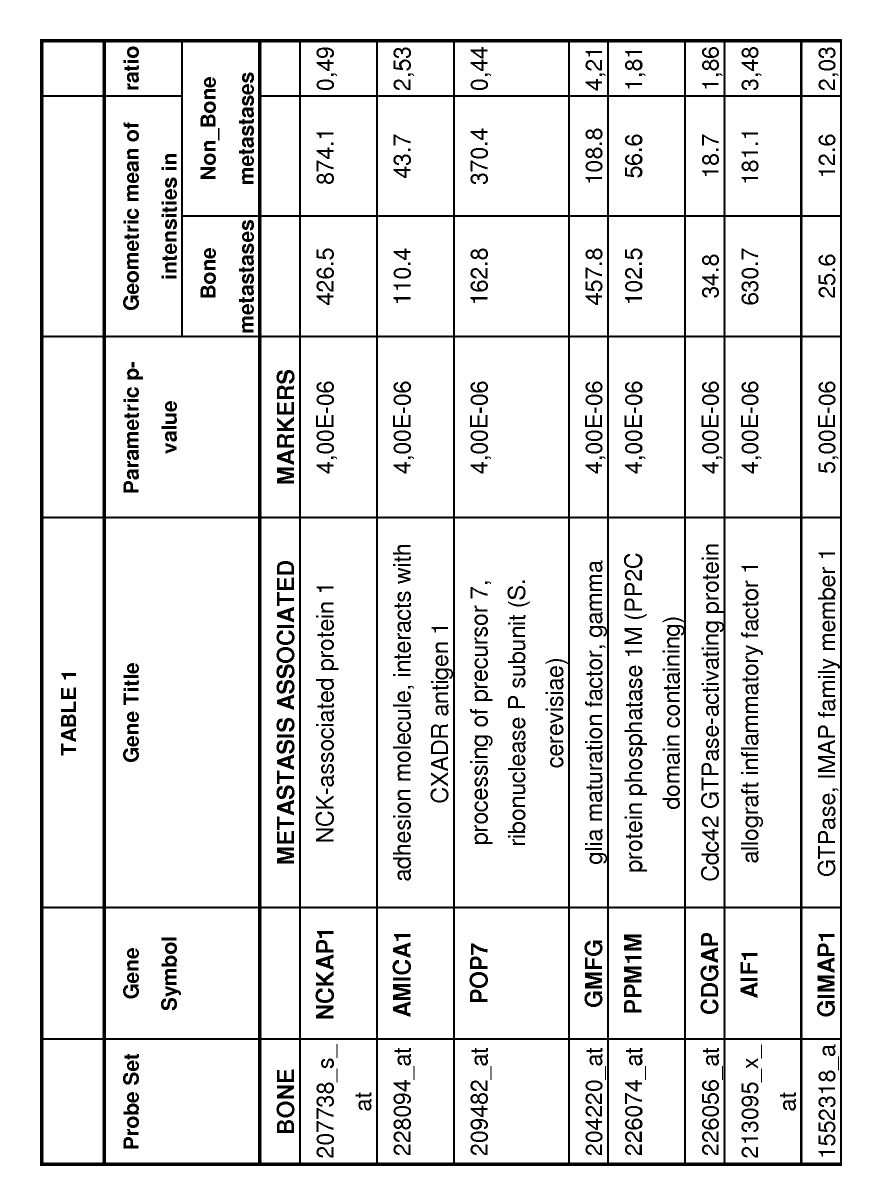

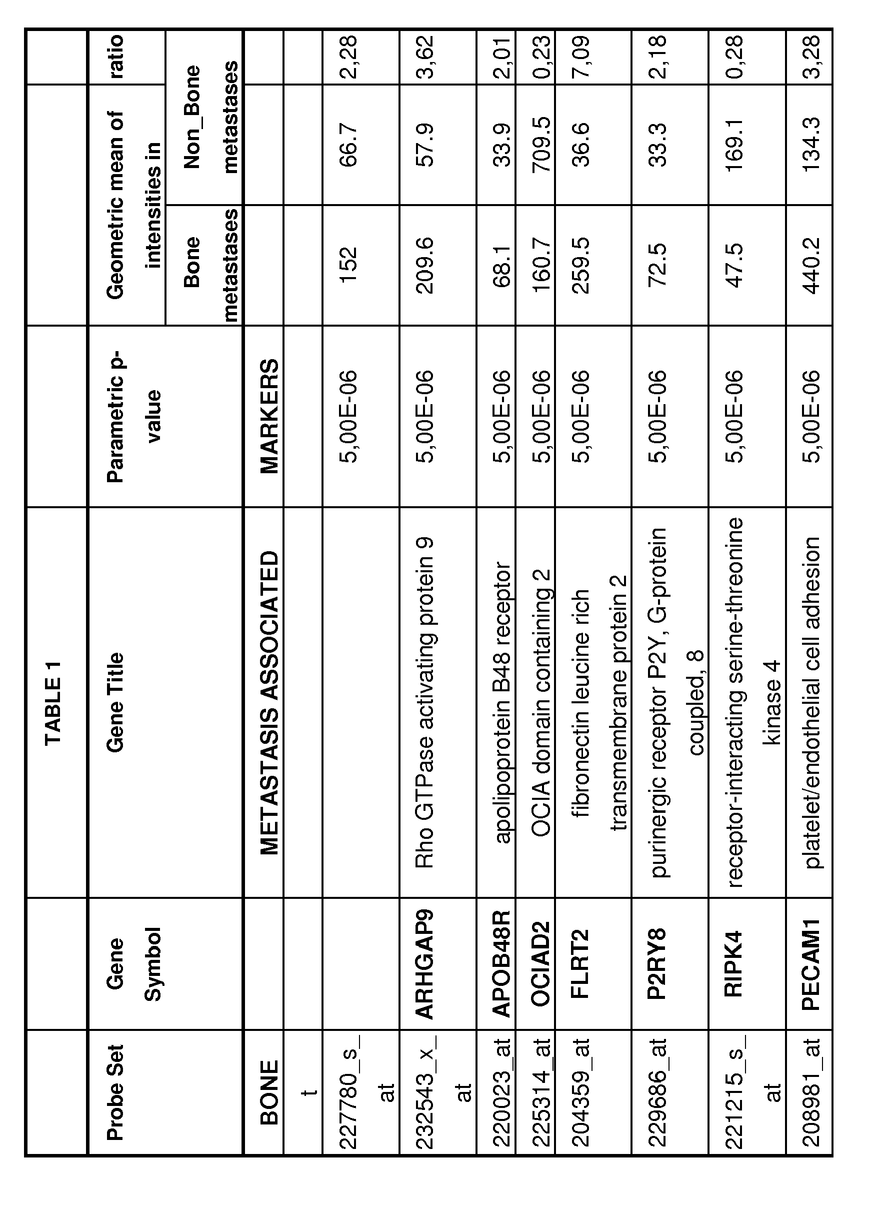

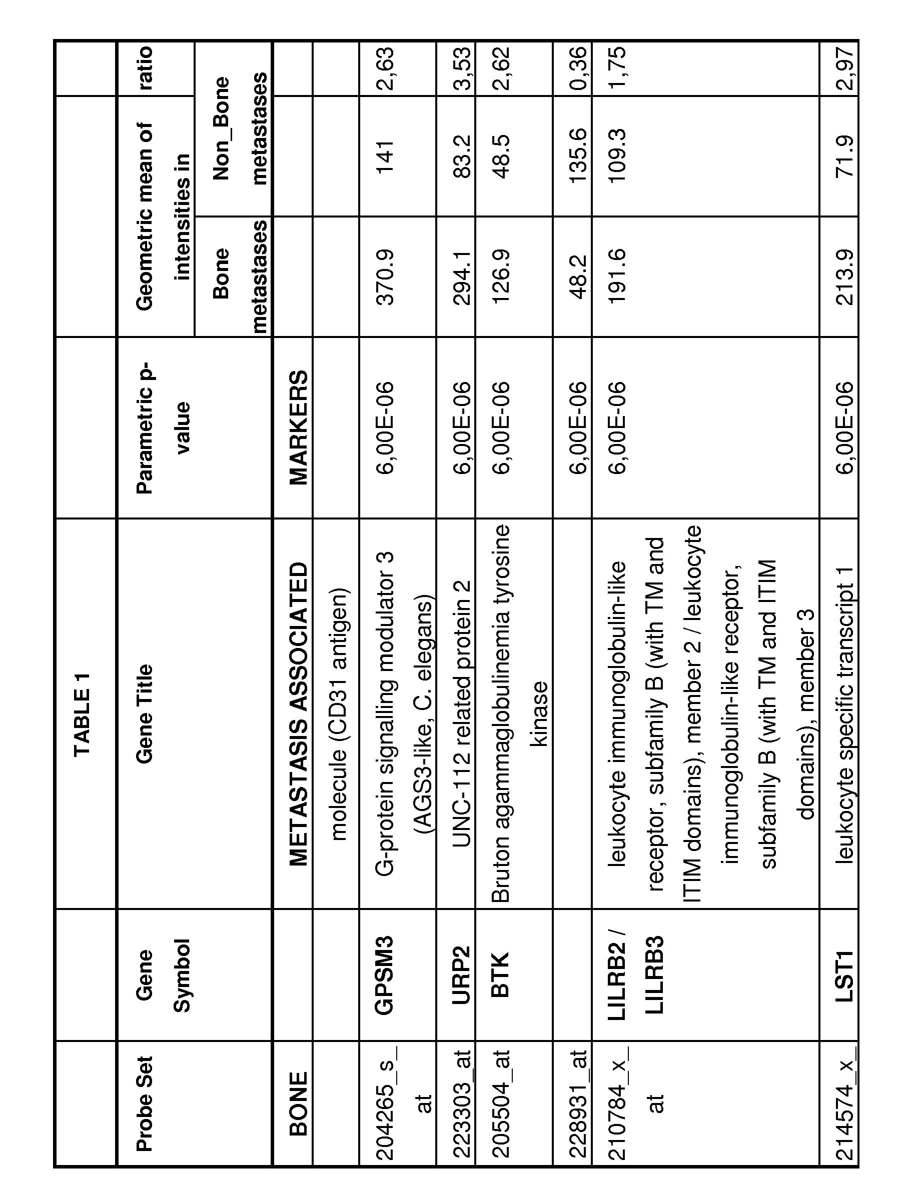

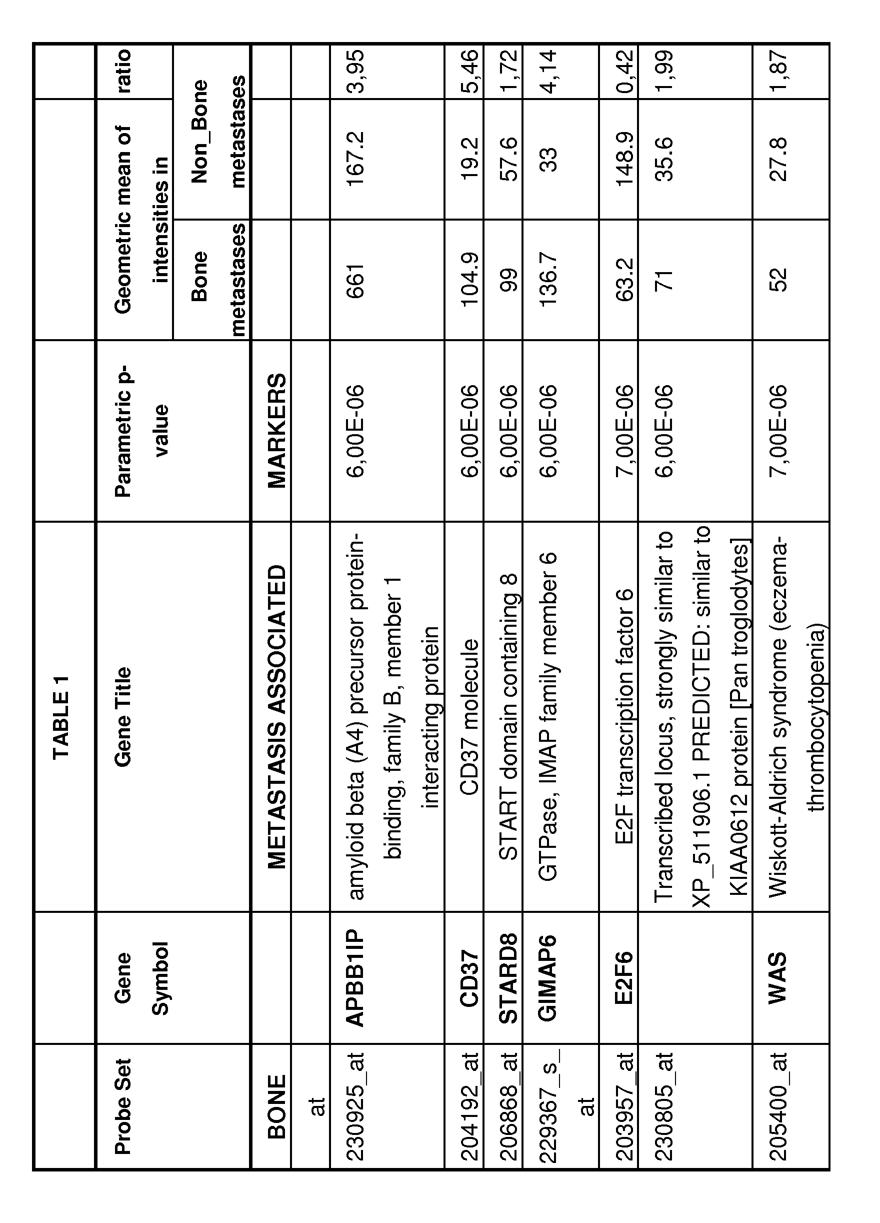

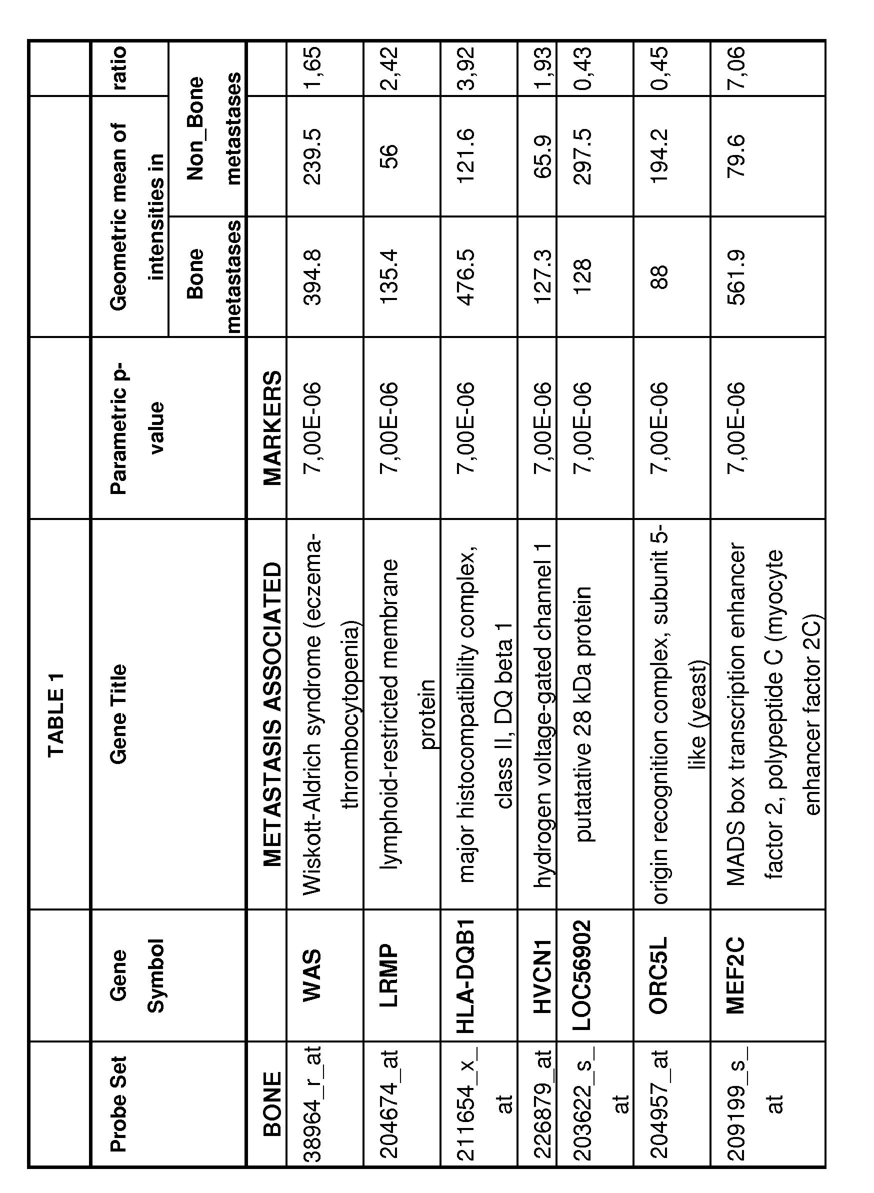

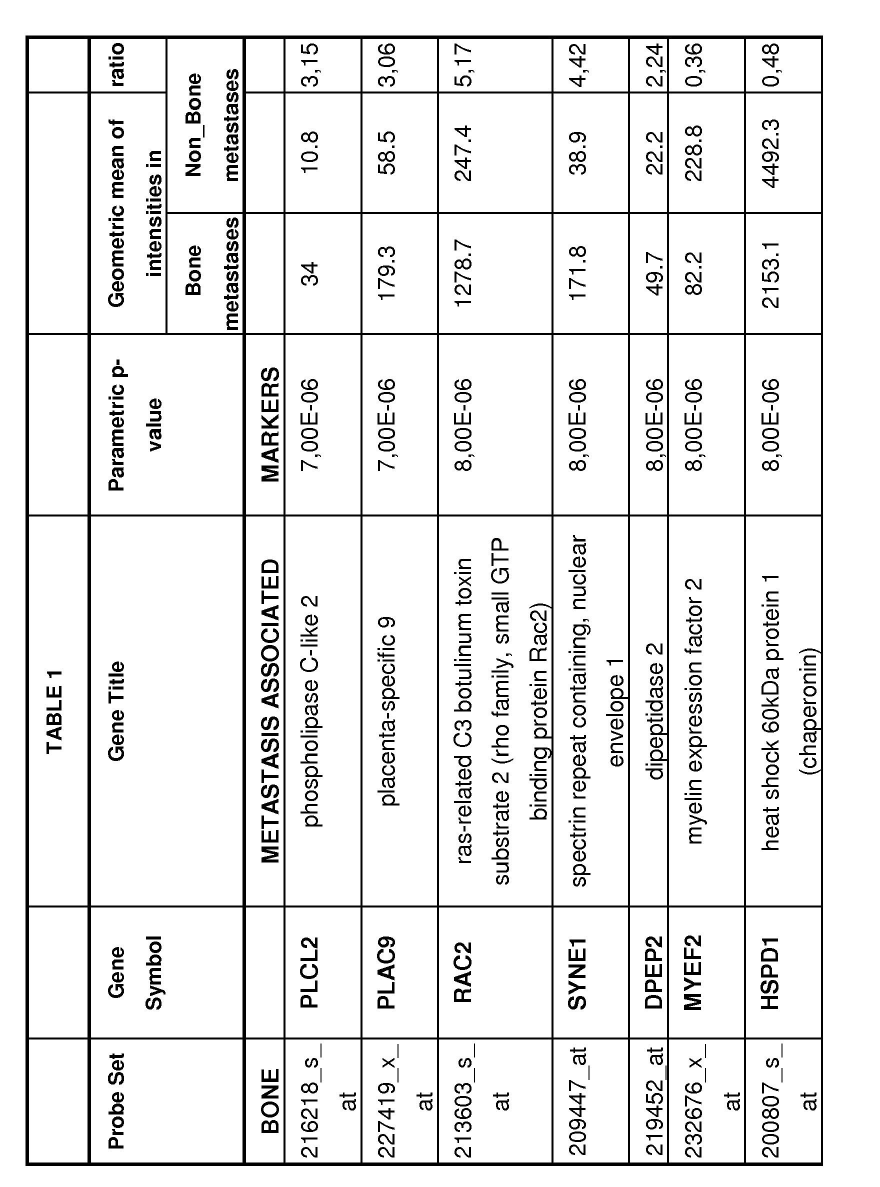

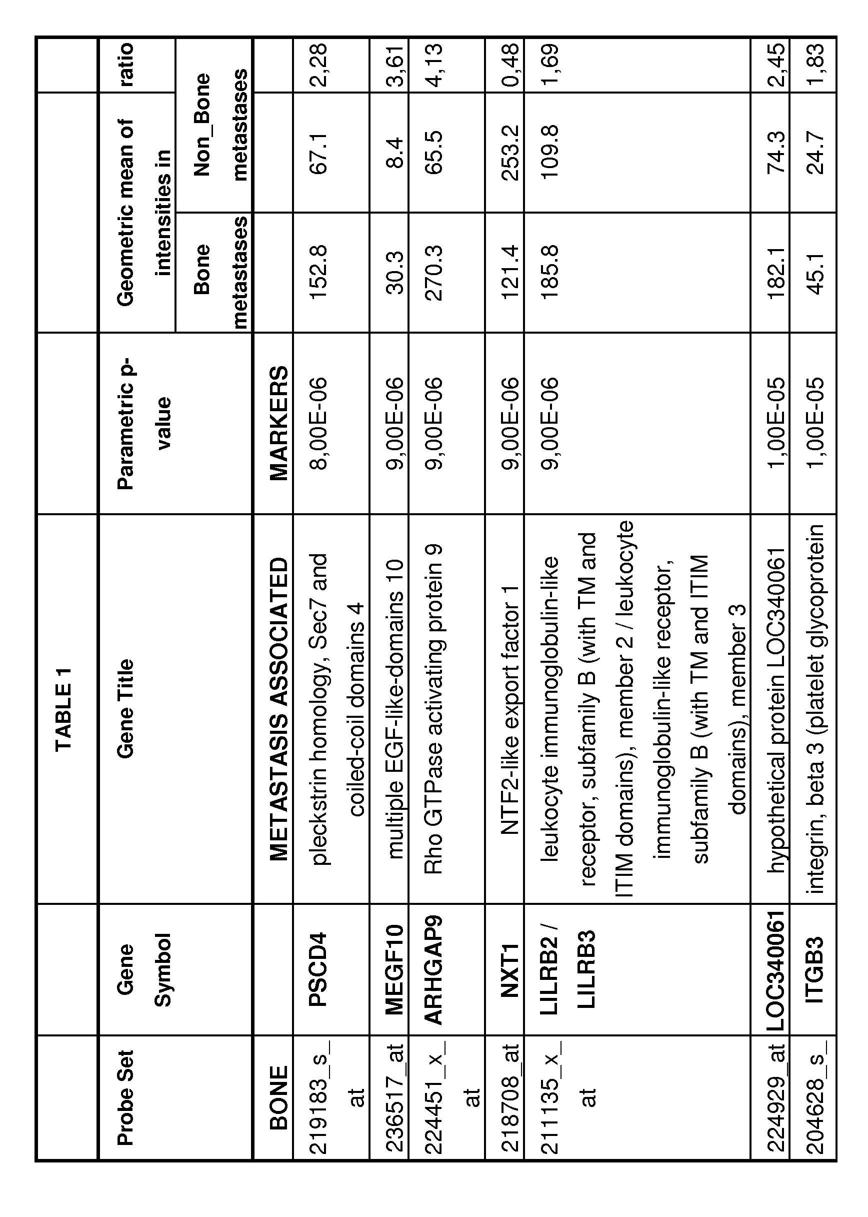

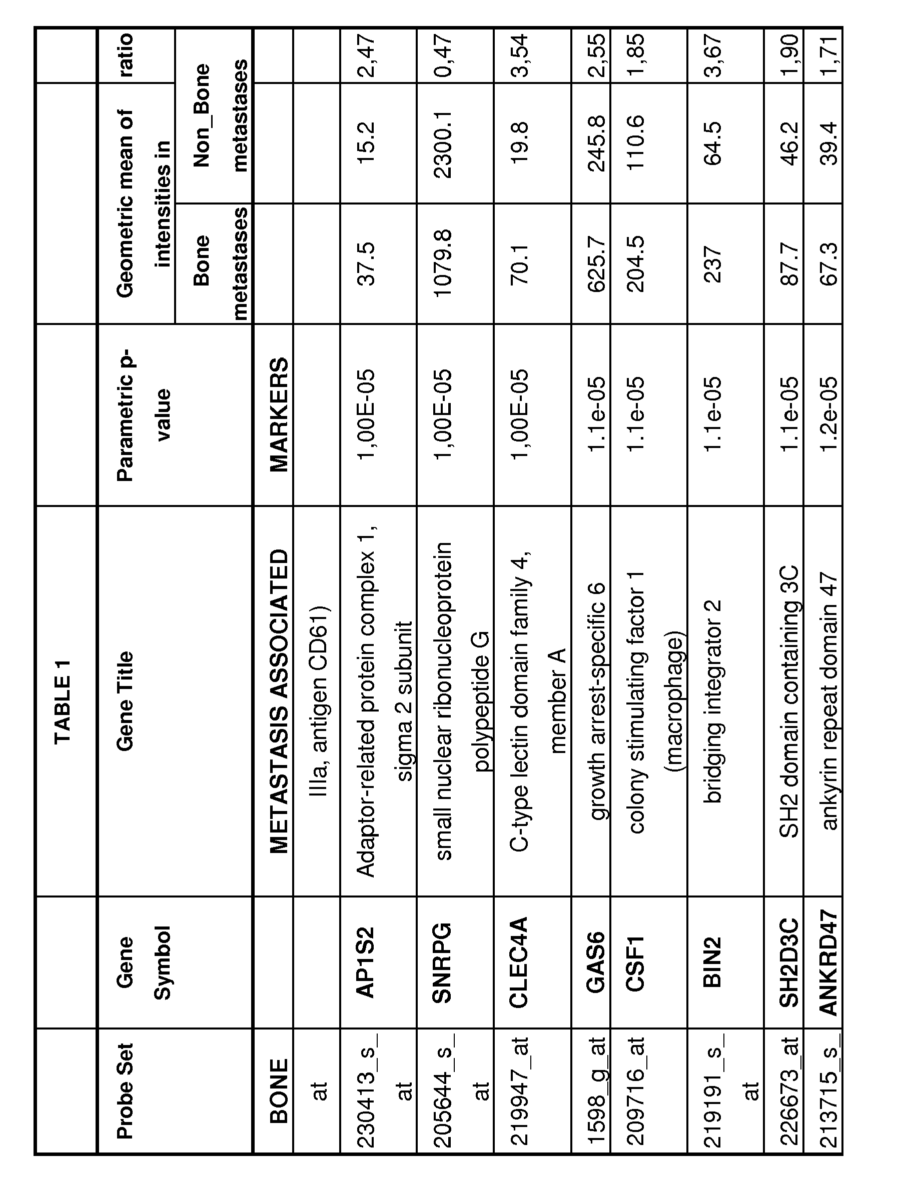

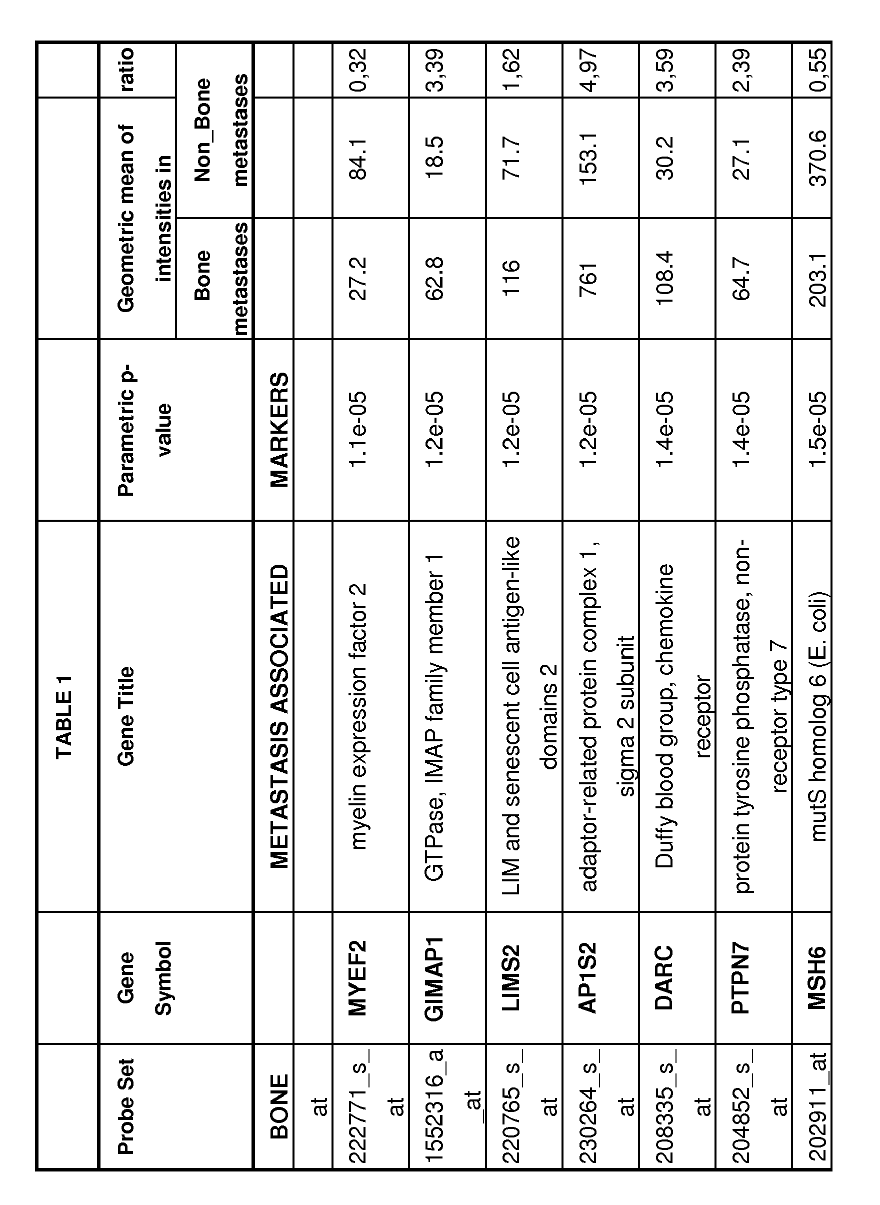

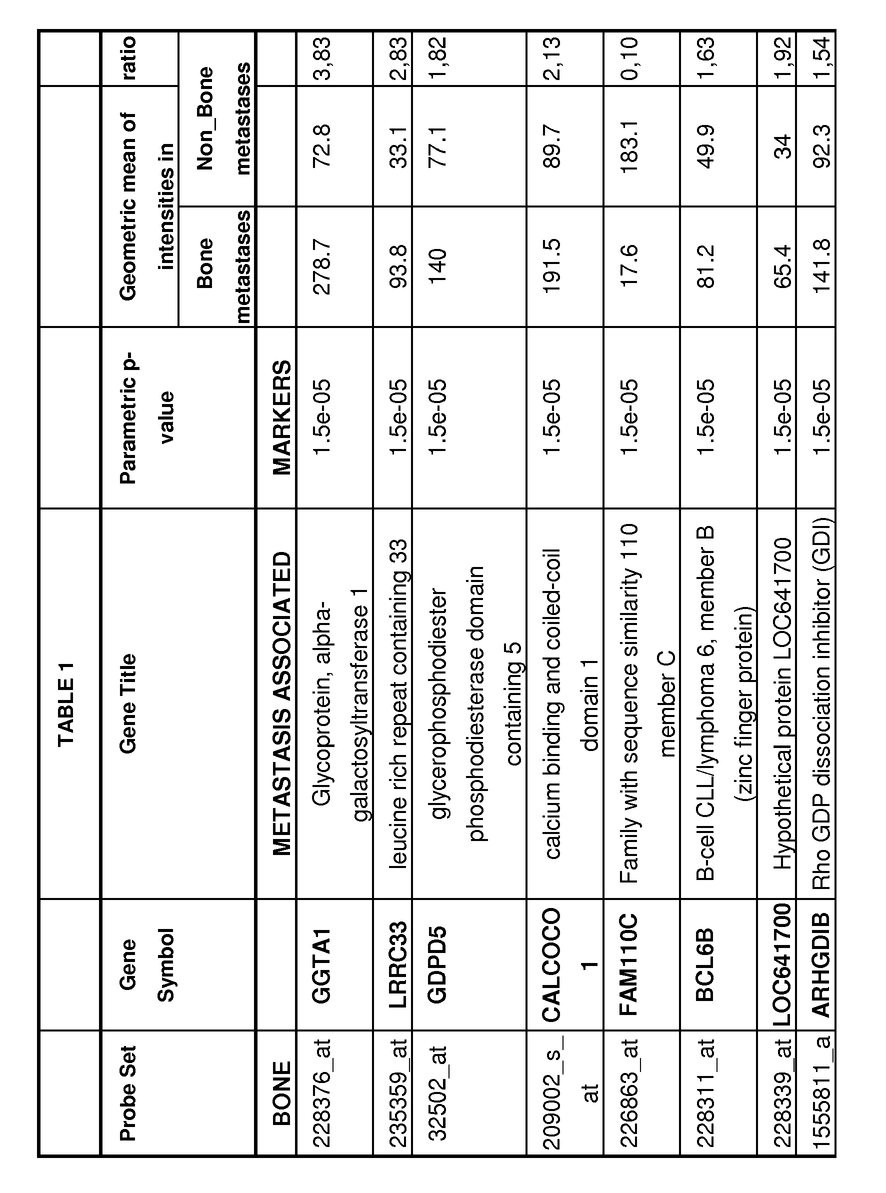

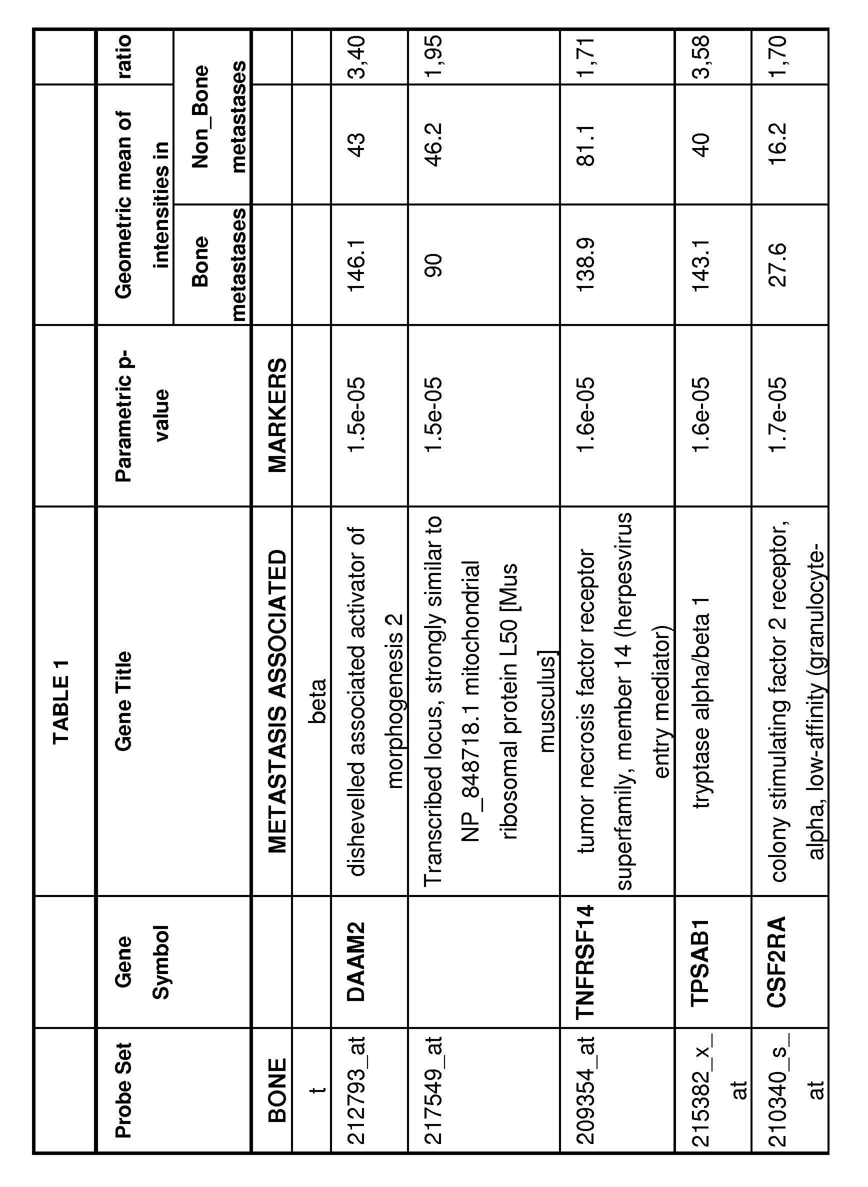

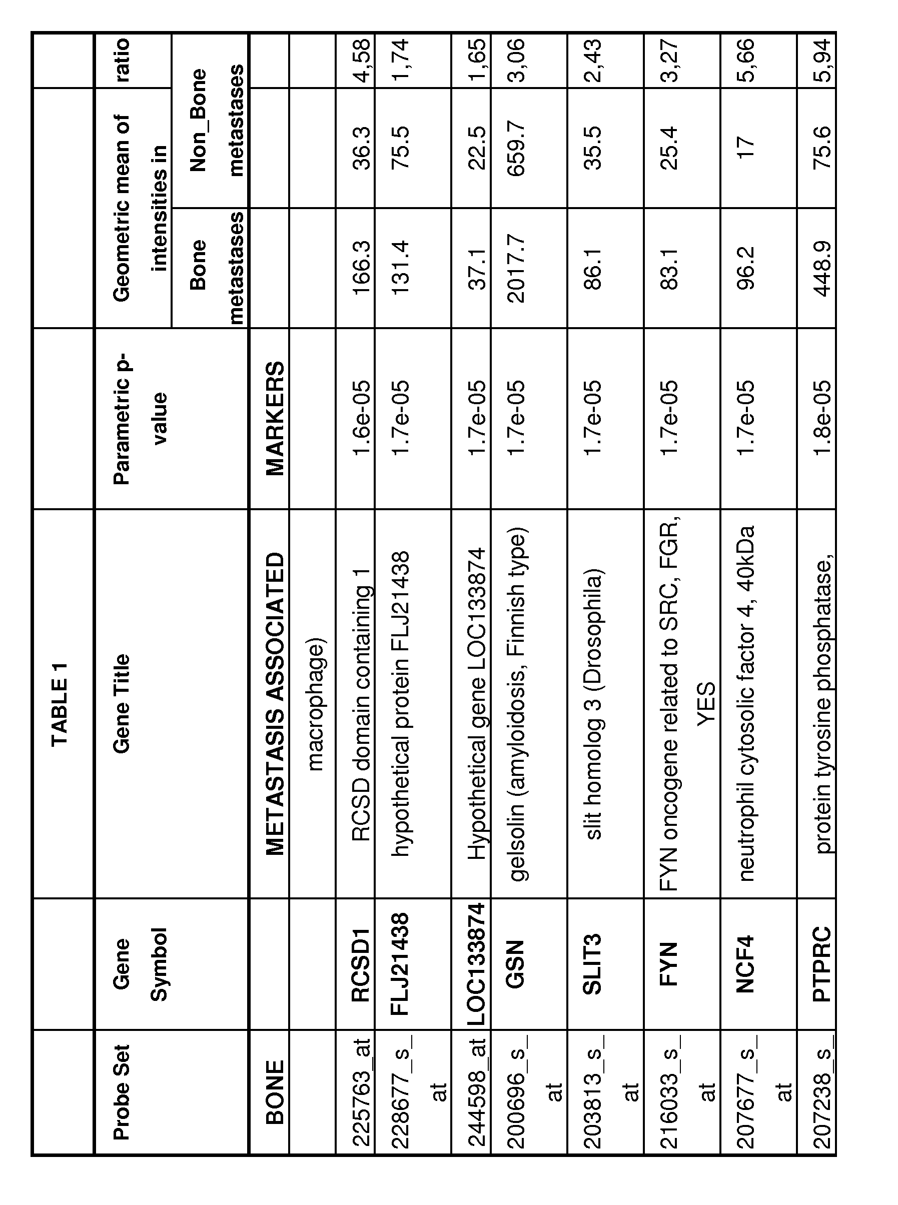

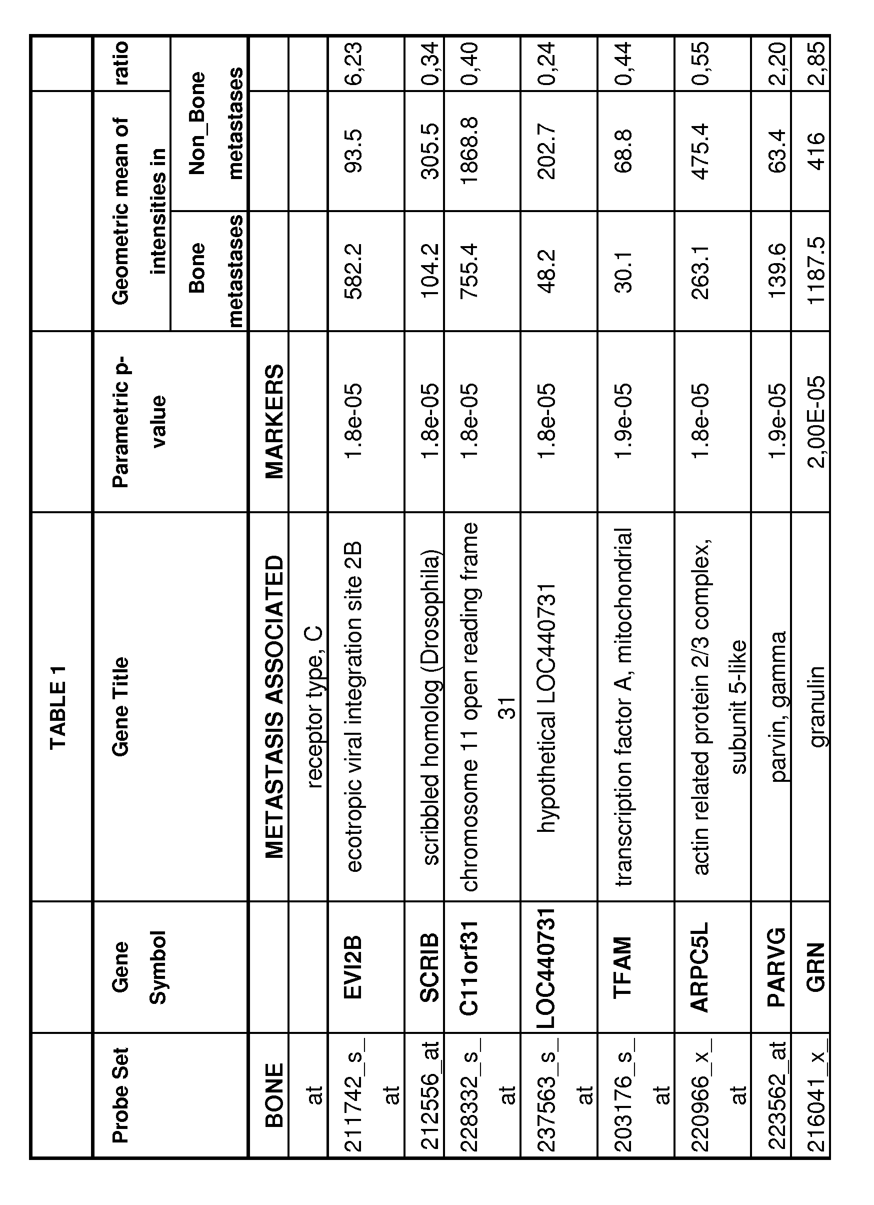

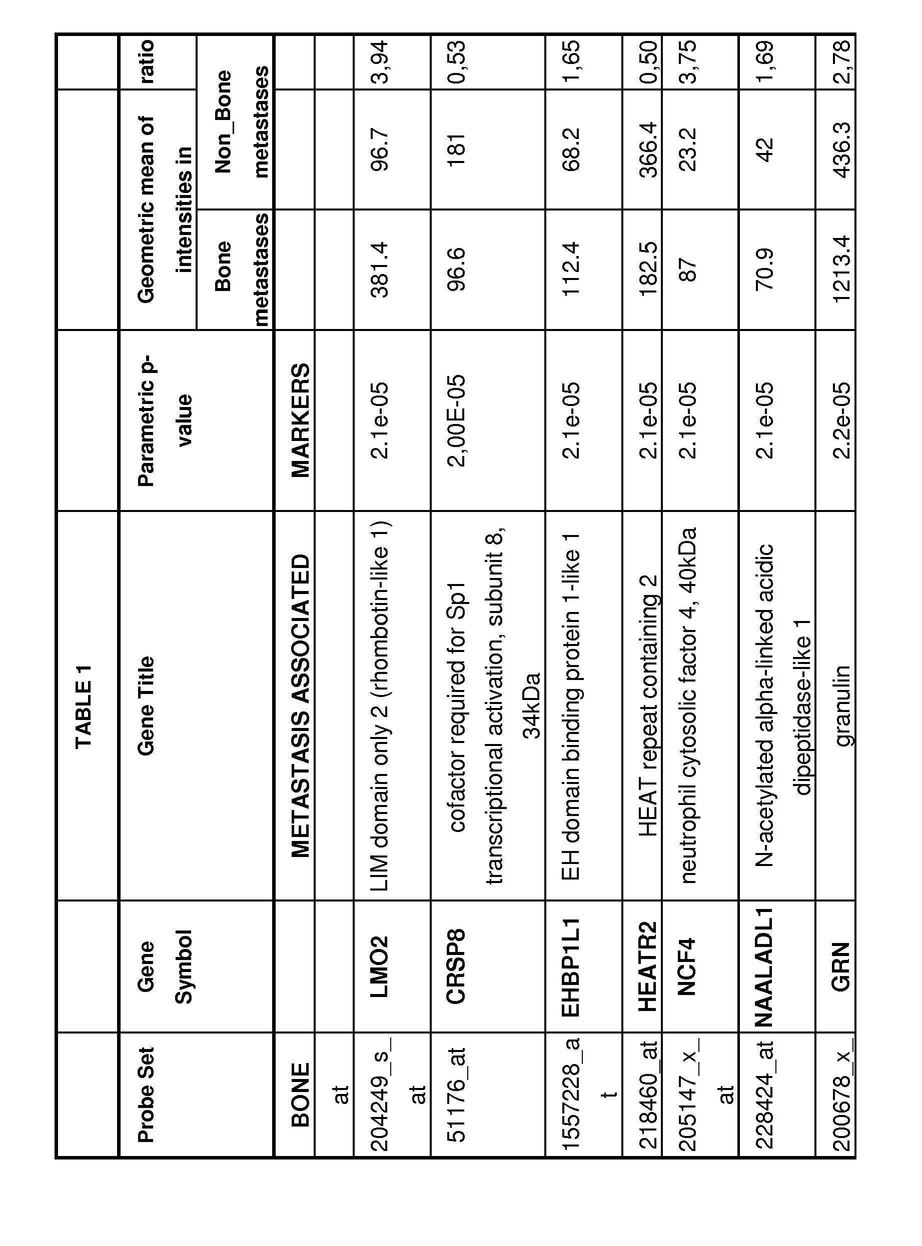

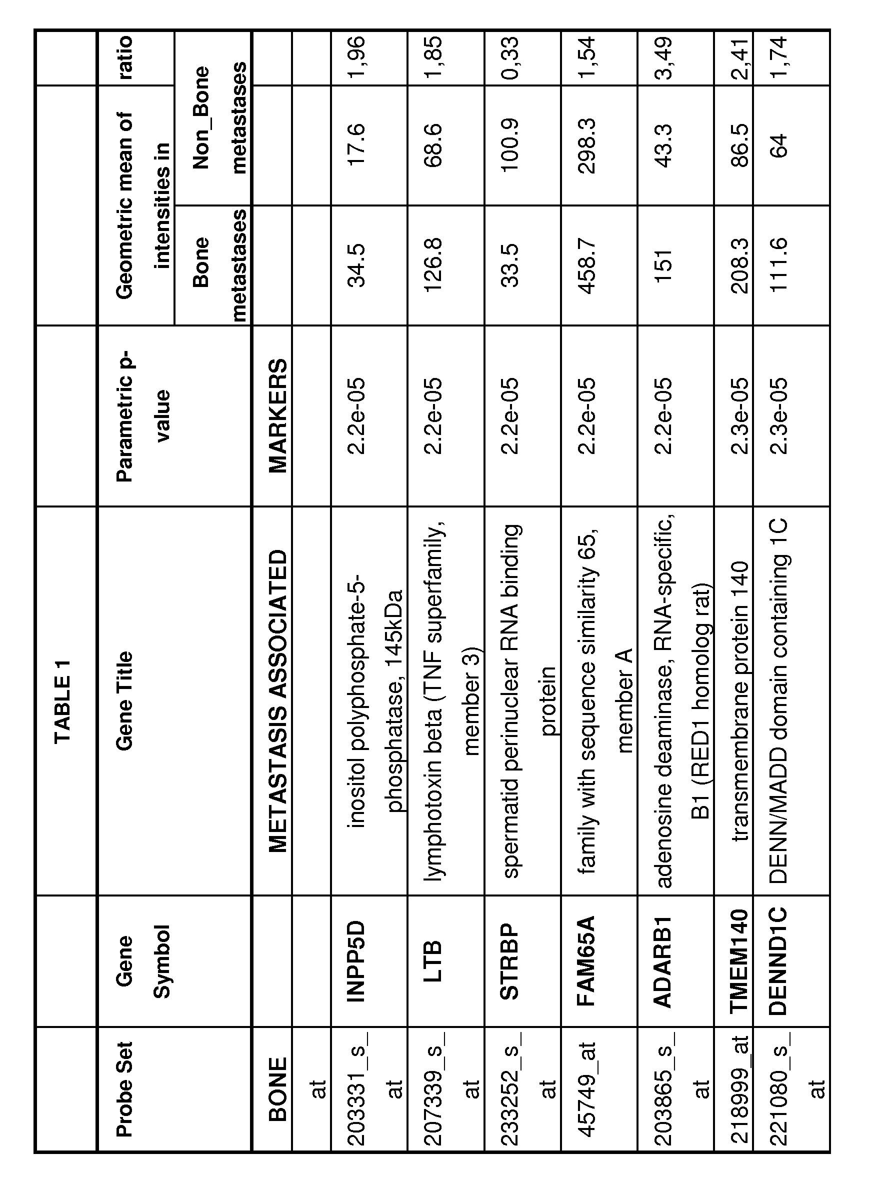

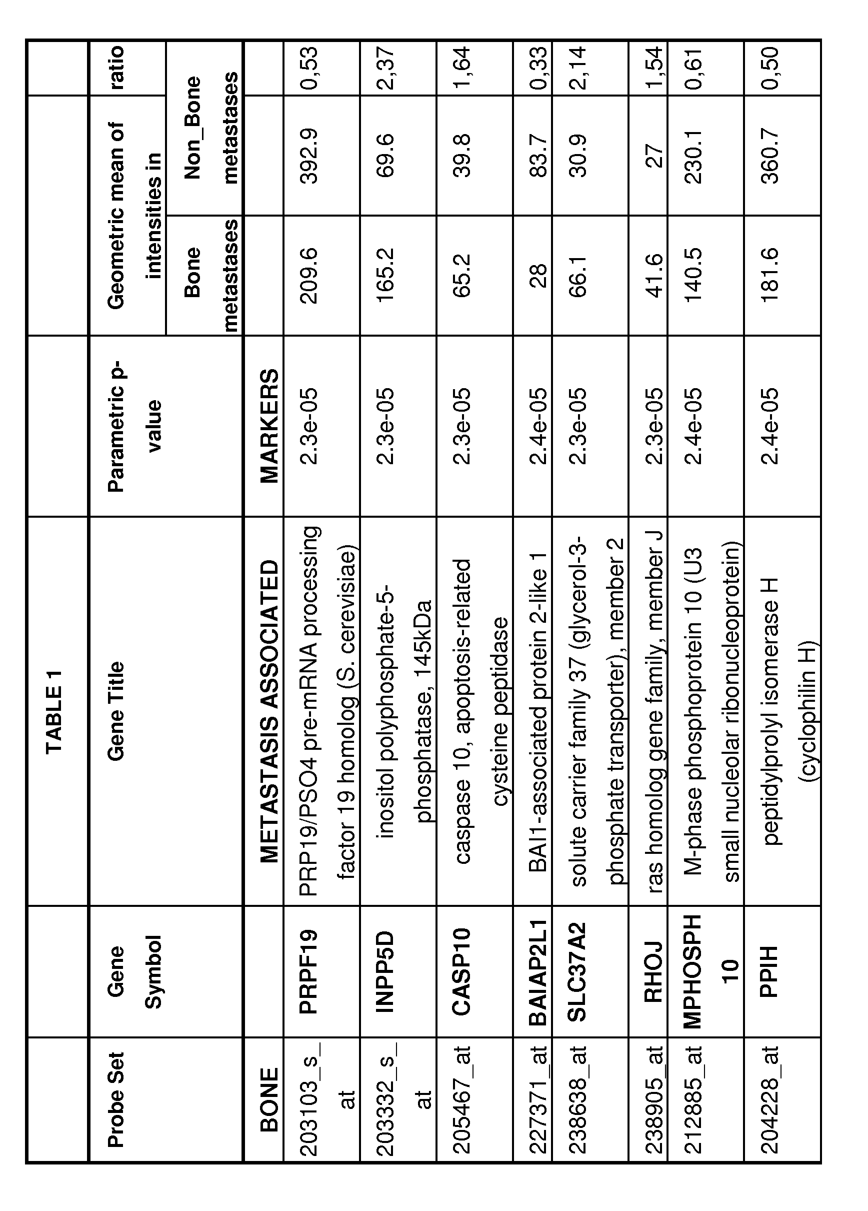

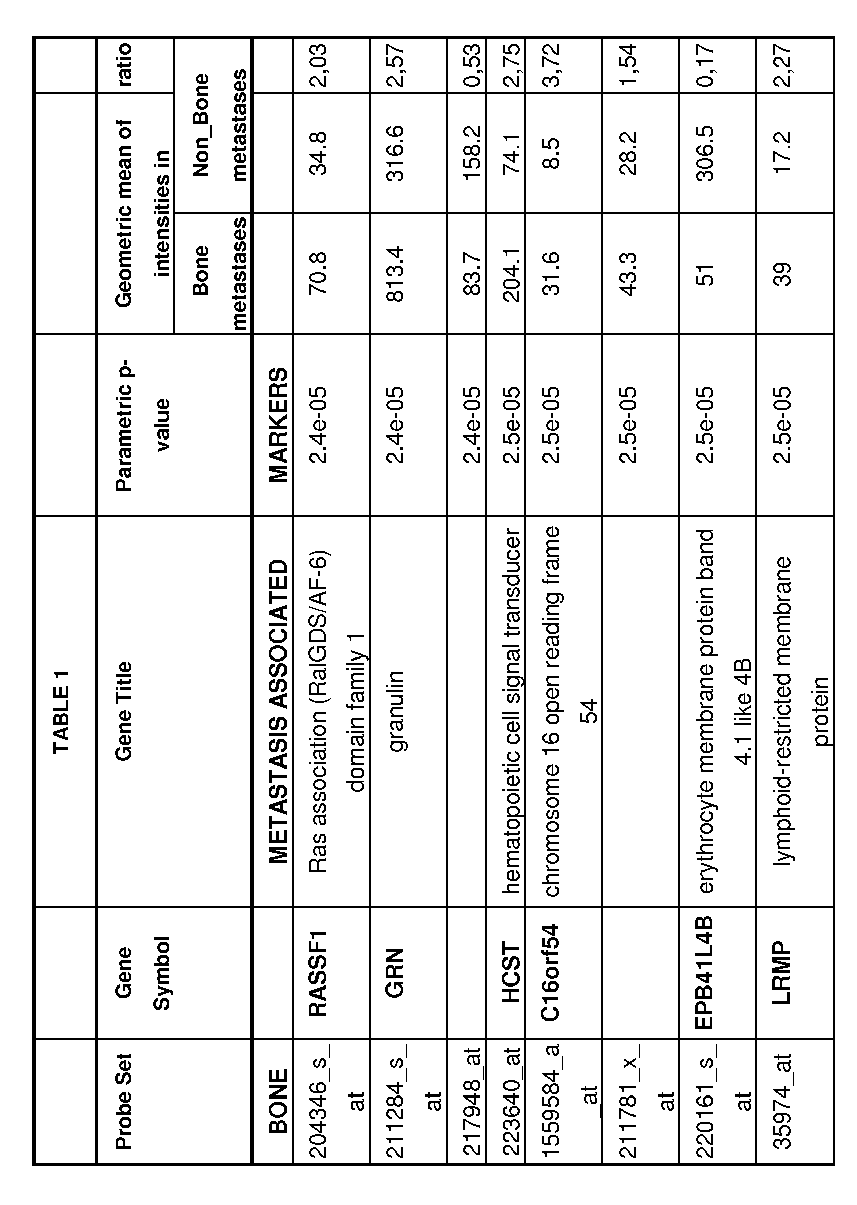

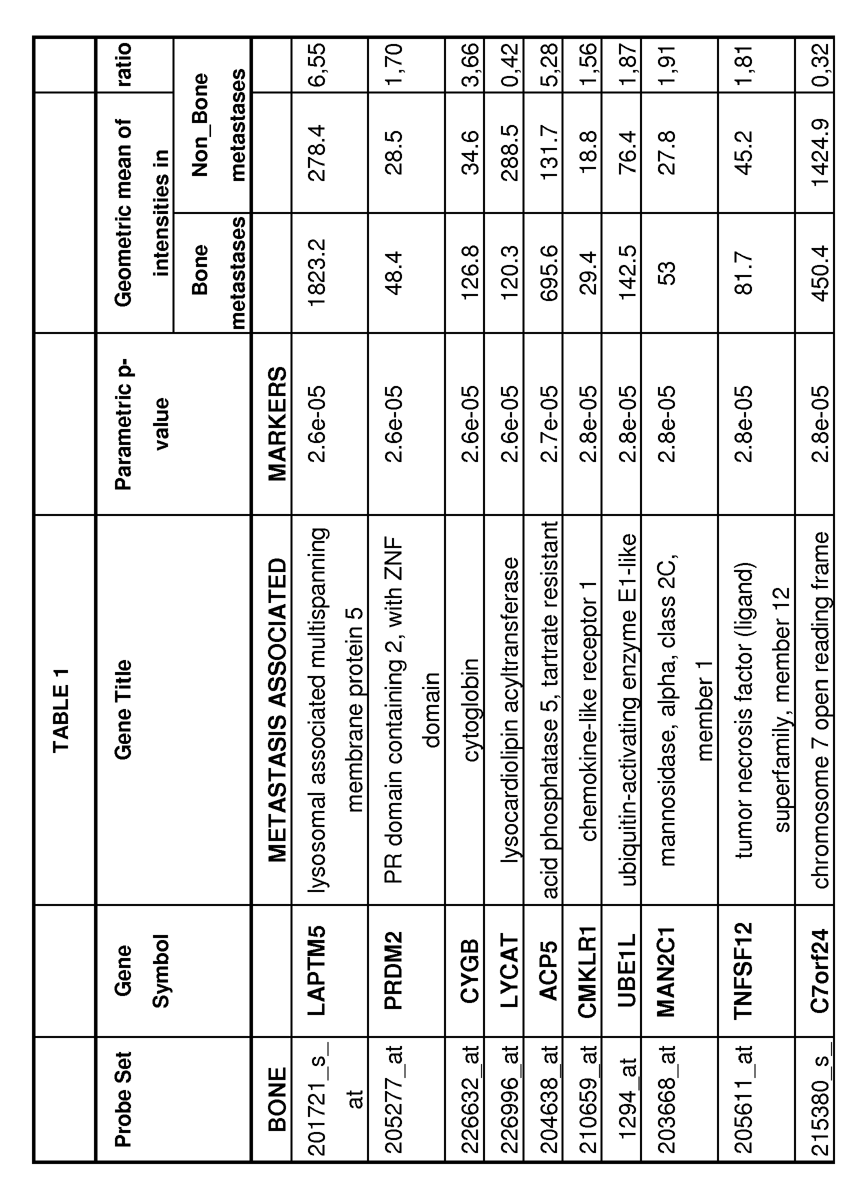

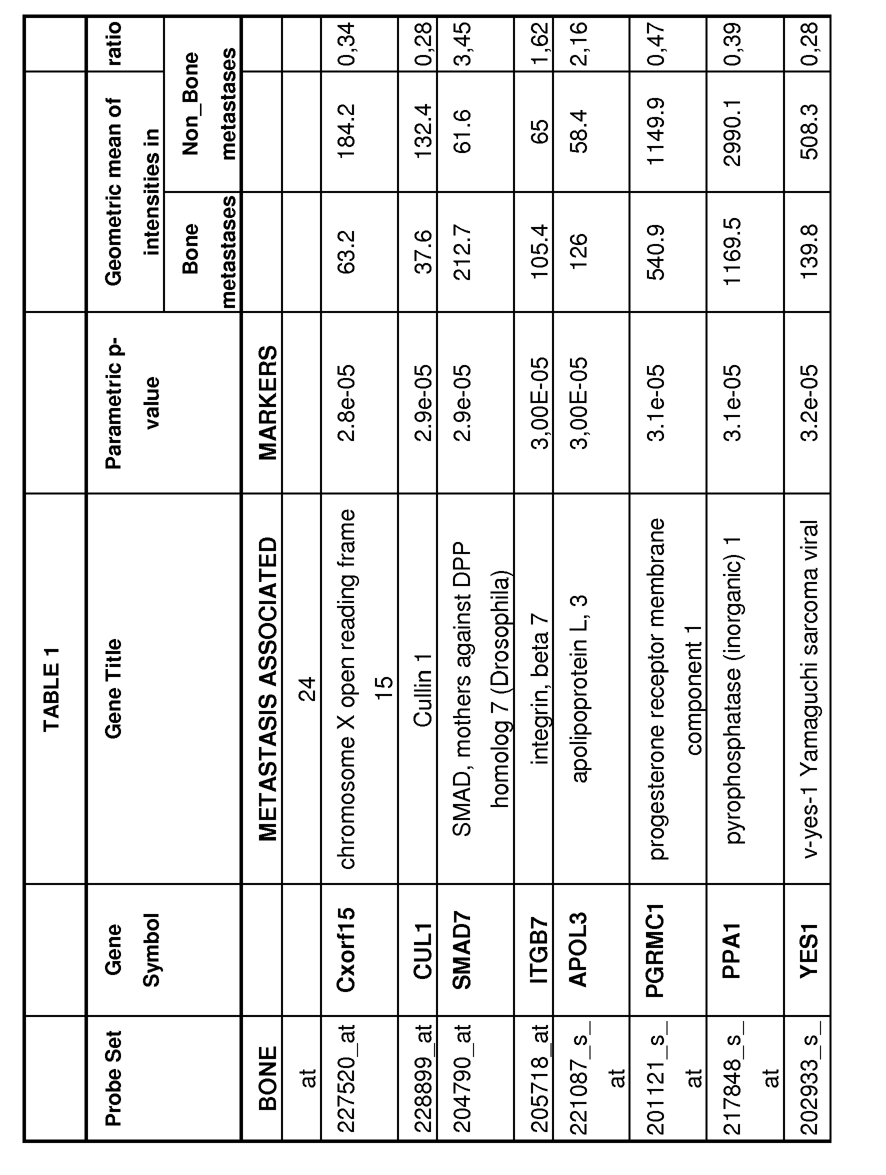

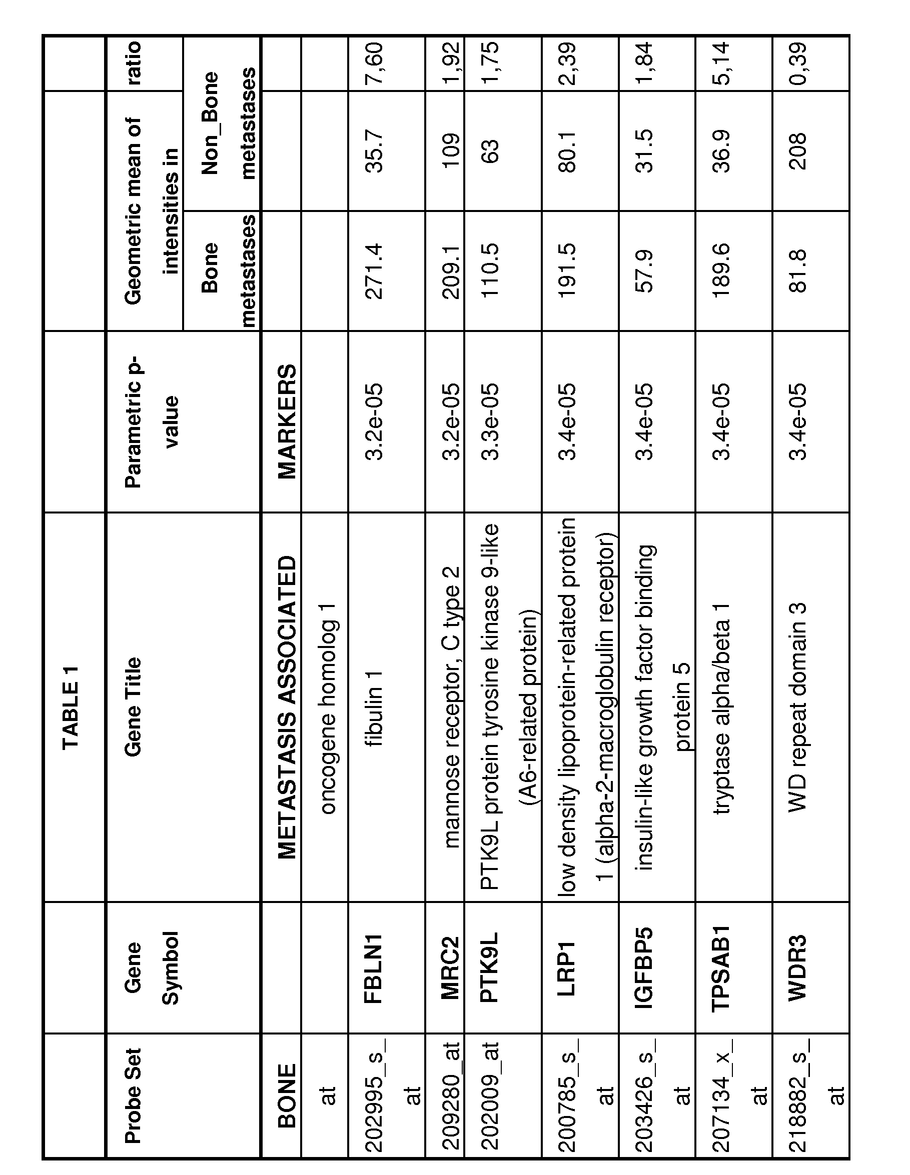

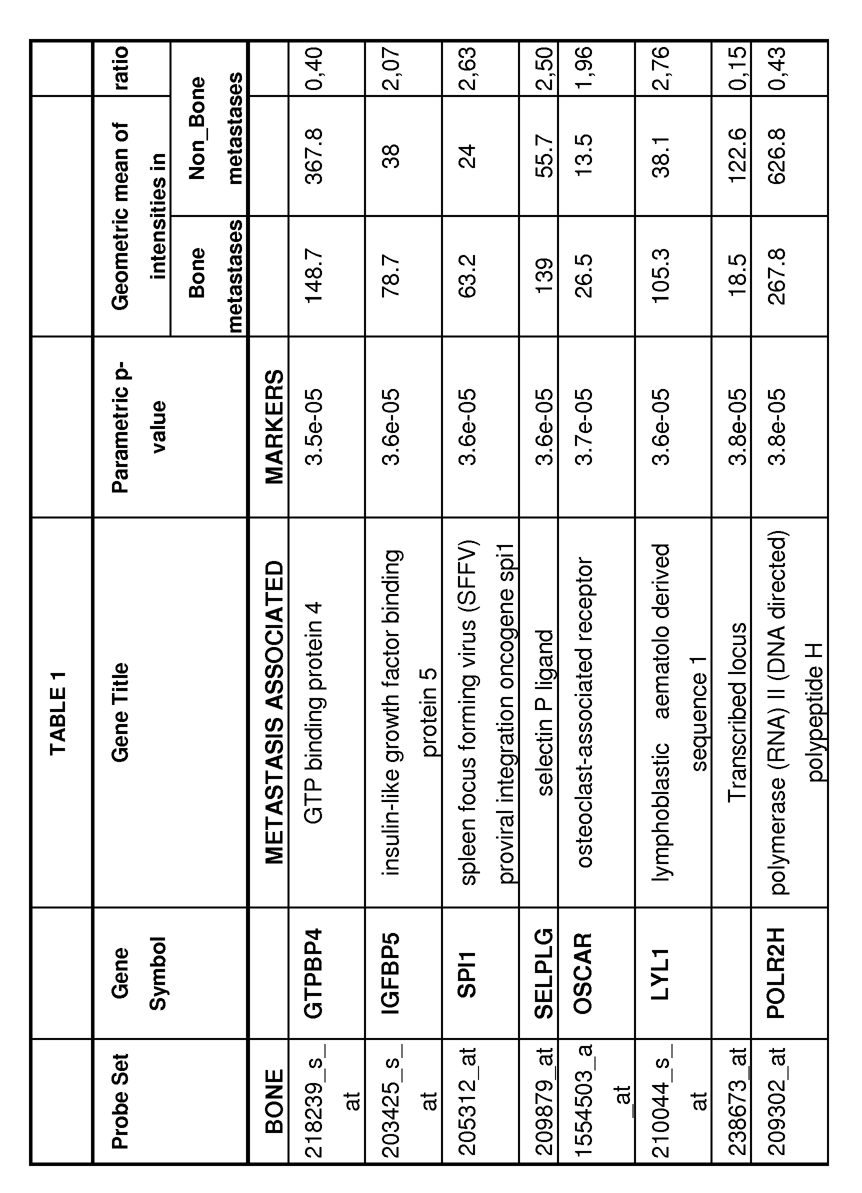

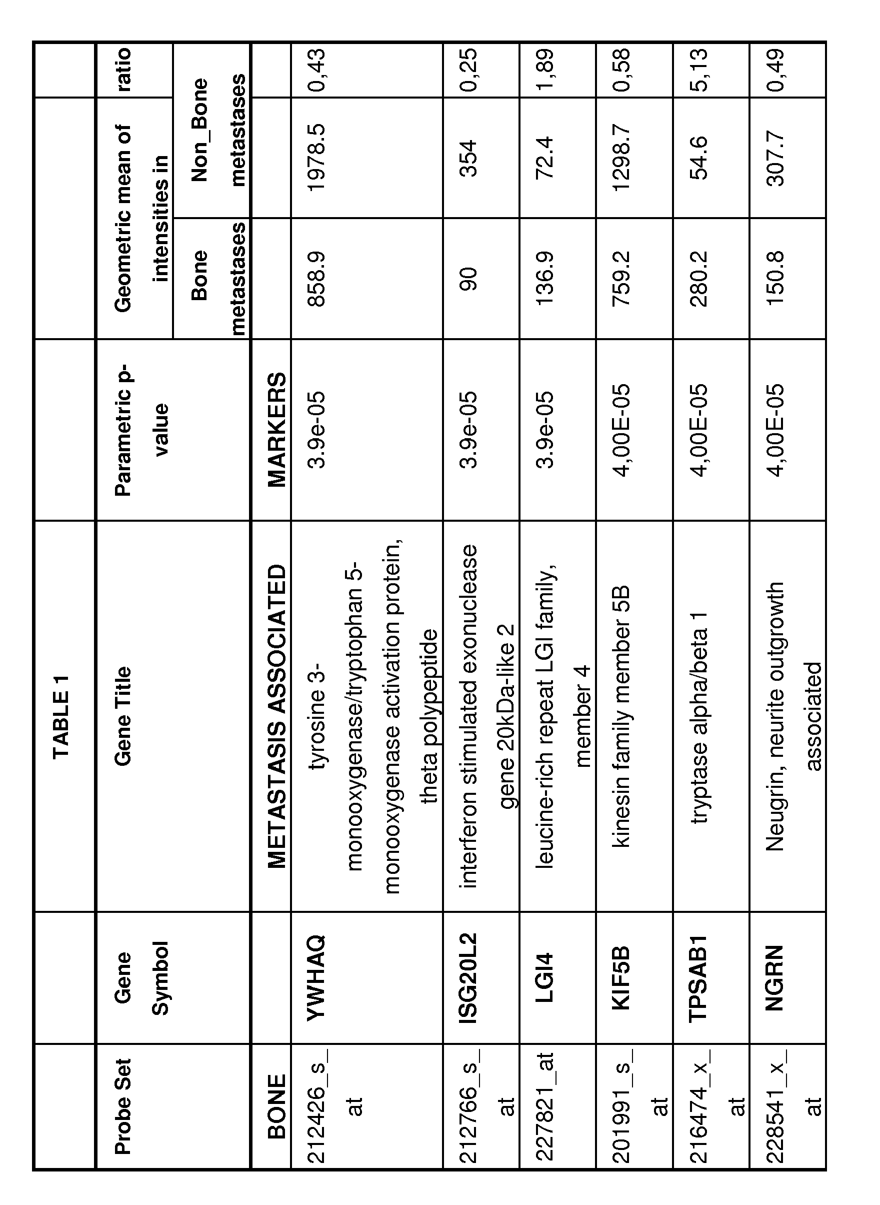

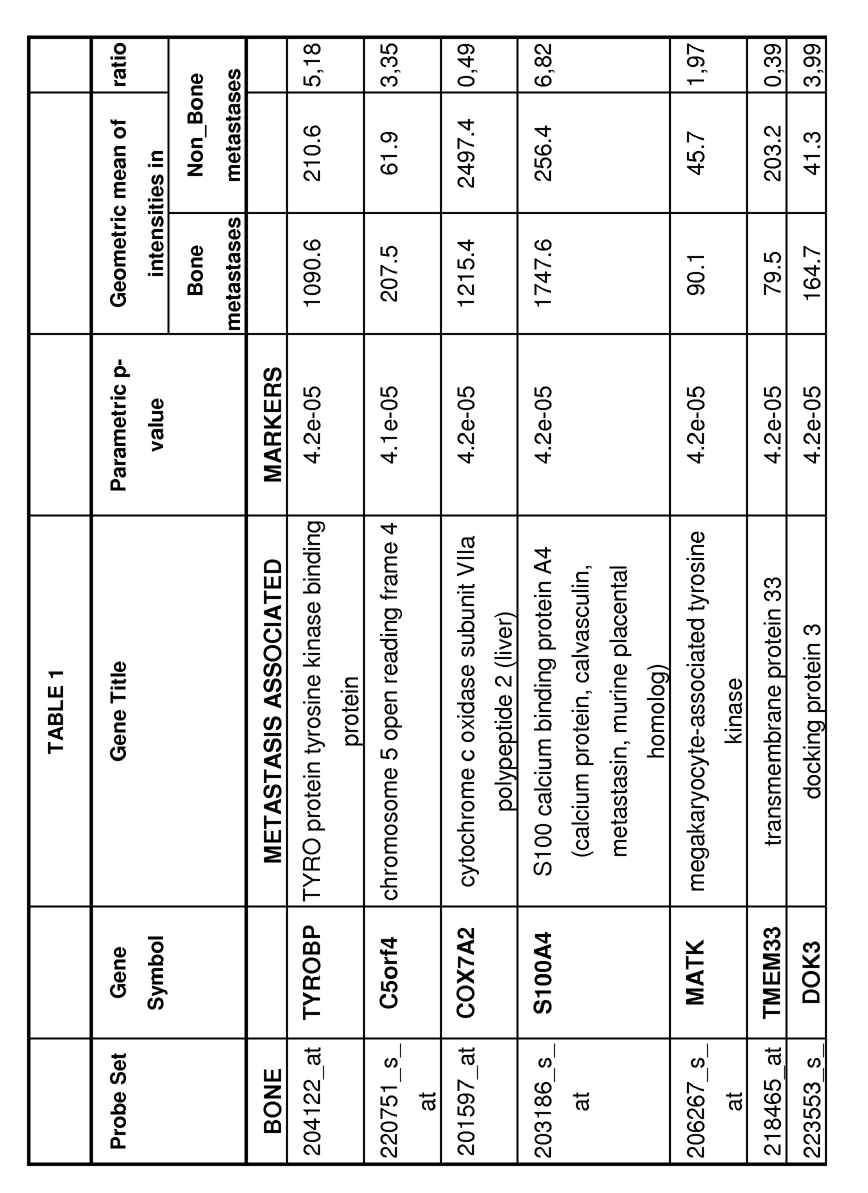

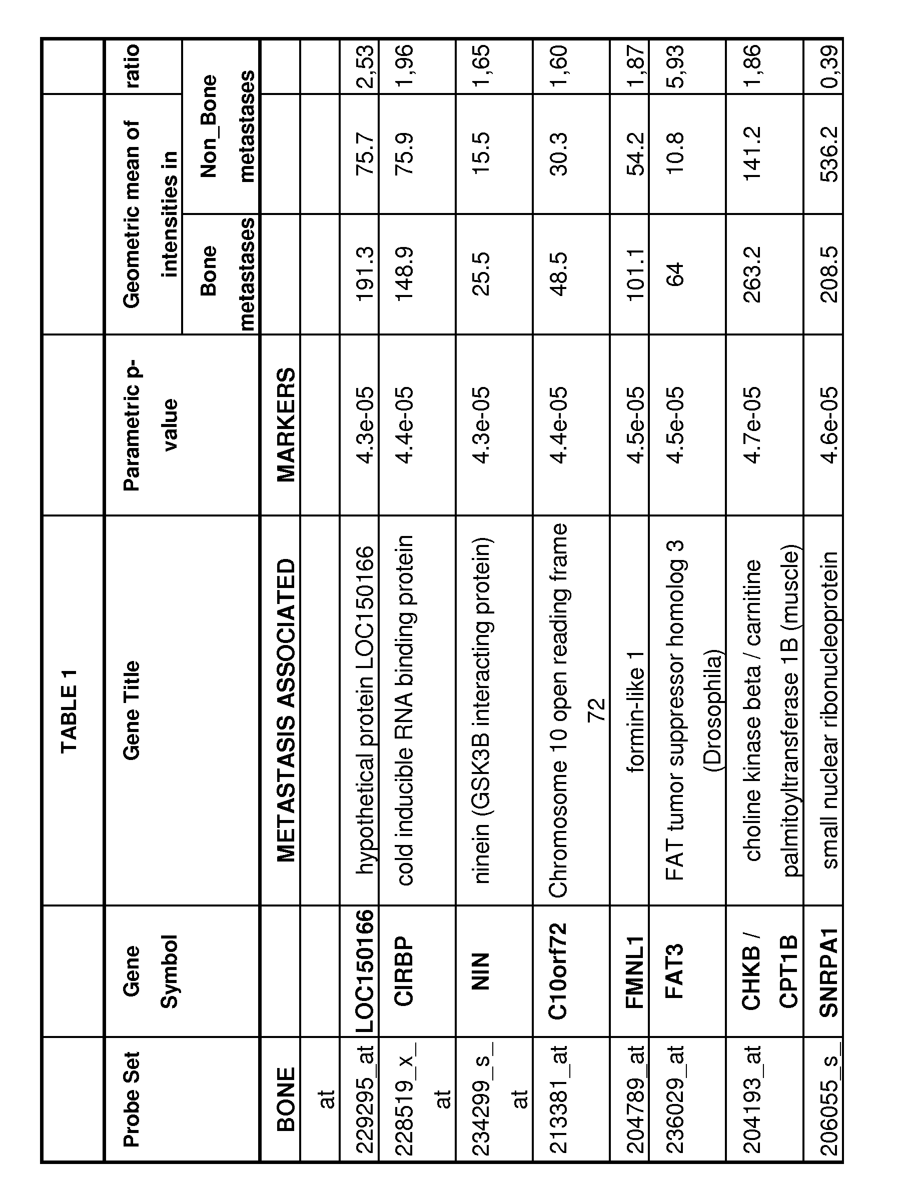

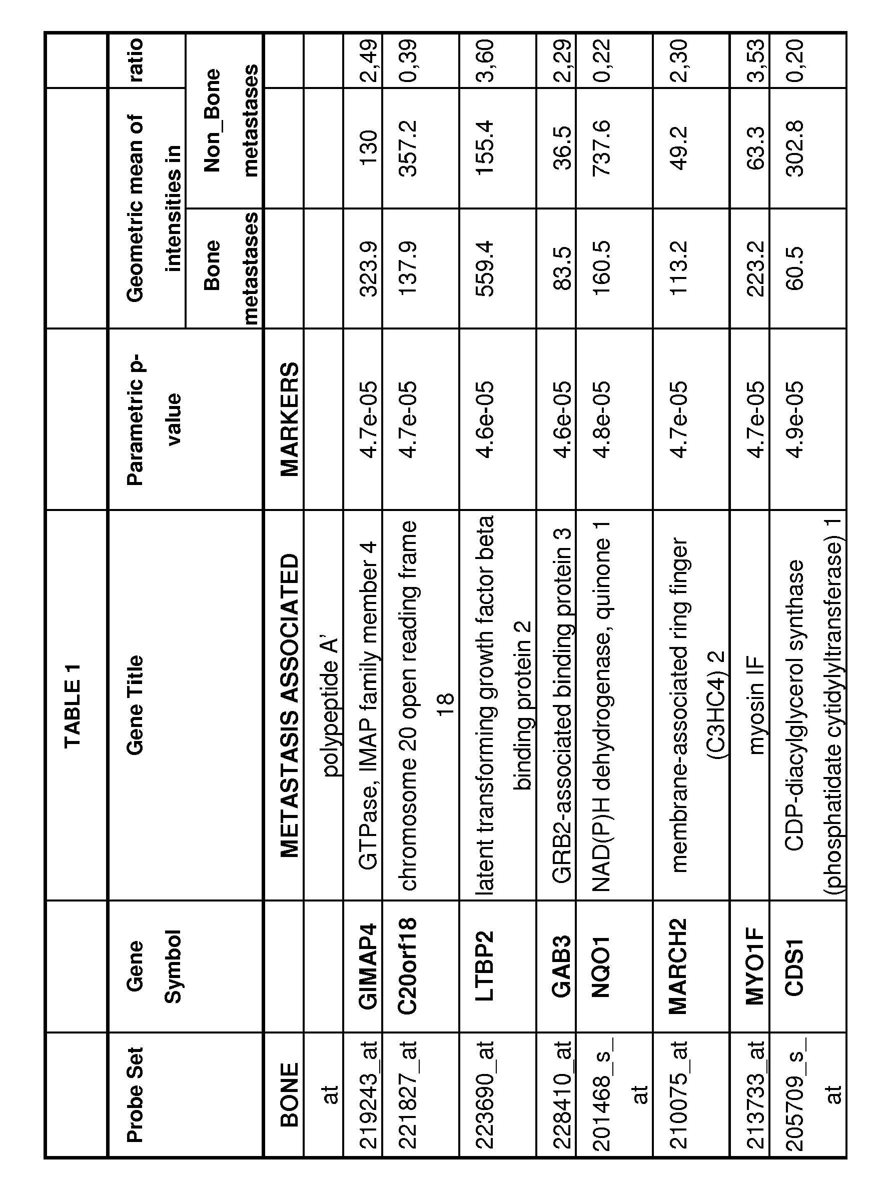

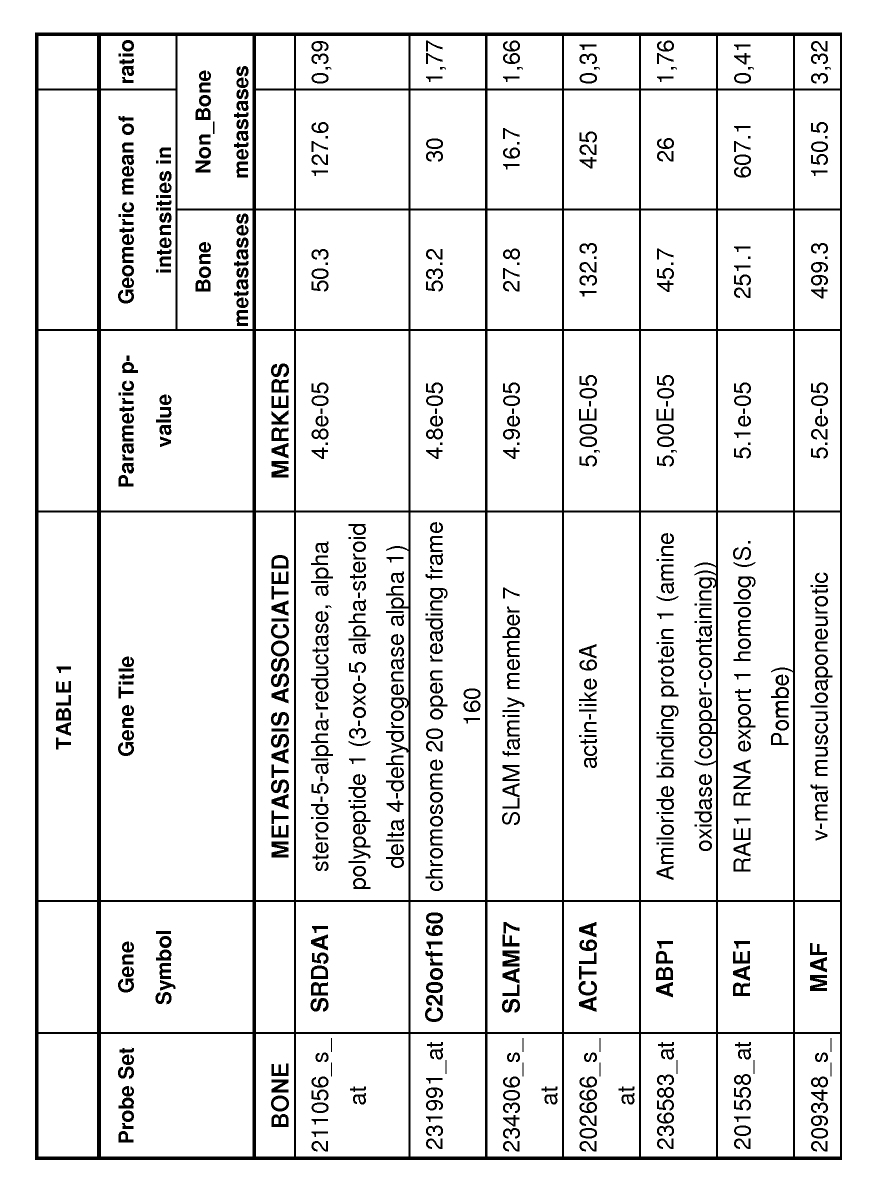

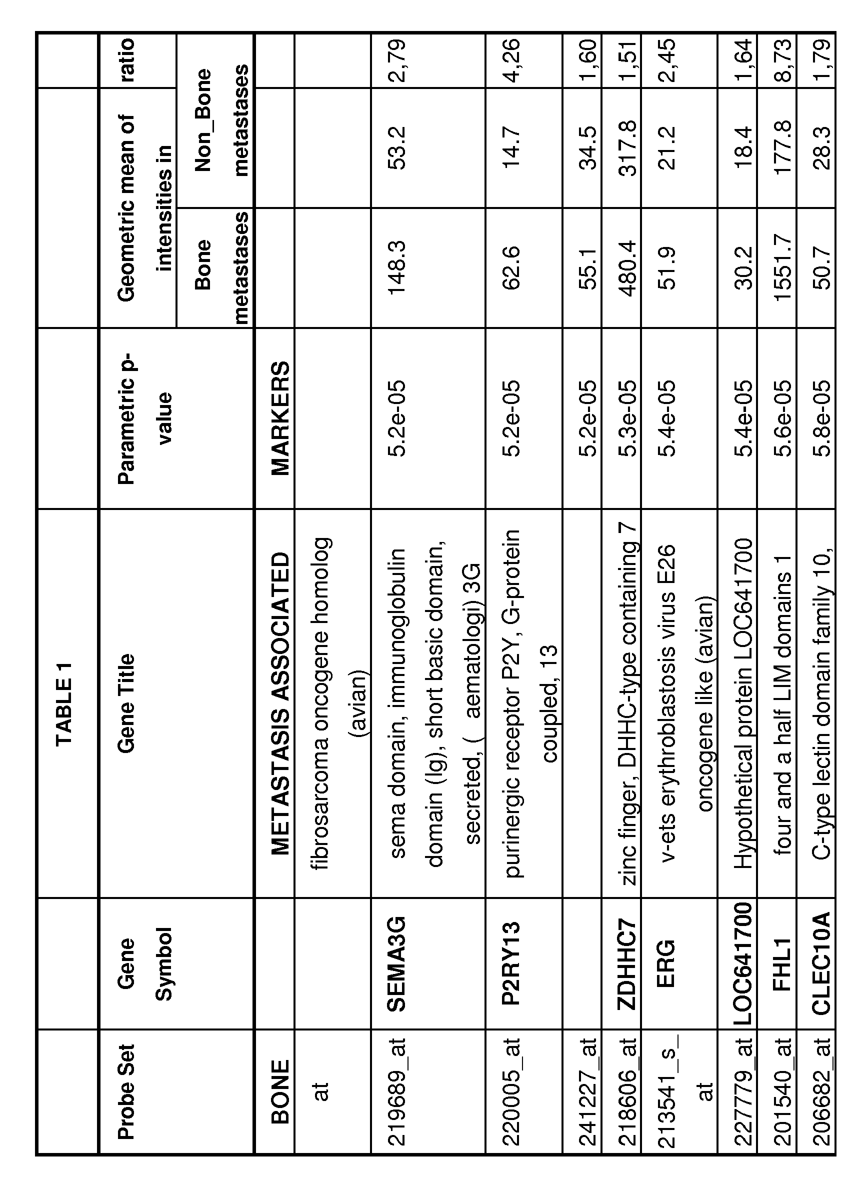

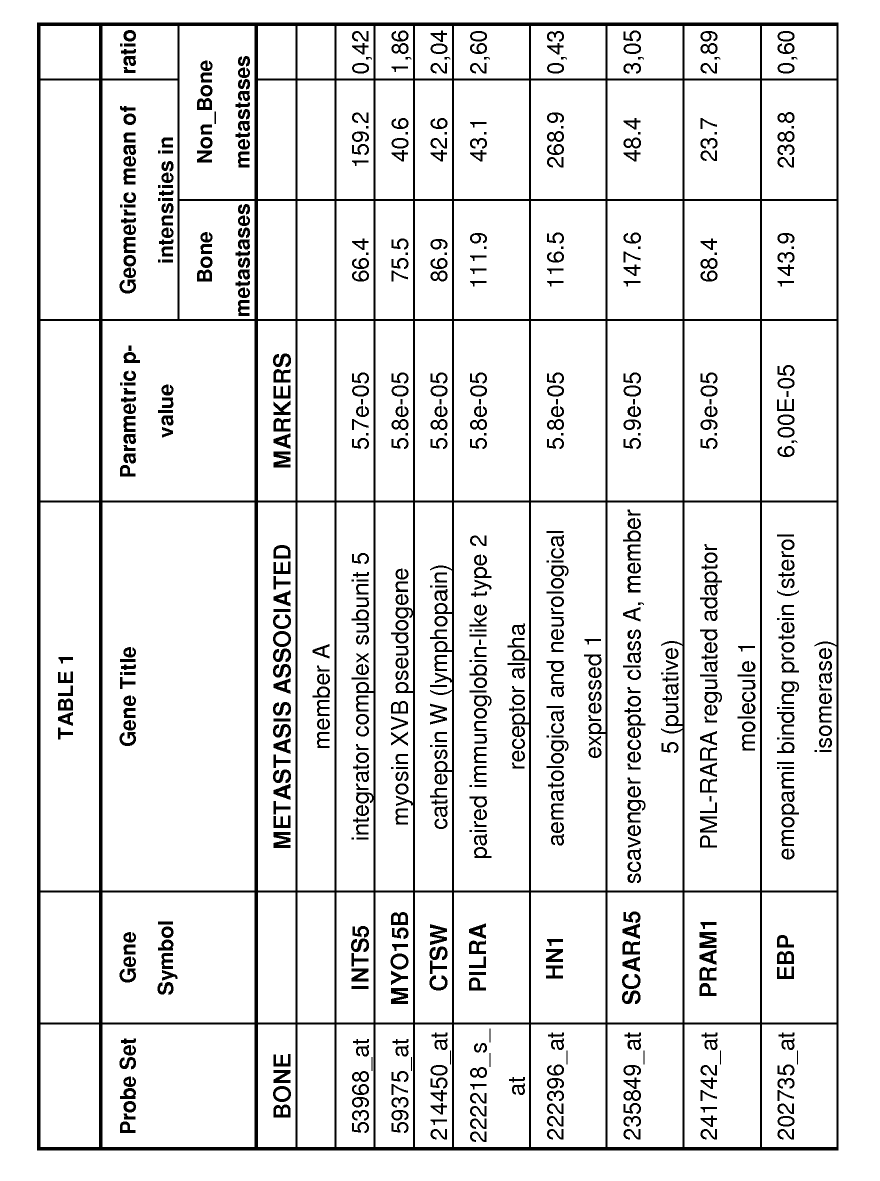

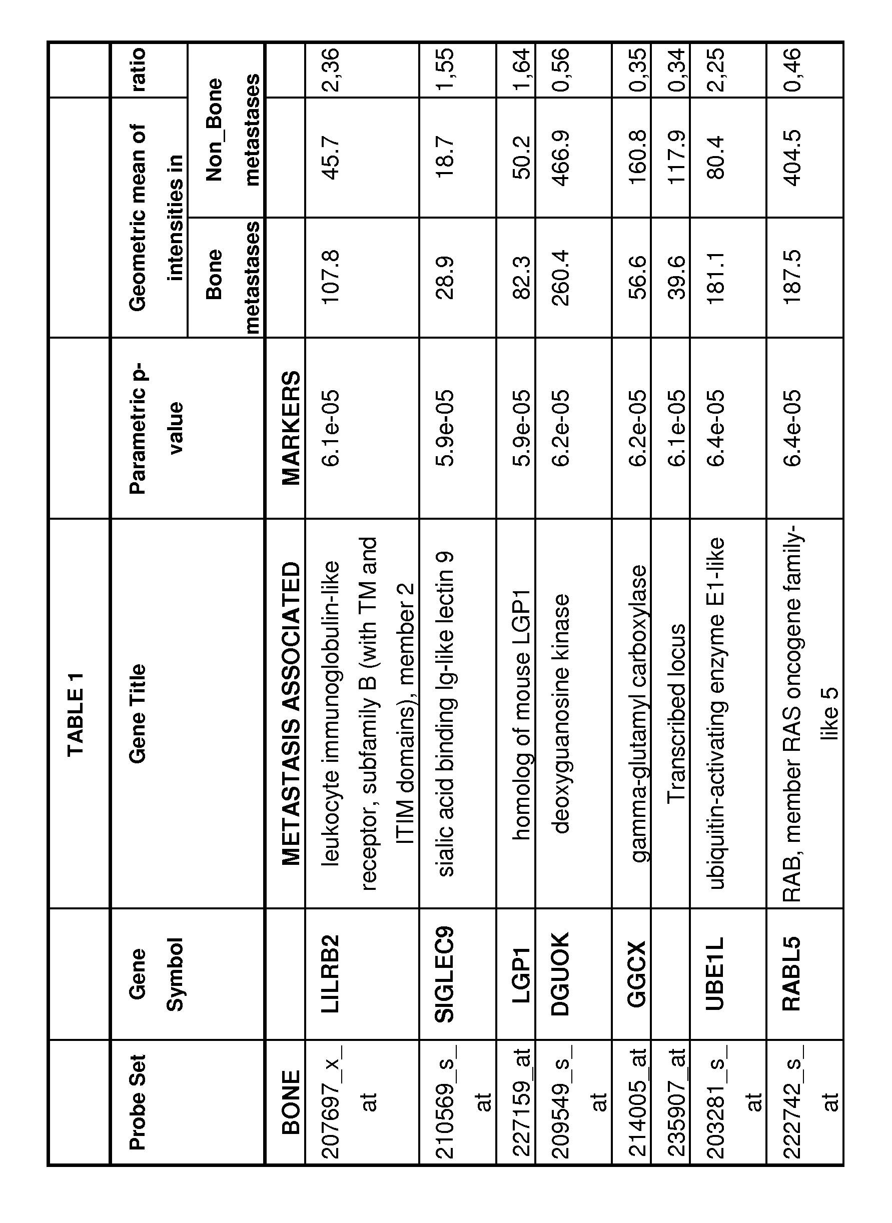

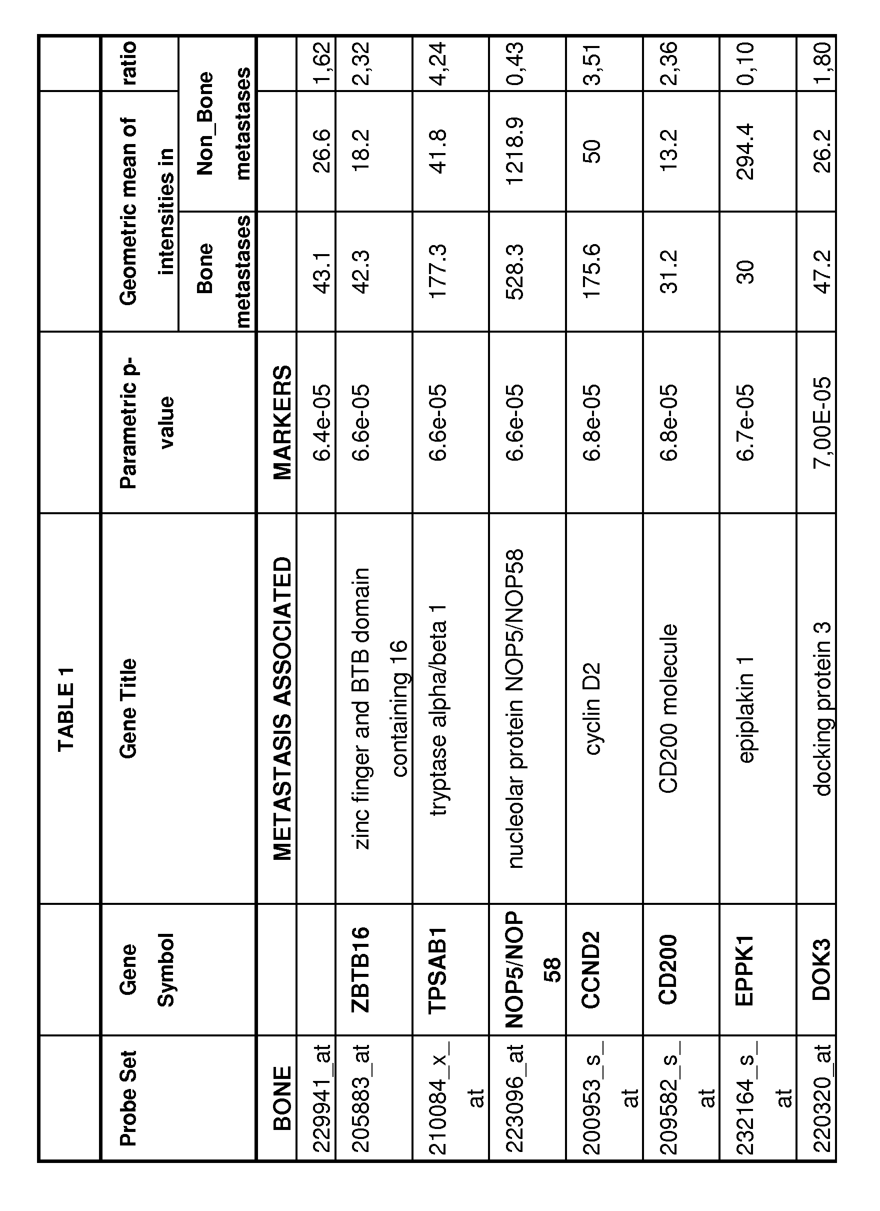

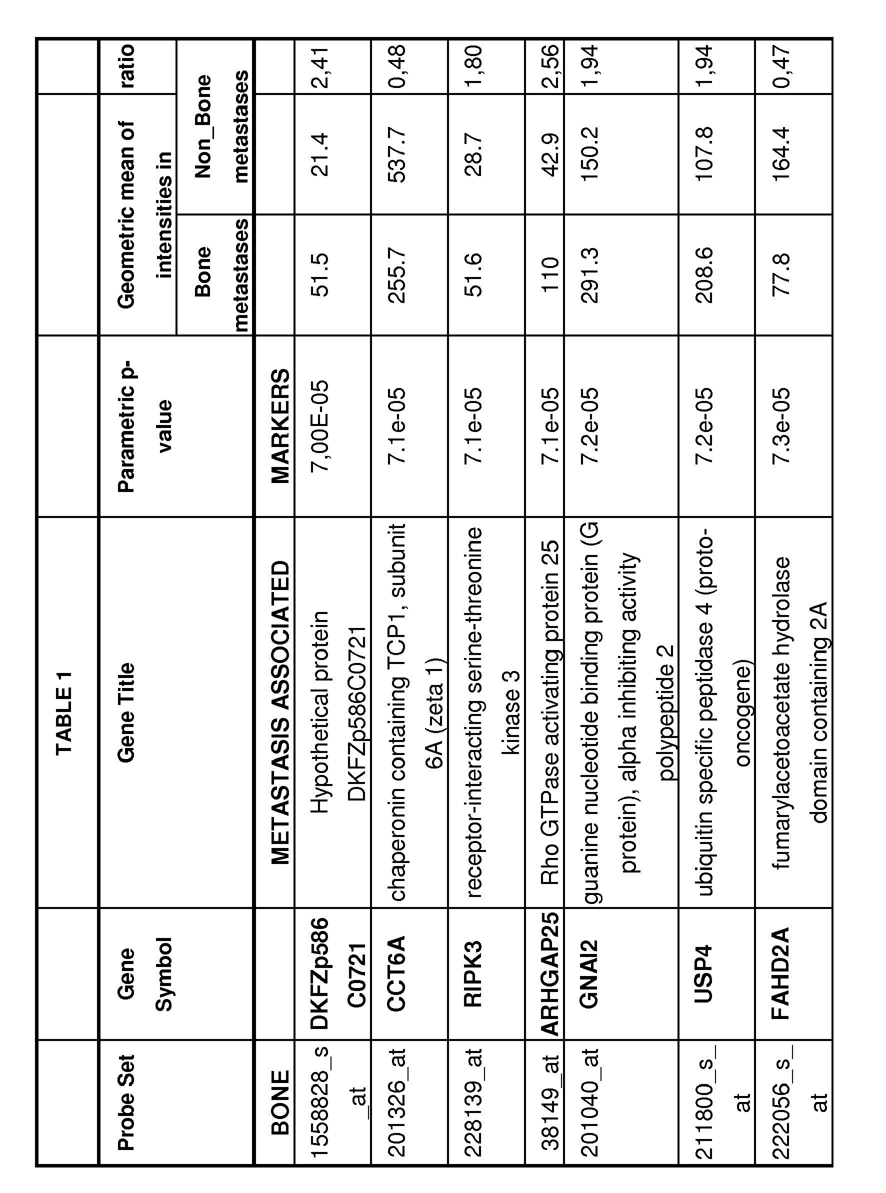

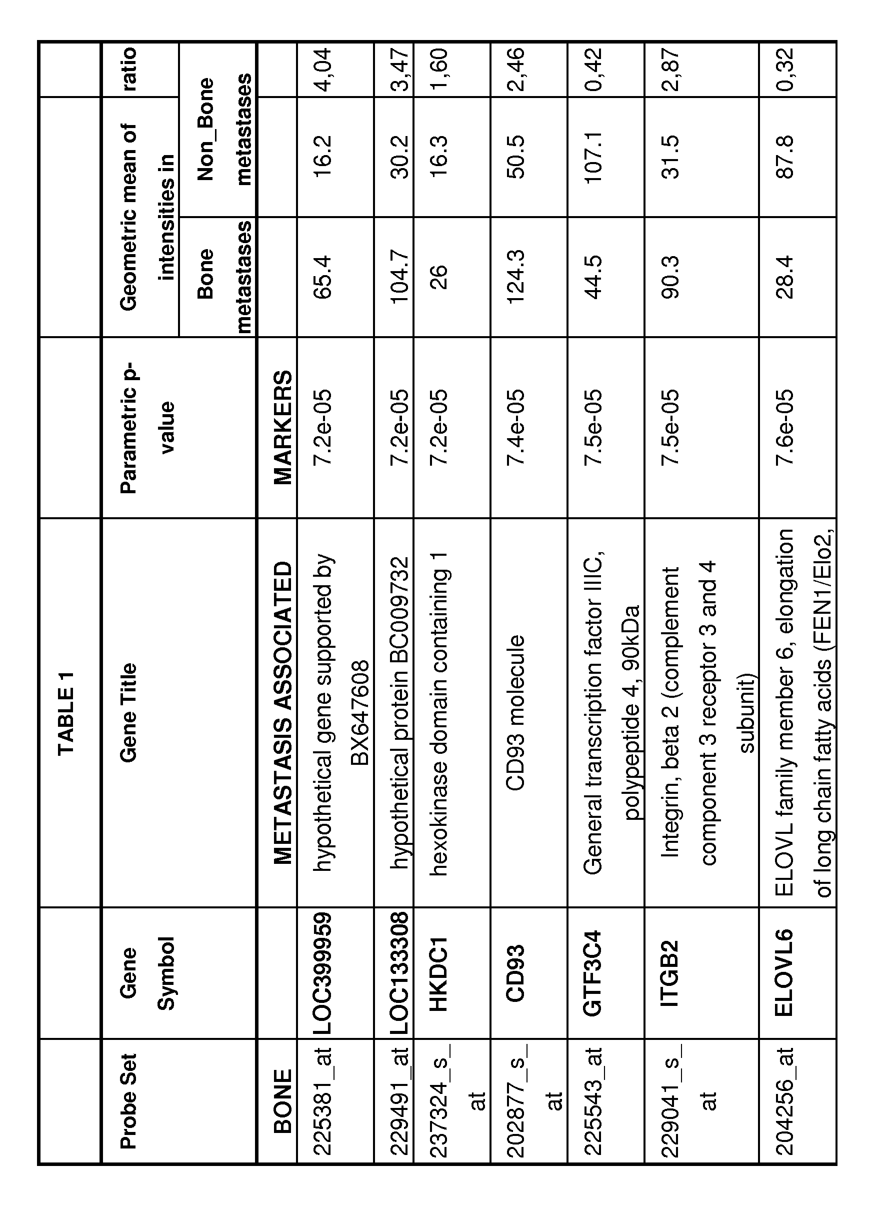

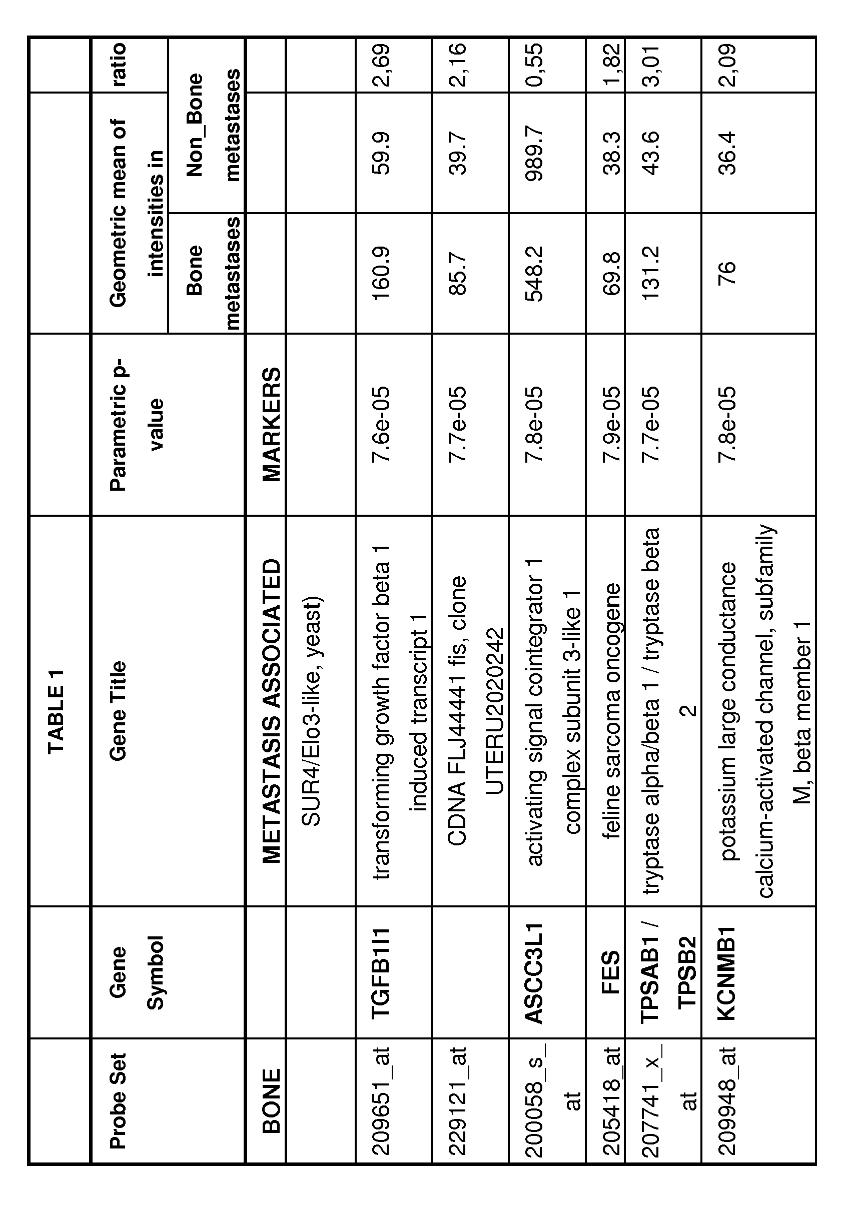

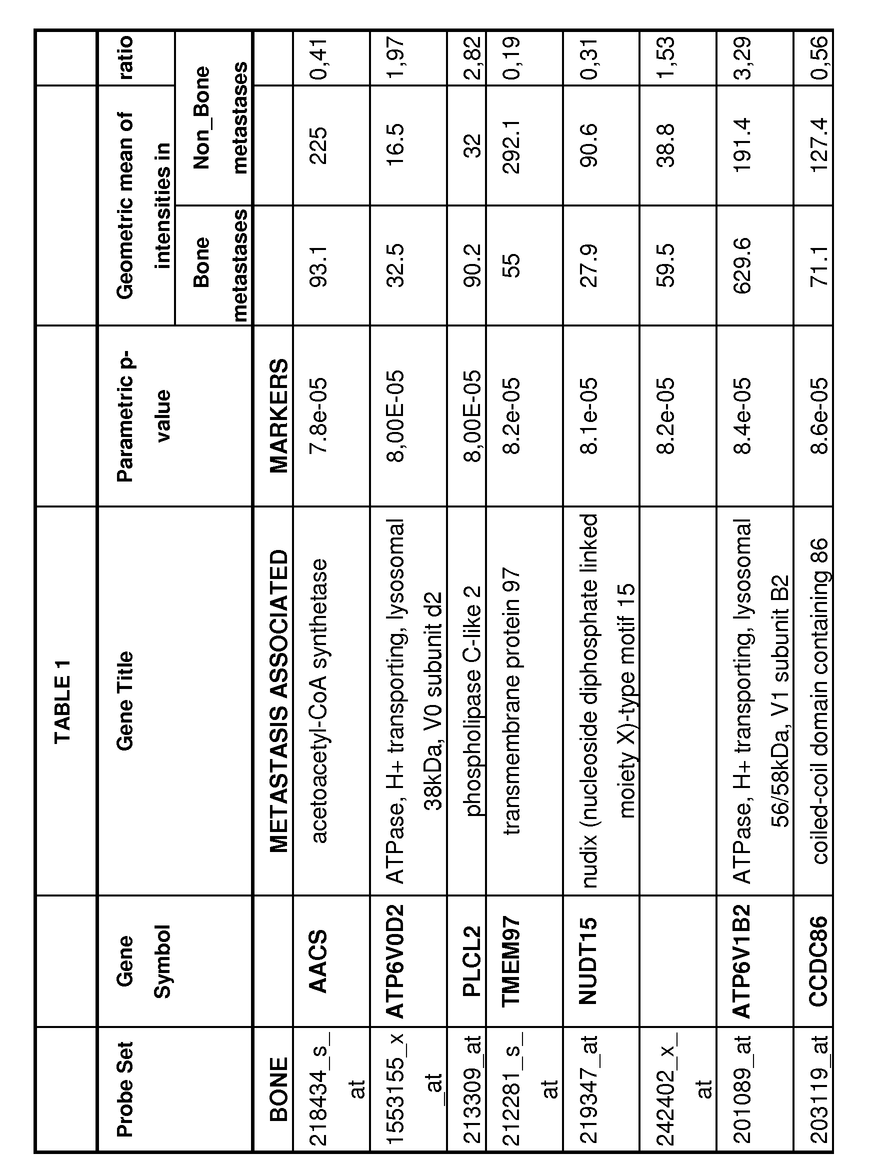

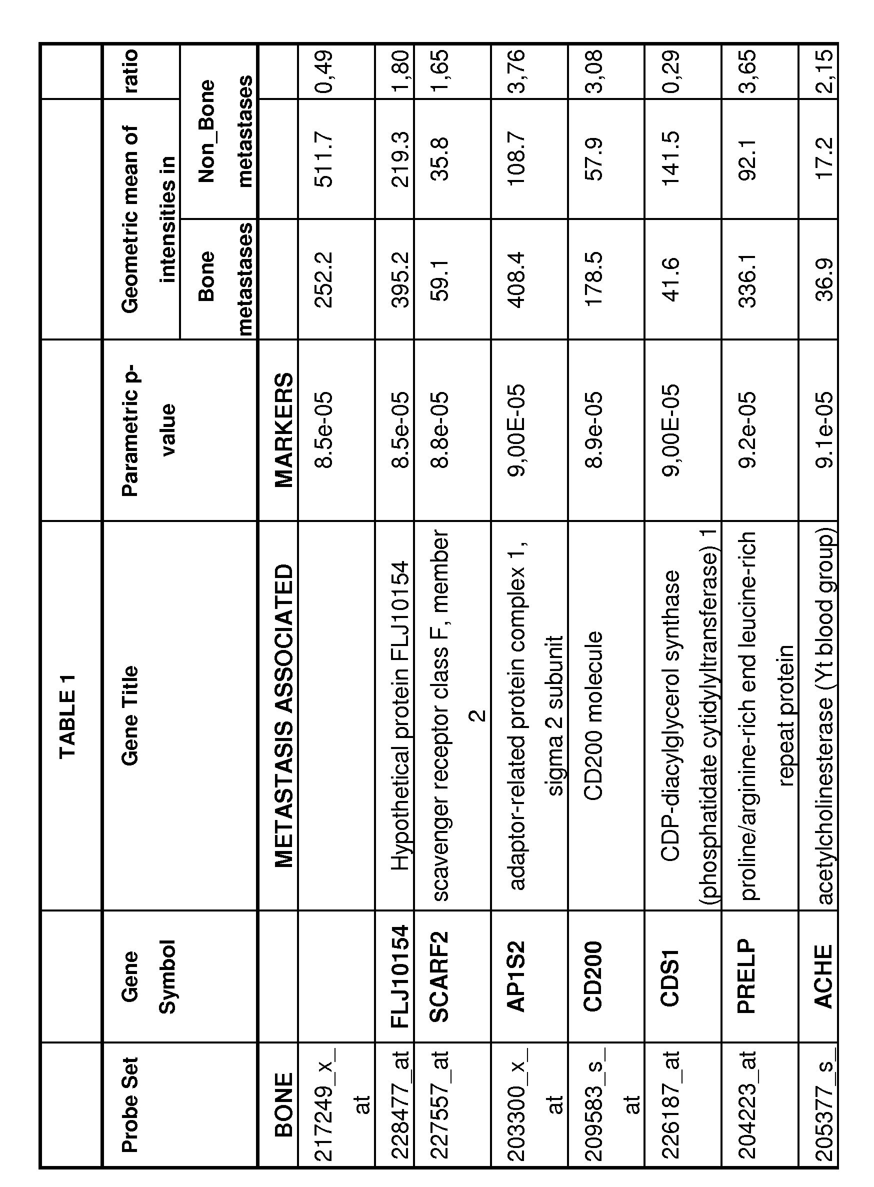

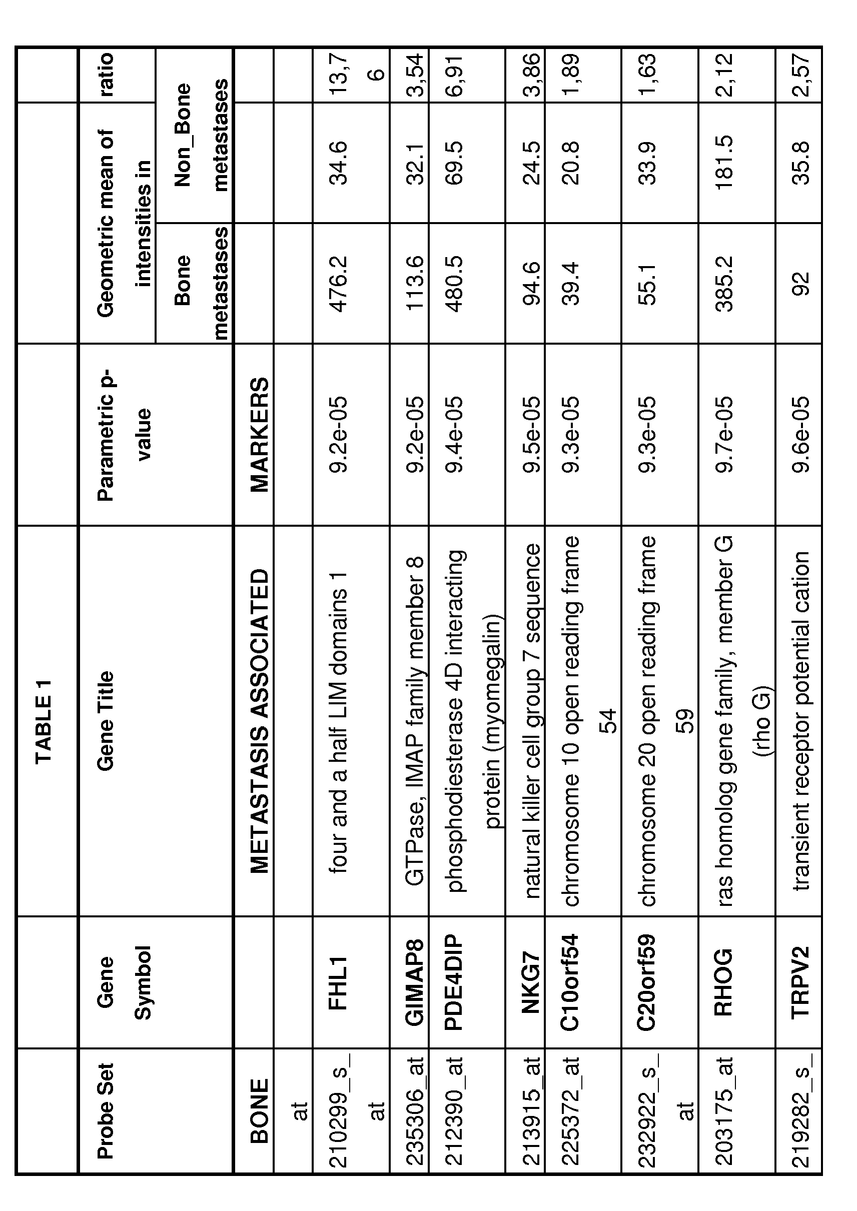

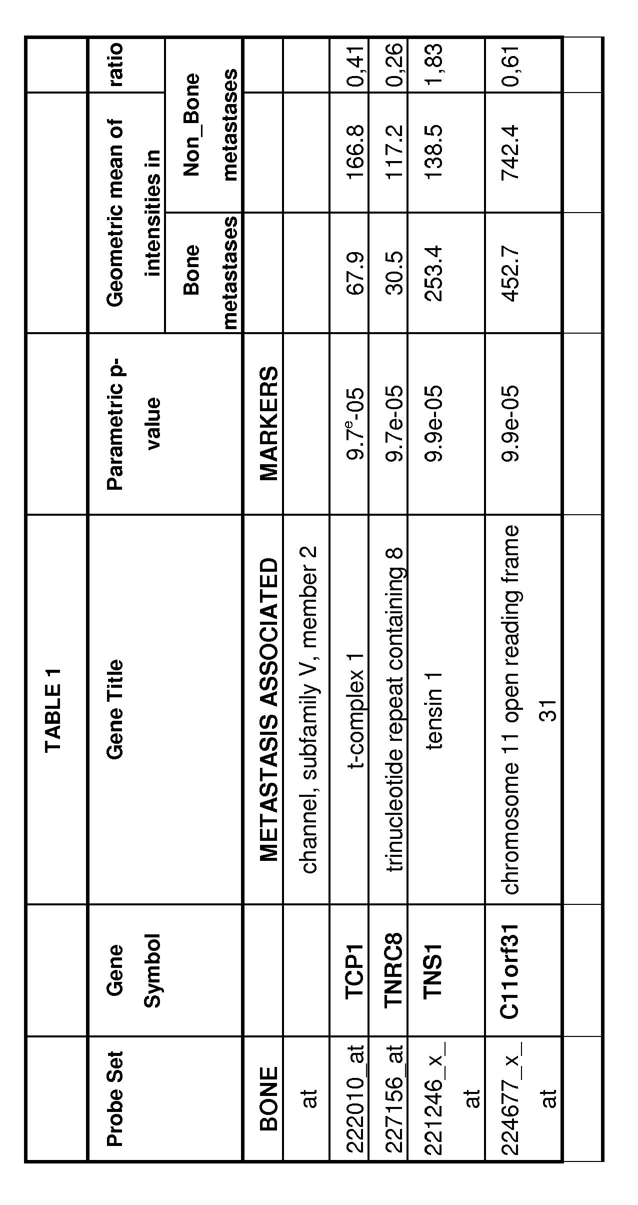

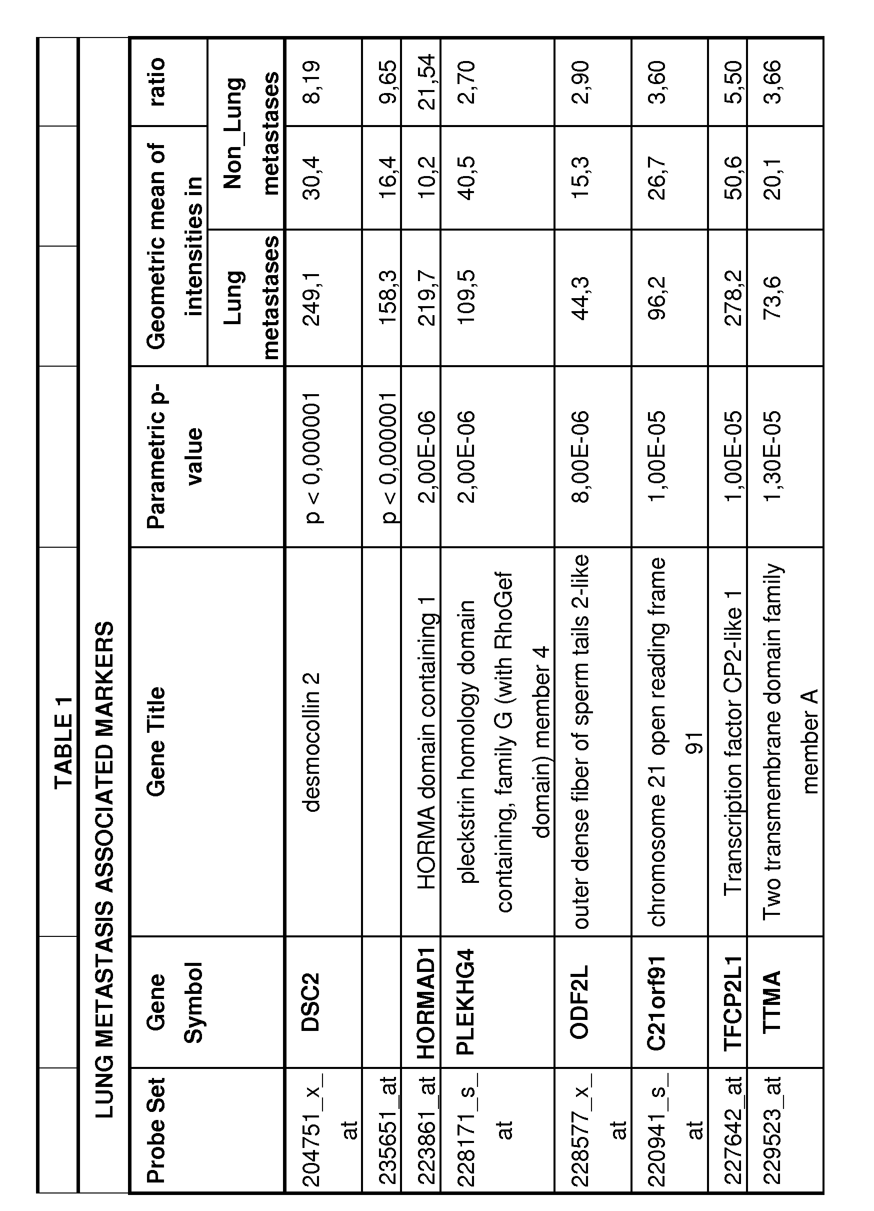

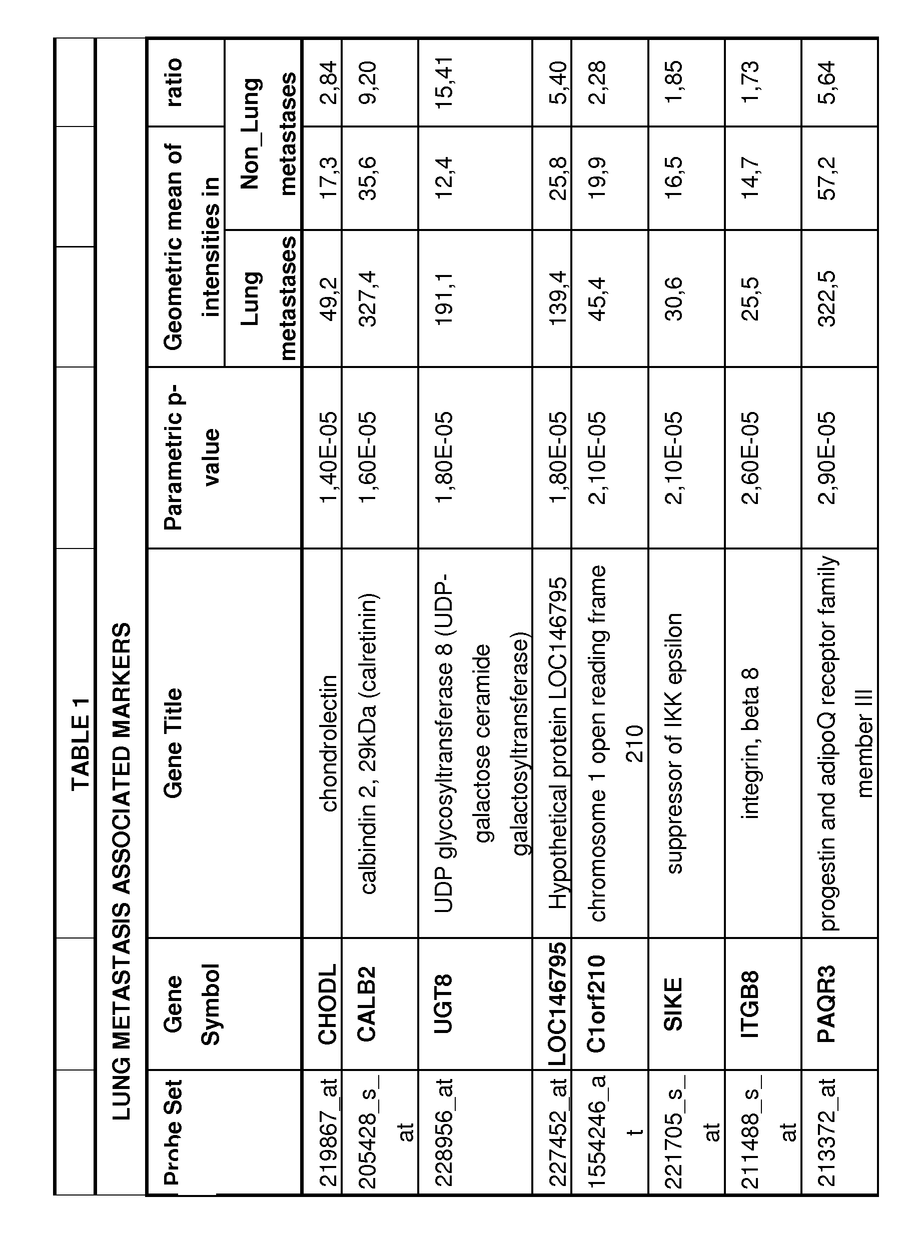

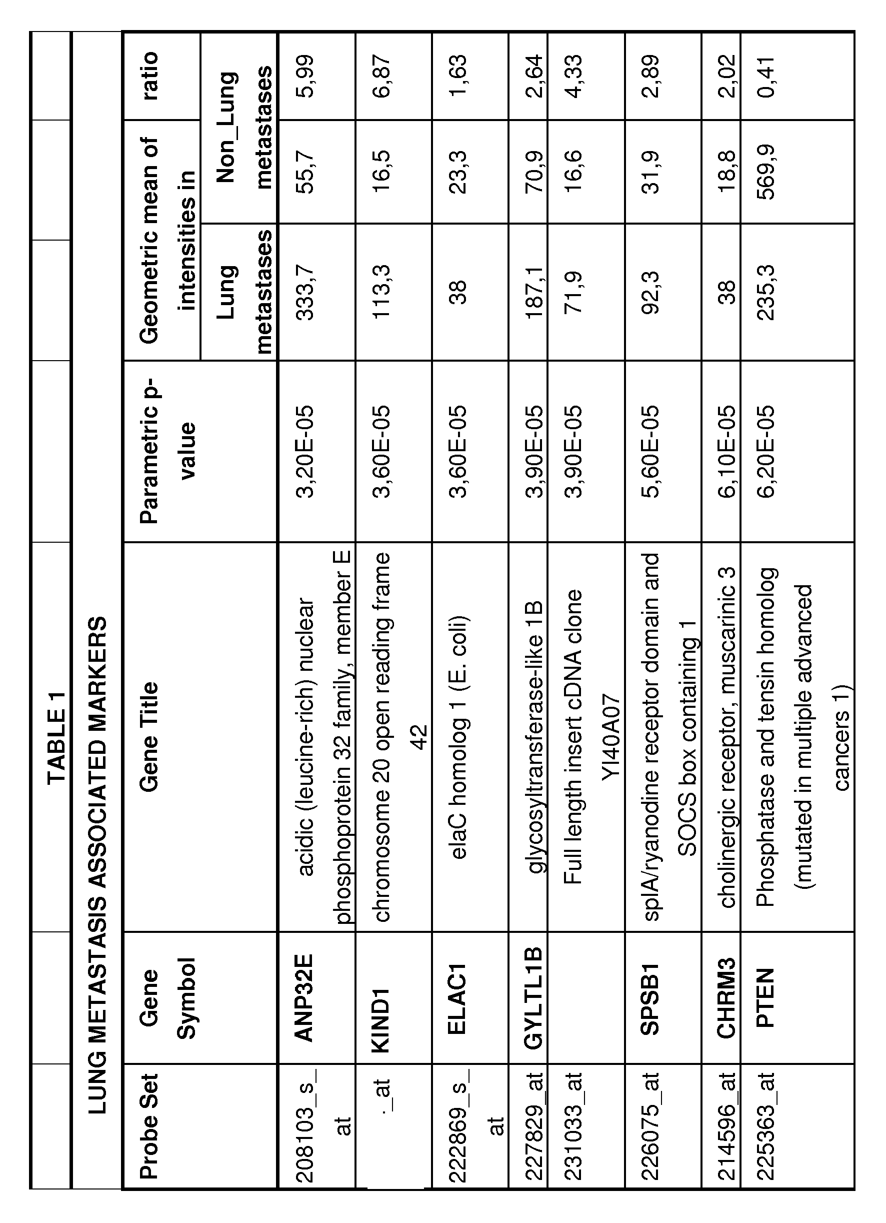

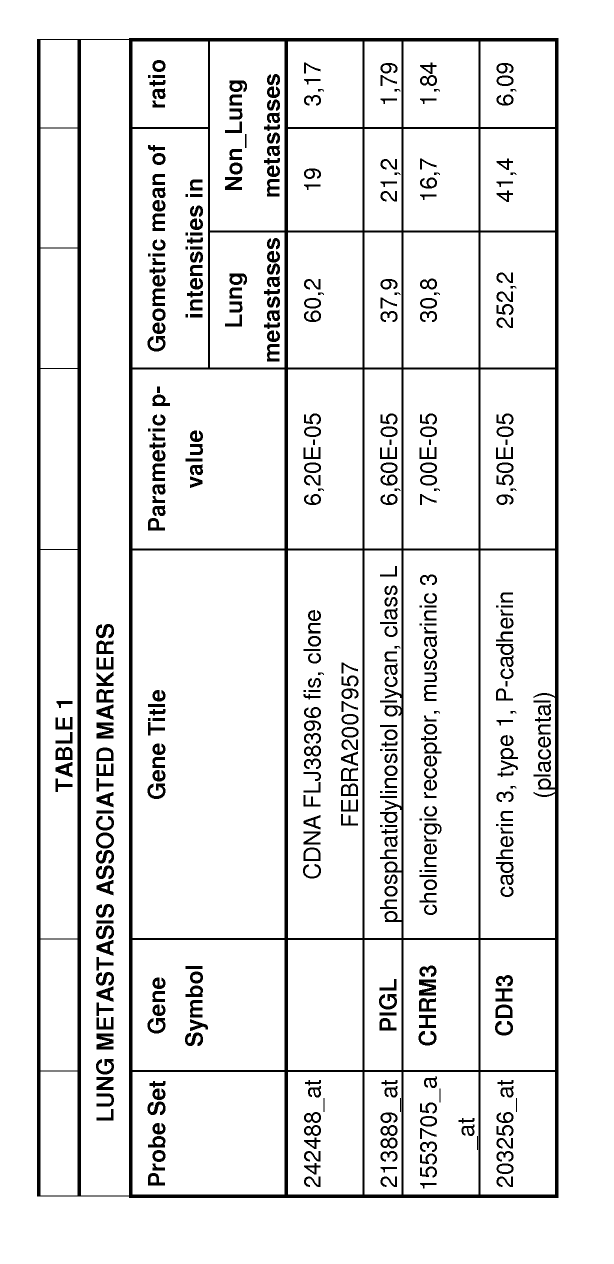

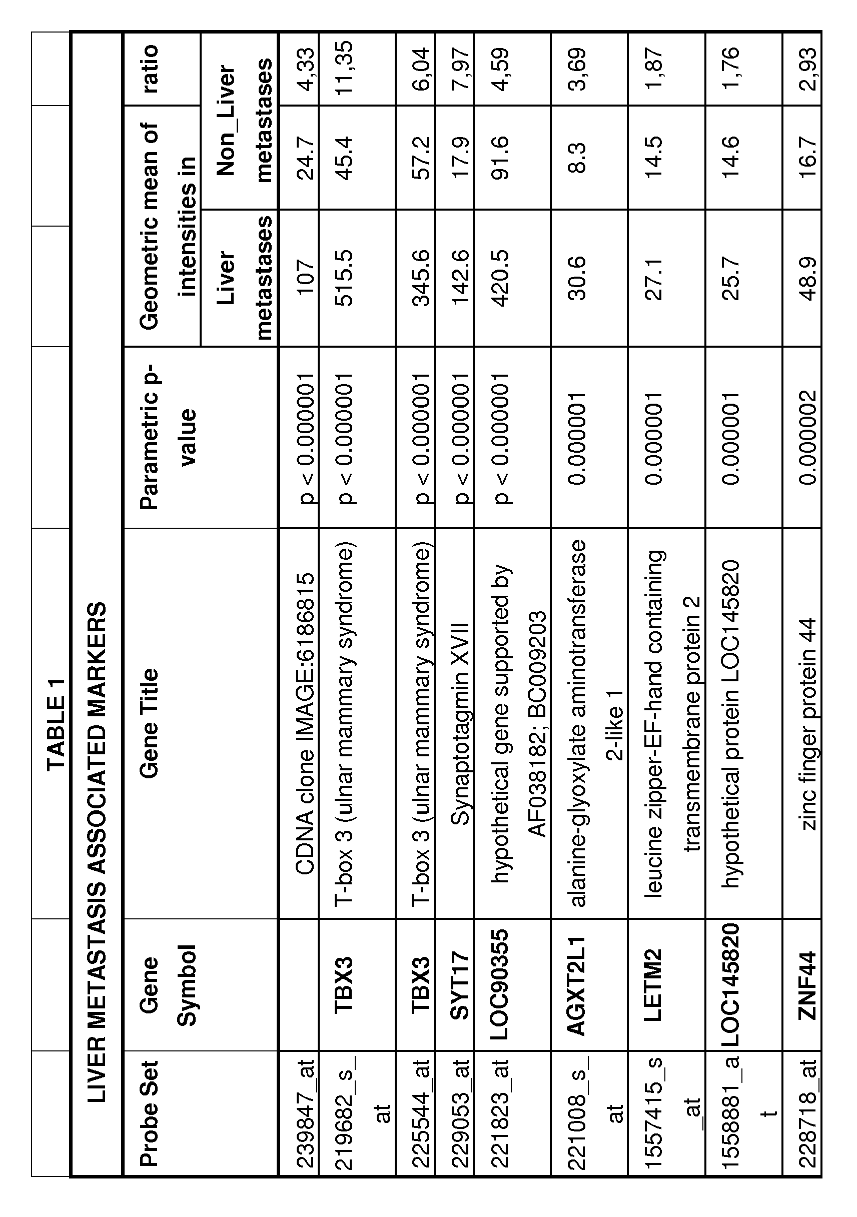

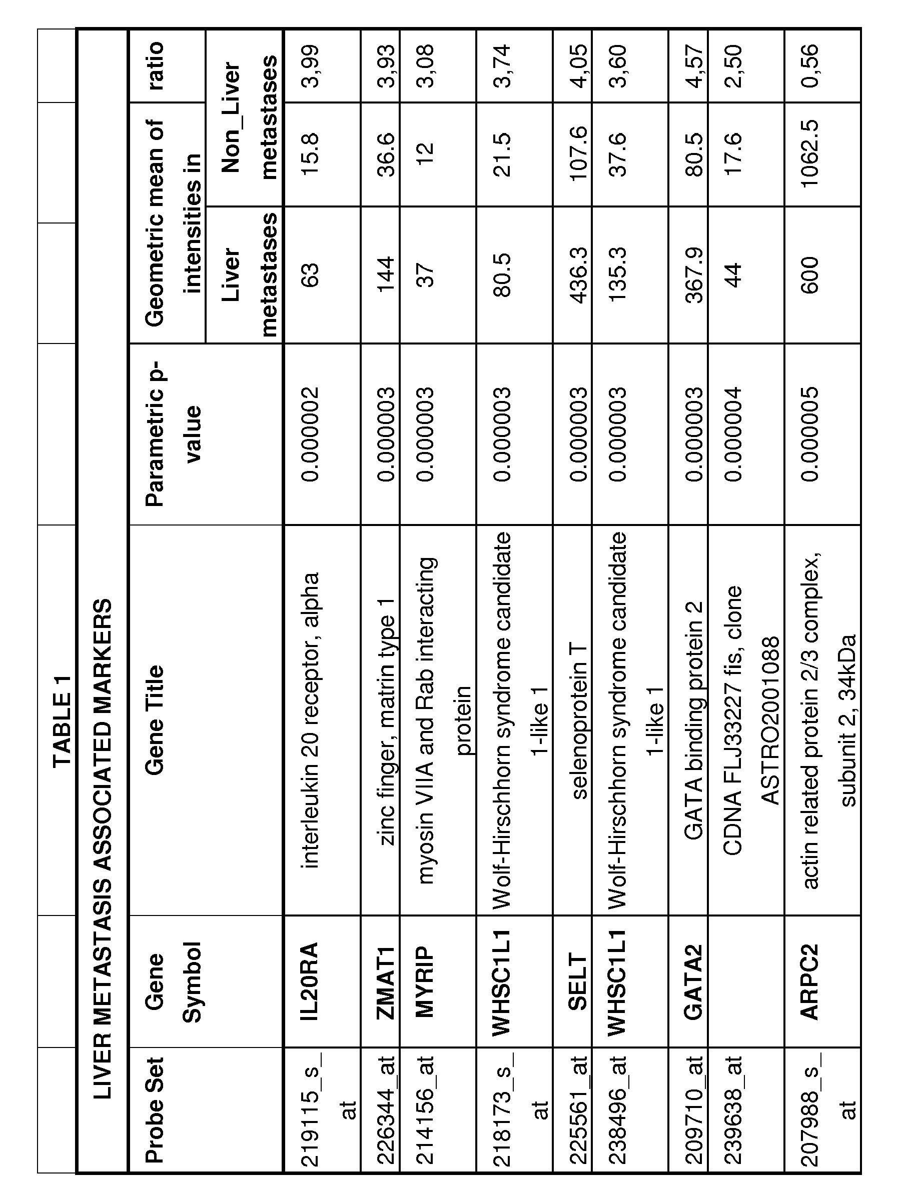

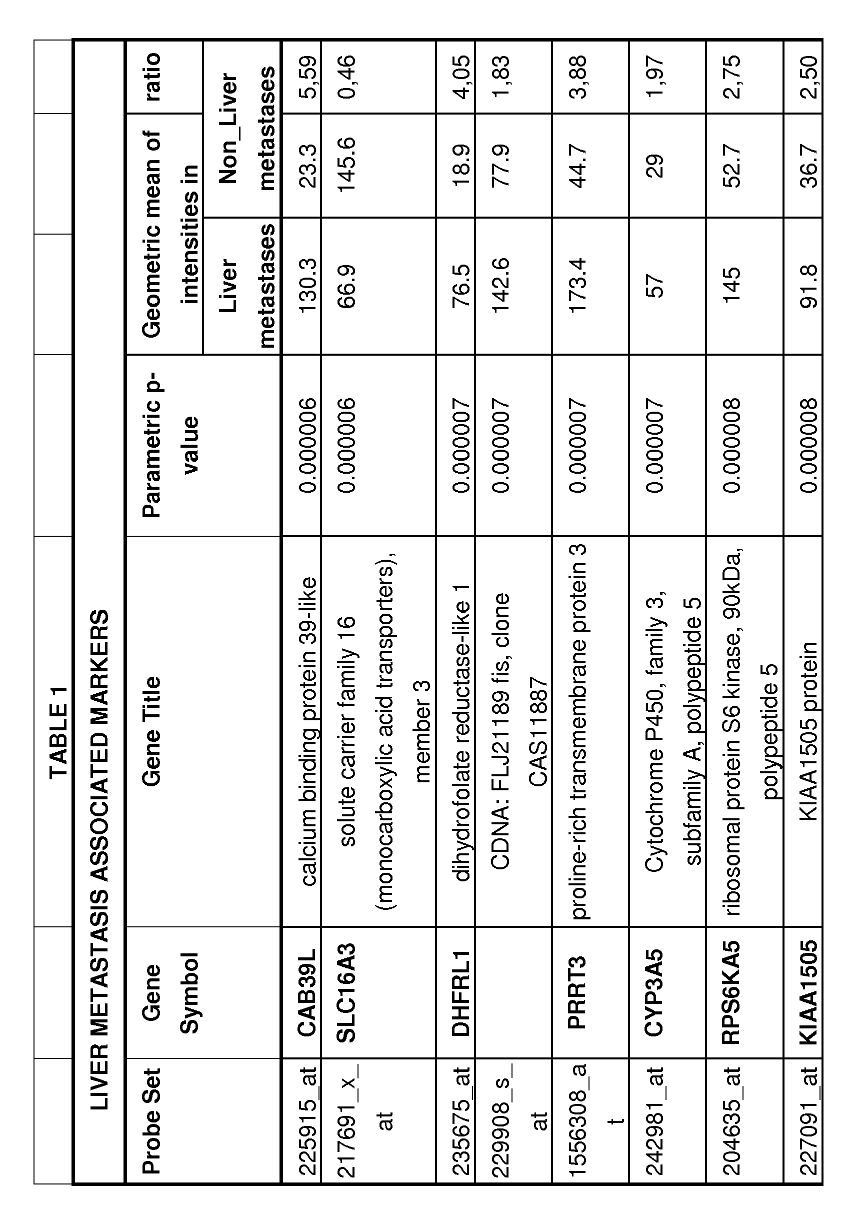

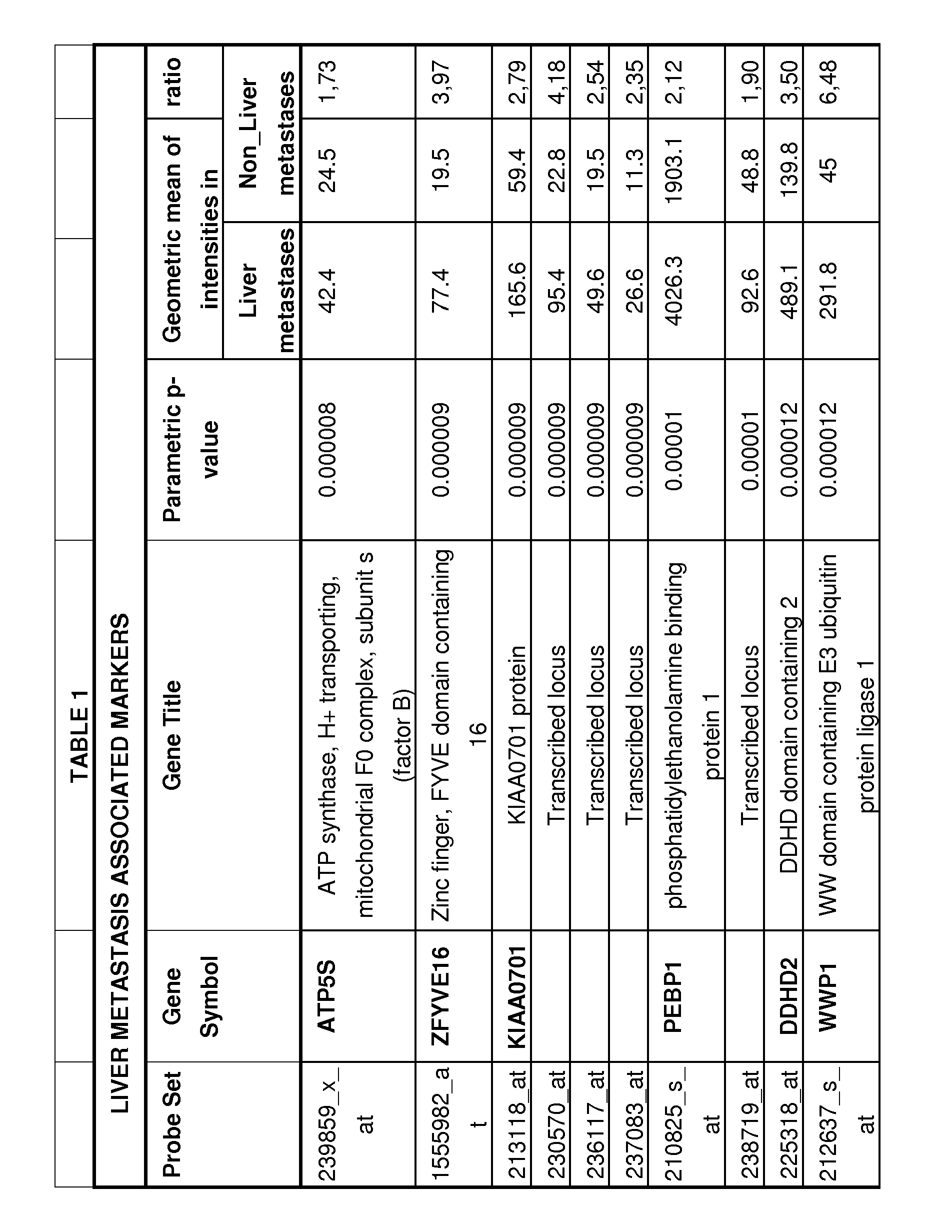

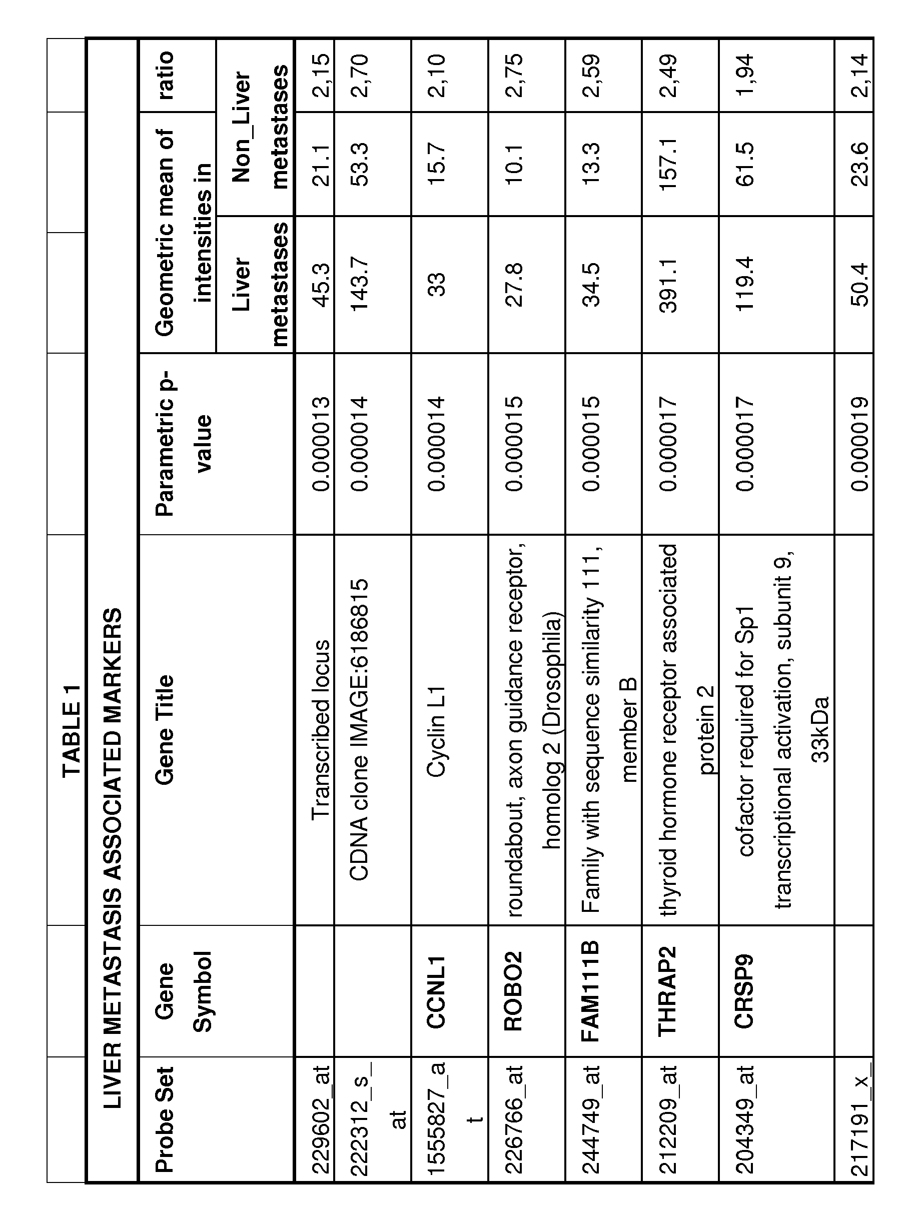

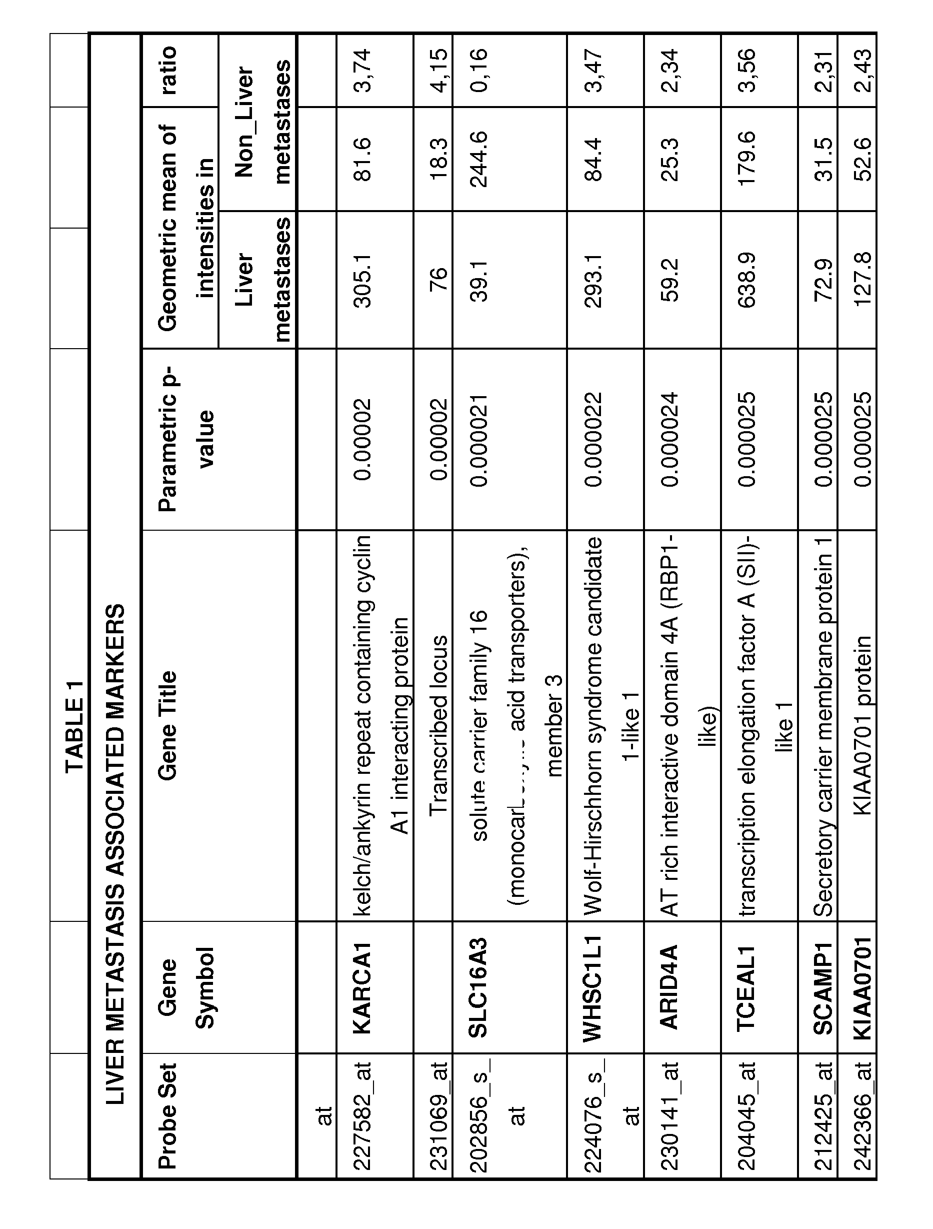

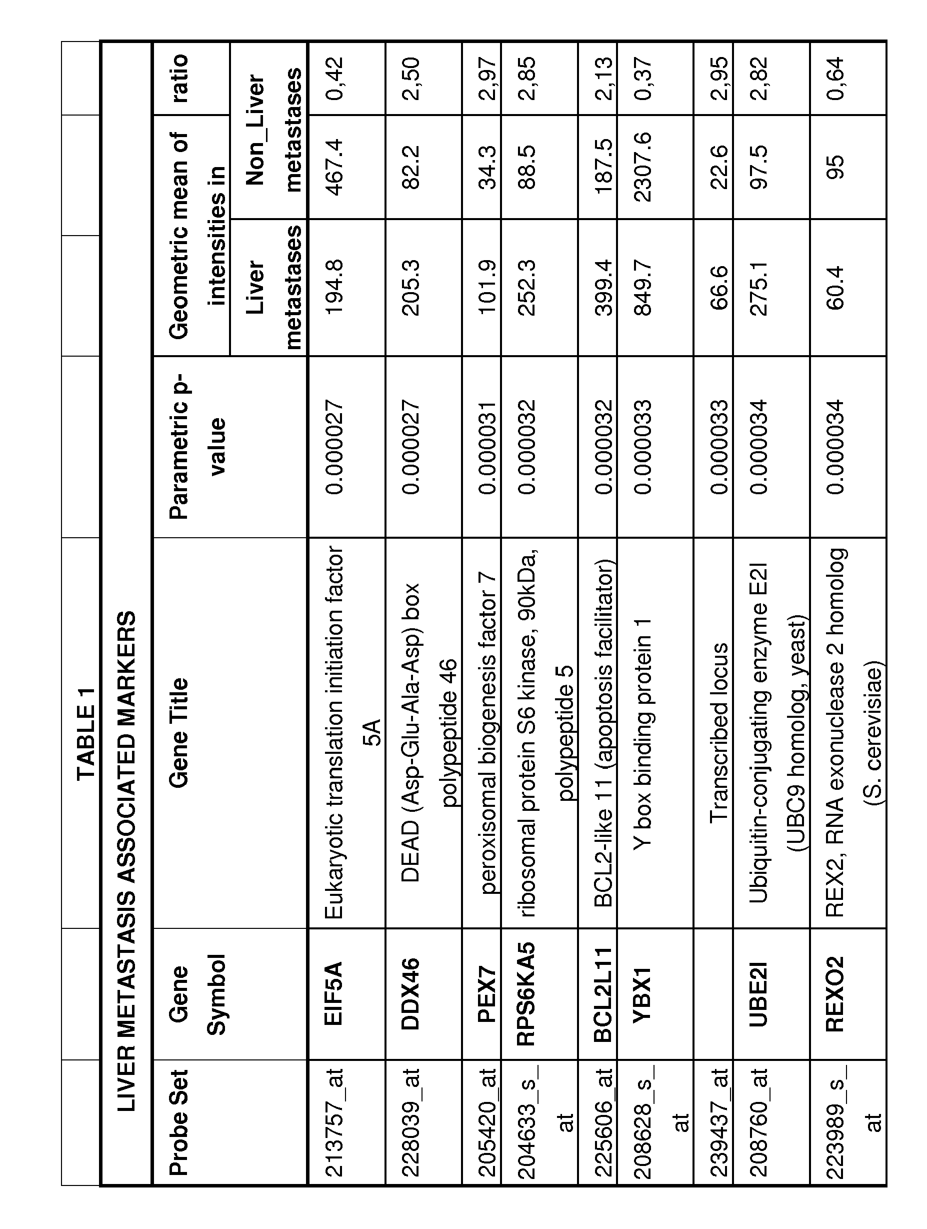

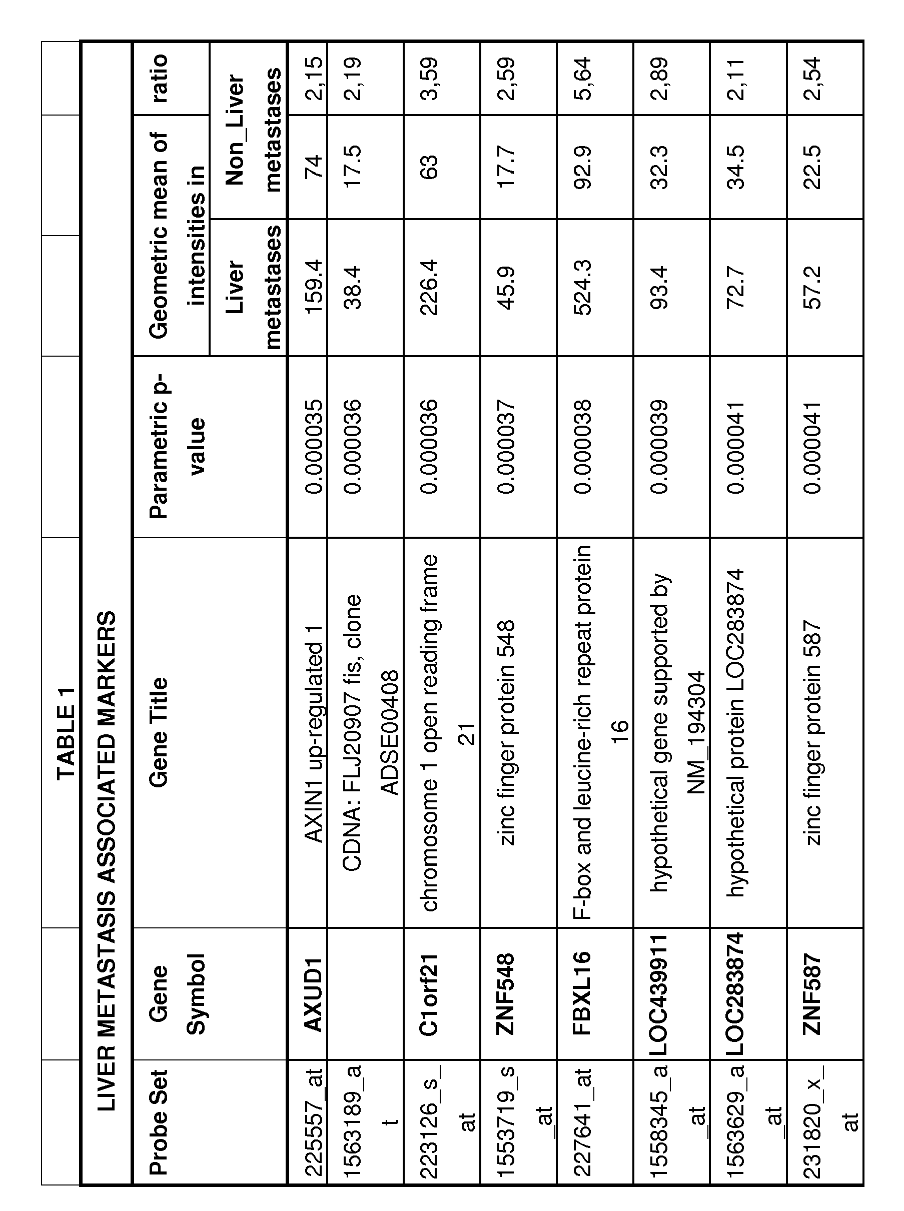

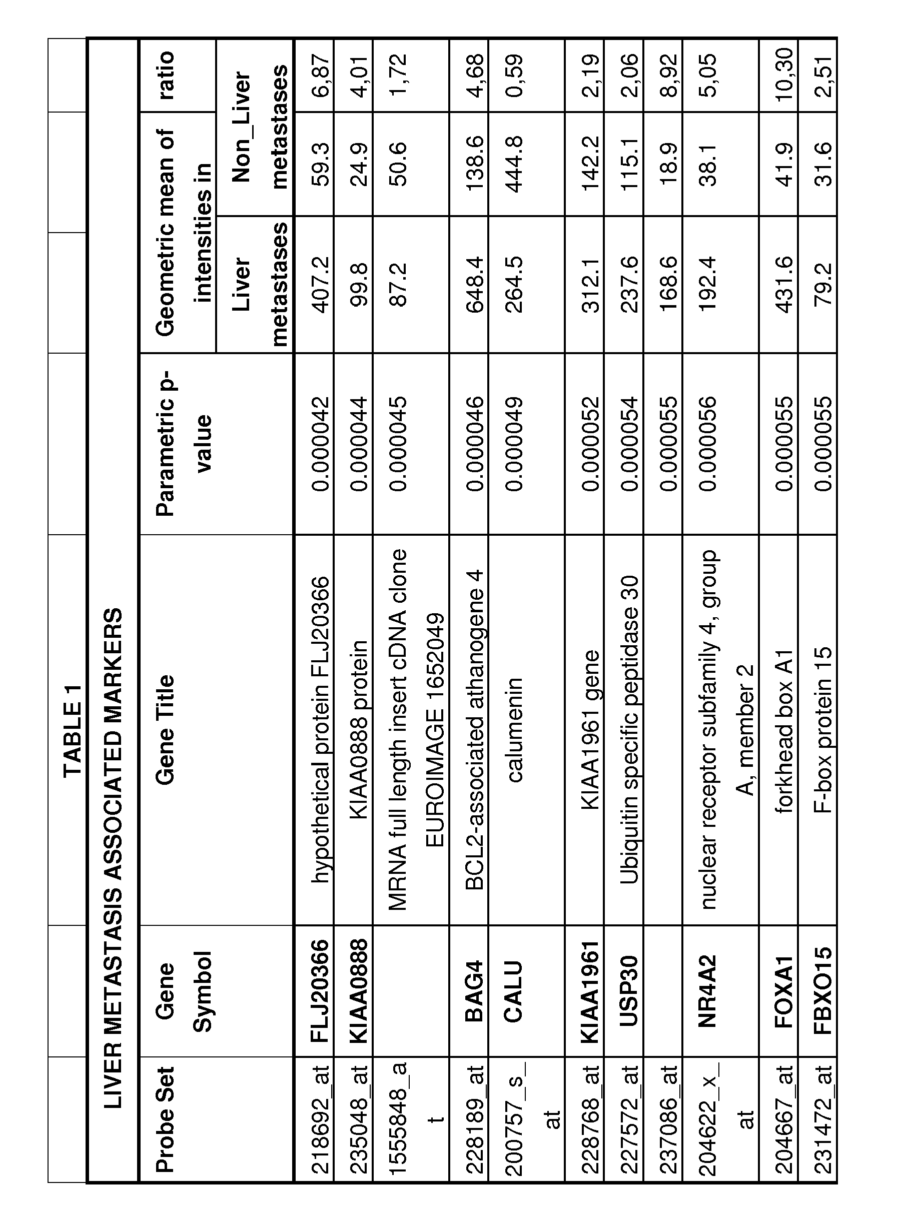

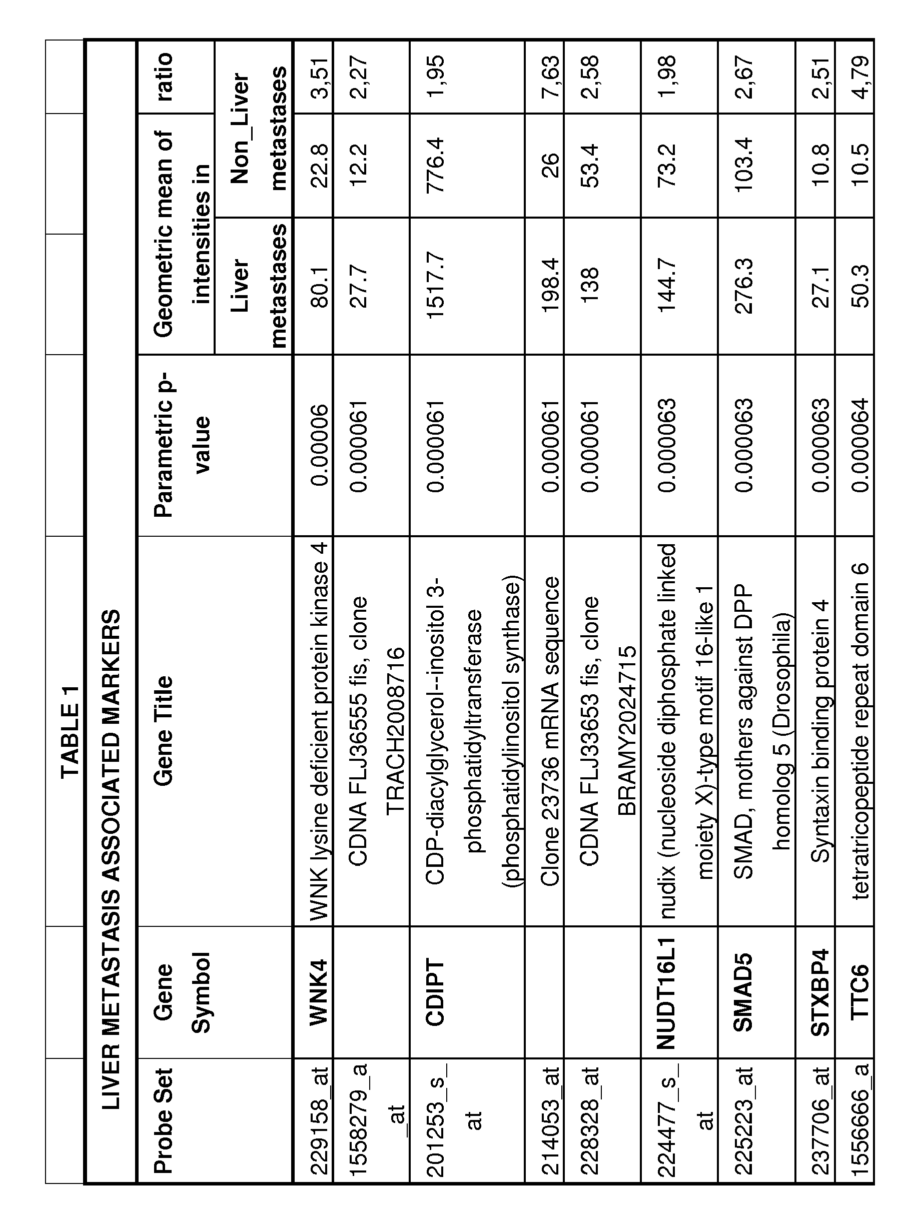

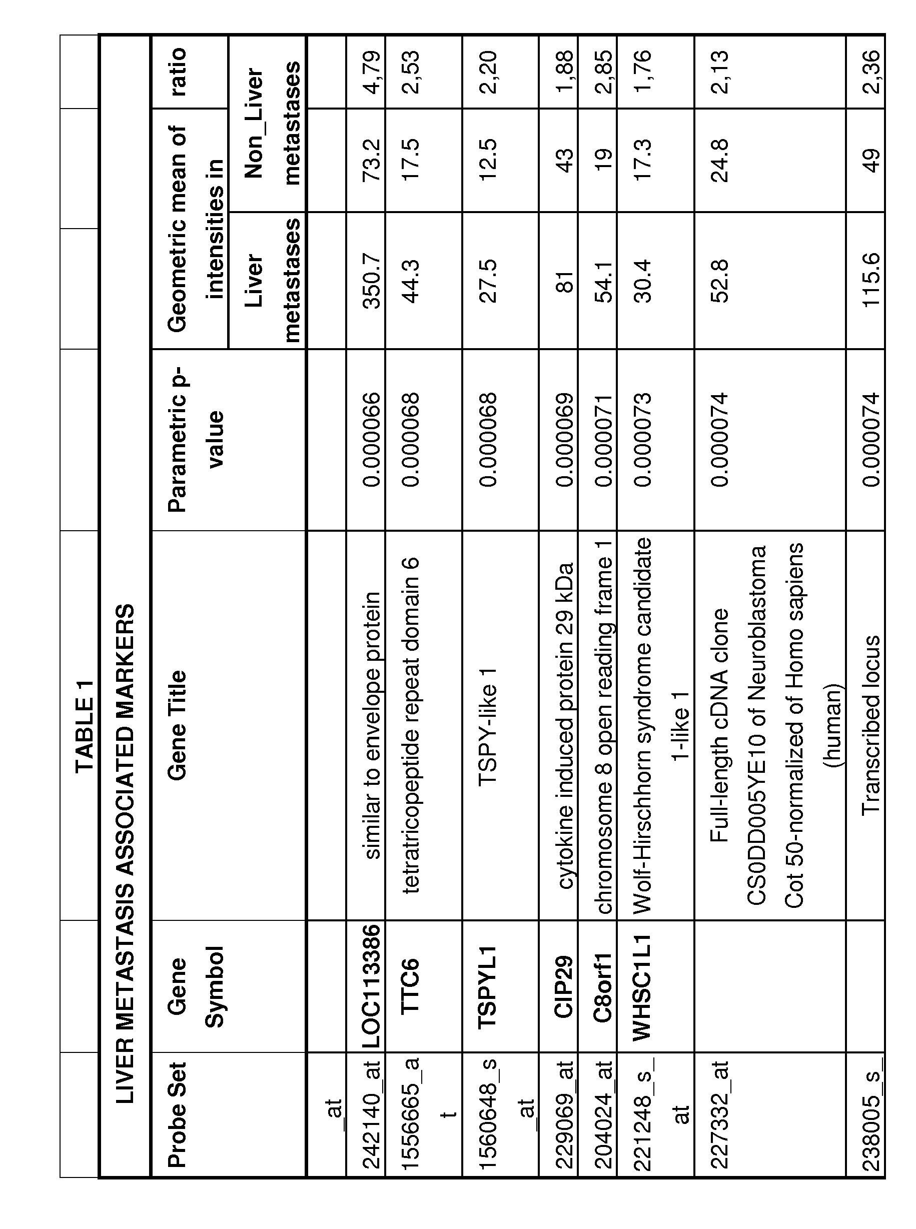

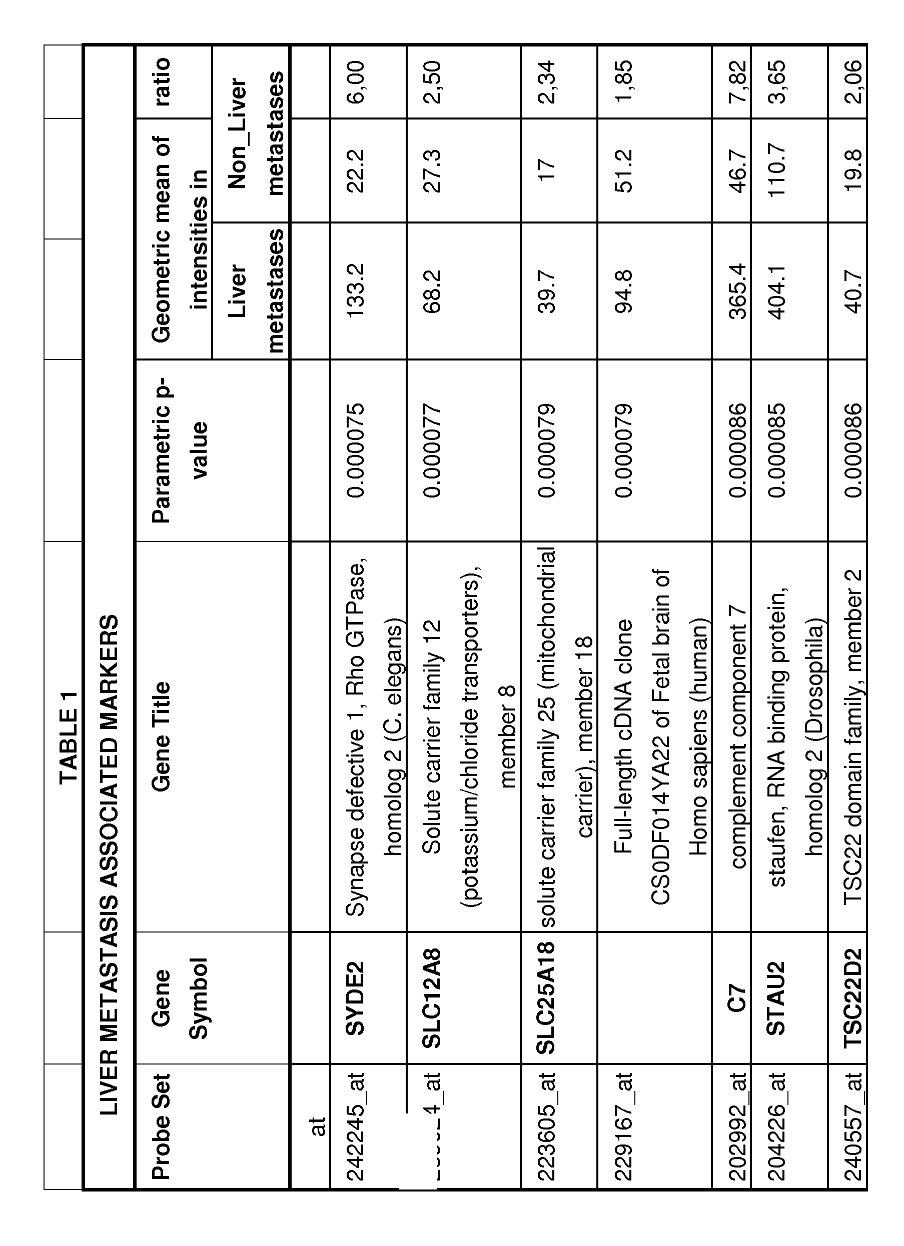

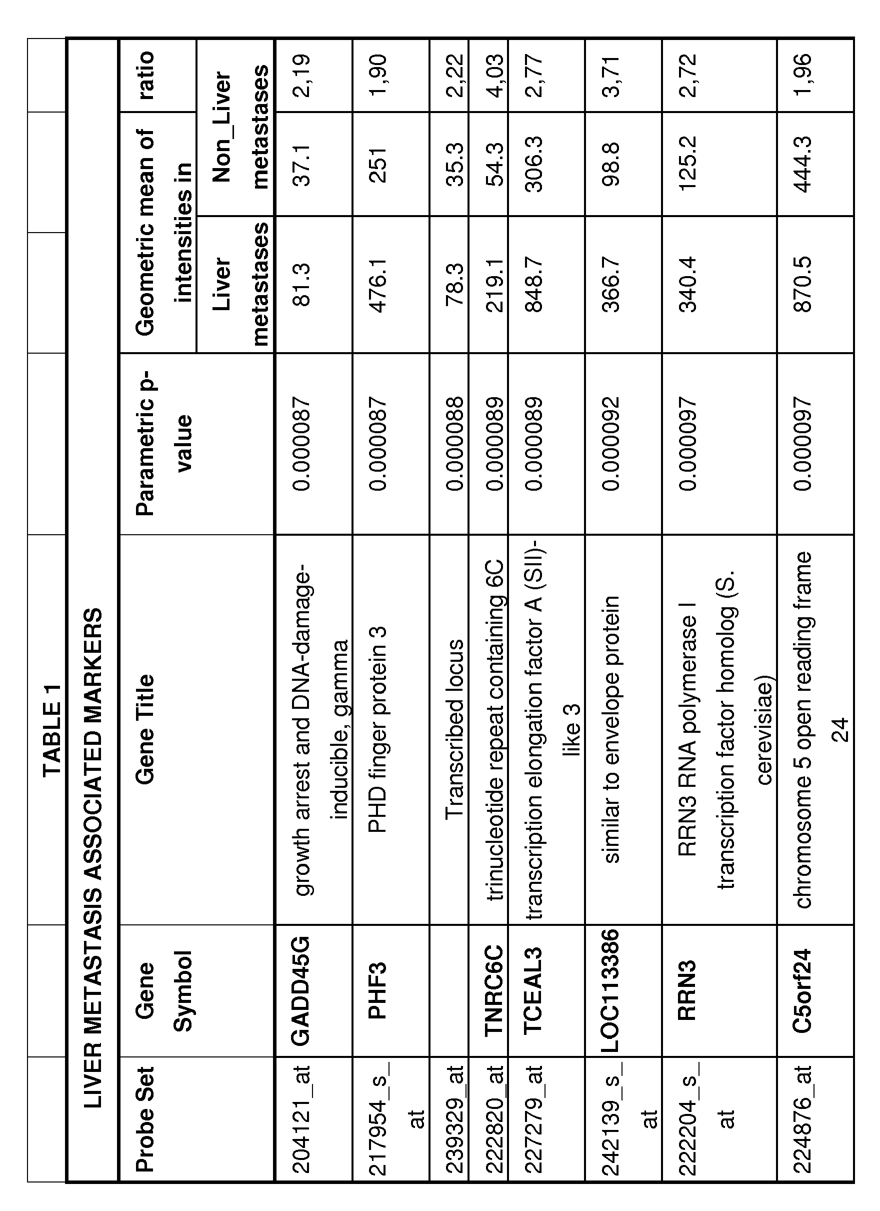

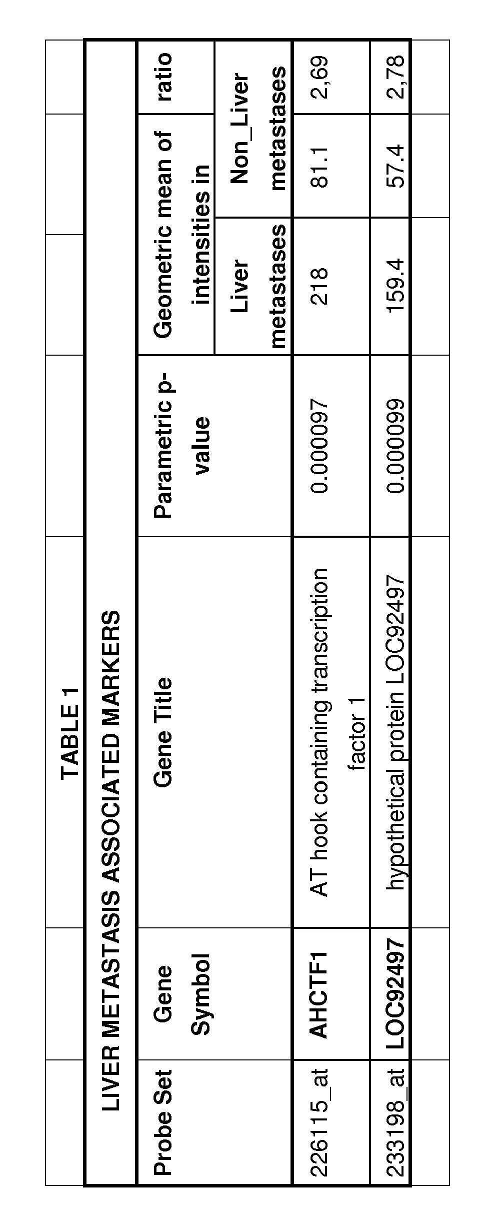

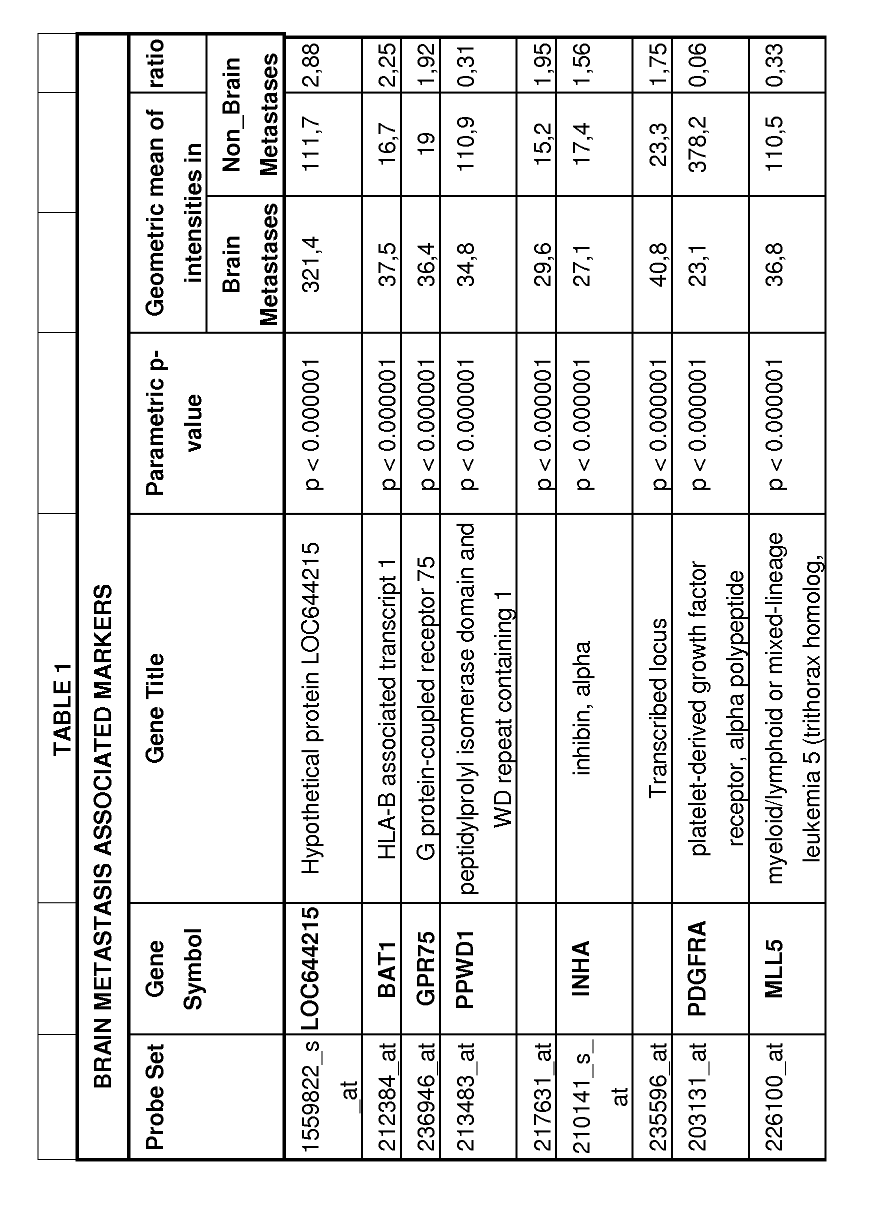

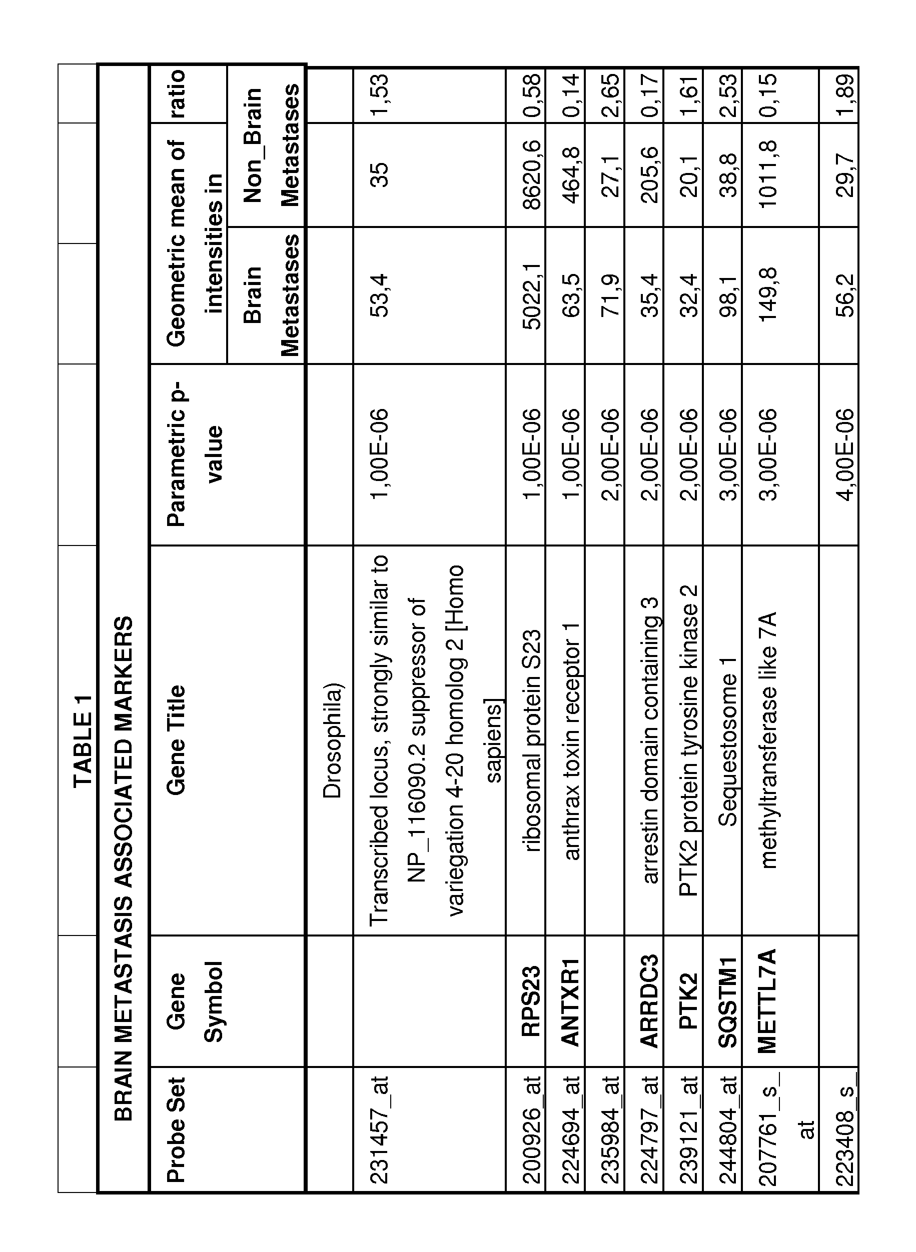

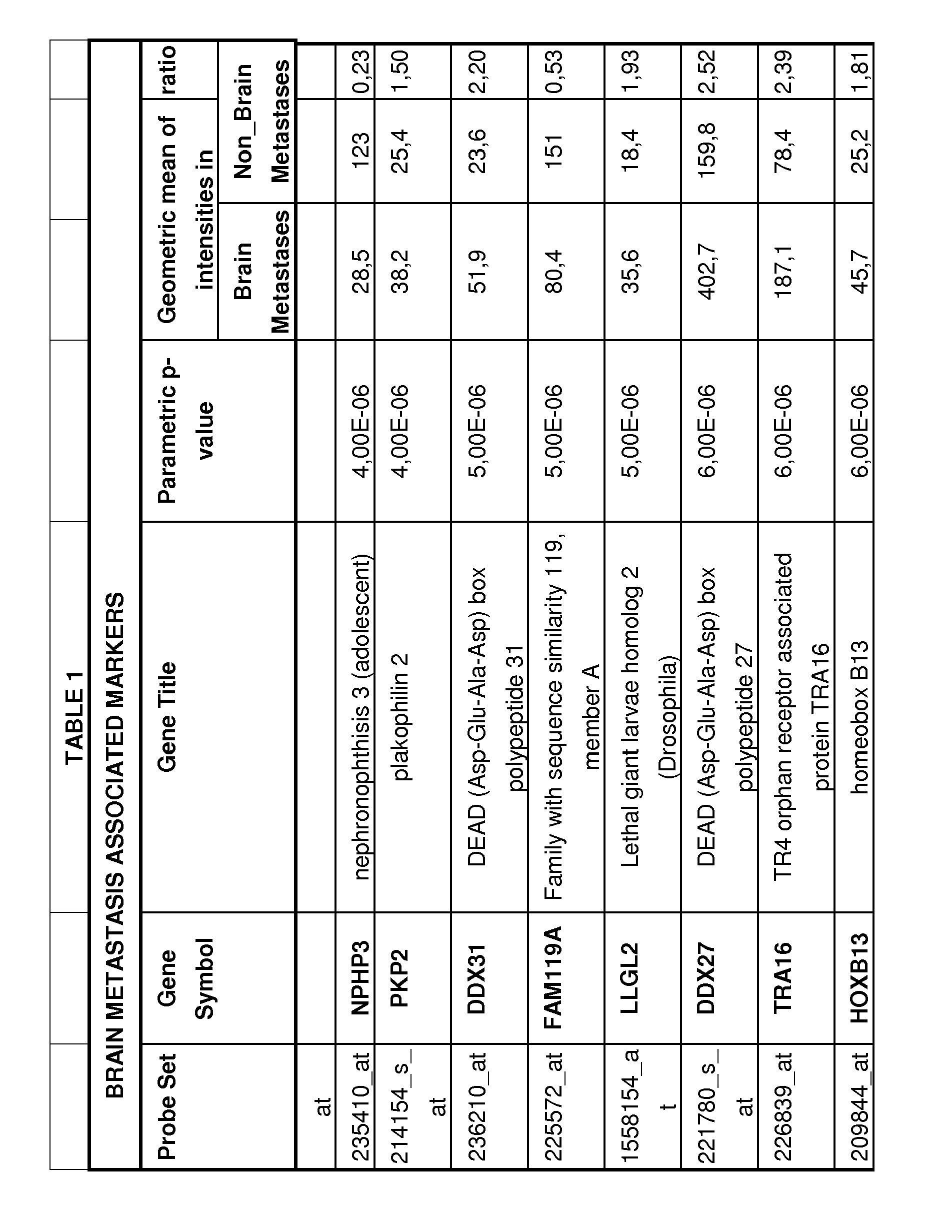

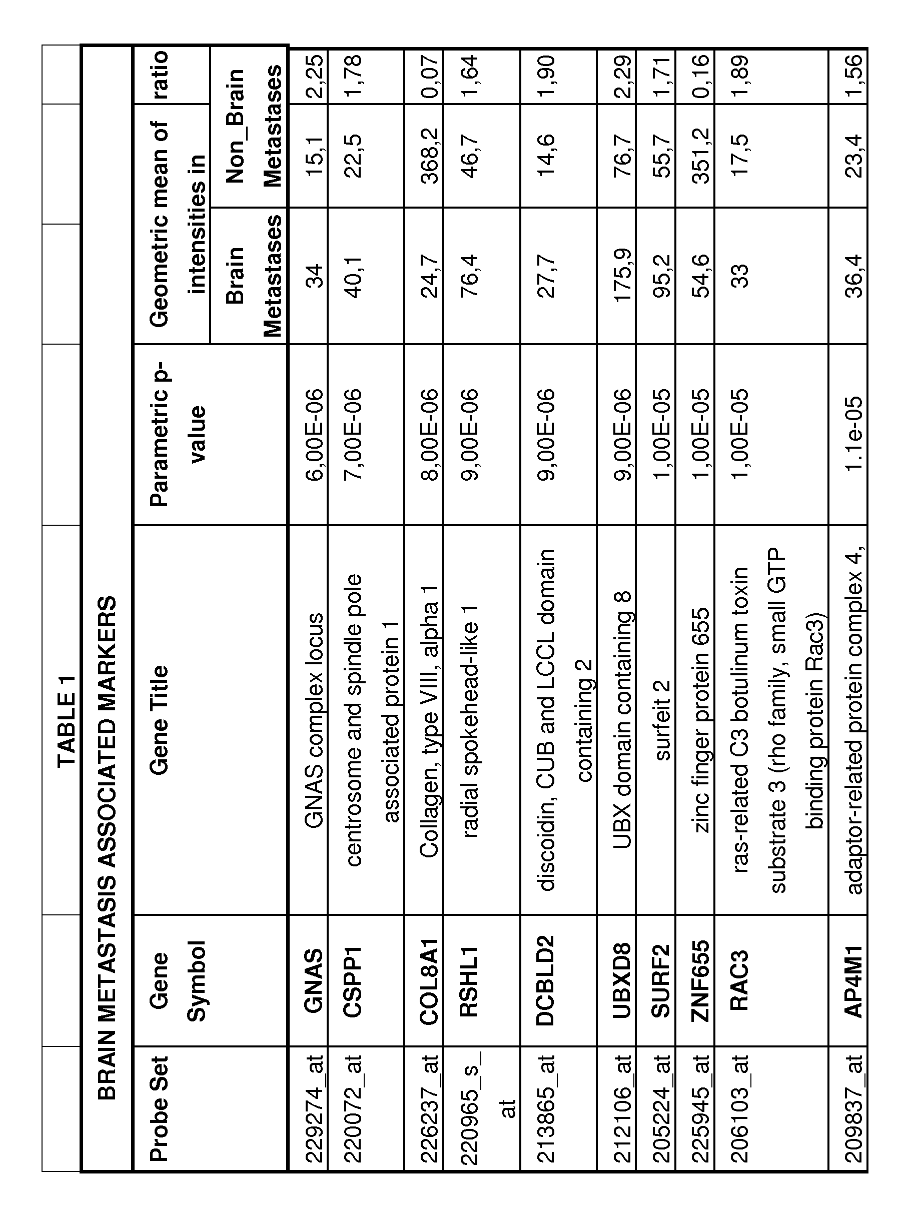

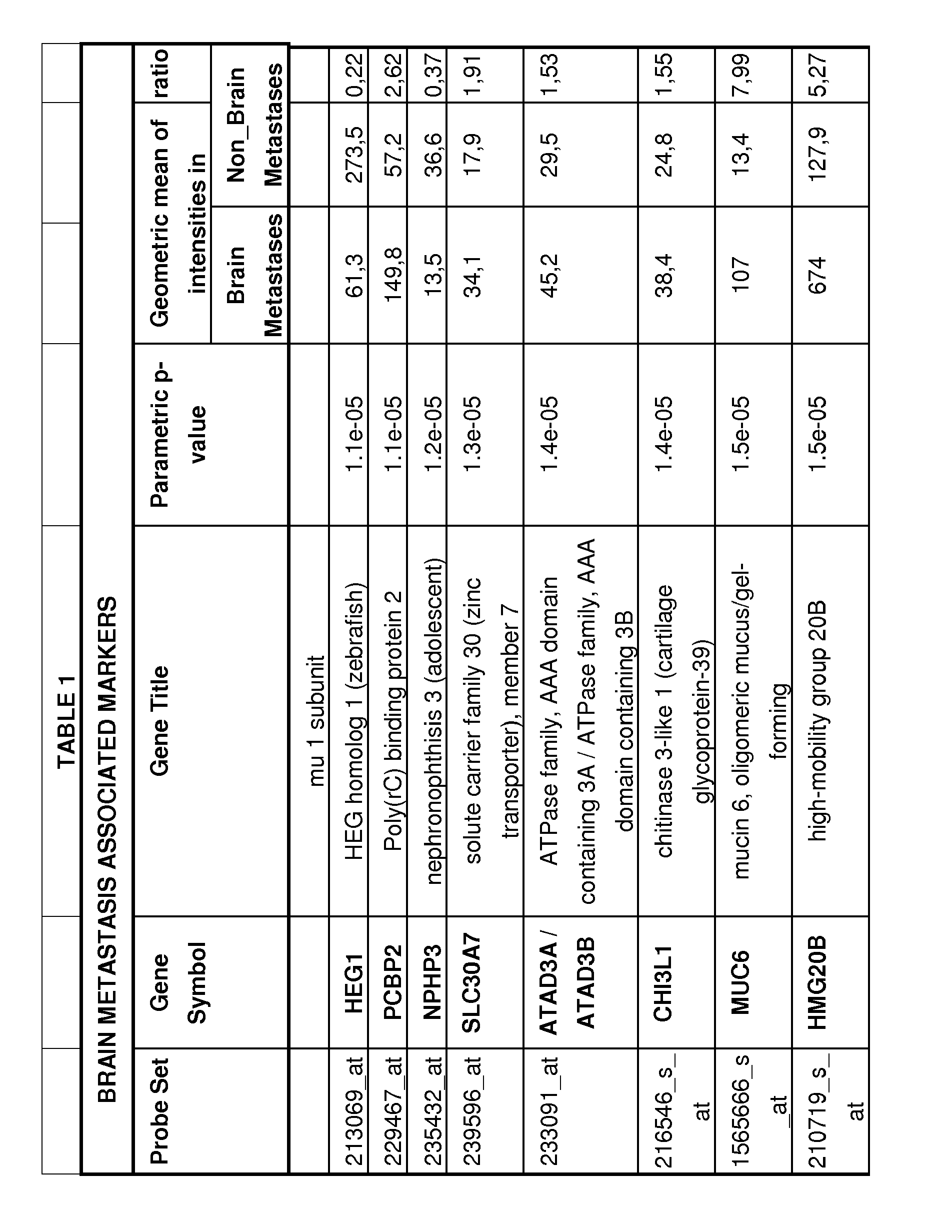

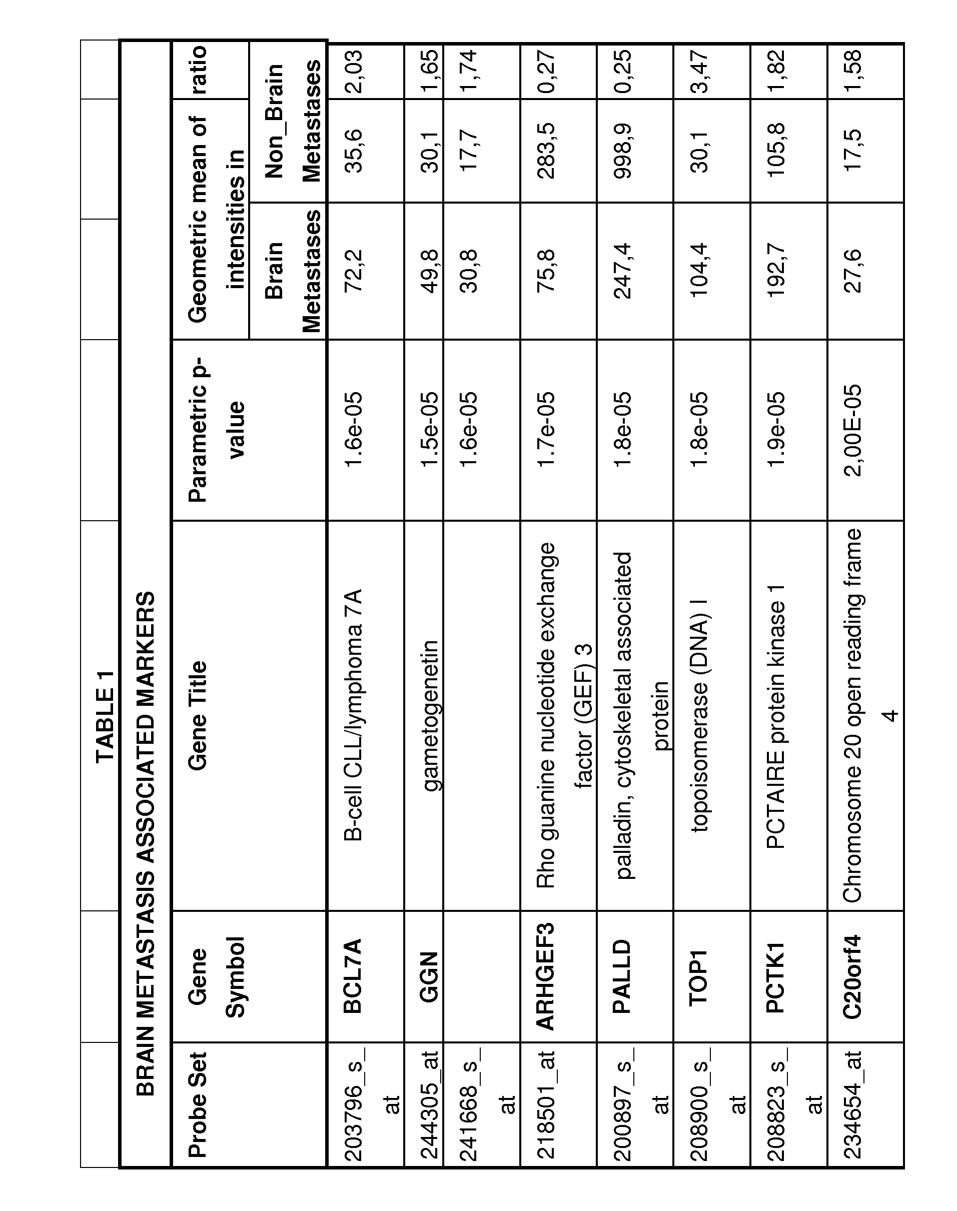

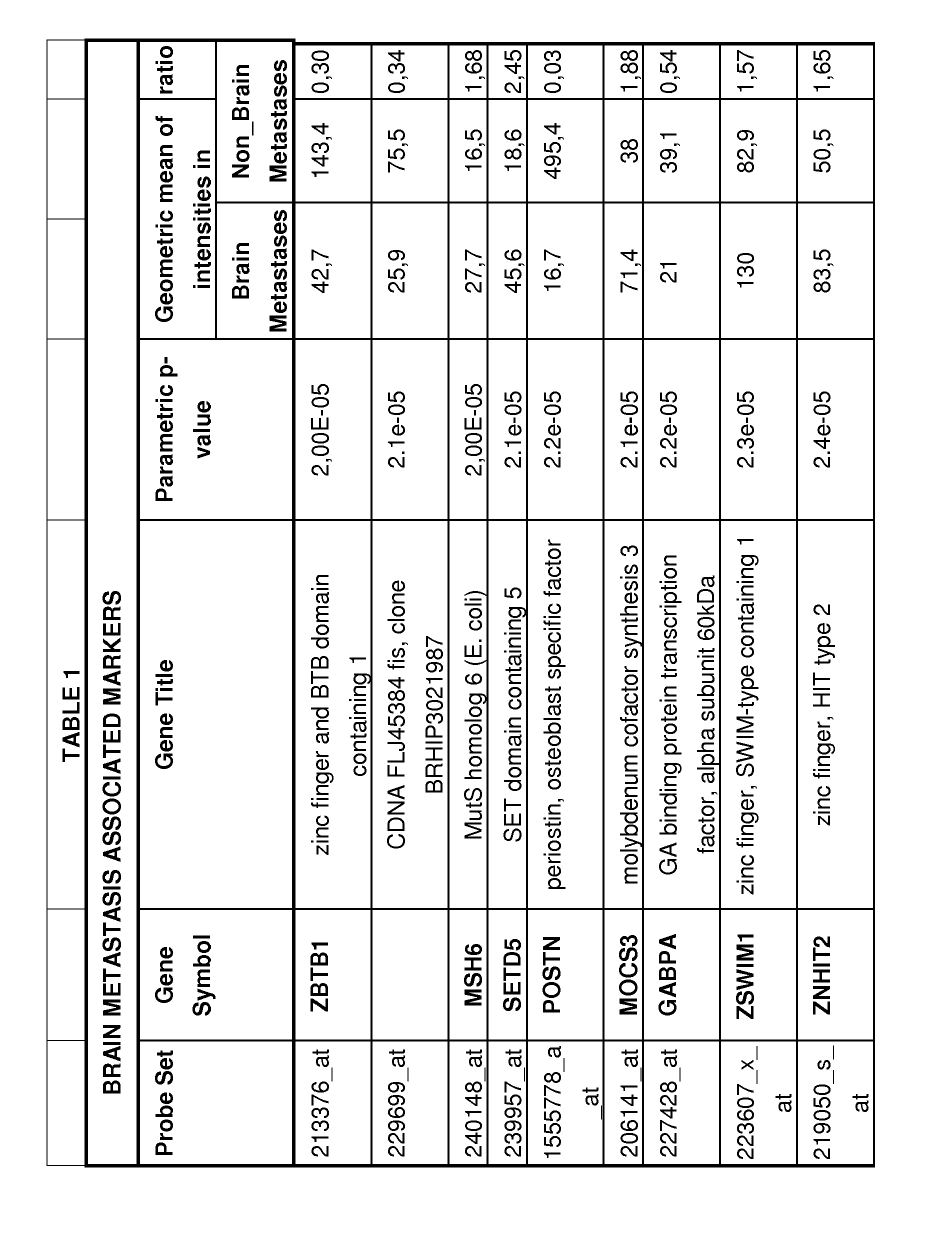

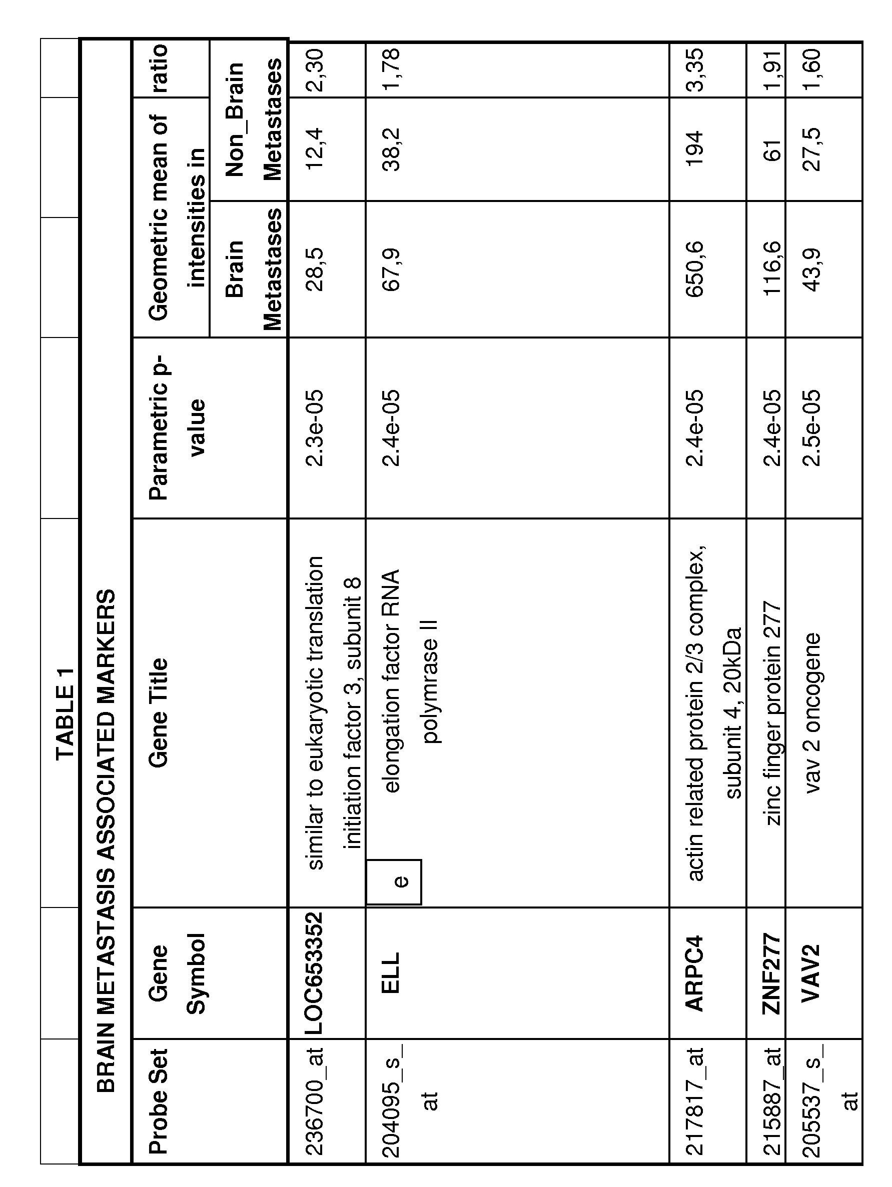

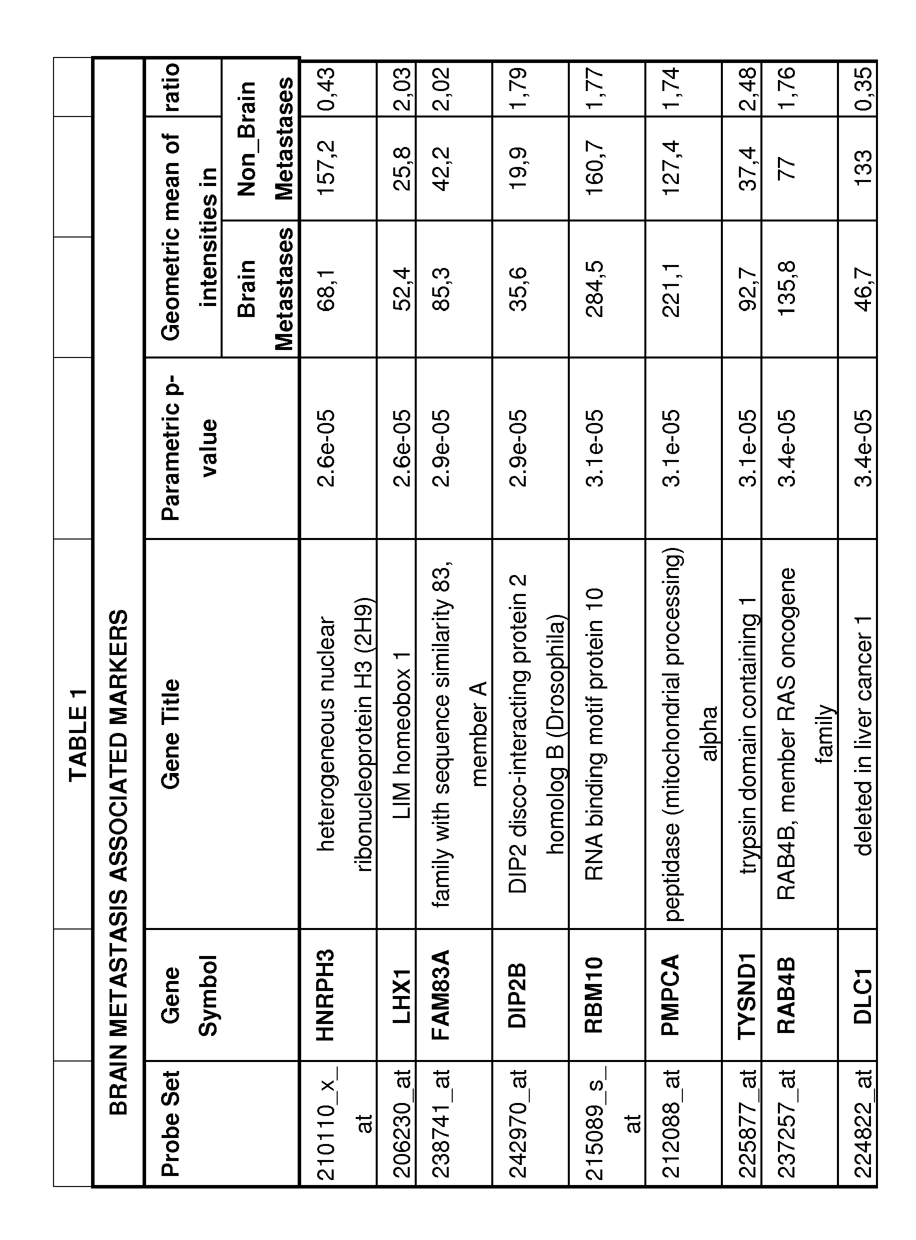

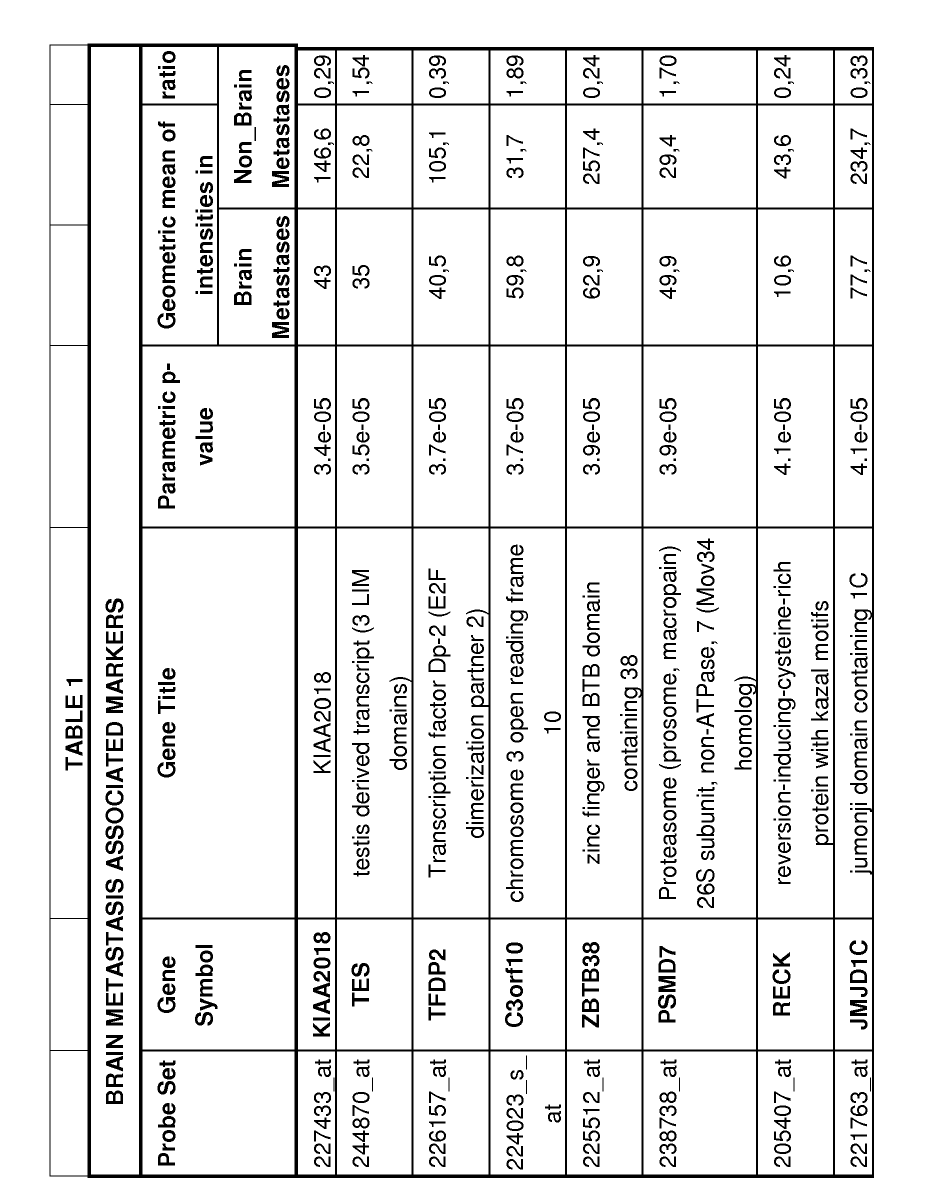

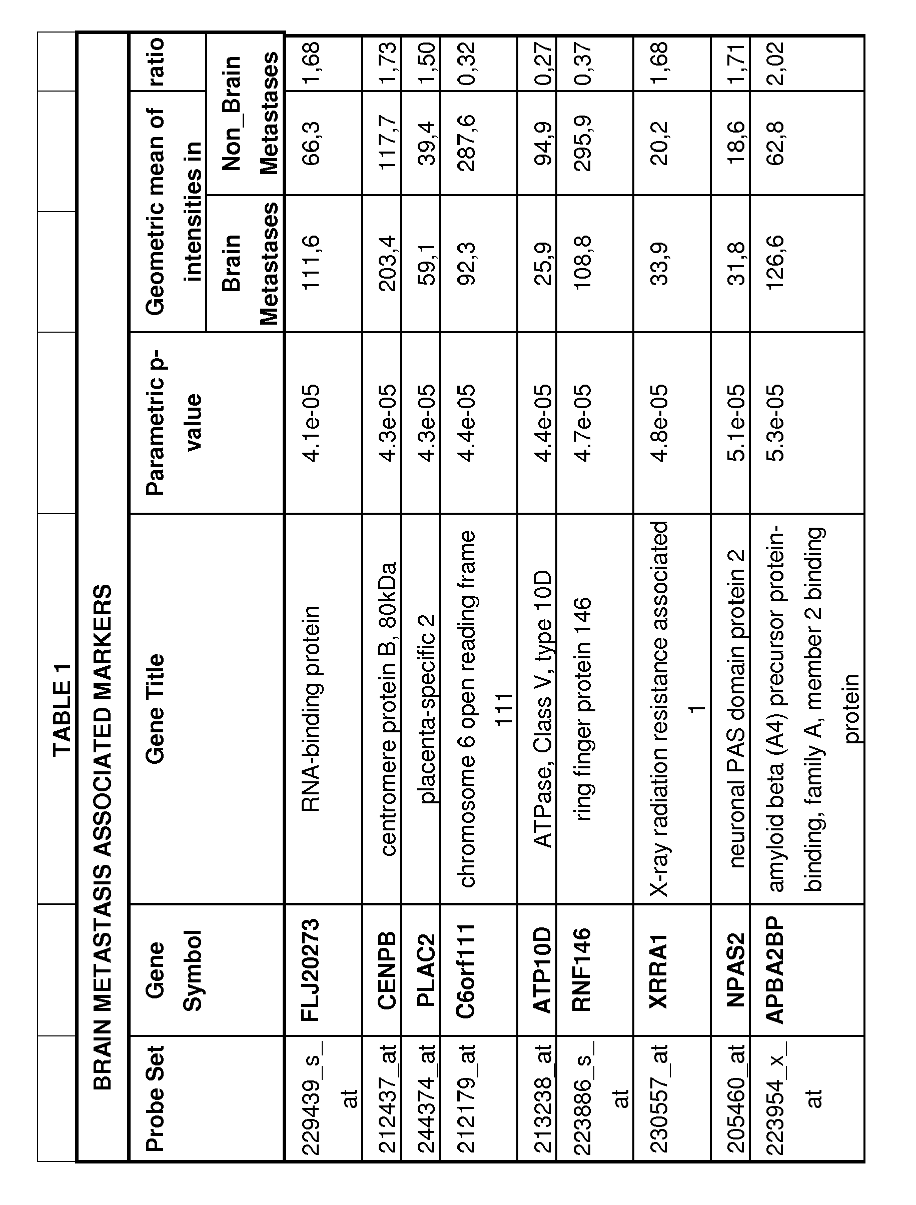

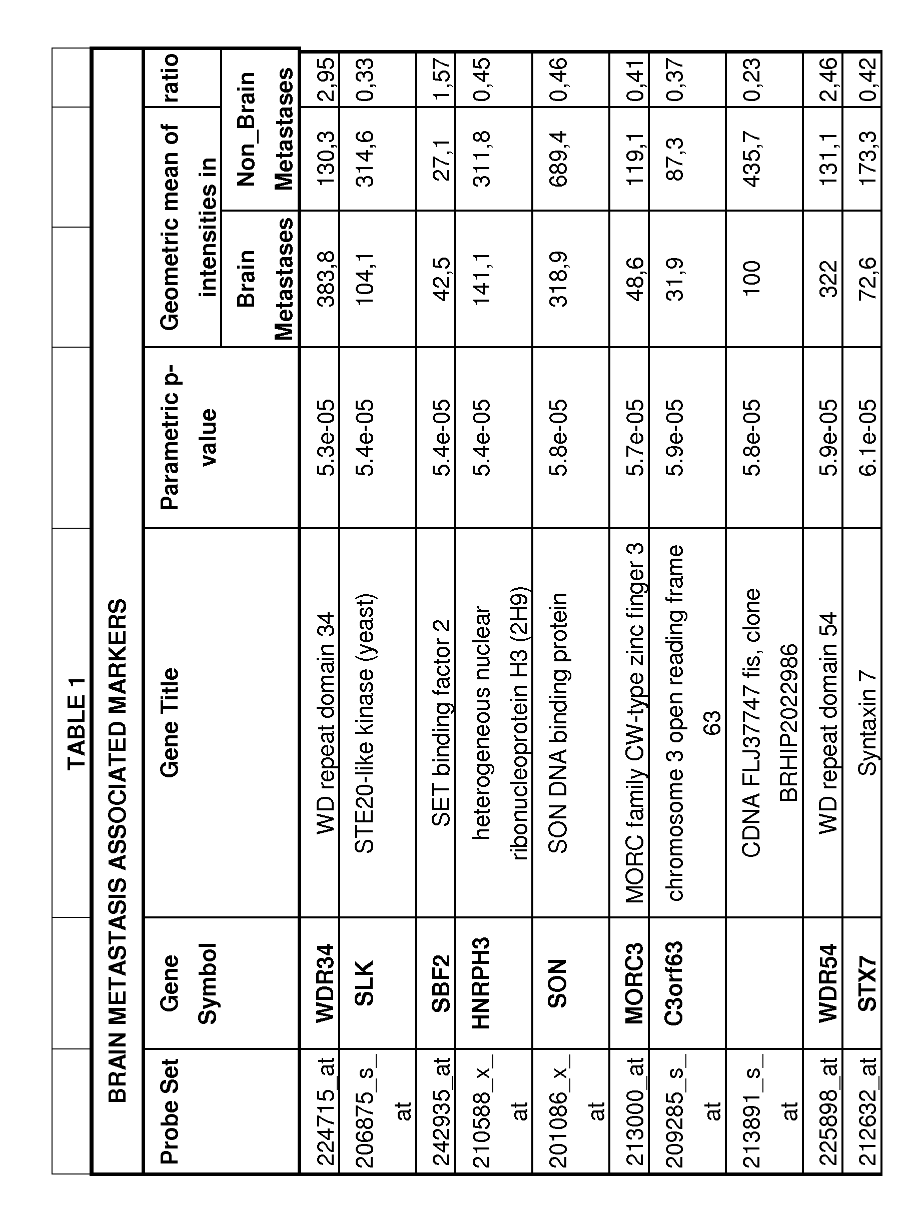

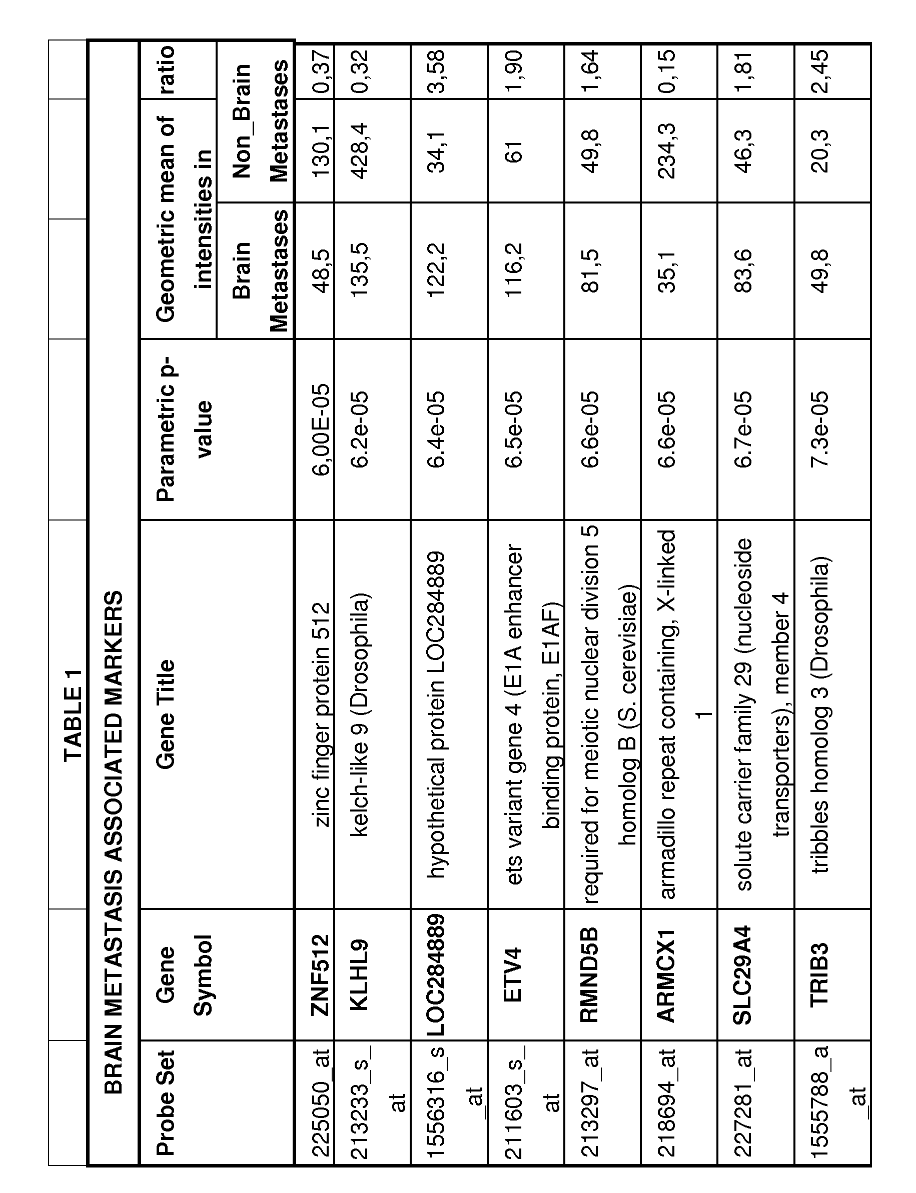

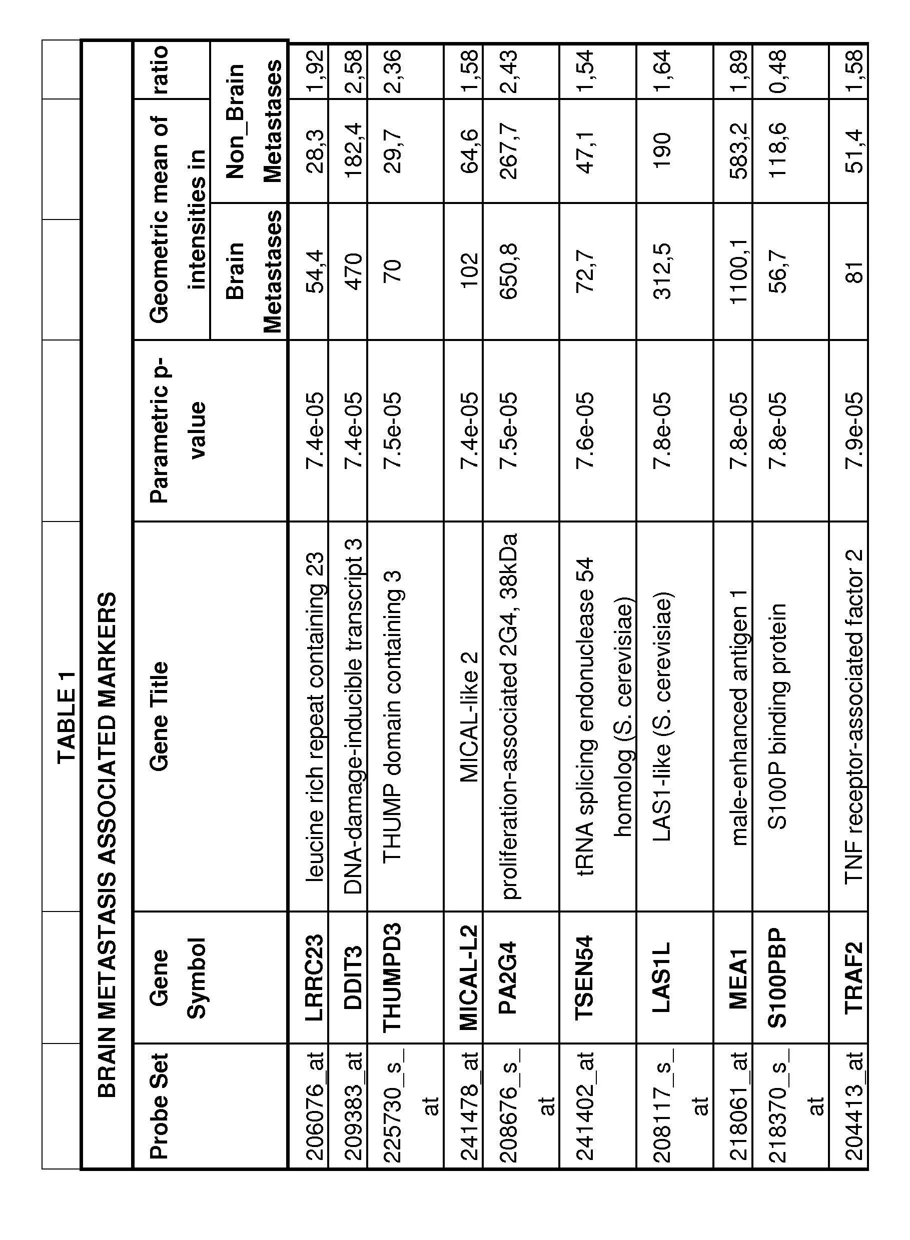

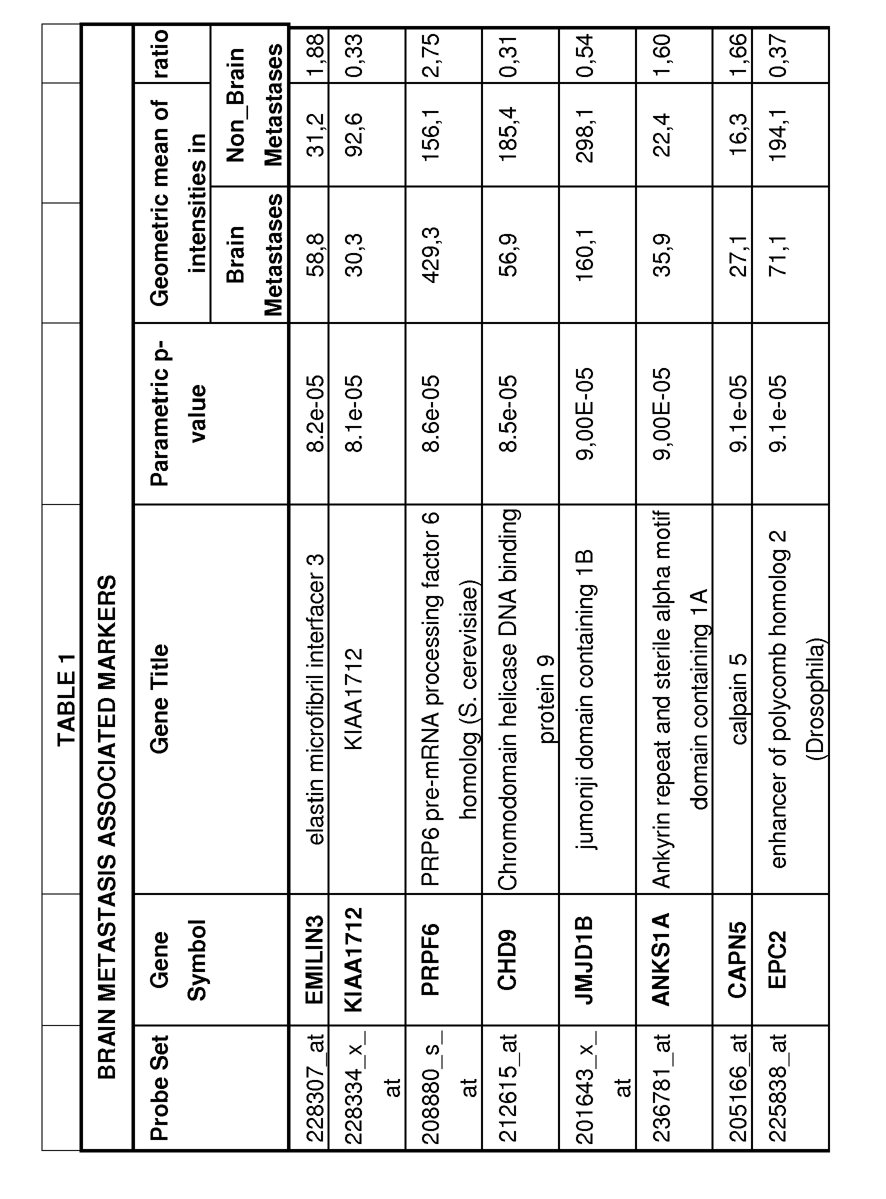

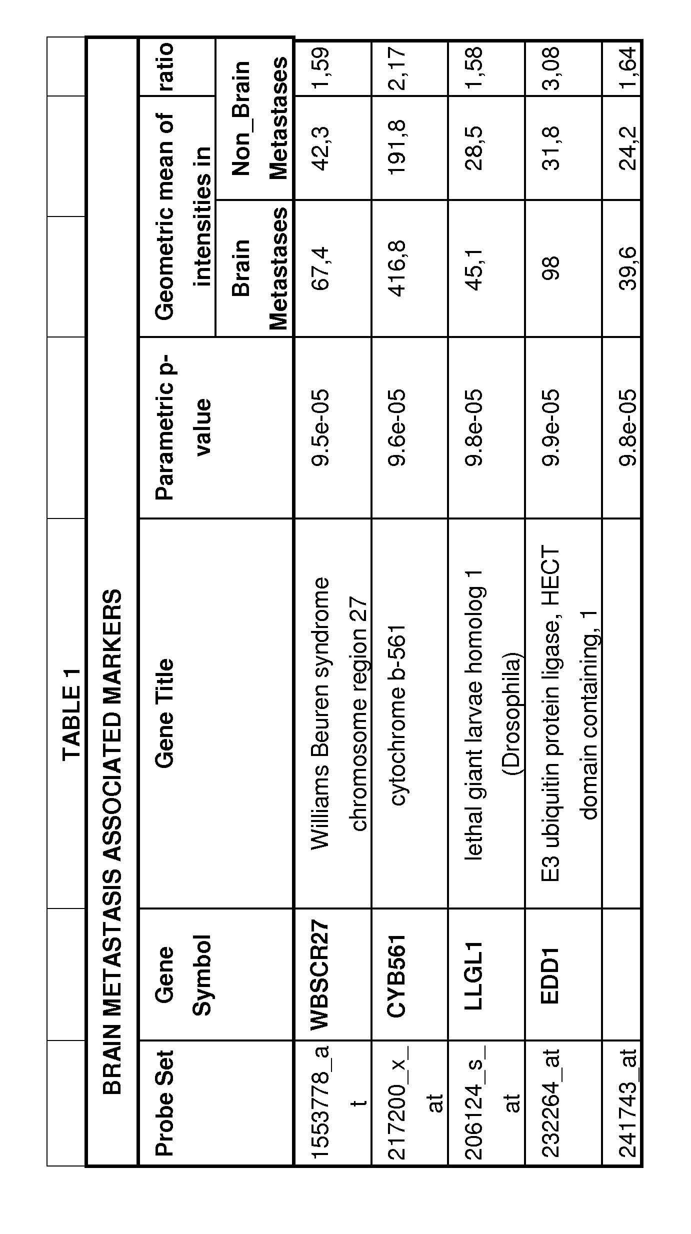

| | | | **TABLE 2** | | | |
|---|---|---|---|---|---|---|
| **Organ specific relapses** | **Probe set** | **Gene symbol** | **Description** | **Parametri c p-value** | **ratio** | **Gene Ontology Biological process and pathway** |
| **BONE** | 221724_s_a t | **CLEC4A** | C-type lectin domain family 4, member A | p < 1e-07 | 2,45 | immune response |
| | 204153_s_a t | **MFNG** | manic fringe homolog (Drosophila) | p < 1e-07 | 2,69 | Notch signaling pathway |
| | 208922_s_a t | **NXF1** | nuclear RNA export factor 1 | p < 1e-07 | 1,61 | mRNA processing |
| | 227002_at | **FAM78A** | family with sequence similarity 78, member A | p < 1e-07 | 2,43 | |
| | 226245_at | **KCTD1** | potassium channel tetramerisation domain containing 1 | 1,00E-07 | 0,30 | potassium ion transport |
| | 227372_s_a t | **BAIAP2L 1** | BAI1-associatedprotein2-like 1 | 1,00E-07 | 0,10 | |
| | 206060_s_a t | **PTPN22** | protein tyrosine phosphatase, non-receptor type 22 (lymphoid) | 1,00E-07 | 4,12 | signal transduction |
| | 232523_at | **MEGF10** | MEGF10 protein | 1,00E-07 | 10,77 | |
| | 222392_x_a t | **PERP** | PERP, TP53 apoptosis effector | 1,00E-07 | 0,09 | regulation of apoptosis |
| | 211178_s_at | **PSTPIP1** | proline-serine-threonine phosphatase interacting protein 1 | 2,00E-07 | 3,01 | cell adhesion |
| | 204236_at | **FLI1** | Friend leukemia virus integration 1 | 2,00E-07 | 4,80 | regulation of transcription |
| | 213290_at | **COL6A2** | collagen, type VI, alpha 2 | 2,00E-07 | 2,63 | extracellular matrix organization |
| | 203547_at | **CD4** | CD4 antigen (p55) | 2,00E-07 | 2,62 | immune response |
| | 205382_s_a t | **CFD** | complement factor D (adipsin) | 2,00E-07 | 7,37 | immune response |
| | 235593_at | **ZFHX1B** | zinc finger homeobox 1b | 2,00E-07 | 2,92 | regulation of transcription |
| | 206120_at | **CD33** | CD33 antigen (gp67) | 3,00E-07 | 2,33 | cell adhesion |
| | 219091_s_a t | **MMRN2** | multimerin 2 | 4,00E-07 | 2,96 | |
| | 214181_x_a t | **LST1** | leukocyte specific transcript 1 | 4,00E-07 | 3,87 | immune response |
| | 1552667_a_ at | **SH2D3C** | SH2 domain containing 3C | 5,00E-07 | 2,31 | intracellular signalling cascade |
| | 205326_at | **RAMP3** | receptor (calcitonin) activity modifying protein 3 | 5,00E-07 | 1,95 | protein transport |
| LUNG | 204751_x_a t | **DSC2** | desmocollin 2 | p < 0,000001 | 8,19 | cell adhesion |
| | 223861_at | **HORMA D1** | HORMA domain containing 1 | 2,00E-06 | 21,54 | |
| | 228171_s_a t | **PLEKHG 4** | pleckstrin homology domain containing, family G (with RhoGef domain) member 4 | 2,00E-06 | 2,70 | regulation of Rho protein signal transduction |
| | 228577_x_a t | **ODF2L** | outer dense fiber of sperm tails 2-like | 8,00E-06 | 2,90 | |
| | 220941_s_a t | **C21orf91** | chromosome 21 open reading frame 91 | 1,00E-05 | 3,60 | |
| | 227642_at | **TFCP2L1** | Transcription factor CP2-like 1 | 1,00E-05 | 5,50 | regulation of transcription |
| | 219867_at | **CHODL** | chondrolectin | 1,40E-05 | 2,84 | |
| | 205428_s_a t | **CALB2** | calbindin 2, 29kDa (calretinin) | 1,60E-05 | 9,20 | calcium ion binding |
| | 228956_at | **UGT8** | UDP glycosyltransferase 8 | 1,80E-05 | 15,41 | nervous system development |
| | 1554246_at | **C1orf210** | chromosome 1 open reading frame 210 | 2.10E-05 | 2,28 | |
| | 221705_s_a t | **SIKE** | suppressor of IKK epsilon | 2,10E-05 | 1,85 | |
| | 211488_s_a t | **ITGB8** | integrin, beta 8 | 2,60E-05 | 1,73 | cell-matrix adhesion |
| | 213372_at | **PAQR3** | progestin and adipoQ receptor family member III | 2,90E-05 | 5,64 | |
| | 208103_s_a t | **ANP32E** | acidic (leucine-rich) nuclear phosphoprotein 32 family, member E | 3,20E-05 | 5,99 | regulation of synaptogenesis |
| | 60474_at | **KIND1** | Kindlin 1 | 3,60E-05 | 6,87 | cell adhesion |
| | 222869_s_a t | **ELAC1** | elaC homolog 1 (E. coli) | 3,60E-05 | 1,63 | regulation of transcription |
| | 227829_at | **GYLTL1 B** | glycosyltransferase-like 1B | 3,90E-05 | 2,64 | synaptic transmission |
| | 226075_at | **SPSB1** | splA/ryanodine receptor domain and SOCS box containing 1 | 5,60E-05 | 2,89 | intracellular signalling cascade |
| | 225363_at | **PTEN** | Phosphatase and tensin homolog (mutated in multiple advanced cancers 1) | 6,20E-05 | 0,41 | regulation of cell proliferation/migration |
| | 1553705_a_ at | CHRM3 | cholinergic receptor, muscarinic 3 | 7,00E-05 | 1,84 | signal transduction |
| LIVER | 219682_s_a t | **TBX3** | T-box 3 (ulnar mammary syndrome) | 1,00E-07 | 11,35 | regulation of transcription |
| | 221823_at | **C5orf30** | Chromosome 5 open reading frame 30 | 1,00E-06 | 4,59 | |
| | 221008_s_a t | **AGXT2L 1** | alanine-glyoxylate aminotransferase 2-like 1 | 1.1e-06 | 3,69 | amino acid metabolism |
| | 1557415_s_ at | **LETM2** | Leucine zipper-EF-hand containing transmembrane protein 2 | 1.2e-06 | 1,87 | |
| | 228718_at | **ZNF44** | zinc finger protein 44 (KOX 7) | 1.9e-06 | 2,93 | regulation of transcription |
| | 219115_s_a t | **IL20RA** | interleukin 20 receptor, alpha | 2.3e-06 | 3,99 | blood coagulation |
| | 226344_at | **ZMAT1** | zinc finger, matrin type 1 | 2.6e-06 | 3,93 | nucleotide biosynthesis |
| | 214156_at | **MYRIP** | myosin VIIA and Rab interacting protein | 2.8e-06 | 3,08 | intracellular protein transport |
| | 218173_s_a t | **WHSC1L 1** | Wolf-Hirschhorn syndrome candidate 1-like 1 | 2.9e-06 | 3,74 | regulation of transcription |
| | 225561_at | **SELT** | selenoprotein T | 3,00E-06 | 4,05 | cell redox homeostasis |
| | 209710_at | **GATA2** | GATA binding protein 2 | 3.3e-06 | 4,57 | regulation of transcription |
| | 235675_at | **DHFRL1** | dihydrofolate reductase-like 1 | 7,00E-06 | 4,05 | nucleotide biosynthesis |
| | 207988_s_a t | **ARPC2** | actin related protein 2/3 complex, subunit 2, 34kDa | 5,00E-06 | 0,56 | cell motility |
| | 217691_x_a t | **SLC16A3** | solute carrier family 16 (monocarboxylic acid transporters), member 3 | 6,00E-06 | 0,46 | transport |
| | 229908_s_a t | **GNPTG** | N-acetylglucosamine-1-phosphate transferase, gamma subunit | 7,00E-06 | 1,83 | lysosome |
| | 1556308_at | **PRRT3** | proline-rich transmembrane protein 3 | 7,00E-06 | 3,88 | |
| | 204635_at | **RPS6KA 5** | ribosomal protein S6 kinase, 90kDa, polypeptide 5 | 8,00E-06 | 2,75 | regulation of transcription |
| | 227091_at | **KIAA150 5** | KIAA1505 protein | 8,00E-06 | 2,50 | |
| | 1555982_at | **ZFYVE1 6** | Zinc finger, FYVE domain containing 16 | 9,00E-06 | 3,97 | regulation of endocytosis |
| | 213118_at | **KIAA070 1** | KIAA0701 protein | 9,00E-06 | 2,79 | |
| BRAIN | 213483_at | **PPWD1** | peptidylprolyl isomerase domain and WD repeat containing 1 | 4,00E-07 | 0,31 | protein folding |
| | 210141_s_a t | **INHA** | inhibin, alpha | 4,00E-07 | 1,56 | cytokine |
| | 203131_at | **PDGFRA** | platelet-derived growth factor receptor, alpha polypeptide | 5,00E-07 | 0,06 | signal transduction |
| | 226100_at | **MLL5** | myeloid/lymphoid or mixed-lineage leukemia 5 | 7,00E-07 | 0,33 | regulation of transcription |
| | 200926_at | **RPS23** | ribosomal protein S23 | 1,40E-06 | 0,58 | protein biosynthesis |
| | 224694_at | **ANTXR1** | anthrax toxin receptor 1 | 1,50E-06 | 0,14 | |
| | 224797_at | **ARRDC3** | arrestin domain containing 3 | 1,70E-06 | 0,17 | |
| | 207761_s_a t | **METTL7 A** | methyltransferase like 7A | 3,20E-06 | 0,15 | |
| | 235410_at | **NPHP3** | nephronophthisis 3 (adolescent) | 3,70E-06 | 0,23 | kinesin complex |
| | 214154_s_a t | **PKP2** | plakophilin 2 | 3,90E-06 | 1,50 | cell adhesion |
| | 221780_s_a t | **DDX27** | DEAD (Asp-Glu-Ala-Asp) box polypeptide 27 | 5,60E-06 | 2,52 | |
| | 226839_at | **TRA16** | TR4 orphan receptor associated protein TRA16 | 5,90E-06 | 2,39 | signal transduction |
| | 209844_at | **HOXB13** | homeo box B13 | 6,40E-06 | 1,81 | regulation of transcription |
| | 220072_at | **CSPP1** | centrosome and spindle pole associated protein 1 | 6,50E-06 | 1,78 | microtubule organization |
| | 220965_s_a t | **RSHL1** | radial spokehead-like 1 | 8,50E-06 | 1,64 | iron homeostasis |
| | 213865_at | **DCBLD2** | discoidin, CUB and LCCL domain containing 2 | 9,00E-06 | 1,90 | cell adhesion / wound healing |
| | 212106_at | **UBXD8** | UBX domain containing 8 | 9,00E-06 | 2,29 | |
| | 205224_at | **SURF2** | surfeit 2 | 1,00E-05 | 1,71 | |
| | 225945_at | **ZNF655** | zinc finger protein 655 | 1,00E-05 | 0,16 | regulation of transcription |
| | 206103_at | **RAC3** | ras-related C3 botulinum toxin substrate 3 | 1,00E-05 | 1,89 | signal transduction |

| | | **TABLE 3** | | | | | | | |
|---|---|---|---|---|---|---|---|---|---|
| **Organ-specific relapses** | **Gene symbol** | **Primers (5' -> 3')** | **SEQ ID N°** | | **SEQ ID N°** | **mean of relative expression in** | | **ratio** | **p value** |
| | | **Forward** | | **Reverse** | | **organ-specific metasta ses** | **other metastases** | | |
| **BONE** | **KCTD1** | AAA TAC CCT GAA TCC AGA ATC GGA A | 1 | TGC TGT TTG AGA CTG TCC AAA ACA AT | 2 | 0,47 | 0,76 | 0,62 | 0,0894 |
| | **BAIAP2L 1** | AGA CCG CGG CTC CTA ACG AT | 3 | AGT CGG GCG GAG TTT CAC AGT | 4 | 0,93 | 2,08 | 0,45 | 0,0250 |
| | **PERP** | CTG TGG TGG AAA TGC TCC CAA GA | 5 | GCT GCT CTA CCC CAC GCG TAC T | 6 | 0,34 | 2,36 | 0,14 | 0,0018 |
| | **CFD** | ACA GCC AGC CCG ACA CCA T | 7 | TGG CCT TCT CCG ACA GCT GTA | 8 | 2,03 | 0,22 | 9,18 | 0,0012 |
| | **CD4** | GTA CAG CTT CCC AGA AGA AGA GCA TA | 9 | CAT TCA GCT TGG ATG GAC CTT TAG T | 10 | 7,82 | 2,09 | 3,74 | 0,0022 |
| | **COL6A2** | GAA ACA ACA ACT GCC CAG AGA AGA | 11 | CCG AGG TGT CCA GCA CGA A | 12 | 1,35 | 1,03 | 1,30 | 0,0073 |
| | **FLI1** | CCT CAG TTA CCT CAG GGA | 13 | GGT CGG TGT GGG AGG | 14 | 2,67 | 0,34 | 7,81 | 0,0007 |
| | | AAG TTC A | | TTG TAT TAT | | | | | |
| | **PSTPIP1** | CAA GAG TTT GAC CGG CTG ACC AT | 15 | CCG CAC TTC CTC GTA GAG CTC AT | 16 | 5,02 | 0,84 | 5,95 | 0,0018 |
| | **MEGF10** | TGC ACG CGG CAC AGA GTC A | 17 | CCC GCT TTC ATA AAA TCC AGG ACA | 18 | 19,45 | 0,77 | 25,26 | 0,0011 |
| | **PTPN22** | ACC ATG GAA AAT TCA ACA TCT TCA A | 19 | GTT TCG CAA AAT TTT CAA ACT CTT ACT | 20 | 10,14 | 1,72 | 5,90 | 0,0012 |
| | **FAM78A** | AGC CAC ATG GAG TTC TAC AAC CAG T | 21 | AGT CGC TGA TGG CTT GGA TCT T | 22 | 6,74 | 1,47 | 4,59 | 0,0022 |
| | **NXF1** | CGA CGT CAA TTC CTT CGT GGT A | 23 | GAA AAA CAC AGC AAT GTG CTT GTC T | 24 | 1,53 | 0,66 | 2,31 | 0,0018 |
| | **MFNG** | CCA GGA CCA GGG AAC AGA CAT T | 25 | CGG AGC AGT TGG TGA CCA CA | 26 | 1,88 | 0,62 | 3,04 | 0,0056 |
| | **CLEC4A** | CAC CAT ACA ATG AAA GTT CCA CAT TCT | 27 | GGG TGA TTT ACG AAA ATT TAG CAC AA | 28 | 5,26 | 0,67 | 7,91 | 0,0009 |
| **LUNG** | **KIND1** | AAG GAA CTT GAA CAA GGA GAA CCA CT | 29 | GGC ACA ACT TCG CAG CCT CTA | 30 | 4,77 | 1,08 | 4,41 | 0,0404 |
| | **ELAC1** | AAA GCC AAC TTA AAG CAG GGA GAA T | 31 | AGC CCA GGA AGG CCA AAG AA | 32 | 0,85 | 0,67 | 1,27 | 0,2220 |
| | **ANP32E** | TTT TGA ACT ACT GCA GCA AAT CAC A | 33 | TCA TCT TCA TCG CCA TCC TCA T | 34 | 3,84 | 1,69 | 2,28 | 0,0168 |
| | **PAQR3** | TCG AAG ATG GAT GGC ATT AGA TTA T | 35 | CAA GTA CAC CTG ACG CCA GTA GTT AT | 36 | 4,01 | 1,67 | 2,41 | 0,1440 |
| | **ITGB8** | GAC TGG GCC AAG GTG AAG ACA | 37 | CCT CTT GAA CAC ACC ATC CAC ATT | 38 | 1,13 | 0,20 | 5,67 | 0,0054 |
| | **C1orf210** | GAG GCT GAG ACT CAC TGG | 39 | CGA AGG CCC AAC AAG | 40 | 1,59 | 0,99 | 1,61 | 0,0797 |
| | | TGT CAT | | TGT TTT | | | | | |
| | **SIKE** | CTT GCA ACA GGA AAA CAG AGA GCT A | 41 | CTG TTT CCG ATA TTT GCT CAT GAT AA | 42 | 1,22 | 1,10 | 1,11 | 0,7500 |
| | **UGT8** | AAA GGC ATG GGG ATA TTG CTA GAA | 43 | CCC TCT GAC GGT AGC TGG GAT | 44 | 3,40 | 0,36 | 9,59 | 0,0021 |
| | **CALB2** | GAA AGG CTC TGG CAT GAT GTC AA | 45 | CTG CCA TCT CGA TTT TCC CAT CT | 46 | 17,30 | 1,32 | 13,11 | 0,1073 |
| | **CHODL** | CTG GAT AGG GCT TTG GAG GAA T | 47 | TTC ATC TGT GTA CCA GTT TCG GTA CT | 48 | 3,17 | 0,49 | 6,51 | 0,1432 |
| | **C21orf91** | AAG AAA CAC TCT CCT TCT GCC ACA T | 49 | ATC AGA CTT TGG TAC CCC CTC AAT | 50 | 1,06 | 0,94 | 1,12 | 0,9150 |
| | **TFCP2L1** | CAG TGG CTT CAC CGC AAC A | 51 | TTC AGC AAG TCA GCA CCT GAG A | 52 | 2,19 | 0,29 | 7,70 | 0,0005 |
| | **ODF2L** | CAA ACA AAG GCT TGA CCA TTT TAC A | 53 | GTT TCA GAC AAC TTG GCT TCC TGA T | 54 | 1,65 | 1,42 | 1,16 | 1,0000 |
| | **HORMA D1** | CAA CAT GAA TCT GGG AGA ATA GTC CT | 55 | TCC TTT TTG GCA CTG ACT CTT GA | 56 | 153,90 | 1,60 | 96,19 | 0,0025 |
| | **PLEKHG 4** | GGT CTC CGC TGT CCC CTG TA | 57 | GAT CTC TGA GTC CTC AGC AGT CAA A | 58 | 0,61 | 0,32 | 1,91 | 0,2427 |
| | **DSC2** | AAT CAA AGT TTT CAG AAG CCT GGA TA | 59 | CGT ACA TGT TCT CCC TCC TTG GT | 60 | 1,48 | 0,43 | 3,41 | 0,0318 |
| **LIVER** | **GATA2** | CAC GAC TAC AGC AGC GGA CTC TT | 61 | CAC TCC CGG CCT TCT GAA CA | 62 | 4,25 | 2,62 | 1,62 | 0,0239 |
| | **SELT** | CTG CTC AAG TTC CAG ATT TGT GTT T | 63 | ATT CTC TCC TTC AAT GCG GAT GT | 64 | 3,00 | 2,40 | 1,25 | 0,0801 |
| | **WHSC1L** | TAC TAA AAG AGG AAG CCC | 65 | CCC ACC TTG GAC CAC | 66 | 1,99 | 0,79 | 2,52 | 0,0062 |
| | **1** | CAG TTC A | | ACA AGA | | | | | |
| | **MYRIP** | CCA CGA CAA TCC TGC AGA AGA TTA T | 67 | TCC ACT TGC TGC TCA CTT TTG CT | 68 | 1,21 | 0,81 | 1,51 | 0,1117 |
| | **ZMAT1** | GCA AGG AAG TGA ACA TCA AAT TAA AGA A | 69 | AAT CTG CAC ACT CAT TTT GGT AAG AGT C | 70 | 1,15 | 0,99 | 1,16 | 0,2267 |
| | **IL20RA** | ACC TTC CTG TTT CCA TGC AAC AA | 71 | GTC ACA CAC TGG GAC CAC GTT CT | 72 | 0,60 | 0,33 | 1,81 | 0,0563 |
| | **ZNF44** | TCA GGA GAA ATC CAA GGT GTG ATG T | 73 | CAA TAC TAT TTC GAA TCT GGC TTA AGG TT | 74 | 0,98 | 0,68 | 1,45 | 0,1433 |
| | **LETM2** | 126 [?] GAC ATT TGG AAC CAA CAA CCT | 75 | CCC CTT CCT TGG CAA TTA TTT CAT | 76 | 4,69 | 1,45 | 3,24 | 0,0186 |
| | **AGXT2L 1** | CAG CAA CTC TGC CGG AGA AAC T | 77 | CGT TGG CTT CGG ATC CTG AAT | 78 | 6,57 | 0,79 | 8,37 | 0,0091 |
| | **C5orf30** | GAT GCG GAG GAC CGT GTC A | 79 | TCT CTT CAC CTC CTG TGC ACT TCT | 80 | 2,10 | 1,71 | 1,23 | 0,0505 |
| | **TBX3** | CCT CTG ATG AGT CCT CCA GTG AAC A | 81 | CCT CGC TGG GAC ATA AAT CTT TGA | 82 | 2,13 | 0,68 | 3,14 | 0,0075 |
| **BRAIN** | **PPWD1** | CTGTGGGTGATGATAAAGCAAT GAA | 83 | CACTGTCCAGGAAAATAGC CAAGTT | 84 | 0,44 | 0,92 | 0,48 | 0,0260 |
| | **PDGFRA** | CAT TTA CAT CTA TGT GCC AGA CCC A | 85 | ATG GCA GAA TCA TCA TCC TCC AC | 86 | 0,02 | 0,51 | 0,05 | 0,0030 |
| | **MLL5** | CGA ATG AAT GTC CAT CCC CAG ATA | 87 | TCC AGG TGA ACC AGG CTT GCT | 88 | 0,42 | 0,60 | 0,71 | 0,3700 |
| | **RPS23** | GGA ATC GTG CTG GAA AAA GTA GGA GT | 89 | CCA TTC TTG ATC AGC TGG ACC CTT A | 90 | 0,27 | 0,54 | 0,50 | 0,1600 |
| | **ANTXR1** | ATT CCC TGA GCC GCG AAA | 91 | CAA GGC ATC GAG TTT | 92 | 0,21 | 0,84 | 0,24 | 0,0220 |
| | | TCT | | | | | | | |
| | **ARRDC3** | TGGGCACGAAAGAGATGATGA TAA | 93 | GAATGAGGTAGCGAGTGGT GTCTGT | 94 | 0,20 | 0,49 | 0,40 | 0,0120 |
| | **METTL7 A** | 127 . GGT GTG CAG AGT GCT GAG A | 95 | ATC CAG GAC TTG TTG CCA GAA GTA AT | 96 | 0,16 | 0,65 | 0,24 | 0,0240 |
| | **NPHP3** | GCA ATG GAG AGA GCA GCA ACA | 97 | TTC TTT GGT CTC CCT AAA AAT CTT GA | 98 | 0,29 | 0,47 | 0,62 | 0,3500 |
| | **RSHL1** | CCA CTT TCA GAA GAT GCA GAA ATC A | 99 | CCA GAG GTT GGA GCG CAC A | 100 | 0,37 | 0,86 | 0,43 | 0,6600 |
| | **CSPP1** | ATG CAG GAA GGT GCC AAA GTT | 101 | CAC TAG TGT CAT CTC TGG GCA TTC T | 102 | 0,94 | 1,20 | 0,78 | 0,6800 |
| | **HOXB13** | GAT GTG TTG CCA GGG AGA ACA GAA | 103 | GCC CGC TGG AGT CTG CAA AT | 104 | 34,57 | 14,75 | 2,34 | 0,9400 |
| | **TRA16** | TGA GTT CAG TGC TGA ATC GCA ACA | 105 | CTG GGA GGG GCC CTG GTC T | 106 | 2,20 | 1,40 | 1,57 | 0,2400 |
| | **DDX27** | CCA GTG AGA GGT CCT GCC AAG A | 107 | AAA GGA AGG GCT AGC TCG ATA CTG TT | 108 | 3,82 | 1,87 | 2,04 | 0,0450 |
| | **PKP2** | CAC CCG AAA GAT GCT GCA TGT T | 109 | AGG GAG AGT TTC TTT GGC AAT TTC A | 110 | 0,54 | 0,38 | 1,40 | 0,3000 |
| | **INHA** | GCC CGA GGA AGA GGA GGA TGT | 111 | GCC CTC TGG CAG CTG ACT TGT | 112 | 3,06 | 3,08 | 0,99 | 0,3400 |
| | **TBP** | TGC ACA GGA GCC AAG AGT GAA | 113 | CAC ATC ACA GCT CCC CAC CA | 114 | | | | |

| **Table 4** | | |
|---|---|---|
| **Bone metastasis associated genes** | | |
| **Gene symbol** | **Description** | **Exemples of Antibodies** |
| **CLEC4A** | C-type lectin domain family 4, member A | Novus biologicals (ab15854); GenWay bitoech (15-288-21195) |
| **MFNG** | manic fringe homolog (Drosophila) (secreted Notch ligand) | Abnova (H00004242-M07) |
| **NXF1** | nuclear RNA export factor 1 | scbt (sc-17310, sc-32319, sc-25768, sc-28377, sc-17311) |
| **FAM78A** | family with sequence similarity 78, member A | |
| **KCTD1** | potassium channel tetramerisation domain containing 1 | |
| **BAIAP2L1** | BAI1-associated protein 2-like 1 | Abnova (H00055971-M01); MBL int.corp (M051-3) |
| **PTPN22** | protein tyrosine phosphatase, non-receptor type 22 (lymphoid) | Abnova (H00026191-M01); R&Dsystems (MAB3428) |
| **MEGF10** | MEGF10 protein | |
| **PERP** | TP53 apoptosis effector | Novus biologicals (NB 500-231); Sigma-Aldrich (P5243); Research diagnostics (RDI-ALS24284); GenWay bitoech (18-661-15116) |
| **PSTPIP1** | proline-serine-threonine phosphatase interacting protein 1 | Abnova (H00009051-M01) |
| | | |

| **Osteomimetism associated genes** | | |
|---|---|---|
| **MMP9** | matrix metalloproteinase 9 (gelatinase B, 92kDa gelatinase, 92kDa type IV collagenase) | NeoMarkers (RB-1539) |
| **IBSP** | integrin-binding sialoprotein (bone sialoprotein, bone | Usbiological (S1013-34B) |
| | sialoprotein II) | |
| **OMD** | osteomodulin | Abnova (H00004958-A01) |
| **COMP** | cartilage oligomeric matrix protein | AbCam (ab11056) |
| **MEPE** | matrix, extracellular phosphoglycoprotein with ASARM motif (bone) | R&Dsystems (AF3140) |
| | | |

| **Lung metastasis associated genes (highest ranking genes)** | | |
|---|---|---|
| **Gene symbol** | **Description** | **Exemples of Antibodies** |
| **DSC2** | desmocollin 2 | scbt (sc-34308, sc-34311, sc-34312); Progen Biotechnik (GP542, 610120); Research diagnostics (RDI-PRO610120, RDI-PROGP542); UsBiological (D3221-50) |
| **HORMAD1** | HORMA domain containing 1 | Abnova (H00084072-M01) |
| **PLEKHG4** | pleckstrin homology domain containing, | family G (with RhoGef domain) member 4 |
| **ODF2L** | outer dense fiber of sperm tails 2-like | |
| **C21orf91** | chromosome 21 open reading frame 91 | |
| **TFCP2L1** | Transcription factor CP2-like 1 | Abcam (ab3962), Novus biological (NB 600-22), Eurogentec (24220), Imgenex (IMG-4094) |
| **CHODL** | chondrolectin | R&Dsystems (AF2576);Abnova (H00140578-A01) |
| **CALB2** | calbindin 2, 29kDa (calretinin) | Chemicon (AB5054); Usbiological (C1036-01 M) |
| **UGT8** | UDP glycosyltransferase 8 (UDP-galactose ceramide galactosyltransferase) | |
| **C1orf210** | chromosome 1 open reading frame 210 | |
| **SIKE** | suppressor of IKK epsilon | |
| **ITGB8** | integrin, beta 8 | Abnova (H00003696-M01, H00003696-A01); GenWay biotech (15-288-21362); sctb (sc-10817, sc-25714, sc-6638) |
| **PAQR3** | progestin and adipoQ receptor family member III | |
| **ANP32E** | acidic (leucine-rich) nuclear phosphoprotein 32 family, member E | GenWay biotech (A21207), UsBiological (L1238) |
| **KIND1** | kindlin | Abcam (ab24152); sctb (sc-30854) |
| **ELAC1** | elaC homolog 1 (E. coli) | Abnova (H00055520-A01) |
| **GYLTL1B** | glycosyltransferase-like 1 B | |
| | | |

| **Liver metastasis associated genes** | | |
|---|---|---|
| **Gene symbol** | **Description** | **Exemples of antibodies** |
| **TBX3** | T-box 3 (ulnar mammary syndrome) | Abnova (H00006926-A01) |
| **C5orf30** | hypothetical gene supported by AF038182; BC009203 | |
| **AGXT2L1** | alanine-glyoxylate aminotransferase 2-like 1 | /// alanine-glyoxylate aminotransferase 2-like 1 |
| **LETM2** | Leucine zipper-EF-hand containing transmembrane protein 2 | |
| **ZNF44** | zing finger protein 44 | |
| **IL20RA** | interleukin 20 receptor, alpha | LifeSpan biosciences (LS-C722) |
| **ZMAT1** | Zinc finger, matrin type 1 | |
| **MYRIP** | myosin VIIA and Rab interacting protein | Novus Biologicals (NB 100-1278); Everest biotech Ltd (EB06023) |
| **WHSC1L1** | Wolf-Hirschhorn syndrome candidate 1-like 1 | Abcam (ab4514); BioCat (AP1904a-AB) |
| **SELT** | selenoprotein T | |
| **GATA2** | GATA binding protein 2 | Abnova (H00002624-M01); R&Dsystems (AF2046) |
| **DHFRL1** | dihydrofolate reductase-like 1 | |
| **ARPC2** | actin related protein 2/3 complex, subunit 2, 34kDa | |
| **SLC16A3** | solute carrier family 16 (monocarboxylic acid transporters), member 3 | |
| **GNPTG** | | |
| **PRRT3** | | |
| **RPS6KA5** | ribosomal protein S6 kinase, 90kDa, polypeptide 5 Santa | cruz (sc-2591, sc-9392, sc-25417); R&Dsystems (AF2518) |
| | | |

| **Brain metastasis associated genes** | | |
|---|---|---|
| **Gene symbol** | **Description** | **Exemples of Antibodies** |
| **PPWD1** | peptidylprolyl isomerase domain and WD repeat containing 1 | |
| **INHA** | Inhibin, alpha | AbCam (Ab10599) |
| **PDGFRA** | platelet-derived growth factor receptor, alpha polypeptide | Abnova (H00005156-M01) |
| **MLL5** | myeloid/lymphoid or mixed-lineage leukemia 5 (trithorax homolog, Drosophila) | Abgent (AP6186a); Orbigen (PAB-10849) |
| **RPS23** | ribosomal protein S23 | Abnova (H00006228-A01) |
| **ANTXR1** | Anthrax toxin receptor 1 | AbCam (Ab21270) |
| **ARRDC3** | arrestin domain containing 3 | |
| **METTL7A** | methyltransferase like 7A | |
| **NPHP3** | nephronophthisis 3 (adolescent) | |
| **PKP2** | plakophilin 2 | Novus biologicals (ab19469) |

**Table 5: Lung metastasis associated genes obtained from a class comparison of lung (n=5) and non-lung metastases of breast cancer (n=18)**

| **Probe Set** | **Gene** | **Gene Title Symbol change** | **Function / biological process** | **Fold** | **Parametric p-value** |
|---|---|---|---|---|---|
| 204751_x_at | *DSC2** | desmocollin 2 | Cell adhesion | 8,2 | p < 0,000001 |
| 223861_at | *HORMAD1** | HORMA domain containing 1 | | 21,5 | 2,00E-06 |
| 228171_s_at | *PLEKHG4** family | pleckstrin homology domain containing, G, member 4 | Rho protein signal transduction | 2,7 | 2,00E-06 |
| 228577_x_at | *ODF2L** | outer dense fiber of sperm tails 2-like | | 2,9 | 8,00E-06 |
| 220941_s_at | *C21orf91** | chromosome 21 open reading frame 91 | | 3,6 | 1,00E-05 |
| 227642_at | *TFCP2L1** | Transcription factor CP2-like 1 | Regulation of transcription | 5,5 | 1,00E-05 |
| 219867_at | *CHODL** | chondrolectin | Hyaluronic acid binding | 2,8 | 1,40E-05 |
| 205428_s_at | *CALB2** | calbindin 2, 29kDa (calretinin) | Calcium ion binding | 9,2 | 1,60E-05 |
| 228956_at | *UGT8** | UDP glycosyltransferase 8 | Glycosphingolipid biosynthetic process | 15,4 | 1,80E-05 |
| 1554246_at | *C1orf210** | chromosome 1 open reading frame 210 | | 2,3 | 2,10E-05 |
| 221705_s_at | *SIKE** | suppressor of IKK epsilon | | 1,9 | 2,10E-05 |
| 211488_s_at | *ITGB8** | integrin, beta 8 | Cell adhesion / Signal transduction | 1,7 | 2,60E-05 |
| 213372_at | *PAQR3** | progestin and adipoQ receptor family member III | Receptor activity | 5,6 | 2,90E-05 |
| 208103_s_at | *ANP32E** 32 | acidic (leucine-rich) nuclear phosphoprotein family, member E | Phosphatase inhibitor activity | 6,0 | 3,20E-05 |
| 60474_at | *FERMT1** | Fermitin family homolog 1 (drosophila) | Cell adhesion | 6,9 | 3,60E-05 |
| 222869_s_at | *ELAC1** | elaC homolog 1 (E. coli) | tRNA processing | 1,6 | 3,60E-05 |
| 227829_at | *GYLTL1B* | glycosyltransferase-like 1B | Glycosphingolipid biosynthetic process | 2,6 | 3,90E-05 |
| 226075_at | *SPSB1* box | splA/ryanodine receptor domain and SOCS containing 1 | Intracellular signalling cascade | 2,9 | 5,60E-05 |
| 1553705_a_at | *CHRM3* | cholinergic receptor, muscarinic 3 | G-protein coupled signal transduction | 2,0 | 6,10E-05 |
| 225363_at | *PTEN* in | Phosphatase and tensin homolog (mutated multiple advanced cancers 1) | Phosphatidylinositol signalling | 0,4 | 6,20E-05 |
| 203256_at | *CDH3* | cadherin 3, type 1, P-cadherin (placental) | Cell adhesion | 6,1 | 9,50E-05 |

| | | | | | |
|---|---|---|---|---|---|
| * Genes tested by qRT-PCR. Colored lines correspond to genes that were validated (Mann-Whitney U test) | | | | | |

**Table 6 : Association between clinical and pathological characteristics and the 6-gene classifier among 72 lymph node-negative patients treated at CRH**

| | | **6-gene classification** | | |
|---|---|---|---|---|
| **Characteristics** | **All patients (n=72)** | **High Risk group (n=18)** | **Low Risk group (n=54)** | **P value*** |
| *Metastases within 10 years* | | | | *0.79* |
| yes | 38 (53%) | 10 (56%) | 28 (52%) | |
| no | 34 (47%) | 8 (44%) | 26 (48%) | |
| *Lung metastases within 10 years* | | | | *0.04* |
| yes | 11 (15%) | 6 (33%) | 5 (9%) | |
| no | 61 (85%) | 12 (67%) | 49 (91%) | |
| *Menopausal status* | | | | *0.58* |
| Pre | 18 (31 %) | 6 (40%) | 12 (28%) | |
| Post | 40 (69%) | 9 (60%) | 31 (72%) | |
| *Macroscopic tumor size* | | | | *0.59* |
| ≤ 20 mm | 23 (33%) | 5 (28%) | 18 (35%) | |
| > 20 mm | 47 (67%) | 13 (72%) | 34 (65%) | |
| *SBR histological grade* | | | | *0.01* |
| I | 7 (11%) | 0 (0%) | 7 (15%) | |
| II | 37 (60%) | 7 (44%) | 30 (65%) | |
| III | 18 (29 %) | 9 (56%) | 9 (20%) | |
| *Estrogen receptor status* | | | | < *0.001* |
| Positive | 44 (61 %) | 3 (17%) | 41 (76%) | |
| Negative | 28 (39%) | 15 (83%) | 13 (24%) | |
| *Progesterone receptor* | | | | *0.004* |
| Positive | 37 (51%) | 4 (22%) | 33 (61 %) | |
| Negative | 35 (49%) | 14 (78%) | 21 (39%) | |

| | | | | |
|---|---|---|---|---|
| * **Chi² test** | | | | |

**Table 7 : Multivariate analysis for lung metastasis in 721 breast cancer patients**

| **Variable** | **Hazard Ratio** | **95% C.I** | **P value** |
|---|---|---|---|
| 6-gene signature (pos.vs. neg.) | 2.12 | 1.2 - 3.76 | 0.01 |
| ER negative (yes vs. no) | 1.83 | 1.03 - 3.26 | 0.04 |
| Lymph node positive (yes vs. no) | 0.92 | 0.52 - 1.62 | 0.77 |

**Table 8 : Highest ranking genes obtained from a class comparison of bone and nonbone metastases of breast cancer (n=23)**

| **Probe Set** | **Gene** | **Gene Title** | ***Function* / *biological process*** | **Fold change** | **p value** |
|---|---|---|---|---|---|
| 204679_at | *KCNK1* | potassium channel, subfamily K, member 1 | potassium ion transport | 0,06 | p < 1e-07 |
| 221724_s_at | *CLEC4A* | C-type lectin domain family 4, member A | immune response / cell adhesion / signal transduction | 2,45 | p < 1e-07 |
| 204153_s_at | *MFNG* | manic fringe homolog (Drosophila) | Notch signaling pathway | 2,69 | p < 1e-07 |
| 208922_s_at | *NXF1* | nuclear RNA export factor 1 | mRNA transport | 1,61 | p < 1e-07 |
| 206060_s_at | *PTPN22* | protein tyrosine phosphatase, non-receptor type 22 | tyrosine phosphatase activity/ signal transduction | 4,12 | 1,00E-07 |
| 222392_x_at | *PERP* | PERP, TP53 apoptosis effector | cell adhesion/ apoptosis | 0,09 | 1,00E-07 |
| 211178_s_at | *PSTPIP1* | proline-serine-threonine phosphatase interacting protein 1 | cell adhesion/ signal transduction | 3,01 | 2,00E-07 |
| 204236_at | *FLI1* | Friend leukemia virus integration 1 | regulation of transcription | 4,80 | 2,00E-07 |
| 213290_at | *COL6A2* | collagen, type VI, alpha 2 | cell adhesion / extracellular matrix organization | 2,63 | 2,00E-07 |
| 203547_at | *CD4* | CD4 antigen (p55) | immune response / cell adhesion/ signaling pathway | 2,62 | 2,00E-07 |
| 205382_s_at | *CFD* | D component of complement (adipsin) | immune response | 7,37 | 2,00E-07 |

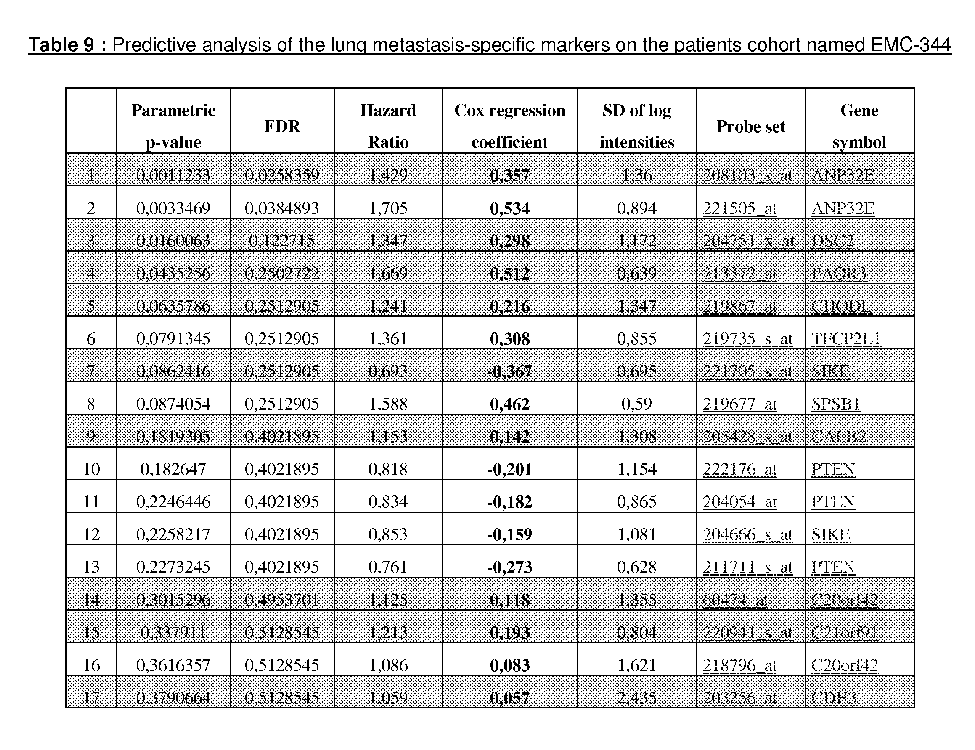

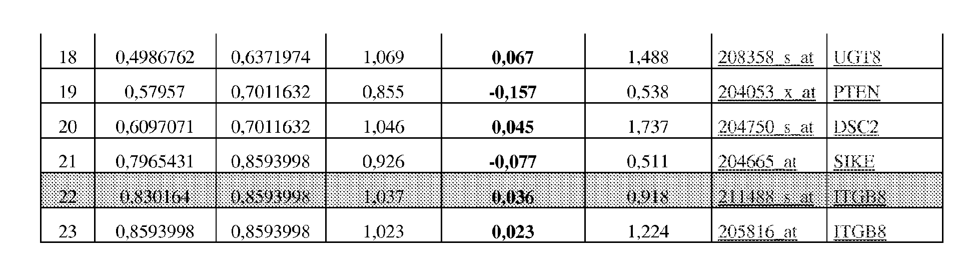

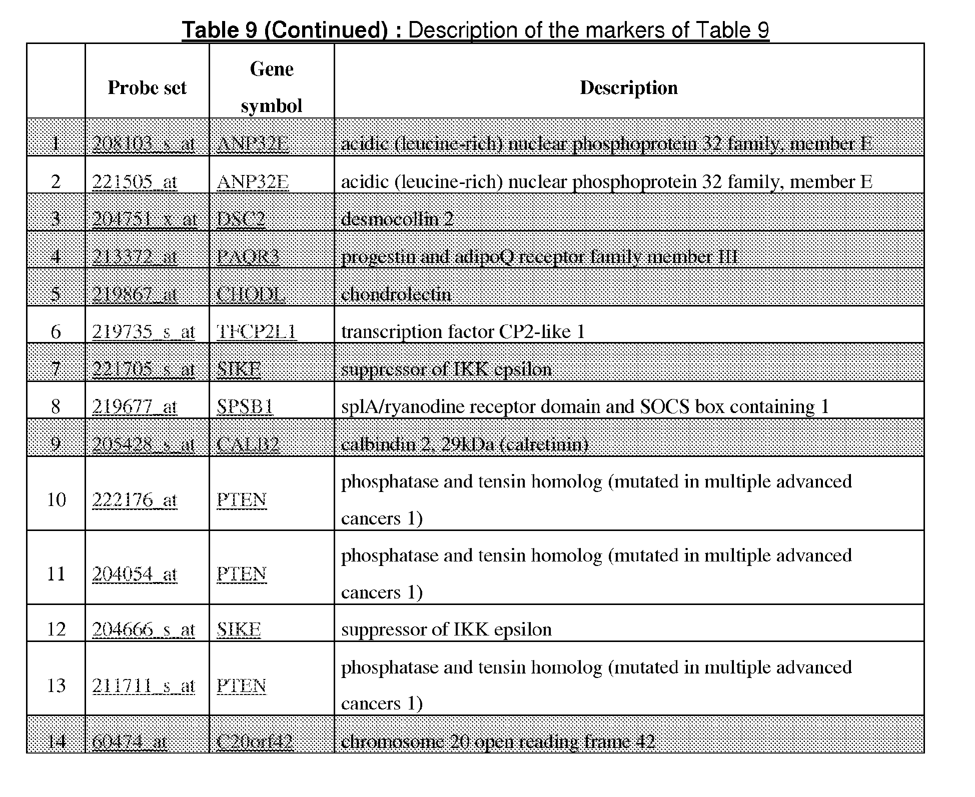

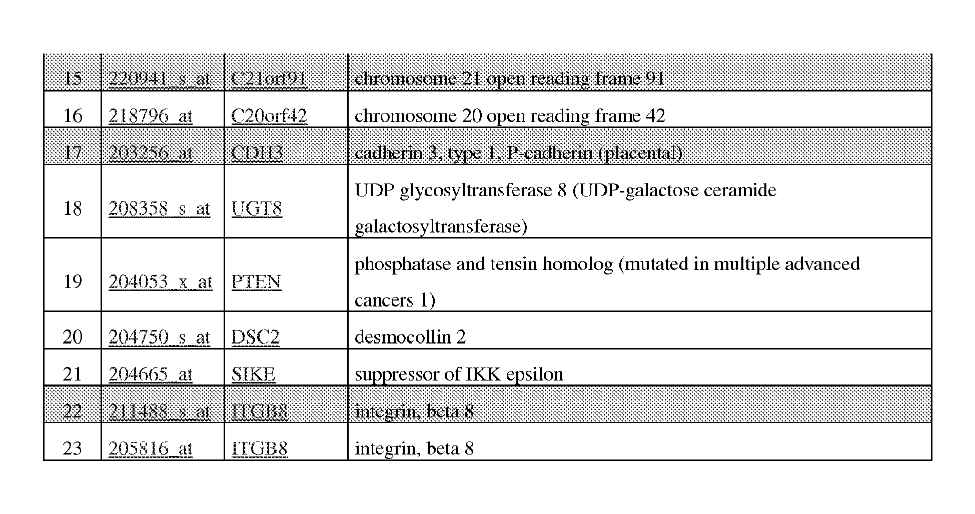

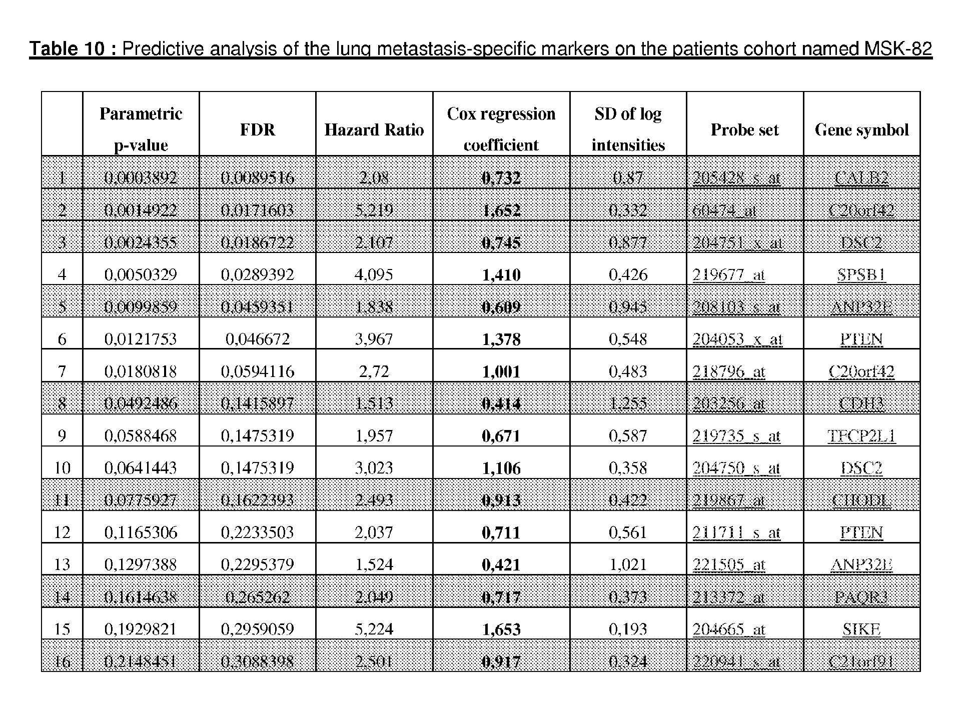

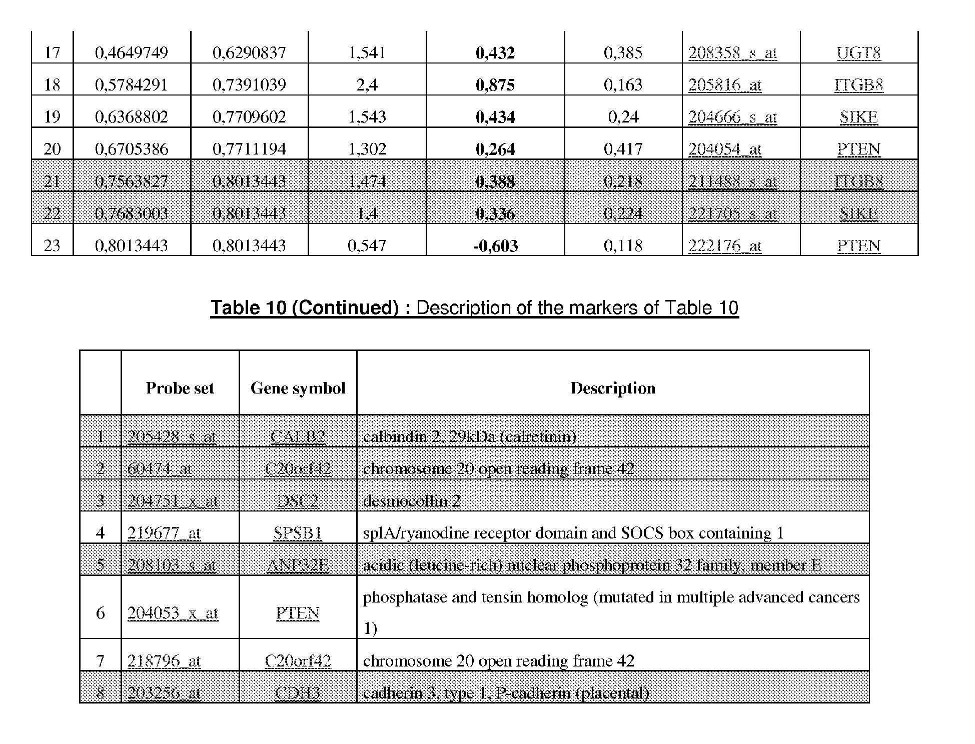

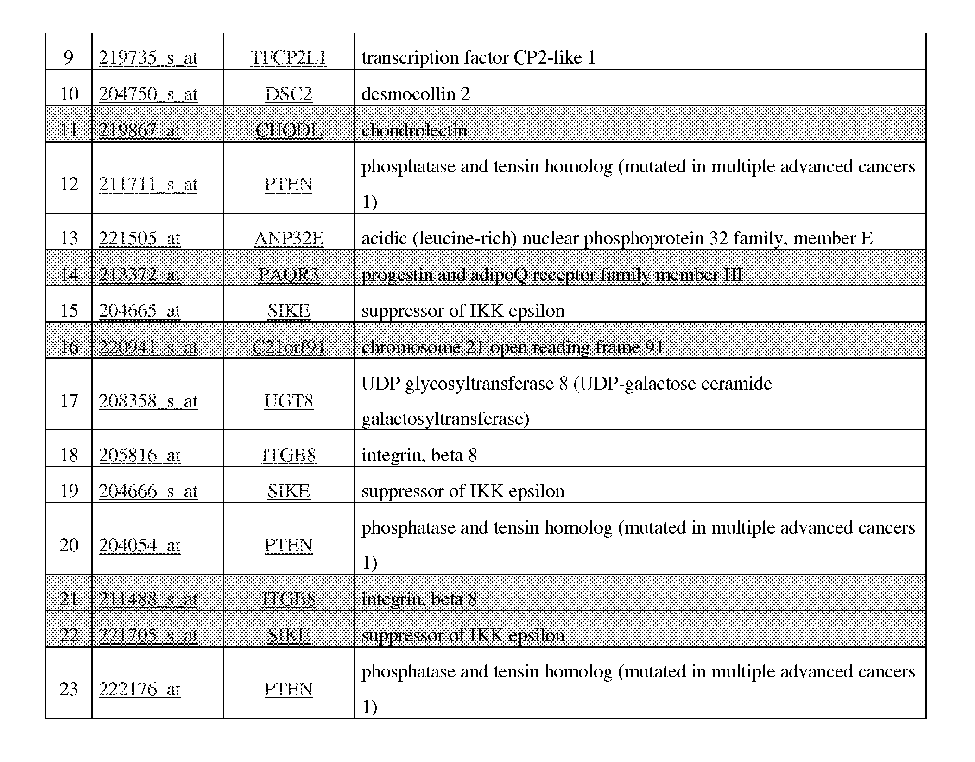

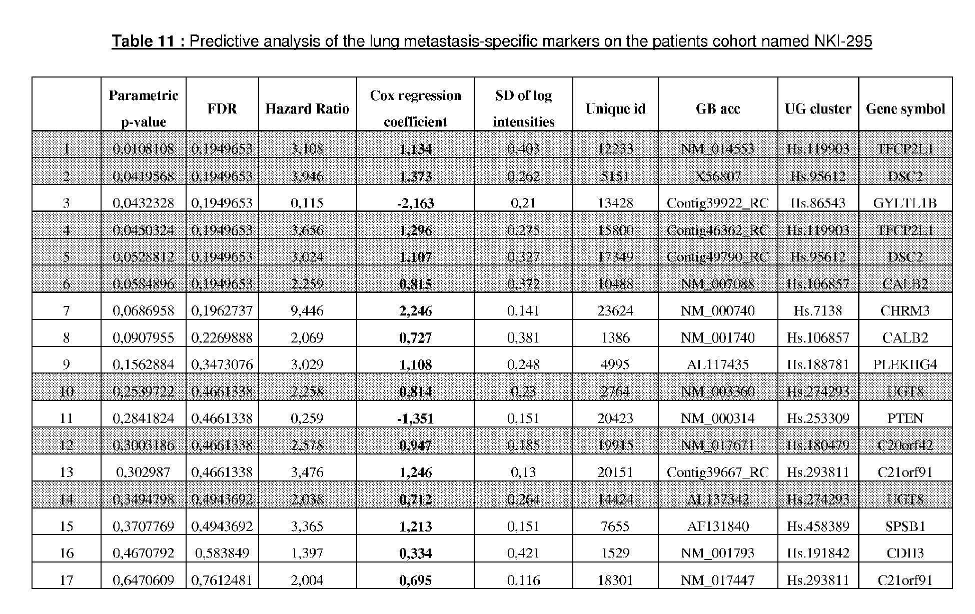

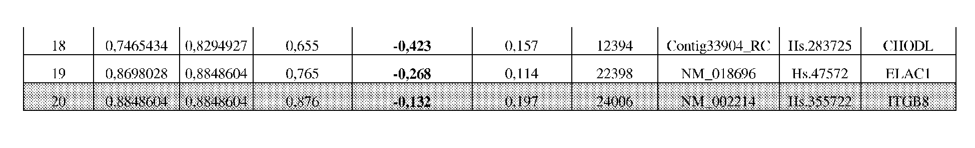

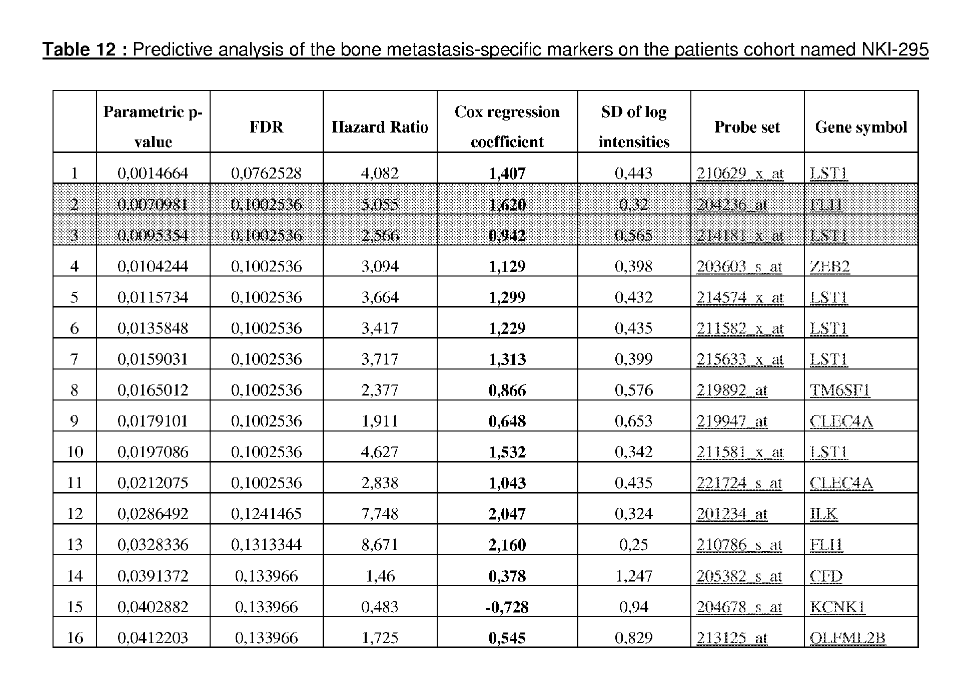

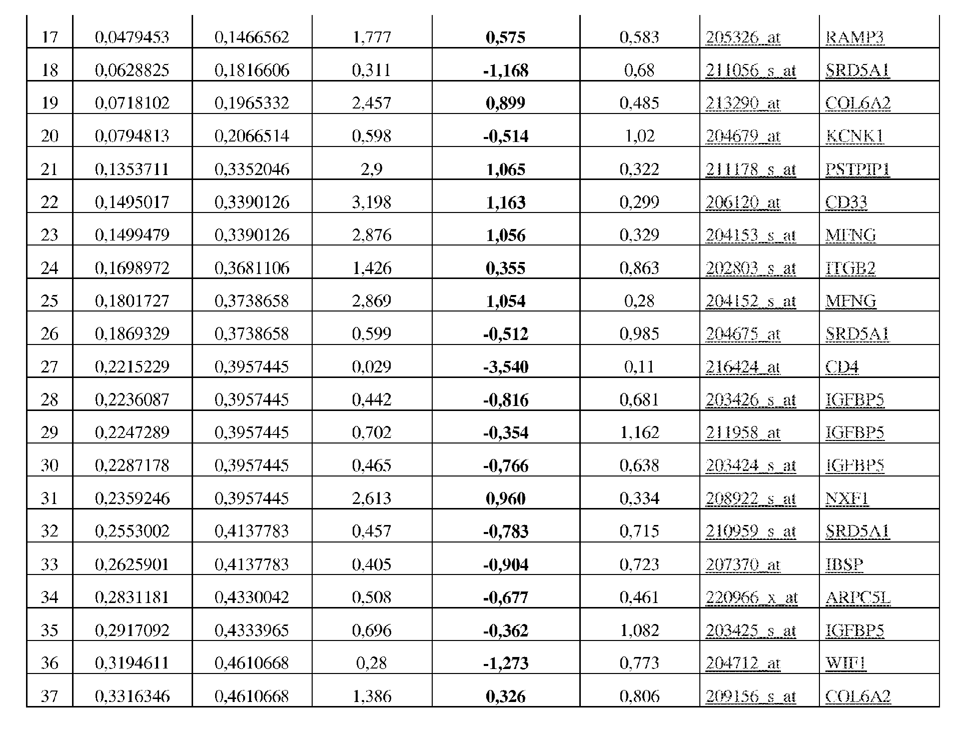

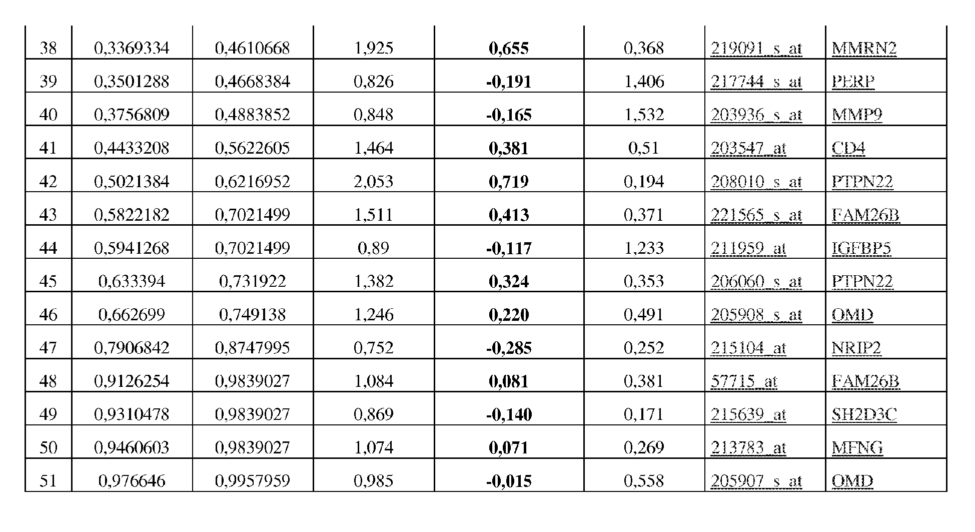

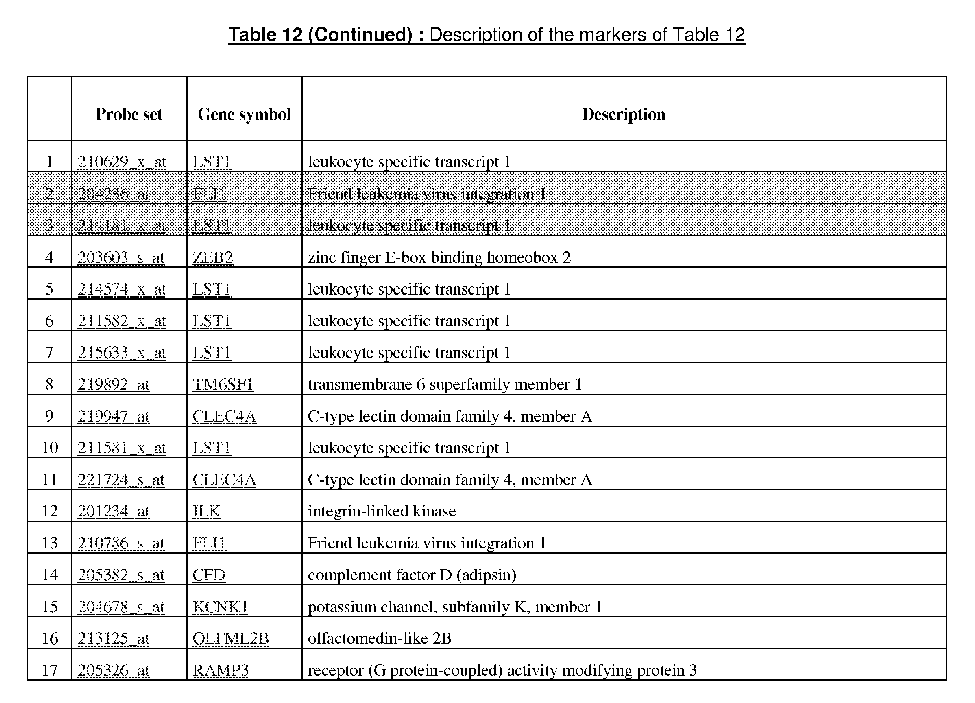

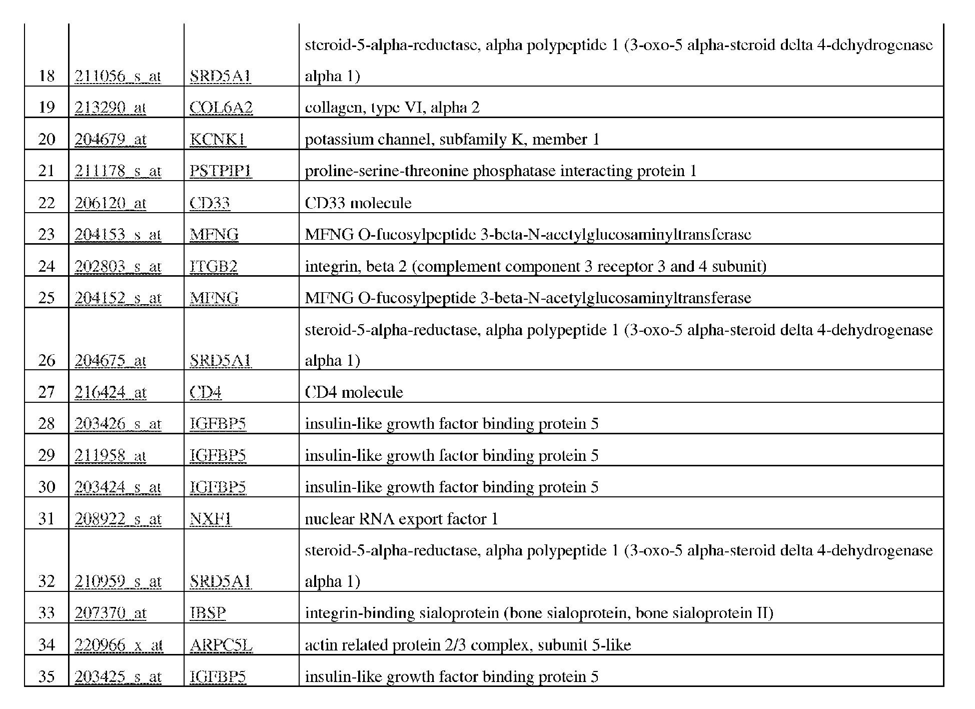

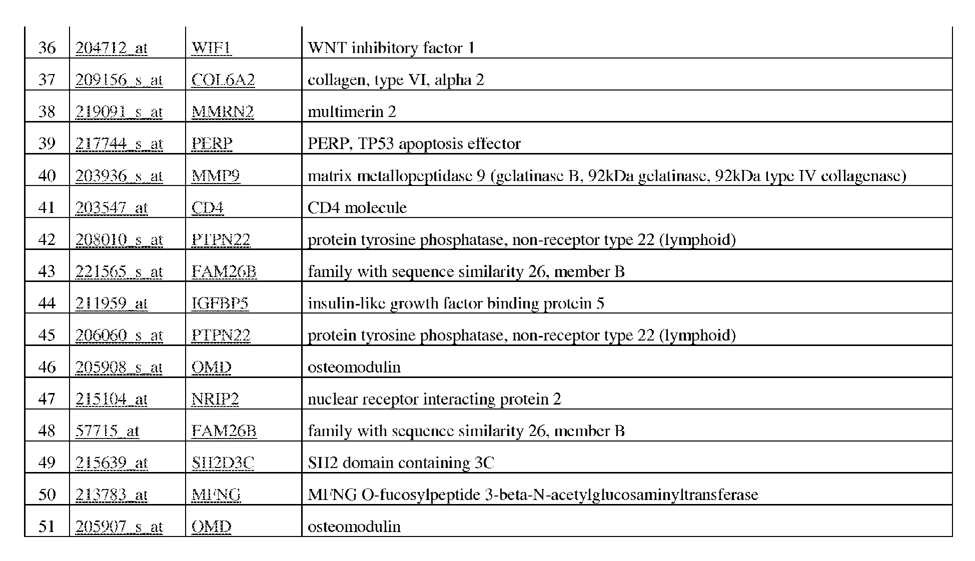

**Table 13: Highest ranking genes obtained from a class comparison of bone and non-bone metastases of breast cancer (n=23)**

| **Probe Set** | **Gene** | **Gene Title** | **Fold** | **p value** |
|---|---|---|---|---|
| 203936_s_at | *MMP9* | matrix metallopeptidase 9 | 36,73 | p < 1e-07 |
| 204679_at | *KCNK1* | potassium channel, subfamily K, member 1 | 0,06 | p < 1e-07 |
| 236028_at | *IBSP* | Integrin-binding sialoprotein (bone sialoprotein) | 123,76 | p < 1e-07 |
| 205907_s_at | *OMD* | osteomodulin | 41,31 | p < 1e-07 |
| 221724_s_at | *CLEC4A* | C-type lectin domain family 4, member A | 2,45 | p < 1e-07 |
| 204153_s_at | *MFNG* | manic fringe homolog (Drosophila) | 2,69 | p < 1e-07 |
| 57715_at | *FAM26B* | family with sequence similarity 26, member B | 2,29 | p < 1e-07 |
| 226914_at | *ARPC5L* | actin related protein 2/3 complex, subunit 5-like | 0,35 | p < 1e-07 |
| 208922_s_at | *NXF1* | nuclear RNA export factor 1 | 1,61 | p < 1e-07 |
| 227002_at | *FAM78A* | family with sequence similarity 78, member A | 2,43 | p < 1e-07 |
| 226245_at | *KCTD1* | potassium channel tetramerisation domain containing 1 | 0,31 | 1,00E-07 |
| 227372_s_at | *BAIAP2L1* | BAI1-associated protein 2-like 1 | 0,10 | 1,OOE-07 |
| 206060_s_at | *PTPN22* | protein tyrosine phosphatase, non-receptor type 22 | 4,12 | 1,00E-07 |
| 232523_at | *MEGF10* | MEGF10 protein | 10,78 | 1,00E-07 |
| 222392_x_at | *PERP* | PERP, TP53 apoptosis effector | 0,09 | 1,00E-07 |
| 231879_at | *COL12A1* | collagen, type XII, alpha 1 | 8,35 | 1,OOE-07 |
| 211178_s_at | *PSTPIP1* | proline-serine-threonine phosphatase interacting protein 1 | 3,01 | 2,00E-07 |
| 204236_at | *FLI1* | Friend leukemia virus integration 1 | 4,80 | 2,00E-07 |
| 213290_at | *COL6A2* | collagen, type VI, alpha 2 | 2,63 | 2,00E-07 |
| 203547_at | *CD4* | CD4 antigen (p55) | 2,62 | 2,00E-07 |
| 205382_s_at | *CFD* | D component of complement (adipsin) | 7,37 | 2,00E-07 |
| 204712_at | *WIF1* | WNT inhibitory factor 1 | 25,94 | 2,00E-07 |
| 235593_at | *ZEB2* | zinc finger homeobox 1b | 2,92 | 2,00E-07 |
| 206120_at | *CD33* | CD33 antigen (gp67) | 2,33 | 3,00E-07 |
| 232204_at | *EBF* | early B-cell factor | 5,00 | 4,00E-07 |
| 219091_s_at | *MMRN2* | multimerin 2 | 2,96 | 4,00E-07 |
| 214181_x_at | *LST1* | leukocyte specific transcript 1 | 3,87 | 4,00E-07 |
| 211958_at | *IGFBP5* | insulin-like growth factor binding protein 5 | 4,04 | 4,00E-07 |
| 1552667_a_at | *SH2D3C* | SH2 domain containing 3C | 2,31 | 5,00E-07 |
| 205326_at | *RAMP3* | receptor (calcitonin) activity modifying protein 3 | 1,95 | 5,00E-07 |
| 202803_s_at | *ITGB2* | integrin, beta 2 (antigen CD18 (p95) | 8,33 | 5,00E-07 |
| 201234_at | *ILK* | integrin-linked kinase | 2,22 | 5,00E-07 |
| 227243_s_at | *EBF3* | early B-cell factor 3 | 3,70 | 6,00E-07 |
| 219892_at | *TM6SF1* | transmembrane 6 superfamily member 1 | 5,42 | 6,00E-07 |
| 215104_at | *NRIP2* | nuclear receptor interacting protein 2 | 1,34 | 7,00E-07 |
| 223245_at | *STRBP* | spermatid perinuclear RNA binding protein | 0,29 | 8,00E-07 |
| 226345_at | *ARL8* | ADP-ribosylation factor-like 8 | 0,31 | 9,00E-07 |
| 204675_at | *SRD5A1* | steroid-5-alpha-reductase, alpha polypeptide 1 | 0,15 | 9,00E-07 |
| 225373_at | *C10orf54* | chromosome 10 open reading frame 54 | 3,10 | 9,00E-07 |
| 213125_at | *OLFML2B* | olfactomedin-like 2B | 8,01 | 9,00E-07 |

## Claims

1. An *in vitro* method for predicting the occurrence of lung metastasis in a patient affected with a breast cancer, comprising the steps of :
a) providing a breast tumour tissue sample previously collected from the patient to be tested;
b) determining, in the said breast tumour tissue sample, the expression level of one or more lung-specific markers in a group of markers comprising FERMT1, and
c) predicting the occurrence of metastasis in the lung when one or more of the said lung-specific markers has a deregulated expression level, as compared to a control expression level value for each marker.

2. The method according to claim 1, wherein at step b), the group of lung-specific markers further comprises one or more lung-specific markers in the group consisting of DSC2, TFCP2L1, UGT8, ITGB8 and ANP32E.

3. The method according to claim 2, wherein, at step b), the number of markers for which the expression level is determined is selected from the group consisting of 2, 3, 4, 5 and 6.

4. The method according to claim 1, wherein at step b), the group of lung-specific markers further comprises every one of the markers comprised in the group consisting of DSC2, TFCP2L1, UGT8, ITGB8 and ANP32E.

5. The method according to claim 1, wherein at step b), the group of lung-specific markers consists of DSC2, TFCP2L1, UGT8, ITGB8, ANP32E and FERMT1.

6. The method according to any one of claims 1 to 5, wherein the control expression level value for each marker consists of the corresponding expression level measured in a breast tumour sample selected from the group consisting of (i) a breast tumour sample from a patient who has not undergone cancer metastasis, and (ii) a breast tumour sample from a patient who has not undergone cancer metastasis in the lung.

7. The method according to any one of claims 1 to 6, wherein the markers are selected from the group consisting of mRNA, cDNA and protein.

8. The method according to any one of claims 1 to 7, wherein step b) is performed by using a DNA microarray having probes specific for the one or more lung-specific markers immobilized thereon.

9. The method according to any one of claims 1 to 7, wherein, at step b), a nucleic acid amplification reaction is performed by using primers, or pairs of primers, specific for each of the one or more lung-specific markers whose expression is determined.

10. The method according to claim 9, wherein the said pairs of primers are selected from the group consisting of SEQ ID N° 59 and 60 (DSC2), SEQ ID N° 51 and 52 (TFCP2L1), SEQ ID N° 43 and 44 (UGT8), SEQ ID N° 37 and 38 (ITGB8), SEQ ID N°33 and 34 (ANP32E) and a probe set specific for FERMT1.

11. The use of a kit comprising means for determining the expression level of one or more lung-specific biological markers in a group of markers comprising FERMT1, for the *in vitro* prediction of the occurrence of lung metastasis in a breast cancer patient.

12. The use of a kit comprising means for determining the expression level of one or more lung-specific biological markers in a group of markers comprising FERMT1, for monitoring the anti-metastasis effectiveness of a therapeutic treatment of a patient affected with a breast cancer with a pharmaceutical agent.

13. The use according to any one of claims 11 and 12, wherein the group of markers further comprises one or more lung-specific markers in the group consisting of DSC2, TFCP2L1, UGT8, ITGB8 and ANP32E.

14. The use according to any one of claims 11 to 13, wherein the said kit comprises one or a combination or set of pair of primers, wherein each primer hybridizes specifically with one of the said one or more biological markers.

15. The use according to any one of claims 11 to 13, wherein the said kit comprises a DNA microarray comprising probes hybridizing to the nucleic acid expression products of the said one or more biological markers.

16. The use according to any one of claims 11 to 13, wherein the said kit comprises a combination or a set of antibodies, wherein each antibody is directed against one of the said one or more biological markers.
